# EUROPEAN PATENT APPLICATION

(11) **EP 1 925 668 A2**
(43) Date of publication of application: **28.05.2008**
(21) Application number: 06807903.7
(22) Date of filing: 01.08.2006
(51) Int. Cl.: C12N 15/52, C12N 15/76, C12N 15/69, C12P 17/12, C07K 14/36

(54) **GENES INVOLVED IN THE BIOSYNTHESIS OF THIOCORALINE AND HETEROLOGOUS PRODUCTION OF SAME**

(30) Priority: 02.08.2005 ES 200501932
(71) Applicant: PHARMA MAR, S.A., 28770 Colmenar Viejo (Madrid) (ES)
(72) Inventor: RAMOS CASTRO, Angelina, Universidad de Oviedo, 33006 Oviedo (ES); FERNÁNDEZ BRAÑA, Alfredo, Universidad de Oviedo, 33006 Oviedo (ES); LOMBÓ BRUGOS, Felipe, Universidad de Oviedo, 33006 Oviedo (ES); MENDEZ FERNÁNDEZ, Carme, Universidad de Oviedo, 33006 Oviedo (ES); SALAS FERNÁNDEZ, José A., Universidad de Oviedo, 33006 Oviedo (ES); VELASCO IGLESIAS, Ana, c/o Pharma Mar, S.A., 28770 Colmenar Viejo - Madrid (ES); SCHLEISSNER SÁNCHEZ, Carmen, c/o Pharma Mar, S.A., 28770 Colmenar Viejo - Madrid (ES)
(74) Representative: ABG Patentes, S.L.
(86) International application number: PCT/ES2006/000455
(87) International publication number: WO 2007/014971

(57) **Abstract**

The invention relates to genes involved in the biosynthesis of thiocoraline and to the heterologous production of same. According to the invention, the cluster of genes responsible for the biosynthesis of thiocoraline was identified and cloned. Said cluster of genes can be used in the heterologous production of thiocoraline which has an antitumor and antibacterial activity.

## Description

### Field of the Invention

The present invention relates to the cluster of genes responsible for the biosynthesis of thiocoraline and its use in the heterologous production of thiocoraline.

### Background of the Invention

Thiocoraline (I) 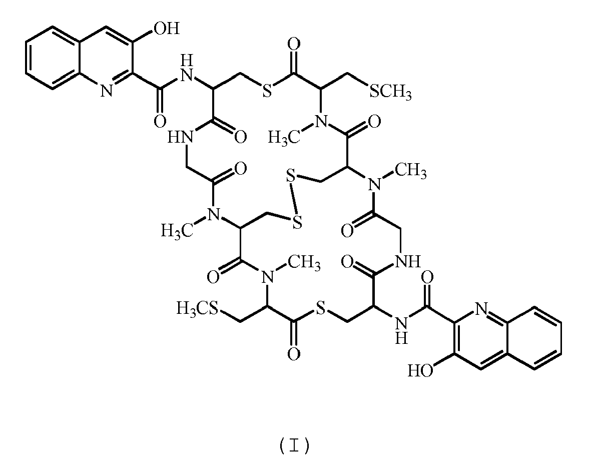 is a cyclodimeric thiodepsipeptide isolated from a marine actinomycete, specifically from the Micromonosporaceae family (Pérez Baz et al., J. Antibiotics, 50(9), 738-741, 1997; Romero et al., J. Antibiotics, 50(9), 734-737, 1997). Although it has been described that thiocoraline is obtained from *Micromonospora marina* or *Micromonospora sp.* L-13-ACM-092, subsequent studies have shown that the compound can also be isolated from the actinomycete *Micromonospora sp.* ML1, which was isolated from a marine mollusk found on the Indian ocean coast, in Mozambique (Espliego, F. Ph.D. Thesis, 1996, University of Leon; de la Calle, F. Ph.D. Thesis, 1998, Autonomous University of Madrid).

*In vitro* studies have shown the capacity of thiocoraline to inhibit the growth of cell lines of different types of solid tumors, such as melanoma, breast, non-microcytic lung and colon cancer. Thiocoraline has also shown that it has a marked antitumor activity in *in vivo* assays against human carcinoma xenografts (Faircloth et al. Eur. J. Cancer, 33, 175, 1997 (abstract)). Thiocoraline further shows antibacterial activity against Gram-positive bacteria.

Although obtaining thiocoraline from said marine actinomycete *(Micromonospora sp.* ML1) is feasible on a small scale, on a large scale said obtainment is limited due to the variability in the production that is observed with this microorganism. Indeed, the production of thiocoraline from said organism is a time-consuming process due to the low growth rate of this organism and shows important fluctuations in the production yields in different batches.

Therefore, due to the fact that on one hand, obtaining thiocoraline from said marine actinomycete is quite limited, and on the other hand, the fact that the thiocoraline molecule also has a complex structure and its synthesis can be complicated on an industrial level, it is desirable to understand the genetic bases of its biosynthesis for the purpose of creating means for affecting its obtainment in a directed manner. This could give rise to an increase in the amounts of thiocoraline produced, given that natural producing strains generally produce the product at low concentration and in a very irregular manner. Likewise, it could also allow the production of thiocoraline in hosts that do not produce this compound naturally.

The development of recombinant DNA technology has opened up an interesting field of research for generating and producing bioactive compounds by means of manipulating genes involved in the biosynthesis of such bioactive compounds, mainly of bacteria from the actinomycete group. These techniques can be used to improve the production of already known natural compounds, because natural strains usually produce low concentrations of the metabolite of interest.

The heterologous expression of the cluster of genes involved in the biosynthesis of thiocoraline in other actinomycetes that are more suitable for genetic manipulation and fermentation would likewise allow producing said compounds with more reproducible yields in shorter fermentation times.

As is known, a number of bacteria and fungi synthesize a wide variety of biologically active peptides with a nonribosomal origin, including antitumor and antibacterial peptides, etc. The biosynthesis of this family of compounds is carried out by nonribosomal peptide synthetases (NRPSs), which are multifunctional enzymes with a modular catalytic domain organization. Each of these modules carries out an elongation cycle, i.e., it activates and incorporates a specific amino acid into the final structure of the compound. A minimal module is formed by three domains: (i) an adenylation domain, (A, with approximately 550 amino acids) which is responsible for selecting a certain amino acid and generating the adenylated aminoacyl version thereof by means of using ATP; (ii) a peptidyl carrier domain (P, with approximately 80 amino acids) containing a phosphopantetheine (PP) prosthetic group acting as cofactor and binding to the P domain by a covalent bond; this domain is responsible for fixing the activated adenylated amino acid before passing to the following reaction centers; and (iii) a condensation domain (C, with approximately 450 amino acids) generating a new peptide bond between two adenylated aminoacyl moieties located in two consecutive P domains. C domains are absent in the modules activating the first amino acid of the system. Some NRPSs have extra domains for carrying out specific activities, such as epimerizations giving rise to D-amino acids, N- or C-type methylations, circularizations acting on the L-Cys or L-Ser amino acids. A final domain located after the last module, is generally responsible for releasing the intermediate enzyme, generating a linear or cyclic peptide. As a general rule, the structure of the different modules reflects the final amino acid sequence of the product peptide. This colinearity rule allows assigning a specific activation function to each module in an NRPS. Information on NRPSs can be found, for example, in Quing-Tao, S. et al., 2004. Dissecting and Exploiting Nonribosomal Peptide Synthetases. Acta Biochimica et Biophysica. Sinica, 36 (4): 243-249.

### Summary of the Invention

An important objective of the present invention consists of isolating and characterizing the complete nucleotide sequence encoding the proteins responsible for the production of thiocoraline. Based on this, the function of the amino acid sequences comprising the proteins involved in the biosynthesis of thiocoraline can be isolated and determined. This objective can be reached by providing an isolated and optionally purified new nucleic acid molecule encoding all the proteins related to the complete biosynthetic thiocoraline production pathway.

The inventors have been able to identify and clone all the genes responsible for the biosynthesis of thiocoraline, i.e., the cluster of genes involved in the biosynthesis of thiocoraline, providing the genetic bases for improving an manipulating the production of this compound in a directed manner.

By means of using initiator oligonucleotides derived from consensus sequences of nonribosomal peptide synthetase (NRPS) adenylation domains, 6 fragments of *Micromonospora* sp. ML1 chromosome were amplified by means of the polymerase chain reaction (PCR), all of which fragments contain putative (hypothetical) NRPS adenylation domain fragments called PSV1, PSV2, PSV3, PSV4, PSV5 and PSV6 (Example 3). The inactivation, by insertion, of said adenylation domains has shown that two of them (PSV2 and PSV5) generated mutants that do not produce thiocoraline, which indicated that they were involved in the biosynthesis of thiocoraline (Examples 7 and 10).

The sequencing of a DNA region of approximately 64.6 kilobases (kb) (SEQ ID NO: 1) showed the presence of 36 complete open reading frames (ORFs) and another 2 incomplete ORFs (Example 12, Table 1). The heterologous expression of a region of approximately 53 kb, containing 26 of said ORFs, in *Streptomyces coelicolor, Streptomyces albus* and *Streptomyces lividans* led to the production of thiocoraline in said actinomycetes (Example 19).

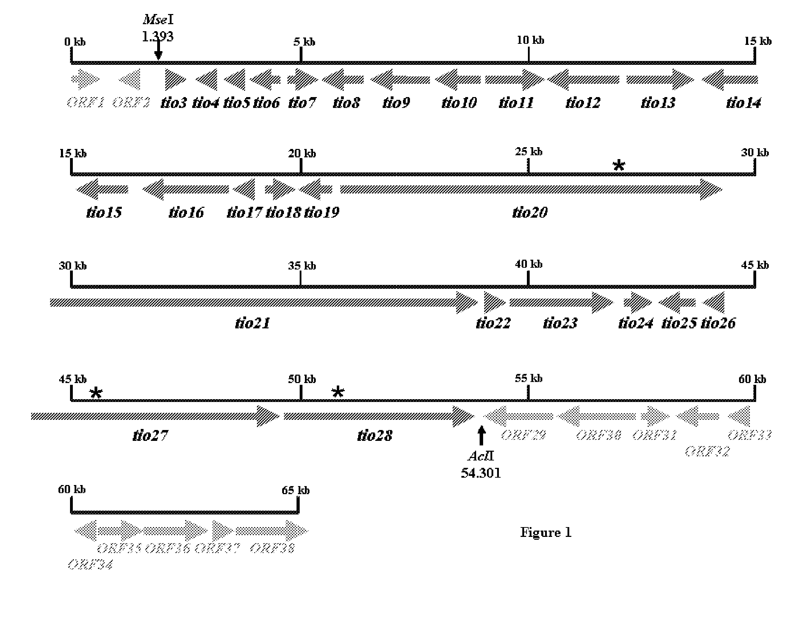

The cluster of genes responsible for the biosynthesis of thiocoraline is schematically shown in Figure 1. Surprisingly enough, the cluster of thiocoraline genes contains more NRPS encoding genes than those expected based on the number of amino acids of the peptide skeleton. Some of the identified proteins are involved in the formation of the thiocoraline peptide structure, such as several of the NRPSs identified as Tio12, Tio17, Tio18, Tio19, Tio20, Tio21, Tio22, Tio27 and Tio28, for example. The proteins identified as Tio20 and Tio21 probably form the NRPSs involved in the biosynthesis of the thiocoraline skeleton and probably, other two NRPSs, identified as Tio27 and Tio 28 could be responsible for the biosynthesis of a small peptide which could be involved in regulating the biosynthesis of thiocoraline in *Micromonospora* sp. ML1. There are also several proteins which could be related to resistance process, such as Tio5, Tio6 and Tio23. The possible regulators of the thiocoraline pathway identified in the sequenced region correspond to Tio3, Tio4, Tio7, Tio24 and Tio25. Finally, there are also several proteins related to the generation of the initiator unit 3-hydroxy-quinaldate, Tio8, Tio9, Tio10 and Tio11. The genes, the gene interruption of which generates a phenotype that does not produce thiocoraline, are indicated in Figure 1 by means of an asterisk *(tio20, tio27* and *tio28*).

The present invention therefore relates to the identification and cloning of the cluster of genes responsible for the biosynthesis of thiocoraline. Said cluster of genes responsible for the biosynthesis of thiocoraline and its expression in a suitable host cell allows the efficient production of thiocoraline.

Consequently, in one aspect, the invention relates to an isolated nucleic acid molecule comprising a nucleotide sequence encoding at least one biosynthetic thiocoraline production pathway protein, or a biologically active fragment thereof.

In another aspect, the invention relates to a composition comprising at least one nucleic acid molecule provided by this invention.

In another aspect, the invention relates to a probe comprising a nucleic acid molecule provided by this invention or a fragment thereof.

In another aspect, the invention relates to a vector comprising a nucleic acid molecule provided by this invention.

In another aspect, the invention relates to a host cell transformed or transfected with a vector provided by this invention.

In another aspect, the invention relates to a protein encoded by a nucleic acid molecule provided by this invention.

In another aspect, the invention relates to a method for producing a protein involved in the biosynthesis of thiocoraline, comprising the use of a thiocoraline-producing organism the genome of which has been manipulated.

In another aspect, the invention relates to a process, based on the use of genes responsible for the biosynthesis of thiocoraline from *Micromonospora sp.* ML1, for the production of thiocoraline in another actinomycete.

### Description of the Drawings

Figure 1. Schematic depiction of the cluster of thiocoraline genes and of the genes surrounding them, including the gene organization of the sequenced *Micromonospora sp.* ML1 chromosome area. The restriction sites used to construct the plasmids for the heterologous expression of the cluster of thiocoraline genes are shown.
Figure 2. Schematic depiction of the cosmids cosV33-D12 and pCT2c. ori: replication origin for *E. coli.* SCP2: replication origin for *Streptomyces. aac(3)*IV*:* apramycin resistance gene. *neo*: neomycin resistance gene. *bla*: ampicillin resistance gene. SV40 ori: eukaryotic origin for episomal replication.
Figure 3. Diagram of clonings carried out for constructing plasmid pFL1036. ori: replication origin for *E. coli.* M13 ori: replication origin for the M13 phage. oriT: conjugative transfer origin. *lacZ:* beta-galactosidase gene. *kan^{R}:* kanamycin resistance gene. *aac(3)*IV*:* apramycin resistance gene. *bla*: ampicillin resistance gene.
Figure 4. Diagram of clonings carried out for constructing plasmid pFL1041. ori: replication origin for *E. coli.* SCP2: replication origin for *Streptomyces.* oriT: conjugative transfer origin. *lacZ:* beta-galactosidase gene. *aac(3)*IV*:* apramycin resistance gene.
Figure 5. Diagram of clonings carried out for constructing plasmid pAR15AT. ori p15A: replication origin for *E. coli.* oriT: conjugative transfer origin. *intφC31:* φC31 phage integrase gene. attP: site-specific recombination site. *kan^{R}:* kanamycin resistance gene. *aac(3)*IV: apramycin resistance gene. ^{K}: cleavage site treated with the Klenow fragment of the *E. coli* DNA polymerase.
Figure 6. Diagram of clonings carried out for constructing plasmid pAPR. ori p15A: replication origin for *E. coli.* oriT: conjugative transfer origin. ori M13: replication origin of the M13 phage. ori: replication origin for *E. coli. lacZ:* beta-galactosidase gene. *lacI:* lactose operon repressor gene. *intφC31:* φC31 phage integrase gene. attP: site-specific recombination site. *kan^{R}:* kanamycin resistance gene. *aac(3)*IV: apramycin resistance gene. ^{K}: cleavage site treated with the Klenow fragment of *E. coli* DNA polymerase. *P_{ermE}:* ermE gene promoter.
Figure 7. Depiction of plasmids pFL1048, pFL1048r and pFL1049. ori pl5A: replication origin for *E. coli*. oriT: conjugative transfer origin. *intφC31:* φC31 phage integrase gene. attP: site-specific recombination site. *aac(3)*IV: apramycin resistance gene.
Figure 8A. HPLC chromatogram of a *Streptomyces albus* (pFL1049) culture extract after 7 days of growth in R5A medium. The peak corresponding to thiocoraline and its retention time, 27 minutes, are shown.
Figure 8B. UV absorption spectrum of the product (thiocoraline) present in the peak of 27 minutes shown in Figure 8A.
Figure 8C. Mass spectrum of the product (thiocoraline) present in the peak of 27 minutes shown in Figure 8A.

### Detailed Description of the Invention

According to the present invention, a new, isolated and optionally purified nucleic acid molecule encoding all or part of the proteins involved in the complete biosynthetic thiocoraline production pathway is provided.

Therefore, in one aspect, the invention relates to a nucleic acid molecule, hereinafter, nucleic acid molecule of the invention, preferably an optionally purified, isolated nucleic acid molecule comprising a nucleotide sequence encoding at least one biosynthetic thiocoraline production pathway protein, or a biologically active fragment thereof. Said biosynthetic thiocoraline production pathway protein is generally a nonribosomal peptide synthetase (NRPS). NRPSs are responsible for the biosynthesis of thiocoraline.

As used herein, the expression "biologically active fragment", applied to a biosynthetic thiocoraline production pathway protein, relates to a part of the protein structure retaining the active function of the full-length protein. Said biologically active fragments can be encoded by the corresponding regions of the nucleic acid molecule of the invention. The size of said regions of the nucleic acid molecule of the invention can vary within a wide range; nevertheless, in one particular embodiment, said regions can have a length of at least 10, 15, 20, 25, 50, 100, 1,000, 2,500, 5,000, 10,000, 20,000, 25,000 or more nucleotides. Said regions normally have a length between 100 and 10,000 nucleotides, preferably between 100 and 7,500, and are biologically functional, i.e., they can encode a biologically active fragment of a biosynthetic thiocoraline production pathway protein.

The nucleic acid molecule of the invention can be a deoxyribonucleic acid (DNA) or ribonucleic acid (RNA) molecule. The nucleic acid molecule of the invention can also be a single-strand nucleic acid molecule or a derived double-strand nucleic acid molecule. Illustrative non-limiting examples of nucleic acid molecules of the invention include genomic DNA (gDNA) molecules, messenger RNA (mRNA) molecules and complementary DNA (cDNA) molecules to mRNA molecules.

The mutants and variants of the nucleic acid molecule of the invention are included within the scope of the present invention. Said mutants and variants include the nucleic acid molecules of the invention in which at least one molecule has been altered, substituted, eliminated or inserted. By way of illustration, the mutants and variants of the nucleic acid molecule of the invention can have 1, 2, 3, 4, 5, 10, 15, 25, 50, 100, 200, 500 and more changes (alterations, substitutions, eliminations or insertions) of nucleotides. Degenerate variants encoding the same protein, as well as non-degenerate variants encoding a different protein are also possible. The nucleotide sequence of said mutants and variants encodes a protein, or a biologically active fragment thereof, conserving at least one of the biological activities or functions of the corresponding protein encoded by any open reading frame (ORF) of the cluster of genes responsible for the biosynthesis of thiocoraline. The allelic forms of the genes of said cluster as well as the polymorphisms are also comprised within the scope of the present invention.

In one particular embodiment, the nucleic acid molecule of the invention is an optionally purified, isolated nucleic acid molecule comprising a nucleotide sequence encoding all the biosynthetic thiocoraline production pathway proteins, or biologically active fragments thereof. In this case, the nucleic acid molecule of the invention comprises the nucleotide sequence containing the complete cluster of genes responsible for the biosynthesis of thiocoraline.

The nucleotide sequence of the complete cluster of genes responsible for the biosynthesis of thiocoraline is included in SEQ ID NO: 1, a 64,650 base pair (bp) genomic DNA sequence of *Micromonospora sp.* ML1. The scope of the invention also includes the complementary strand to the nucleotide sequence shown in SEQ ID NO: 1, i.e., that formed by nucleotides which are complementary to those indicated in SEQ ID NO: 1 (e.g., A substituted with T, C substituted with G and vice versa) and/or reverse nucleotide sequences [i.e., the sequences generated by changing the reading direction e.g., from (5' → 3') to (3' → 5')].

The present invention further includes a nucleic acid molecule hybridizing with the nucleic acid molecule of the invention having the nucleotide sequence shown in SEQ ID NO: 1 or its complementary strand; said molecule can be isolated from a thiocoraline-producing organism and encodes at least one biosynthetic thiocoraline production pathway protein. Typical hybridization techniques and conditions, known by persons skilled in the art, are mentioned, for example, in Sambrook et al., Molecular Cloning: A Laboratory Manual, Second Edition, Cold Spring Harbor Laboratory Press, Cold Spring Harbor, NY (1989). Conventional or severe hybridization techniques are generally used for homologous probes, whereas less severe hybridization conditions are used for partially homologous probes having less than 100% of homology with the target nucleic acid molecule sequence. In the latter case (partially homologous probes), a series of Southern or Northern hybridizations with different conditions can be carried out. By way of illustration, when hybridization is carried out in a solvent containing formamide, the preferred conditions include the use of a constant temperature and ionic strength of approximately 42°C with a solution containing 6X SSC, 50% of formamide. Less severe hybridization conditions can use the same temperature and ionic strength although in this case, the amount of formamide in the annealing buffer will be lower (from approximately 45% to 0%). Alternatively, hybridization can be carried out in aqueous solutions that do not contain formamide. In general, for the hybridization in aqueous medium, the ionic strength of the aqueous solutions is kept the same, typically approximately 1 M Na⁺, whereas the annealing temperature can be reduced from 68°C to 42°C.

The sequencing of the complete cluster of genes responsible for the biosynthesis of thiocoraline (SEQ ID NO: 1) showed the presence of 36 complete open reading frames (ORFs) and of another 2 incomplete ORFs (ORF1 and ORF38, see below). Table 1 (Example 12) shows the position of the different ORFs involved in the biosynthetic thiocoraline production pathway, as well as the amino acid sequences encoded by said ORFs.

The complete chromosomal (genomic) DNA molecule containing the cluster of genes responsible for the biosynthesis of thiocoraline, encoding all the biosynthetic proteins essential for the production of thiocoraline, has been efficiently packaged into two plasmids, specifically into cosmids SuperCos1 and pKC505 (Examples 1 and 2). These two cosmids, containing the cluster of genes responsible for the biosynthesis of thiocoraline, are enough to regenerate the complete biosynthetic pathway for the production of thiocoraline. Therefore, in one particular embodiment, the invention provides the complete cluster of biosynthetic thiocoraline genes in two cosmids which allows having substantially more efficient means for producing thiocoraline.

In one particular embodiment, the nucleic acid molecule of the invention is an optionally purified, isolated nucleic acid molecule comprising a nucleotide sequence encoding a biosynthetic thiocoraline production pathway protein, or a biologically active fragment thereof. In one specific embodiment, the nucleic acid molecule of the invention is selected from the group of genes consisting of:
- the nucleic acid molecule comprising nucleotides 2-535 of SEQ ID NO: 1 *(orf1);*
- the nucleic acid molecule comprising nucleotides 993-1130c of SEQ ID NO: 1 *(orf2);*
- the nucleic acid molecule comprising nucleotides 1517-2131 of SEQ ID NO: 1 *(tio3);*
- the nucleic acid molecule comprising nucleotides 2154-2822c of SEQ ID NO: 1 *(tio4);*
- the nucleic acid molecule comprising nucleotides 2970-3791c of SEQ ID NO: 1 *(tio5);*
- the nucleic acid molecule comprising nucleotides 3794-4777c of SEQ ID NO: 1 *(tio6);*
- the nucleic acid molecule comprising nucleotides 4904-5611 of SEQ ID NO: 1 (*tio7*);
- the nucleic acid molecule comprising nucleotides 5701-6426c of SEQ ID NO: 1 *(tio8);*
- the nucleic acid molecule comprising nucleotides 6426-7688c of SEQ ID NO: 1 *(tio9);*
- the nucleic acid molecule comprising nucleotides 7733-8524c of SEQ ID NO: 1 *(tio10);*
- the nucleic acid molecule comprising nucleotides 8791-10002 of SEQ ID NO: 1 *(tio11);*
- the nucleic acid molecule comprising nucleotides 10002-11590c of SEQ ID NO: 1 *(tio12);*
- the nucleic acid molecule comprising nucleotides 11847-13634 of SEQ ID NO: 1 *(tio13);*
- the nucleic acid molecule comprising nucleotides 13734-15005c of SEQ ID NO: 1 *(tio14);*
- the nucleic acid molecule comprising nucleotides 15005-16354c of SEQ ID NO: 1 *(tio15);*
- the nucleic acid molecule comprising nucleotides 16441-18744c of SEQ ID NO: 1 *(tio16);*
- the nucleic acid molecule comprising nucleotides 18774-19055c of SEQ ID NO: 1 (tio*17*);
- the nucleic acid molecule comprising nucleotides 19260-20036 of SEQ ID NO: 1 *(tio18);*
- the nucleic acid molecule comprising nucleotides 20146-20880c of SEQ ID NO: 1 *(tio19);*
- the nucleic acid molecule comprising nucleotides 21188-28969 of SEQ ID NO: 1 *(tio20);*
- the nucleic acid molecule comprising nucleotides 28979-38398 of SEQ ID NO: 1 *(tio21);*
- the nucleic acid molecule comprising nucleotides 38449-38661 of SEQ ID NO: 1 *(tio22);*
- the nucleic acid molecule comprising nucleotides 38642-41263 of SEQ ID NO: 1 *(tio23);*
- the nucleic acid molecule comprising nucleotides 41835-42368 of SEQ ID NO: 1 *(tio24);*
- the nucleic acid molecule comprising nucleotides 42395-43255c of SEQ ID NO: 1 *(tio25);*
- the nucleic acid molecule comprising nucleotides 43340-43741c of SEQ ID NO: 1 *(tio26);*
- the nucleic acid molecule comprising nucleotides 44152-49563 of SEQ ID NO: 1 *(tio27);*
- the nucleic acid molecule comprising nucleotides 49635-53669 of SEQ ID NO: 1 *(tio28);*
- the nucleic acid molecule comprising nucleotides 53749-55305c of SEQ ID NO: 1 *(orf29);*
- the nucleic acid molecule comprising nucleotides 55384-57222c of SEQ ID NO: 1 *(orf30);*
- the nucleic acid molecule comprising nucleotides 57895-58467c of SEQ ID NO: 1 *(orf31);*
- the nucleic acid molecule comprising nucleotides 58535-59206c of SEQ ID NO: 1 *(orf32);*
- the nucleic acid molecule comprising nucleotides 59298-59564c of SEQ ID NO: 1 *(orf33);*
- the nucleic acid molecule comprising nucleotides 59611-60114c of SEQ ID NO: 1 *(orf34);*
- the nucleic acid molecule comprising nucleotides 60202-60888 of SEQ ID NO: 1 *(orf35);*
- the nucleic acid molecule comprising nucleotides 60960-62240 of SEQ ID NO: 1 *(orf36);*
- the nucleic acid molecule comprising nucleotides 62300-62833 of SEQ ID NO: 1 *(orf37);*
- the nucleic acid molecule comprising nucleotides 62925-64650 of SEQ ID NO: 1 *(orf38);* or
fragments thereof encoding biologically active fragments of biosynthetic thiocoraline production pathway proteins.

In another particular embodiment, the nucleic acid molecule of the invention is an optionally purified, isolated nucleic acid molecule, comprising a nucleotide sequence encoding two or more biosynthetic thiocoraline production pathway proteins, or biologically active fragments thereof. In one specific embodiment, the nucleic acid molecule of the invention comprises a nucleotide sequence comprising two or more genes selected from the genes identified as *orf1, orf2, tio3, tio4, tio5, tio6, tio7, tio8, tio9, tio10, tio11, tio12, tio13, tio14, tio15, tio16, tio17, tio18, tio19, tio20, tio21, tio22, tio23, tio24, tio25, tio26*, *tio27, tio28, orf29, orf30, orf31, orf32, orf33, orf34, orf35, orf36, orf37, orf38* and fragments thereof encoding biologically active fragments of biosynthetic thiocoraline production pathway proteins.

In another particular embodiment, the nucleic acid molecule of the invention is an optionally purified, isolated nucleic acid molecule, comprising a nucleotide sequence encoding at least one biosynthetic thiocoraline production pathway protein, or a biologically active fragment thereof, or a mutant or variant thereof, wherein said protein is selected from the group consisting of the proteins identified as ORF1 (SEQ ID NO: 2), ORF2 (SEQ ID NO: 3), Tio3 (SEQ ID NO: 4), Tio4 (SEQ ID NO: 5), Tio5 (SEQ ID NO: 6), Tio6 (SEQ ID NO: 7), Tio7 (SEQ ID NO: 8), Tio8 (SEQ ID NO: 9), Tio9 (SEQ ID NO: 10), TiolO (SEQ ID NO: 11), Tio11 (SEQ ID NO: 12), Tio12 (SEQ ID NO: 13), Tio13 (SEQ ID NO: 14), Tio14 (SEQ ID NO: 15), Tio1 (SEQ ID NO: 16), Tio16 (SEQ ID NO: 17), Tio17 (SEQ ID NO: 18), Tio18 (SEQ ID NO: 19), Tio19 (SEQ ID NO: 20), Tio20 (SEQ ID NO: 21), Tio21 (SEQ ID NO: 22), Tio22 (SEQ ID NO: 23), Tio23 (SEQ ID NO: 24), Tio24 (SEQ ID NO: 25), Tio25 (SEQ ID NO: 26), Tio26 (SEQ ID NO: 27), Tio27 (SEQ ID NO: 28), Tio28 (SEQ ID NO: 29), ORF29 (SEQ ID NO: 30), ORF30 (SEQ ID NO: 31), ORF31 (SEQ ID NO: 32), ORF32 (SEQ ID NO: 33), ORF33 (SEQ ID NO: 34), ORF34 (SEQ ID NO: 35), ORF35 (SEQ ID NO: 36), ORF36 (SEQ ID NO: 37), ORF37 (SEQ ID NO: 38), ORF38 (SEQ ID NO: 39). Said proteins can be obtained from the corresponding aforementioned *orfs (orf1, orf2, tio3, tio4, tio5, tio6, tio7, tio8, tio9, tio10, tio11, tio12, tio13, tio14, tio15, tio16, tio17, tio18, tio19, tio20, tio21, tio22, tio23, tio24, tio25, tio26, tio27, tio28, orf29, orf30, orf31, orf32, orf33, orf34, orf35, orf36, orf37, orf38)* of the cluster of genes responsible for the biosynthesis of thiocoraline (SEQ ID NO: 1), or from the corresponding regions, mutants or variants thereof.

In another particular embodiment, the nucleic acid molecule of the invention is an optionally purified, isolated nucleic acid molecule comprising a nucleotide sequence encoding at least one variant of a biosynthetic thiocoraline production pathway protein, or a biologically active fragment thereof, wherein said variant is at least 30%, advantageously 50%, preferably 60%, more preferably 70%, even more preferably 80%, particularly 90%, more particularly 95% or more, identical in its amino acid sequence to that of a protein selected from the proteins the amino acid sequences of which are shown in SEQ ID NO: 2-39, or to biologically active fragments thereof. Said variant conserves at least one of the biological activities of functions of the corresponding protein encoded by any of the *orfs* of the cluster of genes responsible for the biosynthesis of thiocoraline.

In another aspect, the present invention relates to a composition comprising at least one nucleic acid molecule of the invention, preferably an isolated nucleic acid molecule. In one particular embodiment, said composition comprises a nucleic acid molecule of the invention. In another particular embodiment, said composition comprises two or more nucleic acid molecules of the invention. Said nucleic acid molecules can be both of DNA and of RNA.

The nucleic acid molecule of the invention can be isolated from any organism producing thiocoraline either naturally or recombinantly, because the cluster of genes responsible for the biosynthesis of thiocoraline has been inserted in a suitable host cell; nevertheless, in one particular embodiment, said nucleic acid molecule of the invention has been isolated from the marine actinomycete *Micromonospora sp.* ML1 (see experimental part, Step 1, Examples 1-4).

The isolation and characterization of (chromosomal) genomic DNA and of cloned recombinant DNA from suitable host cells can be carried out by means of conventional or severe hybridization techniques, using the entire or part of a nucleotide sequence as a probe for tracing a suitable gene library.

Therefore, in another aspect, the invention relates to a probe comprising a nucleic acid molecule of the invention or a fragment thereof. In general, the suitably comprise a sequence of at least 5, 10, 15, 20, 25, 30, 40, 50, 60 or more nucleotides. The sequences with a length of 20 to 60 nucleotides are preferred. In one particular embodiment, said probe can be used to detect genes involved in the biosynthesis of thiocoraline in *Micromonospora sp.* The use of said probe to detect a nucleic acid, e.g., gDNA, cDNA or mRNA, related to the biosynthesis of thiocoraline forms an additional aspect of this invention.

Alternatively, the isolation and characterization of (chromosomal) genomic DNA and of the cloned recombinant DNA from suitable host cells can be carried out by means of techniques based on the enzymatic amplification of nucleic acids. By way of illustration, initiator oligonucleotides can be designed (based on the known sequences of DNA and of proteins involved in the biosynthesis of thiocoraline) which can be used in enzymatic amplification reactions, PCR for example, to amplify and identify other identical or related sequences.

The nucleic acid molecules of the invention can be isolated and, if desired, purified by conventional methods. Although the nucleic acid molecules of the invention will generally be obtained by recombinant or isolation methods, the invention also contemplates the possibility that the nucleic acid molecules of the invention are obtained by chemical synthesis, which molecules will have the same, or substantially the same structure as those derived from both wild-type (wt) and mutant thiocoraline-producing organisms.

In another aspect, the invention relates to a vector, hereinafter vector of the invention, comprising a nucleic acid molecule of the invention encoding at least one biosynthetic thiocoraline production pathway protein, or a biologically active fragment thereof. In one particular embodiment, the vector of the invention is a biologically functional vector or plasmid, such as cloning vector or an expression vector.

In one specific embodiment, the vector of the invention is a cloning vector, preferably a cosmid. Preferred cloning vectors are selected by their capacity to incorporate large DNA sequences (e.g., complete clusters of genes involved in the biosynthesis of products of interest). Said vectors are generally conventional vectors and are commonly available. The present invention further contemplates that the genetic material can be reduced so as to be finally contained in a single cloning vector or plasmid (e.g., cosmid) by means of genetic manipulation by techniques known by persons skilled in the art. The rearrangement can be carried out by means of cloning, PCR or synthetic genes or combination of any of these techniques known in the state of the art.

In another particular embodiment, the vector of the invention is an expression vector suitable for its insertion into a suitable host cell. The insertion of said vector into said suitable host cell can be carried out by any conventional genetic material transfer method (e.g., transformation, transfection, etc.).

Therefore, in another aspect, the invention relates to a host cell, hereinafter host cell of the invention, transformed or transfected with a vector of the invention. Said host cell of the invention contains one or more nucleic acid molecules of the invention. In one particular embodiment, the host cell of the invention contains a nucleic acid molecule of the invention. In another particular embodiment, the host cell of the invention contains two or more nucleic acid molecules of the invention; in this case, said nucleic acid molecules of the invention can be identical of different from one another.

A preferred host cell of the invention is a host cell stably transformed or transfected with a vector of the invention comprising an (exogenous) nucleic acid molecule of the invention comprising a nucleotide sequence encoding at least one biosynthetic thiocoraline production pathway protein, or a biologically active fragment thereof, in a manner sufficient to direct the biosynthesis and/or rearrangement of thiocoraline. The host cell is preferably a microorganism, more preferably a bacterium. In one particular embodiment, said host cell is a Gram-positive bacterium, such as an actinomycete, a streptomycete for example.

Although different streptomycete species such as *Streptomyces coelicolor, Streptomyces lividans, Streptomyces albus* and *Streptomyces avermitilis* have been used in the examples of the present invention as heterologous hosts, the heterologous expression of the genes involved in the biosynthesis of thiocoraline can be carried out in other streptomycetes, actinomycetes, etc., provided that they can be transformed, preferably in a stable manner, with the vectors of the invention. The *in vitro* expression of the proteins can be carried out, if desired, using conventional methods.

In one particular embodiment, the invention provides a host cell of the invention, such as a recombinant bacterium for example, in which at least one region of the nucleic acid molecule of the invention has been altered to give rise to a recombinant host cell, such as a recombinant bacterium, producing altered thiocoraline levels compared to the corresponding non-recombinant, i.e. wt, thiocoraline-producing cell (bacterium). To that end, conventional techniques known by persons skilled in the art can be used, which include for example increasing the number of copies of the genes responsible for the most important domains of the NRPSs involved in the production of thiocoraline or increasing the gene expression-regulating sequences of those genes by genetic engineering techniques known in the state of the art and thus increasing the yield in the production of thiocoraline.

In another aspect, the invention relates to a protein, hereinafter protein of the invention, encoded by the nucleic acid molecule of the invention.

As used herein, the term "protein" means polypeptides, enzymes and the like, encoded by the nucleic acid molecule of the invention comprised by the biosynthetic pathway for the production of thiocoraline. The proteins of the invention include amino acid chains with variable lengths, including full-length amino acid chains, wherein the amino acid moieties are joined by covalent peptide bonds, as well as biologically active fragments of said proteins involved in the biosynthesis of thiocoraline, as well as the biologically active variants thereof. The proteins of the invention can be natural, recombinant or synthetic. By way of illustration, said proteins involved in the biosynthesis of thiocoraline can be produced through conventional recombinant DNA technology, inserting a nucleotide sequence encoding the protein into a suitable expression vector and expressing the protein in a suitable host cell or through conventional chemical peptide synthesis, for example, by means of the solid-phase peptide synthesis of Merrifield (Merrifield, J. Am. Chem. Soc. 85:2149-2154 (1963)) in which the amino acids are individually and sequentially joined to the amino acid chain. Alternatively, the proteins of the invention can be synthesized using equipment for automated protein synthesis marketed by different manufacturers (e.g., Perkin-Elmer, Inc.).

The biologically active variants included within the scope of the present invention comprise at least one biologically active fragment of the amino acid sequence encoded by the nucleic acid molecule of the invention, i.e., a part of the protein structure retaining the active function of the protein, for example, the thioesterase part encoded by the *tio18* gene having the same or substantially the same activity as the Tio18 protein encoded by said *tio18* gene, i.e., it has at least a similarity or power of at least approximately 70%, advantageously of at least 80%, preferably of at least 90%, more preferably of about 95% approximately.

The biologically active variants of the proteins of the invention include active amino acid structures in which amino acids, naturally occurring alleles, etc. have been eliminated, substituted or added. The biologically active fragment can be easily identified by subjecting the full-length protein to chemical or enzymatic digestion in order to prepare fragments and then assaying the amino acid structure fragments conserving the same or substantially the same biological activity as the full-length protein.

In one particular embodiment, the protein of the invention is an optionally purified, isolated protein involved in the biosynthesis of thiocoraline encoded by a gene selected from the group consisting of the genes identified as *orf1, orf2, tio3, tio4, tio5, tio6, tio7, tio8, tio9, tio10, tio11, tio12, tio13, tio14, tio15, tio16, tio17, tio18, tio19, tio20, tio21, tio22, tio23, tio24, tio25, tio26, tio27, tio28, orf29, orf30, orf31, orf32, orf33, orf34, orf35, orf36, orf37* and *orf38.*

In another particular embodiment, the protein of the invention is an optionally purified, isolated protein involved in the biosynthesis of thiocoraline selected from the group consisting of the proteins identified as ORF1 (SEQ ID NO: 2), ORF2 (SEQ ID NO: 3), Tio3 (SEQ ID NO: 4), Tio4 (SEQ ID NO: 5), Tio5 (SEQ ID NO: 6), Tio6 (SEQ ID NO: 7), Tio7 (SEQ ID NO: 8), Tio8 (SEQ ID NO: 9), Tio9 (SEQ ID NO: 10), Tio10 (SEQ ID NO: 11), Tio11 (SEQ ID NO: 12), Tio12 (SEQ ID NO: 13), Tio13 (SEQ ID NO: 14), Tio14 (SEQ ID NO: 15), Tio15 (SEQ ID NO: 16), Tio16 (SEQ ID NO: 17), Tio17 (SEQ ID NO: 18), Tio18 (SEQ ID NO: 19), Tio19 (SEQ ID NO: 20), Tio20 (SEQ ID NO: 21), Tio21 (SEQ ID NO: 22), Tio22 (SEQ ID NO: 23), Tio23 (SEQ ID NO: 24), Tio24 (SEQ ID NO: 25), Tio25 (SEQ ID NO: 26), Tio26 (SEQ ID NO: 27), Tio27 (SEQ ID NO: 28), Tio28 (SEQ ID NO: 29), ORF29 (SEQ ID NO: 30), ORF30 (SEQ ID NO: 31), ORF31 (SEQ ID NO: 32), ORF32 (SEQ ID NO: 33), ORF33 (SEQ ID NO: 34), ORF34 (SEQ ID NO: 35), ORF35 (SEQ ID NO: 36), ORF36 (SEQ ID NO: 37), ORF37 (SEQ ID NO: 38), ORF38 (SEQ ID NO: 39), and combinations thereof, or biologically active fragments thereof. The hypothetical functions of said proteins are included in Table 1.

The *orfs* of the cluster of genes responsible for the biosynthesis of thiocoraline, encoding the proteins involved in the biosynthesis of said compound, can be identified using conventional techniques. Illustrative non-limiting examples of said techniques include computational analysis for locating the stop and start codons, the putative locations of the reading frames based on the frequencies of the codons, alignments by similarity to genes expressed in other actinomycetes and the like. The proteins of the invention can thus be identified using the nucleotide sequence of the present invention and the *orfs* or the proteins encoded by them can be isolated and if desired, purified, or alternatively, synthesized by chemical methods. Gene constructs for the expression of said products based on the *orfs* can be designed and the suitable expression regulating elements (promoters, terminators, etc.) can be included and said gene constructs can be introduced in suitable host cells for expressing the protein or proteins encoded by one ore more *orfs.*

The proteins of the invention can be isolated and, if desired, purified by conventional methods. The proteins are preferably obtained in a substantially pure form, although a lower degree of purity, typically from 80% to 90% approximately, can also be acceptable. The invention also contemplates the possibility that the proteins of the invention are obtained by chemical synthesis, which proteins will have the same or substantially the same structure as those directly derived from both wild-type (wt) and mutant thiocoraline-producing organisms.

In another aspect, the invention relates to a process for producing a protein of the invention involved in the biosynthesis of thiocoraline which comprises growing, under suitable (nutrient and environmental) conditions, a thiocoraline-producing organism and, if desired, isolating one or more of said proteins involved in the biosynthesis of thiocoraline. If desired, said protein of the invention can be isolated and purified by conventional methods, such as those described previously.

In another aspect, the invention relates to a method for producing thiocoraline which comprises growing, under suitable conditions for producing said compound, a thiocoraline-producing organism in which the number of copies of genes encoding proteins involved in the biosynthesis of thiocoraline has been increased, and, if desired, isolating thiocoraline.

In one particular embodiment, the thiocoraline-producing organism is an actinomycete such as *Micromonospora sp* for example, in which the number of copies of genes encoding proteins involved in the biosynthesis of thiocoraline has been increased. The increase in the number of copies of genes encoding proteins involved in the biosynthesis of thiocoraline can be carried out by conventional methods known by persons skilled in the art. In this case, the previously described method comprises fermenting said organism under suitable nutrient and environmental conditions for the expression of the genes involved in the production of thiocoraline. If desired, the thiocoraline produced can be isolated and purified from the culture medium by conventional methods.

In another aspect, the invention relates to a method for producing thiocoraline which comprises growing, under suitable conditions for producing said compound, a thiocoraline-producing organism in which the expression of the genes encoding the proteins responsible for the biosynthesis of thiocoraline has been modulated by means of manipulating or substituting one or more genes encoding proteins involved in the biosynthesis of thiocoraline or by means of manipulating the sequences responsible for regulating the expression of said genes, and, if desired, isolating thiocoraline. The expression of the genes encoding said proteins responsible for the biosynthesis of thiocoraline has preferably been improved. To that end, the unessential gene sequences in the thiocoraline biosynthesis process can be eliminated, or the efficiency of the gene expression-regulating sequences of said genes can be increased by genetic engineering sequences known by persons skilled in the art. The yield in the production of thiocoraline can thus be increased. The genetic manipulation for eliminating the unessential gene sequences in the thiocoraline biosynthesis process or for increasing the efficiency of the gene expression-regulating sequences of said genes can be carried out by genetic engineering techniques known by persons skilled in the art.

In one particular embodiment, the thiocoraline-producing organism is an actinomycete such as *Micromonospora sp* for example, in which the expression of the genes encoding the proteins responsible for the biosynthesis of thiocoraline has been modulated by means of manipulating or substituting one or more genes encoding proteins involved in the biosynthesis of thiocoraline or by means of manipulating the sequences responsible for regulating the expression of said genes, which can be carried out by conventional methods known by persons skilled in the art. In this case, the previously described method comprises fermenting said organism under suitable nutrient and environmental conditions for the expression of the genes involved in the production of thiocoraline. If desired, the thiocoraline produced can be isolated and purified from the culture medium by conventional methods.

In another aspect, the invention relates to a method for producing thiocoraline which comprises growing, under suitable conditions for producing said compound, a host cell of the invention transformed or transfected with a vector of the invention comprising the cluster of genes responsible for the biosynthesis of thiocoraline, and, if desired, isolating thiocoraline. The (nutrient, environmental, etc.) conditions will be selected according to the nature of the host cell.

In one particular embodiment, the host cell of the invention is selected from an organism producing thiocoraline natively, an organism that does not produce thiocoraline natively and an organism that has been genetically manipulated to produce thiocoraline. In one particular embodiment, said host cell of the invention is an actinomycete or a streptomycete.

In another aspect, the invention relates to a process, based on the use of genes responsible for the biosynthesis of thiocoraline from *Micromonospora sp.* ML1, for the production of said compound in another actinomycete, which comprises:
(1) obtaining mutants affected in specific genes of the thiocoraline biosynthesis pathway;
(2) isolating the *Micromonospora sp.* ML1 chromosome region containing the cluster of genes responsible for the biosynthesis of thiocoraline;
(3) obtaining and analyzing the nucleotide sequence of the cluster of genes responsible for the biosynthesis of thiocoraline; and
(4) heterologously producing thiocoraline in other actinomycetes.

The identification and isolation of the *Micromonospora sp.* ML1 chromosome region containing the cluster of genes responsible for the biosynthesis of thiocoraline, as well as the analysis of the nucleotide sequence of said cluster can be carried out based on the teachings provided by this invention, illustrated in a non-limiting manner in the Examples attached to this description.

The mutants affected in specific genes of the thiocoraline biosynthesis pathway can be identified by conventional methods. In one particular embodiment, said mutants can be identified by means of culturing and measuring the production of thiocoraline by conventional methods, by HPLC-MS for example, as mentioned in Example 5.

The entire or part of the cluster of genes responsible for the biosynthesis of thiocoraline can be introduced in an actinomycete by conventional methods, e.g., by transformation or transfection, for the heterologous production of thiocoraline by fermenting a suitable nutrient medium under the suitable conditions for the production of thiocoraline and, if desired, the thiocoraline thus obtained can be isolated and/or purified by conventional methods.

The determination of the cluster of genes responsible for the biosynthesis of thiocoraline has a great commercial importance. The isolation and complete description of the cluster of genes responsible for the biosynthesis of thiocoraline provided by this invention allows increasing the production of thiocoraline and manipulating thiocoraline-producing organisms. In this sense, the number of copies of the genes responsible for the most important domains of the NRPSs involved in the production of thiocoraline can be increased or the efficiency of the gene expression-regulating sequences of those genes can be increased by genetic engineering techniques known in the state of the art and the yield in its production can thus be increased.

Another advantage associated to the identification and cloning of the complete cluster of thiocoraline genes provided by the present invention relates to the efficient production of thiocoraline. In fact, it allows obtaining a compound of great interest in a smaller number of steps. The elimination of unessential sequences in the biosynthesis process in cluster mutants considerably reduces the time necessary for producing the compound of interest. The remaining sequences are sufficient and maintain their functionality for producing thiocoraline.

### EXPERIMENTAL PART

The experimental procedures of the present invention include conventional molecular biology methods in the current state of the art. Detailed descriptions of the techniques that are not explained herein can be found in the manuals of Kieser et al. (Practical Streptomyces genetics. The John Innes Foundation, Norwich, Great Britain, 2000) and Sambrook et al. (Molecular cloning: a laboratory manual. Cold Spring Harbor Laboratory Press, Cold Spring Harbor, New York, USA, 2001). The following steps describe in detail the present invention without limitation.

### Step 1. Isolating the Micromonospora sp. ML1 chromosome region containing the thiocoraline biosynthesis pathway genes

### Example 1. Construction of a gene library in SuperCos1 from Micromonospora sp. ML1 chromosomal DNA

Chromosomal DNA was obtained using the salting out protocol (Kieser et al. 2000) from a *Micromonospora sp.* ML1 culture (Espliego, F. Ph.D. Thesis, 1996, University of Leon; de la Calle, F. Ph.D. Thesis, 1998, Autonomous University of Madrid), available in the Pharma Mar, S.A. culture collection, in MIAM2 medium (5 g/l of yeast extract, 3 g/l of meat extract, 5 g/l of tryptone, 5 g/l of glucose, 20 g/l of dextrin, 4 g/l of CaCO₃, 10 g/l of sea salts. pH 6.8). This chromosomal DNA was subjected to partial digestion with the *Bam*HI endonuclease and the fragments obtained were used to generate a gene library in the cosmid SuperCos 1 (Stratagene), digested with *Bam*HI*.* The generation of this gene library in *E. coli* XL-1 Blue MR (Stratagene) was carried out according to already described procedures (Sambrook et al. 2001) and the *in vitro* packaging kit Gigapack III Gold Packaging Extract Kit (Stratagene).

1,000 *E. coli* transducing colonies were deposited on nylon membranes in order to conduct an *in situ* colony hybridization analysis by means of usual protocols (Sambrook et al. 2001).

### Example 2. Construction of a gene library in pKC505 from Micromonospora sp. ML1 chromosomal DNA

Chromosomal DNA was obtained using the salting out protocol (Kieser et al. 2000) from a *Micromonospora sp.* ML1 culture in MIAM2 medium. This chromosomal DNA was subjected to partial digestion with the *Sau*3AI endonuclease and the fragments obtained were used to generate a gene library in the bifunctional cosmid *Escherichia coli*/*Streptomyces* pKC505 (Richardson at al. 1987, Gene 61, 231-241), digested with BamHI. The generation of this gene library in *E. coli* ED8767 was carried out according to already described procedures (Sambrook et al. 2001) and the *in vitro* packaging kit Gigapack III Gold Packaging Extract Kit (Stratagene).

3,300 *E. coli* transducing colonies were deposited on 96-well microtiter plates containing TSB medium (Merck) with 25 µg/ml of apramycin and incubated at 30°C for 24 hours. These clones were replicated to TSA (Tryptic Soy Agar) plates with 25 µg/ml of apramycin, and after one night at 30°C, the colonies were transferred to nylon membranes in order to conduct an *in situ* colony hybridization analysis by means of usual protocols (Sambrook et al. 2001).

### Example 3. Design of specific oligonucleotides for adenylation domains in NRPS and their PCR amplification from Micromonospora sp. ML1 chromosomal DNA

Based on the structure of thiocoraline, the NRPSs responsible for its biosynthesis were expected to have from one to three adenylation domains activating L-cysteine and one domain activating glycine. On this basis, degenerated oligonucleotides, based on conserved regions inside the NRPS adenylation domains, which can specifically amplify DNA fragments encoding NRPS adenylation domains which were combined with oligonucleotides described in the literature for the amplification of NRPS adenylation domains were designed.

The PCR amplification with the initiator oligonucleotides:
MTF2 (5'-GCNGGYGGYGCNTAYGTNCC-3'; Neilan et al. 1999. J. Bacteriol. 181(13):4089-4097) and
PSV-4 (5'-SAGSAGGSWGTGGCCGCCSAGCTCGAAGAA-3')
resulted in a 1.3 kb band which was cloned into a pGEM-T Easy vector (Promega). The PCR program used was an initial cycle of 95°C-2 min; 60°C-15 min; 72°C-6 min followed by 20 cycles of 95°C-1 min; 60°C-2 min; 72°C-2 min. *Micromonospora sp.* ML1 chromosomal DNA was used as a template.

The analysis of the clones by restriction fragment length polymorphism (RFLP) showed that there were three types of different clones corresponding to peptide synthetases, pGPSV1, pGPSV2 and pGPSV3, which contained the adenylation domains fragments called PSV1, PSV2 and PSV3, respectively.

The insert of the clones was subsequently released with an EcoRI digestion and the fragment was cloned into pBBR1-MCS2 (Kovach, M.E. et al. 1995. Gene. 166:175-176) to construct plasmids pBPSV1, pBPSV2 and pBPSV3, respectively, which contained the adenylation domains fragments called PSV1, PSV2 and PSV3, respectively.

From the PCR band obtained with initiator oligonucleotides MTF2 and PS4, a nested-PCR (30 cycles of 95°C-1 min; 60°C-1 min; 72°C-1 min) was carried out with the initiator oligonucleotides PS2-TG: 5'-ACNGGNMRNCCNAARGG-3' and MTR: 5'-CCNCGDATYTTNACY-3' (Neilan et al. 1999. J. Bacteriol. 181(13):4089-4097) in order to obtain a 750 bp band which was cloned into a pGEM-T Easy vector (Promega). The analysis of the clones by RFLP showed that there were two new types of different clones corresponding to peptide synthetases, pGPSV4 and pGPSV5 respectively, which contained the adenylation domain fragments called PSV4 and PSV5, respectively.

The PCR amplification with the initiator oligonucleotides PS2M: 5'- TACACSGGCWSSACSGG-3' and PSV-4 resulted in a 1.3 kb band which was cloned into a pGEM-T Easy vector (Promega). The program used was a Touch-down starting with 5 cycles at the annealing temperature of 72°C, followed by 10 cycles at 70°C of annealing to end with 20 cycles at 68°C (96°C-1 min; 72°C- 68°C-2 min; 72°C-3 min). The analysis of the clones by RFLP showed that was a new type of clone corresponding to a peptide synthetase, pGPSV6, which contained the adenylation domain fragment called PSV6.

### Example 4. Analysis of the gene libraries by colony hybridization

The gene libraries constructed in SuperCos1 and in pKC505 (Examples 1 and 2) were subjected to respective *in situ* colony hybridization analyses (Sambrook et al. 2001) using the DIG DNA Labeling and Detection Kit system (Roche). The 6 adenylation domain fragments called PSV1- PSV6 were used as probes.

The following was obtained from the gene library constructed in SuperCos1:
- 3 positive cosmids (clones) which hybridized with fragment PSV1, called pCT1a, pCT1b and pCT1c;
- 3 positive cosmids (clones) which hybridized with fragment PSV2, called pCT2a, pCT2b and pCT2c; from these fragments, pCT2c also hybridized with fragment PSV5;
- 2 positive cosmids (clones) which hybridized with fragment PSV3, called pCT3a and pCT3b; furthermore, both of them also hybridized with fragment PSV6; and
- 1 positive cosmid (clone) which hybridized with PSV4, called pCT4a.

55 positive cosmids were obtained from the gene library constructed in pKC505:
- 10 positive cosmids (clones) which hybridized with fragment PSV2, called cosV1-F8, cosV7-D2, cosV7-D12, cosV14-H4, cosV19-B4, cosV29-B9, cosV31-B11, cosV31-H10, cosV33-D12, cosV33-F7;
- 7 positive cosmids which hybridized with fragment PSV5, called cosV1-B6, cosV6-H8, cosV11-F10, cosV20-F8, cosV22-F7, cosV25-B3, cosV32-B4; and
- 38 positive cosmids which hybridized with fragments PSV1, PSV3, PSV4 or PSV6, called cosV1-B7, cosV1-F5, cosV2-E5, cosV2-F11, cosV3-D9, cosV4-D2, cosV5-D7, cosV5-G6, cosV6-A7, cosV6-A12, cosV7-E7, cosV8-F8, cosV9-H7, cosV10-A3, cosV11-B4, cosV11-G2, cosV12-B12, cosV13-B2, cosV16-H11, cosV17-A3, cosV19-F4, cosV20-B3, cosV20-H5, cosV21-H6, cosV22-Bll, cosV23-F8, cosV26-H11, cosV28-G1, cosV29-E1, cosV29-G6, cosV30-G5, cosV31-A12, cosV31-E10, cosV32-A7, cosV32-D10, cosV33-A8, cosV33-D10, cosV33-F10.

### Step 2. Generating mutants in six isolated adenylation regions

The six adenylation domain fragments previously amplified from *Micromonospora sp.* ML1 chromosomal DNA (PSV1, PSV2, PSV3, PSV4, PSV5 and PSV6) were used for independent gene interruption experiments for the purpose of evaluating the regions involved in the biosynthesis of thiocoraline (Examples 6-11).

The conjugative plasmid *E. coli*/*Streptomyces* pOJ260 (Bierman et al. 1992, Gene 116, 43-49) was used to generate constructs pFL903, pFL904, pFL905, pFL906, pFL940 and pFL941 which contained regions PSV1 to PSV6, respectively. These constructs were introduced in the conjugative *E. coli* ET12567 (pUB307) strain (Kieser et al. 2000) and from here, by conjugation, in the *Micromonospora sp.* ML1 strain, using described procedures (Kieser et al. 2000). The transconjugant clones were selected with apramycin and the integration in the suitable chromosomal region was verified by means of Southern hybridization using the corresponding regions of the adenylation domain fragments PSV1 to PSV6. The transconjugants selected from each region of the PSV adenylation domains (PSV1-PSV6) were grown in thiocoraline production medium MT4 and their mycelium was subsequently extracted with acetonitrile and analyzed by HPLC-MS (Example 5). Only the mutants affected in the adenylation domains PSV2 and PSV5 has a phenotype that does not produce thiocoraline (Examples 7 and 10). The production of thiocoraline in mutants with deletions in PSV1, PSV3, PSV4 and PSV6 was similar to that of the wt strain (Examples 6, 8, 9 and 11). These experiments showed that the adenylation domains PSV2 and PSV5 were involved in the biosynthesis of thiocoraline.

### Example 5. HPLC detection of the production of thiocoraline

The extracts with acetonitrile of the different analyzed strains were concentrated in the rotary evaporator and resuspended in DMSO before being used in HPLC-MS analysis.

The samples (10 µl) were analyzed by HPLC, using a reversed-phase column (Symmetry C₁₈, 2.1 X 150 mm, Waters), using acetonitrile and a mixture of 0.1% of trifluoroacetic acid in water as solvents. During the first 4 minutes, a concentration of the mobile phase with 10% of acetonitrile was maintained isocratically. Then, up to 30 minutes, a linear gradient from 10% to 100% of acetonitrile is started. The flow used was 0.25 ml/min. The spectral detection and characterization of the peaks was carried out using a photodiode detector and by means of using the Millennium computer software (Waters). The chromatograms were extracted at an absorbance of 230 nm.

### Example 6. Gene interruption in the PSV1 region

The PSV1 region was obtained from plasmid pBPSV1 as an 1.3 kb *Eco*RI band and was cloned into the *Eco*RI site of conjugative plasmid *E. coli*/*Streptomyces* pOJ260, generating pFL903. pOJ260 contains a gene conferring apramycin resistance in *Streptomyces* and in these cells it is a suicide plasmid.

The construct pFL903 was introduced in the conjugative *E. coli* ET12567 (pUB307) strain and from there, by conjugation, in the *Micromonospora sp.* ML1 strain, using described procedures (Kieser et al. 2000). The transconjugant clones were selected with 25 µg/ml of apramycin and, from the chromosomal DNA thereof, it was verified that the PSV1 region has indeed been interrupted by means of Southern hybridization. The probe used in this case was the PSV1 band.

The mutant *Micromonospora sp.* ΔPSV1 was grown in thiocoraline production medium MT4 and its mycelium was subsequently extracted with acetonitrile and analyzed by HPLC-MS (see Example 5), proving to be a thiocoraline producer. The composition of the culture medium MT4 per liter is as follows: 6 g soy flour, 2.5 g of malt extract, 2.5 g of peptone, 5 g of dextrose, 20 g of dextrin, 4 g of CaCO₃, 10 g of sea salts, adjust the pH to 6.8.

### Example 7. Gene interruption in the PSV2 region

The PSV2 region was obtained from plasmid pBPSV2 as a 1.3 kb *Eco*RI band and was cloned into the *Eco*RI site of plasmid pOJ260, generating pFL904.

The construct pFL904 was introduced in the conjugative *E. coli* ET12567 stain (pUB307) and from there, by conjugation, in the *Micromonospora sp.* ML1 strain. The transconjugant clones were selected with 25 µg/ml of apramycin and, from the chromosomal DNA thereof, it was verified that the PSV2 region has indeed been interrupted by means of Southern hybridization. The probe used in this case was the PSV2 band.

The mutant *Micromonospora sp*. ΔPSV2 was grown in thiocoraline production medium MT4 and its mycelium was subsequently extracted with acetonitrile and analyzed by HPLC-MS (Example 5), giving as a result that this strain did not produce thiocoraline.

### Example 8. Gene interruption in the PSV3 region

The PSV3 region was obtained from plasmid pBPSV3 as a 1.4 kb *Eco*RI band and was cloned into the *Eco*RI site of plasmid pOJ260, generating pFL905.

The construct pFL905 was introduced in the conjugative *E. coli* ET12567 (pUB307) strain and from there, by conjugation, in the *Micromonospora sp.* ML1 strain. The transconjugant clones were selected with 25 µg/ml of apramycin and, from the chromosomal DNA thereof, it was verified that the PSV3 region had indeed been interrupted by means of Southern hybridization. The probe used in this case was the PSV3 band.

The mutant *Micromonospora sp*. ΔPSV3 was grown in thiocoraline production medium MT4 and its mycelium was subsequently extracted with acetonitrile and analyzed by HPLC-MS (Example 5), proving to be a thiocoraline producer.

### Example 9. Gene interruption in the PSV4 region

The PSV4 region was obtained from plasmid pGPSV4 as a 1.2 kb *Eco*RI band and was cloned into the *Eco*RI site of plasmid pOJ260, generating pFL906.

The construct pFL906 was introduced in the conjugative *E. coli* ET12567 (pUB307) strain and from there, by conjugation, in the *Micromonospora sp.* ML1 strain. The transconjugant clones were selected with 25 µg/ml of apramycin and from the chromosomal DNA thereof, it was verified that the PSV4 region had indeed been interrupted by means of Southern hybridization. The probe used in this case was the PSV4 band.

The mutant *Micromonospora sp.* ΔPSV4 was grown in thiocoraline production medium MT4 and its mycelium was subsequently extracted with acetonitrile and analyzed by HPLC-MS (Example 5), proving to be a thiocoraline producer.

### Example 10. Gene interruption in the PSV5 region

The PSV5 region was obtained from plasmid pGPSV5 as a 1.1 kb *Eco*RI band and was cloned into the *Eco*RI site of plasmid pOJ260, generating pFL940.

The construct pFL940 was introduced in conjugative *E. coli* ET12567 (pUB307) strain and from there, by conjugation, in the *Micromonospora sp.* ML1 strain. The transconjugant clones were selected with 25 µg/ml of apramycin and, from the chromosomal DNA thereof, it was verified that the PSV5 region had indeed been interrupted by means of Southern hybridization. The probe used in this case was the PSV5 band.

The mutant *Micromonospora sp.* ΔPSV5 was grown in thiocoraline production medium MT4 and its mycelium was subsequently extracted with acetonitrile and analyzed by HPLC, giving as a result that this strain did not produce thiocoraline.

### Example 11. Gene interruption in the PSV6 region

The PSV6 region was obtained from plasmid pGPSV6 as a 1.1 kb *Eco*RI band and was cloned into the *Eco*RI site of plasmid pOJ260, generating pFL941.

The construct pFL941 was introduced in the conjugative *E. coli* ET12567 (pUB307) strain and from there, by conjugation, in the *Micromonospora sp.* ML1 strain. The transconjugant clones were selected with 25 µg/ml of apramycin and, from the chromosomal DNA thereof, it was verified that the PSV6 region had indeed been interrupted by means of Southern hybridization. The probe used in this case was the PSV6 band.

The mutant *Micromonospora sp.* ΔPSV6 was grown in thiocoraline production medium MT4 and its mycelium was subsequently extracted with acetonitrile and analyzed by HPLC, proving to be a thiocoraline producer.

### Step 3. Obtaining and analyzing the nucleotide sequence of the gene cluster responsible for the biosynthesis of thiocoraline

Based on the previous results, in which the amplified areas of the adenylation domains PSV2 and PSV5 were the only ones the gene interruption of which caused a phenotype that did not produce thiocoraline, two overlapping cosmids, cosV33-D12 (containing the region of adenylation domain PSV2) and pCT2c (containing the regions of adenylation domains PSV2 and PSVS), were chosen to be sequenced. The analysis of the 64,650 bp obtained from said cosmids showed the presence of 36 complete ORFs and 2 incomplete ORFs, the organization of which is shown in Figure 1. The comparison with the protein sequences existing in the databases of the products deduced from the different genes allowed deducing the functions for most of them (Table 1).

### Example 12. Determination and analysis of the nucleotide sequence of the insert of cosmids cosV33-D12 and pCT2c

Both cosmids were sequenced using the usual methodology and the program package GCG, from the Genetics Computer Group of the University of Wisconsin, was used for the computer analysis of the sequence (Devereux et al. 1984, Nucleic Acid Res. 12, 387-395).

A sequence of 64,650 nucleotides was thus obtained, the computer analysis of which showed the existence of 38 ORFs [36 complete ORFs and 2 incomplete ORFs], the organization of which is in Figure 1. The gene expression products of said ORFs were compared with proteins having a known function present in the databases using the BLAST program (Altschul et al. 1997, Nucleic Acid Res. 25, 3389-3402), whereby the probable functions for most of these ORFs were assigned (Table 1).

**Table 1**

| **Gene** | **Position** | **Amino acids** | **Deduced Function** | **Notes** |
|---|---|---|---|---|
| *ORF1* | 2-535 | 178* | Transposase | SEQ ID NO:2 |
| *ORF2* | 993-1130c | 46 | Unknown | SEQ ID NO:3 |
| *Tio3* | 1517-2131 | 205 | OmpR family regulator | SEQ ID NO:4 |
| *Tio4* | 2154-2822c | 223 | Possible regulator | SEQ ID NO:5 |
| *Tio5* | 2970-3791c | 274 | ABC transporter (permease subunit) | SEQ ID NO:6 |
| *Tio6* | 3794-4777c | 328 | ABC transporter (ATPase subunit) | SEQ ID NO:7 |
| *Tio7* | 4904-5611 | 236 | MerR family regulator | SEQ ID NO:8 |
| *Tio8* | 5701-6426c | 242 | Tryptophan 2,3-dioxygenase | SEQ ID NO:9 |
| *Tio9* | 6426-7688c | 421 | Kynurenine aminotransferase | SEQ ID NO:10 |
| *Tio10* | 7733-8524c | 264 | NAD- or NADP-oxidoreductase | SEQ ID NO:11 |
| *Tio11* | 8791-10002 | 404 | Quinaldate 3-hydroxylase (Cytochrome P₄₅₀) | SEQ ID NO:12 |
| *Tio12* | 10022-11590c | 523 | 3-hydroxy-quinaldate-AMP-Ligase | SEQ ID NO:13 |
| *Tio13* | 11847-13634 | 596 | NRPS | SEQ ID NO:14 |
| *Tio14* | 13734-15005c | 424 | Unknown | SEQ ID NO:15 |
| *Tio15* | 15005-16354c | 450 | V-chloroperoxidase | SEQ ID NO:16 |
| *Tio16* | 16441-18744c | 768 | NRPS | SEQ ID NO:17 |
| *Tio17* | 18774-19055c | 94 | 3-hydroxy-quinaldate-Carrier Protein | SEQ ID NO:18 |
| *Tio18* | 19260-20036 | 259 | Thioesterase | SEQ ID NO:19 |
| *Tiol9* | 20146-20880c | 245 | Thioesterase | SEQ ID NO:20 |
| *Tio20* | 21188-28969 | 2594 | NRPS | SEQ ID NO:21 |
| *Tio21* | 28979-38398 | 3140 | NRPS | SEQ ID NO:22 |
| *Tio22* | 38449-38661 | 71 | Unknown (similar to MbtH) | SEQ ID NO:23 |
| *Tio23* | 38642-41263 | 874 | DNA excisionase | SEQ ID NO:24 |
| *Tio24* | 41835-42368 | 178 | OmpR family regulator | SEQ ID NO:25 |
| *Tio25* | 42395-43255c | 287 | Possible regulator | SEQ ID NO:26 |
| *Tio26* | 43340-43741c | 134 | Unknown | SEQ ID NO:27 |
| *Tio27* | 44152-49563 | 1804 | NRPS | SEQ ID NO:28 |
| *Tio28* | 49635-53669 | 1345 | NRPS | SEQ ID NO:29 |
| *ORF29* | 53749-55305c | 519 | Glucoside permease | SEQ ID NO:30 |
| *ORF30* | 55384-57222c | 613 | Glucoside permease | SEQ ID NO:31 |
| *ORF31* | 57895-58467 | 191 | MarR family regulator | SEQ ID NO:32 |
| *ORF32* | 58535-59206c | 224 | Anti anti-σ factor | SEQ ID NO:33 |
| *ORF33* | 59298-59564c | 89 | Unknown | SEQ ID NO:34 |
| *ORF34* | 59611-60114c | 168 | Anti anti-σ factor | SEQ ID NO:35 |
| *ORF35* | 60202-60888 | 229 | Regulator system of two components (Response regulator) | SEQ ID NO:36 |
| *ORF36* | 60960-62240 | 427 | Regulator system of two components (Histidine kinase) | SEQ ID NO:37 |
| *ORF37* | 62300-62833 | 178 | Unknown | SEQ ID NO:38 |
| *ORF38* | 62925-64650 | 574* | Chaperon DnaK | SEQ ID NO:39 |

| | | | | |
|---|---|---|---|---|
| * Incomplete ORF | | | | |

Some of the identified proteins are involved in the formation of the thiocoraline peptide structure, such as for example several of the identified NRPSs, Tio12, Tio17, Tio18, Tio19, Tio20, Tio21, Tio22, Tio27 and Tio28. There are also several proteins which can be related to resistance processes, such as Tio5, Tio6, and Tio23. The possible thiocoraline pathway regulators identified in the sequences region correspond to Tio3, Tio4, Tio7, Tio24, Tio25. Finally, there are also several proteins related to the generation of the initiator unit 3-hydroxy-quinaldate, Tio8, Tio9, Tio10 and Tio11.

The genes, the gene interruption of which generates a phenotype that does not produce thiocoraline, are indicated in Figure 1 by means of an asterisk *(tio20, tio27* and *tio28*).

### Example 13. Gene interruption in tio28

For the purpose of demonstrating the involvement or not of the Tio28 protein in the biosynthesis of thiocoraline, the inactivation by gene interruption of the *tio28* gene, and specifically of the single one of the adenylation domains it has, was carried out.

Two initiator oligonucleotides inside this adenylation domain (FL-T-102up and FL-T-102rp) were designed and used to amplify a 1,428 base pair area in *tio28.* The sequences of said initiator oligonucleotides are the following:
FL-T-102up: 5'-ACCTGAGGTACTGGGCGCAGC-3' (21 nucleotides)
FL-T-102rp: 5'- CCGATCACCACCACCGTGGC-3' (20 nucleotides)
The PCR program used was: 2 min at 94°C, 30 cycles (30 s at 94°C, 60 s at 53°C, 90 s at 68°C), 5 min at 68°C and 15 min at 4°C. The PCR reaction mixture contained: 1 µl of template DNA of cosmid pCT2c, 1 µl of each oligonucleotide at a 30 pmol/µl concentration, 7.5 µl of 2 mM dNTPs solution (dATP, dTTP, dCTP and dGTP), 1 µl of 50 mM MgSO₄, 5 µl of reaction buffer for Pfx (Invitrogene), 5 µl of Enhancer solution for Pfx (Invitrogene), 28 µl of distilled water and 0.5 µl of Pfx polymerase (Invitrogene).

The PCR product obtained, called PSV7, was cloned into the EcoRV site of plasmid pOJ260, generating pFL971.

The construct pFL971 was introduced in the conjugative *E. coli* ET12567 (pUB307) strain and from there, by conjugation, in the *Micromonospora sp.* ML1 strain. The transconjugant clones were selected with 25 µg/ml of apramycin and from the chromosomal DNA thereof, it was verified that the PSV7 region had indeed been interrupted by means of Southern hybridization. The probe used in this case was the PCR product PSV7.

The mutant *Micromonospora sp*. ΔPSV7 was grown in thiocoraline production medium MT4 and its mycelium was subsequently extracted with acetonitrile and analyzed by HPLC-MS (Example 5), giving as a result that this strain did not produce thiocoraline.

### Step 4. Heterologously expressing thiocoraline in other actinomycetes

To verify the involvement of the genes identified in the biosynthesis of thiocoraline, the heterologous expression of the cluster of thiocoraline genes in several *Streptomyces* species was assayed. The DNA region comprised between positions 1,393 (*Mse*I restriction site) and 54,301 (*Acl*I restriction site) of SEQ ID NO: 1 was chosen as the DNA fragment to be cloned into a plasmid replicative in *E. coli* and subsequently, into a plasmid replicative in *E. coli*/integrative in *Streptomyces.* This DNA region contains all the ORFs located between *tio3* and *tio28,* both of them inclusive and complete (Figure 1). The choice of this DNA region was due to the fact that the Tio3 and Tio28 proteins are the outermost proteins within the sequenced region which showed similarities with secondary metabolism proteins.

Due to its large size, said DNA region was obtained in steps, joining 3 independent DNA fragments which were obtained from 3 different cosmids (cosV33-D12, cosVl9-B4 and pCT2c):
- fragment A (20.2 kb): *Mse*I (position 1,393 of SEQ ID NO:1) - *Nsi*I (position 21,585 of SEQ ID NO:1);
- fragment B (19 kb): *Nsi*I (position 21,585 of SEQ ID NO:1) - *Eco*RI (position 40,636 of SEQ ID NO:1); and
- fragment C (13.7 kb): *Eco*RI (position 40,636 of SEQ ID NO:1) - *Acl*I (position 54,301 of SEQ ID NO:1).

To facilitate the subcloning, the complete DNA fragment was first subcloned into the plasmid replicative in *E. coli* pOJ260 (Example 14). The insert was rescued and subcloned into a vector replicative in *E. coli*/integrative of *Streptomyces* which contained the erythromycin resistance promoter (*ermEp*) (pARP) [Example 16] or without said promoter (pAR15AT) [Example 15]. This selected DNA region was cloned into said plasmids integrative of *Streptomyces* pAR15AT, in both directions (Example 17) and pARP (Example 18). Finally, said constructs were introduced in several streptomycetes by means of intergenus conjugation (Example 19).

### Example 14. Cloning of the selected DNA region into E. coli plasmid pOJ260

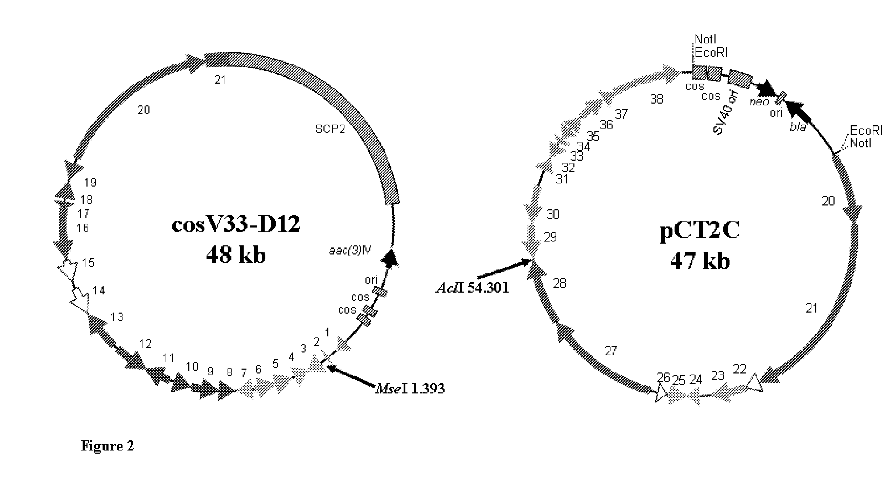

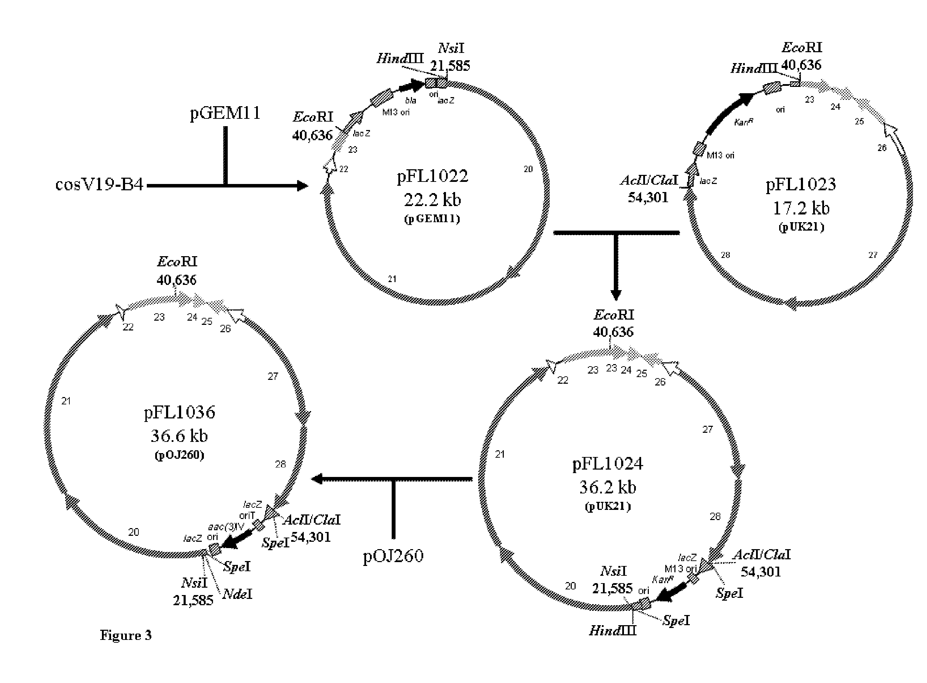

The DNA region located between the restriction sites *Eco*RI (position 40,636 of SEQ ID NO:1) and *Acl*I (position 54,301 of SEQ ID NO:1) was obtained from cosmid pCT2c (Figure 2) by means of usual procedures (Sambrook et al. 2001). This DNA fragment was cloned into the unique restriction sites *Eco*RI and *Cla*I of *E. coli* plasmid pUK21 (Vieira et al. 1991, Gene 100, 189-194), generating the construct pFL1023 (Figure 3).

The DNA region located between the restriction sites *NsiI* (position 21,585 of SEQ ID NO:1) and *Eco*RI (position 40,636 of SEQ ID NO:1) was obtained from cosmid cosV19-B4 by means of usual procedures (Sambrook et al. 2001). This DNA fragment was cloned into the unique restriction sites *Nsi*I and *Eco*RI of *E. coli* plasmid pGEM-11Zf (Promega), generating the construct pFL1022 (Figure 3).

These two DNA fragments were then joined. To that end, the DNA fragment located between the restriction sites *Nsi*I (position 21,585 of SEQ ID NO:1) and *Eco*RI (position 40,636 of SEQ ID NO:1) present in pFL1022 was rescued by digesting with the restriction enzymes *Hind*III (located in the multiple cloning site immediately before the *Nsi*I restriction site) and *Eco*RI. This fragment was then cloned into the unique restriction sites *Hind*III and *Eco*RI present in construct pFL1023, thus generating plasmid pFL1024 (Figure 3).

The entire region cloned into pFL1024 was rescued as a *Spe*I band (thanks to these two restriction sites present at both ends of the multiple cloning site of pUK21) and cloned into the unique *Spe*I site of plasmid pOJ260, thus generating plasmid pFL1036 (Figure 3).

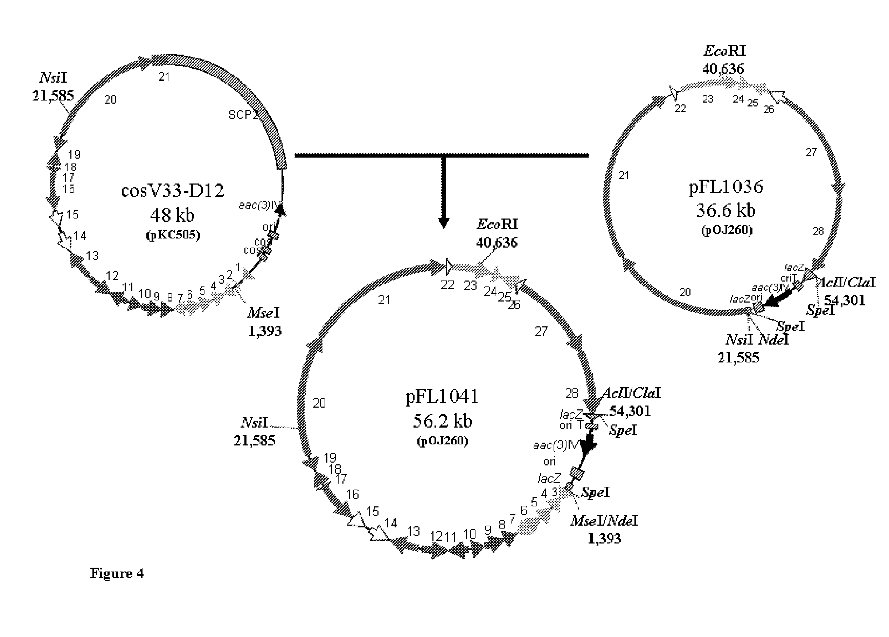

Finally, the fragment located between the cleavage sites *Mse*I (position 1,393 of SEQ ID NO:1) and *Nsi*I (position 21,585 of SEQ ID NO:1) was obtained from cosmid cosV33-D12 and it was cloned into the *Nde*I and *Nsi*I sites, respectively, of pFL1036, generating construct pFL1041 (Figure 4) containing in pOJ260 (Bierman et al. 1992, Gene 116, 43-49) the entire region comprised between the positions 1,393 (*Mse*I restriction site) and 54,301 (*Acl*I restriction site) of SEQ ID NO:1, i.e., from ORF *tio3* to *tio28*, both of them inclusive and complete. Furthermore, in pFL1041, this region is flanked by two *Spe*I restriction sites. pFL1041 is a plasmid replicative in *E. coli.*

### Example 15. Construction of the plasmid integrative of Streptomyces pAR15AT

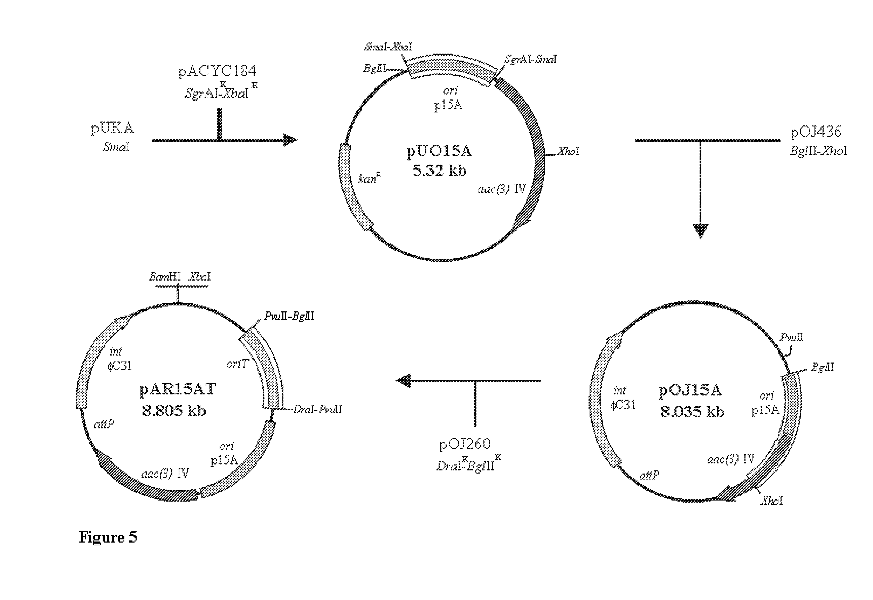

The replication origin of plasmid pACYC184 (Rose 1988, Nucleic Acids Res. 16, 355), ori p15A, was obtained as a *Sgr*AI-*Xba*I fragment and was treated with the Klenow fragment of the *E. coli* DNA polymerase. This replication origin was cloned into the *Sma*I site of plasmid pUKA, thus obtaining plasmid pUO15A (Figure 5). pUKA is a derivative of plasmid pUK21 (Vieira et al. 1991, Gene 100, 189-194) containing, cloned into its *Pst*I*-Acc*I restriction sites, the apramycin resistance gene obtained from cosmid pKC505 (Richardson at al. 1987, Gene 61, 231-241) as a *Pst*I*-Eco*RI band.

A DNA fragment containing ori p15A next to the apramycin resistance gene *aac(3)IV* was obtained by means of a *Bgl*II-*Xho*I digestion on pUO15A. This fragment was cloned into plasmid pOJ436 using the same restriction enzymes (Bierman et al. 1992, Gene 116, 43-49), giving rise to construct pOJ15A (Figure 5).

The *Dra*I-*Bgl*II fragment (treated with Klenow) from plasmid pOJ260 and containing the conjugation origin oriT was cloned into the *Pvu*II restriction site of pOJ15A. Plasmid pAR15AT is finally thus obtained (Figure 5).

### Example 16. Construction of the plasmid integrative of Streptomyces pARP

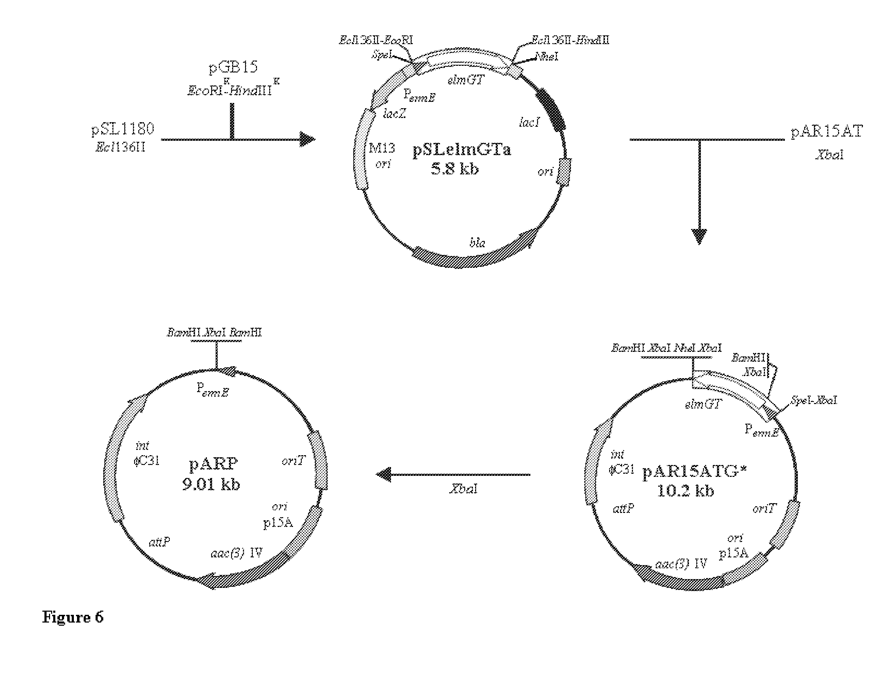

The *elmGT* glycosyltransferase gene from the elloramycin biosynthesis pathway, as a *Eco*RI-*Hind*III DNA fragment treated with Klenow obtained from plasmid pGB15 (Blanco et al. 2001, Chem. Biol. 8, 253-263), was cloned into the *Ecl*136II restriction site of pSL1180 (Amersham Pharmacia). Construct pSLelmGTa was thus obtained (Figure 6), in which the *elmGT* gene is under the control of the constitutive ermE erythromycin resistance gene promoter (P_{ermE}).

A *Spe*I-*Nhe*I fragment obtained from pSLelmGTa which contained *P_{ermE}-elmGT* was cloned into the *Xba*I site of plasmid pAR15AT described in Example 15, obtaining construct pAR15ATG* (Figure 6).

By means of *Xba*I digestion on plasmid pAR15ATG* and subsequent religation, the *elmGT* gene was eliminated, the P_{*erm*E} promoter being maintained, which gave rise to plasmid pARP (Figure 6).

### Example 17. Cloning of the selected DNA region into the plasmid integrative of Streptomyces pAR15AT, in both orientations

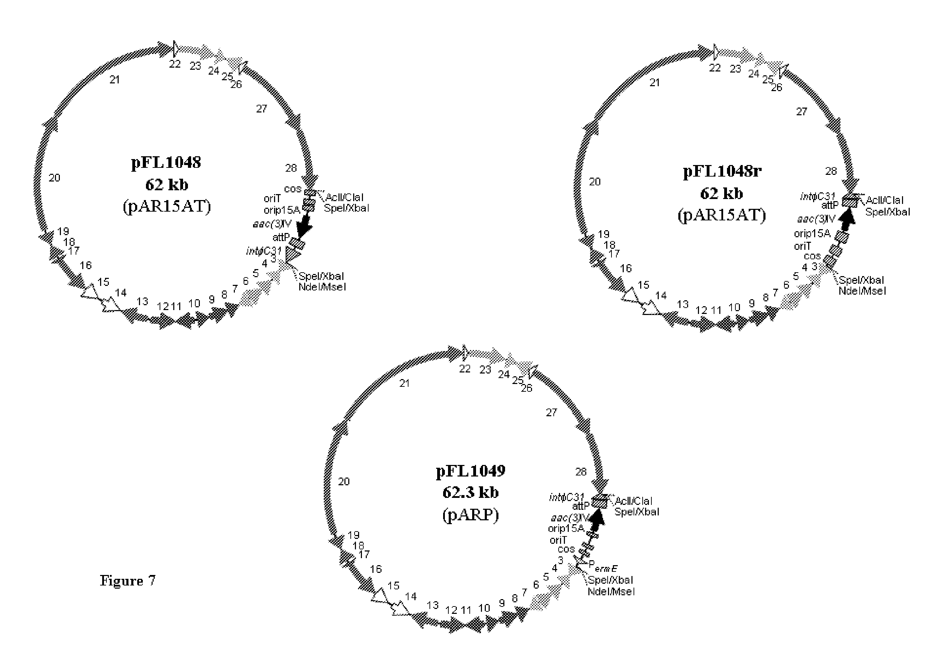

The *Spe*I DNA fragment from pFL1041 (Figure 4) containing the region comprised between positions 1,393 (*Mse*I restriction site) and 54,301 (*Acl*I restriction site) of SEQ ID NO: 1 was cloned, in both orientations, into the *Xba*I restriction site of plasmid pAR15AT (Figure 5). Two new plasmids, called pFL1048 and pFL1048r (Figure 7), with the apramycin resistance gene, replicative in *E. coli* and integrative in *Streptomyces* were thus generated by means of the system using the attP region of the φC31 phage.

### Example 18. Cloning of the selected DNA region into the plasmid integrative of Streptomyces pARP after the ErmE gene promoter

In a similar way, the *Spe*I DNA fragment from pFL1041 (Figure 4) containing the region comprised between positions 1,393 (*Mse*I restriction site) and 54,301 (*Acl*I restriction site) of SEQ ID NO:1 was cloned into the *Xba*I restriction site of plasmid pARP (Figure 6). pFL1049 (Figure 7) was thus generated, in which the ORF corresponding to *tio3* (SEQ ID NO:4) is under the control of the constitutive promoter P*_{ermE}* present in pARP. This plasmid has the apramycin resistance gene, it is replicative in *E. coli* and integrative in *Streptomyces* by means of the system using the attP region of the φC31 phage.

### Example 19. Heterologous expression of the thiocoraline biosynthesis pathway in different streptomycetes

Plasmid pFL1048 (Figure 7) was introduced by conjugation from the *E. coli* ET12567 (pUB307) strain (Kieser et al. 2000) in the *Streptomyces lividans* TK21 (Kieser et al. 2000) and *Streptomyces albus* J1074 species (Chater et al. 1980, J. Gene. Microbiol. 116, 323-334).

Plasmid pFL1049 (Figure 7) was introduced by conjugation from the *E. coli* ET12567 (pUB307) strain in the *Streptomyces coelicolor* M145 (Redenbach et al., 1996, Mol. Microbiol., 21, 77-96), *Streptomyces lividans* TK21, *Streptomyces albus* J1074 and *Streptomyces avermitilis* ATCC 31267 species.

Finally, plasmid pFL1048r (Figure 7) was introduced by conjugation from the *E. coli* ET12567 strain (pUB307) in the *Streptomyces lividans* TK21 species.

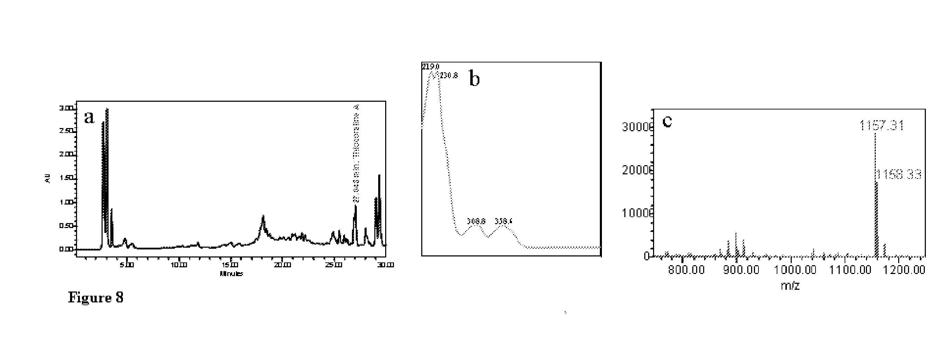

The results of the culture of the *Streptomyces albus* (pFL1049) clone in production medium R5A (Fernàndez et al. 1998, J. Bacteriol. 180, 4929-4937) are shown in Figure 8A. Figure 8B shows the absorption spectrum of the peak with a retention time of 27 minutes in this chromatogram, and its mass spectrum (Figure 8C), both of them being identical to those of purified thiocoraline.

## Claims

1. An isolated nucleic acid molecule comprising a nucleotide sequence encoding at least one biosynthetic thiocoraline production pathway protein, or a biologically active fragment thereof.

2. A nucleic acid molecule according to claim 1, comprising a nucleotide sequence encoding all the biosynthetic thiocoraline production pathway proteins, or biologically active fragments thereof.

3. A nucleic acid molecule according to claim 1 or 2, comprising the nucleotide sequence shown in SEQ ID NO: 1 or its complementary strand.

4. A nucleic acid molecule hybridizing with the nucleic acid molecule of claim 3 and encoding at least one biosynthetic thiocoraline production pathway protein, or a biologically active fragment thereof.

5. A nucleic acid molecule according to claim 1, comprising a nucleotide sequence encoding a biosynthetic thiocoraline production pathway protein, or a biologically active fragment thereof.

6. A nucleic acid molecule according to claim 5, selected from the group consisting of:
- the nucleic acid molecule comprising nucleotides 2-535 of SEQ ID NO: 1 *(orf1);*
- the nucleic acid molecule comprising nucleotides 993-1130c of SEQ ID NO: 1 *(orf2);*
- the nucleic acid molecule comprising nucleotides 1517-2131 of SEQ ID NO: 1 *(tio3);*
- the nucleic acid molecule comprising nucleotides 2154-2822c of SEQ ID NO: 1 *(tio4);*
- the nucleic acid molecule comprising nucleotides 2970-3791c of SEQ ID NO: 1 *(tio5);*
- the nucleic acid molecule comprising nucleotides 3794-4777c of SEQ ID NO: 1 *(tio6);*
- the nucleic acid molecule comprising nucleotides 4904-5611 of SEQ ID NO: 1 *(tio7);*
- the nucleic acid molecule comprising nucleotides 5701-6426c of SEQ ID NO: 1 *(tio8);*
- the nucleic acid molecule comprising nucleotides 6426-7688c of SEQ ID NO: 1 *(tio9);*
- the nucleic acid molecule comprising nucleotides 7733-8524c of SEQ ID NO: 1 *(tio10);*
- the nucleic acid molecule comprising nucleotides 8791-10002 of SEQ ID NO: 1 *(tio11);*
- the nucleic acid molecule comprising nucleotides 10002-11590c of SEQ ID NO: 1 *(tio12);*
- the nucleic acid molecule comprising nucleotides 11847-13634 of SEQ ID NO: 1 *(tio13);*
- the nucleic acid molecule comprising nucleotides 13734-15005c of SEQ ID NO: 1 *(tio14);*
- the nucleic acid molecule comprising nucleotides 15005-16354c of SEQ ID NO: 1 *(tio15);*
- the nucleic acid molecule comprising nucleotides 16441-18744c of SEQ ID NO: 1 *(tio16);*
- the nucleic acid molecule comprising nucleotides 18774-19055c of SEQ ID NO: 1 *(tio17);*
- the nucleic acid molecule comprising nucleotides 19260-20036 of SEQ ID NO: 1 *(tio18);*
- the nucleic acid molecule comprising nucleotides 20146-20880c of SEQ ID NO: 1 *(tio19);*
- the nucleic acid molecule comprising nucleotides 21188-28969 of SEQ ID NO: 1 *(tio20);*
- the nucleic acid molecule comprising nucleotides 28979-38398 of SEQ ID NO: 1 *(tio21);*
- the nucleic acid molecule comprising nucleotides 38449-38661 of SEQ ID NO: 1 *(tio22);*
- the nucleic acid molecule comprising nucleotides 38642-41263 of SEQ ID NO: 1 *(tio23);*
- the nucleic acid molecule comprising nucleotides 41835-42368 of SEQ ID NO: 1 *(tio24);*
- the nucleic acid molecule comprising nucleotides 42395-43255c of SEQ ID NO: 1 *(tio25);*
- the nucleic acid molecule comprising nucleotides 43340-43741c of SEQ ID NO: 1 *(tio26);*
- the nucleic acid molecule comprising nucleotides 44152-49563 of SEQ ID NO: 1 *(tio27);*
- the nucleic acid molecule comprising nucleotides 49635-53669 of SEQ ID NO: 1 *(tio28);*
- the nucleic acid molecule comprising nucleotides 53749-55305c of SEQ ID NO: 1 *(orf29);*
- the nucleic acid molecule comprising nucleotides 55384-57222c of SEQ ID NO: 1 *(orf30);*
- the nucleic acid molecule comprising nucleotides 57895-58467c of SEQ ID NO: 1 *(orf31);*
- the nucleic acid molecule comprising nucleotides 58535-59206c of SEQ ID NO: 1 *(orf32);*
- the nucleic acid molecule comprising nucleotides 59298-59564c of SEQ ID NO: 1 *(orf33);*
- the nucleic acid molecule comprising nucleotides 59611-60114c of SEQ ID NO: 1 *(orf34);*
- the nucleic acid molecule comprising nucleotides 60202-60888 of SEQ ID NO: 1 *(orf35);*
- the nucleic acid molecule comprising nucleotides 60960-62240 of SEQ ID NO: 1 *(orf36);*
- the nucleic acid molecule comprising nucleotides 62300-62833 of SEQ ID NO: 1 *(orf37);*
- the nucleic acid molecule comprising nucleotides 62925-64650 of SEQ ID NO: 1 *(orf38);* or
fragments thereof encoding biologically active fragments of biosynthetic thiocoraline production pathway proteins.

7. A nucleic acid molecule according to claim 1, comprising a nucleotide sequence encoding two or more biosynthetic thiocoraline production pathway proteins, or biologically active fragments thereof.

8. A nucleic acid molecule according to claim 7, comprising two or more genes selected from the genes identified as *orf1, orf2, tio3, tio4, tio5, tio6, tio7, tio8, tio9, tio10, tio11, tio12, tio13, tio14, tio15, tio16, tio17, tio18, tio19, tio20, tio21, tio22, tio23, tio24, tio25, tio26, tio27, tio28, orf29, orf30, orf31, orf32, orf33, orf34, orf35, orf36, orf37, orf38* and fragments thereof encoding biologically active fragments of biosynthetic thiocoraline production pathway proteins.

9. A nucleic acid molecule according to claim 1, comprising a nucleotide sequence encoding at least one biosynthetic thiocoraline production pathway protein, or a biologically active fragment thereof, or a mutant or variant thereof, wherein said protein is selected from the group consisting of the proteins identified as ORF1 (SEQ ID NO: 2), ORF2 (SEQ ID NO: 3), Tio3 (SEQ ID NO: 4), Tio4 (SEQ ID NO: 5), Tio5 (SEQ ID NO: 6), Tio6 (SEQ ID NO: 7), Tio7 (SEQ ID NO: 8), Tio8 (SEQ ID NO: 9), Tio9 (SEQ ID NO: 10), TiolO (SEQ ID NO: 11), Tio11 (SEQ ID NO: 12), Tio12 (SEQ ID NO: 13), Tio13 (SEQ ID NO: 14), Tio14 (SEQ ID NO: 15), Tio15 (SEQ ID NO: 16), Tio16 (SEQ ID NO: 17), Tio17 (SEQ ID NO: 18), Tio18 (SEQ ID NO: 19), Tio19 (SEQ ID NO: 20), Tio20 (SEQ ID NO: 21), Tio21 (SEQ ID NO: 22), Tio22 (SEQ ID NO: 23), Tio23 (SEQ ID NO: 24), Tio24 (SEQ ID NO: 25), Tio25 (SEQ ID NO: 26), Tio26 (SEQ ID NO: 27), Tio27 (SEQ ID NO: 28), Tio28 (SEQ ID NO: 29), ORF29 (SEQ ID NO: 30), ORF30 (SEQ ID NO: 31), ORF31 (SEQ ID NO: 32), ORF32 (SEQ ID NO: 33), ORF33 (SEQ ID NO: 34), ORF34 (SEQ ID NO: 35), ORF35 (SEQ ID NO: 36), ORF36 (SEQ ID NO: 37), ORF37 (SEQ ID NO: 38), ORF38 (SEQ ID NO: 39) and combinations thereof.

10. A nucleic acid molecule according to claim 1, comprising a nucleotide sequence comprising an *orfs* selected from the group consisting of *orf1, orf2, tio3, tio4, tio5, tio6, tio7, tio8, tio9, tio10, tio11, tio12, tio13, tio14, tio15, tio16, tio17, tio18, tio19, tio20, tio21, tio22, tio23, tio24, tio25, tio26, tio27, tio28, orf29, orf30, orf31, orf32, orf33, orf34, orf35, orf36, orf37, orf38* and combinations thereof, or of the corresponding regions, mutants or variants thereof.

11. A nucleic acid molecule according to claim 1, isolated from *Micromonospora sp.*

12. A composition comprising at least one nucleic acid molecule according to any of claims 1 to 11.

13. A probe comprising a nucleic acid molecule according to any of claims 1 to 11 or a fragment thereof.

14. A vector comprising a nucleic acid molecule according to any of claims 1 to 11 or a composition according to claim 12.

15. A host cell transformed or transfected with a vector of the invention.

16. A host cell according to claim 15, wherein said host cell is a microorganism, preferably a bacterium.

17. A host cell according to claim 16, wherein said bacterium is a Gram-positive bacterium, preferably an actinomycete or a streptomycete.

18. A protein encoded by the nucleic acid molecule of the invention.

19. A protein according to claim 18, selected from the group consisting of the proteins identified as ORF1 (SEQ ID NO: 2), ORF2 (SEQ ID NO: 3), Tio3 (SEQ ID NO: 4), Tio4 (SEQ ID NO: 5), Tio5 (SEQ ID NO: 6), Tio6 (SEQ ID NO: 7), Tio7 (SEQ ID NO: 8), Tio8 (SEQ ID NO: 9), Tio9 (SEQ ID NO: 10), Tio10 (SEQ ID NO: 11), Tio11 (SEQ ID NO: 12), Tio12 (SEQ ID NO: 13), Tio13 (SEQ ID NO: 14), Tio14 (SEQ ID NO: 15), Tio15 (SEQ ID NO: 16), Tio16 (SEQ ID NO: 17), Tio17 (SEQ ID NO: 18), Tio18 (SEQ ID NO: 19), Tio19 (SEQ ID NO: 20), Tio20 (SEQ ID NO: 21), Tio21 (SEQ ID NO: 22), Tio22 (SEQ ID NO: 23), Tio23 (SEQ ID NO: 24), Tio24 (SEQ ID NO: 25), Tio25 (SEQ ID NO: 26), Tio26 (SEQ ID NO: 27), Tio27 (SEQ ID NO: 28), Tio28 (SEQ ID NO: 29), ORF29 (SEQ ID NO: 30), ORF30 (SEQ ID NO: 31), ORF31 (SEQ ID NO: 32), ORF32 (SEQ ID NO: 33), ORF33 (SEQ ID NO: 34), ORF34 (SEQ ID NO: 35), ORF35 (SEQ ID NO: 36), ORF36 (SEQ ID NO: 37), ORF37 (SEQ ID NO: 38), ORF38 (SEQ ID NO: 39), and combinations thereof, or biologically active fragments thereof.

20. A process for producing a protein involved in the biosynthesis of thiocoraline according to any of claims 18 or 19, which comprises growing, under suitable conditions, a thiocoraline-producing organism, and, if desired, isolating one or more of said proteins involved in the biosynthesis of thiocoraline.

21. A method for producing thiocoraline which comprises growing, under suitable conditions for producing said compound, a thiocoraline-producing organism in which the number of copies of genes encoding proteins involved in the biosynthesis of thiocoraline has been increased, and, if desired, isolating thiocoraline.

22. A method for producing thiocoraline which comprises growing, under suitable conditions for producing said compound, a thiocoraline-producing organism in which the expression of the genes encoding the proteins responsible for the biosynthesis of thiocoraline has been modulated by means of manipulating or substituting one or more genes encoding proteins involved in the biosynthesis of thiocoraline or by means of manipulating the sequences responsible for regulating the expression of said genes, and, if desired, isolating thiocoraline.

23. A method according to any of claims 21 or 22, wherein said thiocoraline-producing organism is an actinomycete, preferably *Micromonospora sp.*

24. A method for producing thiocoraline which comprises growing, under suitable conditions for producing said compound, a host cell according to any of claims 15 to 17, and, if desired, isolating thiocoraline.

25. A method according to claim 24, wherein said host cell is an actinomycete or a streptomycete.

26. A process, based on the use of genes responsible for the biosynthesis of thiocoraline from *Micromonospora sp.* ML1, for the production of said compound in another actinomycete, comprising:
(1) obtaining mutants affected in specific genes of the thiocoraline biosynthesis pathway;
(2) isolating the *Micromonospora sp.* ML1 chromosome region containing the cluster of genes responsible for the biosynthesis of thiocoraline;
(3) obtaining and analyzing the nucleotide sequence of the cluster of genes responsible for the biosynthesis of thiocoraline; and
(4) heterologously producing thiocoraline in other actinomycetes.


LIST OF SEQUENCES

<110> Pharma Mar, S.A.

<120> GENES INVOLVED IN THE BIOSYNTHESIS OF THIOCORALINE AND
HETEROLOGOUS PRODUCTION OF SAME

<160> 39

<170> PatentIn version 3.1

<210> 1
<211> 64,650
<212> DNA
<213> Micromonospora sp., ML1 strain

<220>
<221> CDS
<222> (2)..(535)
<223> Open Reading Phase of orf1

<220>
<221> CDS
<222> (993)..(1130c)
<223> Open Reading Phase of orf2

<220>
<221> CDS
<222> (1517)..(2131)
<223> Open Reading Phase of tio3

<220>
<221> CDS
<222> (2154)..(2822)
<223> Open Reading Phase of tio4

<220>
<221> CDS
<222> (2970)..(3791)
<223> Open Reading Phase of tio5

<220>
<221> CDS
<222> (3794)..(4777c)
<223> Open Reading Phase of tio6

<220>
<221> CDS
<222> (4904)..(5611)
<223> Open Reading Phase of tio7

<220>
<221> CDS

<222>  (5701)..(6426c)
<223>  Open Reading Phase of tio8

<220>
<221>  CDS
<222>  (6426)..(7688c)
<223>  Open Reading Phase of tio9

<220>
<221>  CDS
<222>  (7733)..(8524c)
<223>  Open Reading Phase of tio10

<220>
<221>  CDS
<222>  (8791)..(10002)
<223>  Open Reading Phase of tio11

<220>
<221>  CDS
<222>  (10002)..(11590c)
<223>  Open Reading Phase of tio12

<220>
<221>  CDS
<222>  (11847)..(13634)
<223>  Open Reading Phase of tio13

<220>
<221>  CDS
<222>  (13734)..(15005c)
<223>  Open Reading Phase of tio14

<220>
<221>  CDS
<222>  (15005)..(16354c)
<223>  Open Reading Phase of tio15

<220>
<221>  CDS
<222>  (16441)..(18744c)
<223>  Open Reading Phase of tio16

<220>
<221>  CDS
<222>  (18774)..(19055c)
<223>  Open Reading Phase of tio17

<220>
<221>  CDS
<222>  (19260)..(20036)
<223>  Open Reading Phase of tio18

<220>
<221>  CDS
<222>  (20146)..(20880c)
<223>  Open Reading Phase of tio19

<220>
<221>  CDS
<222>  (21188)..(28969)
<223>  Open Reading Phase of tio20

<220>
<221>  CDS
<222>  (28979)..(38398)
<223>  Open Reading Phase of tio21

<220>
<221>  CDS
<222>  (38449)..(38661)
<223>  Open Reading Phase of tio22

<220>
<221>  CDS
<222>  (38642)..(41263)
<223>  Open Reading Phase of tio23

<220>
<221>  CDS
<222>  (41835)..(42368)
<223>  Open Reading Phase of tio24

<220>
<221>  CDS
<222>  (42395)..(43255c)
<223>  Open Reading Phase of tio25

<220>
<221>  CDS
<222>  (43340)..(43741c)
<223>  Open Reading Phase of tio26

<220>
<221>  CDS
<222>  (44152)..(49563)
<223>  Open Reading Phase of tio27

<220>
<221>  CDS
<222>  (49635)..(53669)
<223>  Open Reading Phase of tio28

<220>
<221>  CDS
<222>  (53749)..(55305c)
<223>  Open Reading Phase of orf29

<220>
<221>  CDS
<222>  (55384)..(57222c)
<223>  Open Reading Phase of orf30

<220>
<221>  CDS
<222>  (57895)..(58467c)
<223>  Open Reading Phase of orf31

<220>
<221>  CDS
<222>  (58535)..(59206c)
<223>  Open Reading Phase of orf32

<220>
<221>  CDS
<222>  (59298)..(59564c)
<223>  Open Reading Phase of orf33

<220>
<221>  CDS
<222>  (59611)..(60114c)
<223>  Open Reading Phase of orf34

<220>
<221>  CDS
<222>  (60202)..(60888)
<223>  Open Reading Phase of orf35

<220>
<221>  CDS
<222>  (60960)..(62240)
<223>  Open Reading Phase of orf36

<220>
<221>  CDS
<222>  (62300)..(62833)
<223>  Open Reading Phase of orf37

<220>
<221>  CDS
<222>  (62925)..(64650)
<223>  Open Reading Phase of orf38

<400>  1
   gatccccagc cgcgaatggc agccacacag ctgcggcccc ggggccaaag gaccacgcga      60
   gtacctgtgg gcctggatca ccaccgccac cagcgcaggt gagcaccggt ggctgctgat     120
   gcgccgcaac cgcagcaccg gtgagctggc cttctacctg tgctggtcac cccgcccggc     180
   gccgctgcac accctcgtga ccgtcgctgg ttcccgctgg agcatcgagg agctgttcca     240
   gtccggcaaa ggccaggtcg gcctggacca ctaccaggtc cgcggctgga ccggctggca 300
   ccgccacgtc accctggcca tgctcgccct ggccgtcctg accatcctcg ccgctcggca 360
   gcccgacccc gacccggaga tcatcgcgct gaccgtcgcc gaaatccgcg ggctcctcaa 420
   cgccttcgtc ctcgccgtga cgctcccacc cgcgcacacc ctgcactggt cgacctggcg 480
   acgaacatcc caagcccgcg cccgacgagc ccactaccag cgcagacagg cgaagtgacg 540
   ttggagtact agccattgag ccgataattc agcgcacgac ttgccggaat cggtagggat 600
   ggtgtggcga ccatggccgc gatcgatgcc tgggcgcgtc ggcccggtcg acgccgagtc 660
   gggcgacgcg ctggcgggtg aaggtggccg cccgtcgaac tttacgactt gaataccagt 720
   atcggtgaag aggccgaaga ccgatatctg ccattacgta ccgatcatgt tgcaggaatg 780
   cccggaacct ttcggatgtc aggctgccag ttggcaagcg ggtgggtgcc ctctcccgca 840
   gggcttcgcg tagtcgcctg aacgtcttcc gacctcgcgt tcgtggtgtc gcggacctga 900
   ttggagggtg atttagcacg ggtgtggtcc atcggtgatc tgcagttgct gagacaacat 960
   gagacggaga aggaccacac cctcgcatgt catcgtcgct gatcgacgta acccgtcagc 1020
   atgccccgcc ccgcccgact caccgactgc tggccgcgag gcgcttggag gccctggcgc 1080
   gggttcccga tccgcgtgac ccgtgccaac cgccgcccac acgacctcat cgccaccgtg 1140
   aacagcagct accccactat gcaatgaccc tgctctgtcc cgtcgacggg acagagcgcc 1200
   cgccgccgga ggaagaaacc tgttgccgtc gcgggatgct gattatattc agcctcatga 1260
   ccctgctcat gaagcggcgg gcttcggtcg cgatgcgttt gttccgccga tatctcacta 1320
   aacgccggga ctggacagtg tgaacatcat tgcacttcta gctggttgac aacgtatcta 1380
   cggtcgccat ggttaattgt ggttgacggc gcgtgtcgcg actgcggagc gccatgtcgt 1440
   cgaatctcgg ccagccgtcg ccgtgacatt tgtcaaccag ctccgatgaa ttctgtgcac 1500
   tctgggggag ttgctgatgt cggtttcggg aagccgccgg agcgaaggct cctaccgtcg 1560
   ttcgggtggt tgtgcccagg gccgctcgcc ccggccagcc agggaagacg tcaagttcgt 1620
   ccgttggccg gccgagcggc acctacgcga cctgtgccgt caggagcgga tcccgtgcct 1680
   cctggtggtc gagggcggag cagagccgcc ggtctgcagt gatccgcgtg aggactgggt 1740
   tcgtacgccg atctcgccat ccgaccttga cgctcgggtg accgctctcc ggcaacgttc 1800
   tcacgaccgg cgtacgcctg ttctcgaccc ggccgggacg ttgtcgttca ggactgcgtc 1860
   cgtcgtcatg tccagtacgc acctcgaact catggatctg ttcgtcgaac gcttcggcca 1920
   ggtggtctac cgctgggagt tgatgaccat cctcacgaag cgcgactcga catcaacgcg 1980
   aaacgccctc gatctgcaca tcatgagaat gaggaagcgg atcgcgccgc tcgatctgga 2040
   tatagttact gcctggggcc gtggctacgc gctggaagaa aagtcctcat gggccgcggt 2100
   ggccagcaag cccgcggctg tcgatcattg ctgacatcgc cggccgatgg ctactcagtt 2160
   tcgtcgtggg tagtcttgtc gagggtctct gctgcggccc ggtcgagggt ggagacgatc 2220
   cactgatcga gttcgacgct tgattggtgc gcggcctggc gccatcgggt aaggcgtcgg 2280
   tcgggtatgg gaatgcgggg catcagtgcg gtcttgatgg tttctgttgt gcggtcggag 2340
   cgggtcgctt tgatggccgc gcggagttgt ttgaccgacc acatctgggt ttcggcttcg 2400
   tcgagccaac ggtcctgatc gtcgggagac attgcggcta cttcggcgtg gtgttggaaa 2460
   ctgaggccgc ttcgacggcg ggccagtggg aagcggccgg cgacccaggc gtagttgcgt 2520
   agggtctggt agtccagtcc tacagcctgg acgacgtgcc gatagcggtc ggcgtattgt 2580
   tccttgccgt acaccagcca gtcgcccagg caccaggacg aggagtcgac gataccggag 2640
   agttggcgcc cggttcgctc ccagtgttcg aagctcattc gttcgggcaa ccgtagaccg 2700
   accctggtca gcatcacctg ggccggctct gctgggcgtt gcttcggaac gccagccgtt 2760
   gctgcaggga ccacccggag cgctgctacg gcgttgcgtg acgatcggct acccgcgctc 2820
   atcccactca acctccccga cagtcggata actcgctatc tctgctctct ttttccgtct 2880
   cgcgccgtgc atgtgtgatc ggcgcgcgcg ggatgccacc cgtaaggccg cccttccgac 2940
   aaaggcggcc ttgcgggtgt ttccggttac ctcgaccgca ggctgtactt acgggtcgcc 3000
   agcggagcga agatggtgat cagcaggacc gaccaggcaa ccgtcagcag cacatccagc 3060
   cccggcggat tgccgttgaa caggtcgcgc agcgcgttga ccgtcgcggt catgggactg 3120
   gcatcggcgt acacccgcag ccaggtcggc atcgactcgg taggtacgaa cgccgacgag 3180
   atcagactga acgggaagat ccagatcagg ccggccgact gcgccgcctc cgggttgggc 3240
   accgagaggc cgatcatggc gccgacccag gtgagcgcgt agccgaacag caacagcagc 3300
   gcgagtccgc cgagggcaga caggaccccg ccctggaagc ggaagccgat cagcagaccc 3360
   atgagcacca cgaccagcat cgacacgacg ttgcgaagga tctccgacag cgtacgcccg 3420
   acgaggaccg cggaccggct catcggcatc gagcggaacc ggtccatcag gccgcggtcc 3480
   atgtcggtgg agatgccgat cgtggtgccg gcgaccgaac cgaacaacac catctgcacc 3540
   aggatgccgg gcagcaggaa ctgggggtag ttcccgccct cgacattgat cgccccgccg 3600
   aagacgtagg cgaacagcag gatgaacatg acgggctgaa ccagcgagaa catcaccagt 3660
   tcggggctgc gcgtcaggtg ccgcagattg cgcagcgcca tctgccaggc gtcggacagc 3720
   caccaggaca ggccggtctg ctgctgtgcg gcggcggcat cgagccgcgg tttcagggac 3780
   gtggagacca tcaccgctta ccacccttct tcttgctgtt cacgtcttcg gacgcccggg 3840
   ccgtggcggt gtgcccggtg agggcgagga acacgtcatc gagcgtgggc cggcggacgg 3900
   ccacgtcggc gattgcgatg ccggaatcgc gcagcggatt gagtagctcg ttgagcgtca 3960
   taccctcggg gaggggcgcg tgcacctgca tgccctccgg atcgccgacc ggctcggtcc 4020
   ccaacctctc gcgcaggatc tgggtggtct tctcgaccgc ggcggcgtcg atgagagtca 4080
   cgtcagccca cgcctggccg acctccgcct tgagttcgtc cggcgcgccg ctggcgatca 4140
   ccttgccgct gtcgatcacg gacagcgagt cggcgaggtg gtcggcctcc tcgaggtact 4200
   gggtcgtgag cagaaccgtg gtgccctcct cgaccaactc gttgacgatg cgccacacgt 4260
   ccatgcgact gcgcggatcg agggcggcgg taggttcgtc gaggaagatg agctggggcc 4320
   ggctcatcag ggtggcggcg atgtccagcc ggcggcgcat accgcccgag tacgtcttcg 4380
   cctgccggcc ggccgcctcg gtcaggtcga agcgctccag cagttcggtc gcccgcacct 4440
   tgatgtccgg cgtggacatc cggaacagct tgccgaccat gtgcaggttc tcccgcccgg 4500
   tcagcatgtc gtcgacggtg gtgtactgac cggtcaggcc gatgatggag cgcacctgta 4560
   gcgcgttgcg ccgcacgtcg aagccgaata tctcggcctt cccctcatcc ggcctgagaa 4620
   gggtggtcag aacccgaacg gtggtggtct tgcccgcacc gttaggtccg agcagaccgt 4680
   gcaccttacc ctccggcacg gtgagttcga ggtgatcgag agcggtcacc gagccgaatc 4740
   tcttgcacag tccagctact tcaatcgctg ctggcatctc caggattcct ctcaaagggg 4800
   ccaacgaagg tcggcgtgct gatcctggaa tctacccctg aacttccctt agaagctttg 4860
   accgcagaat gagcggcagc gagtcgctat agtctcaaac gtgatgtatt tccgtccggc 4920
   tgatctcgcc catgagcacg gcctttcccc gcaatcggta cgcaactacg agcgtgatgg 4980
   tctcatcccg cccgctcggc gtacggagag cggctaccgg cggtacaccg agaagcacgc 5040
   cgcagcgcta cgggcgtacc gcgccctcat cccggcgcac gggtacgccg aaagcggcgc 5100
   gatcatgcgg gacatcactg ccggacggtt gaacgaggca ttgacaacca tcgaccgcag 5160
   tcacgccgaa ctgctgcgag accgcggcac cctggacgct gtcgccgagg tcctgactca 5220
   cctgacggga cgctcgaccg gcacctggcg tgccccgaag agcattcagc cctacagcat 5280
   cggggagttg gcgcaccggc tcggcgtcaa tgtggcgacc atccggaaat gggagacgtc 5340
   gggcgtgctc gcgcccgtcc gccggccgga gaccgggcac cggatgtacg acgccagcga 5400
   cgtccgcgac gcggaactgg cgcacttcct gcggcgcggt cgctatccgc tcgagctgat 5460
   agccaccgtg gtgcagcagg tccgggcggc cggagacacc caggcgttgg ccgcggccct 5520
   cgcggactgg cggcagcgcg tcaccacgcg cggcctggcc atgctgaacg ccgccgcaca 5580
   actcgccagc tacctggcga tggaggagaa ctgatccggg gcccgacgcc cccgtgaccc 5640
   gctgcgccgg gactgttccg ggcgggtggg gccgtaccga ctccacccac cccgatctca 5700
   ctgcgtgtgg gcagtcggct gctcccacac caacgcgtcg tggacctgcg cccgaagctg 5760
   ccgcagctgg gcgaagggca gggtgatccg gcgggccaga tagtcgacgc cggtgctgcc 5820
   gccggtgccg ttgcgattgc ccaccatctt cgagaccagc gccaggtggg tcatcttcca 5880
   ccgccagtag ccgttggcca cgtcgagcag gctgtcggcg atgcggttga gcacgccggc 5940
   gccggggccc cgtgcgcaga cctcggcgac gctgatgccg ttgtgctcgg tcagccgctc 6000
   gaaggcccgg tagaggcggc cctcctgacg ctcgccgccg agcagccgcg cgagcaggcg 6060
   gaactgcgcc gactgcgcac cgctcgcccc gtcgaggaac gggcggaatt cggcgaagtg 6120
   acgcagcggc aaccgttcca gcgcgaccac ctgctcgtgc agtacccgga gcagctcagc 6180
   ggtgcgctgg agaaattcca gcgccagctc ctcgtcctcg ggtccgtagg gcggcgtcat 6240
   cgcgtccgcc gcggcgtcga ggtcgtacgt cacctgcttg agccacagct cgcaggcttg 6300
   atgggtgacg atgaagaagt gctcagccag taccacggac cggctcgcgg tgtccggggc 6360
   gcgggtctcc tgcagggaca gcagcgtgtc gacctgcagg tagtccgcgt aggtcagggt 6420
   cgtcatcggg cggtcccggg gcgttctcgt gcgaactcca gcagctcgcg tacgccgctg 6480
   cgcagcaccg acggcgctac gcacatggcg atccggatga ggtgacgcga ccagggcgcc 6540
   ggttcgacgg tgatgacccg gccccgggcg tccaggcggg gcatcagcgc gaacccggtg 6600
   ccgggaacca ccgcgacgtt ccgggtctcc agcagcccgg tggcgaactc ttcggcggtc 6660
   aggccggtcg gcgcgacgtc gagcactgcg taccagccac cggcgggcag ttcgggcagc 6720
   aggccggcgt cgcgtacggc gggcagcacc gcatcgcggt tgctgagcac catggcacgg 6780
   ttctcgcgta ccacgtgtcc cagccgcagc gcctccagcc ccgcgtattg caccggggtc 6840
   gacatcgcga tgaggttggc ctcctgcacg atccggaagg cgtgcgcggt ccggtcgttg 6900
   gcggtgctga cgtaccccaa tcggtagccg gtcatcgcca ggctcttcga gaaggagtag 6960
   gagctgtgca cgatccgctc ggagaccggc acatcccact ccagtgacgc ggtcgagacg 7020
   tgttcgtcgt cgaagacgaa gtgctcgtac gcctcgtcgc tgatgacctg ccagccccgg 7080
   gcgcgcgcca tgtcgatcag cgtgctcagg gtcgccgtgt cgaggacggc cccggtcggg 7140
   ttggcgggtg agttgatcaa cagcactctg gtgttcgcgg tcgaggccgc cagcaccgac 7200
   tcggggtccg tccggtagtc gcgtcccagc gggtagaaca ccggacggag accggcgagc 7260
   agcacctgct gcaggtggat gggccagtgc agctccggca gcaggatctc cgcgtcgggt 7320
   tcggccacgg cctgcatcag cgcggccagg ccctgacagg agccgggggt gacgaagagc 7380
   cgggacacat cggtgtccac gccgttctgg tcagcgagct tctccaccag acgctcgcgg 7440
   agctccagca gcccctcgac cgccgtgtag tcggtgcgcc ctgcggtcac cgcctccgcc 7500
   agcgcctgcg cgacctcggg tggcggggtg aagtagggct cgccgacatg cagcttggtc 7560
   accggaacac cgctgatgcg ctcgcgctcg ccggcatcgc ggaagacgcg atgcagctcc 7620
   gacgacggaa tcgtcgacgc gaccgaggga ccgtcgagca ggttgggata ggggcttggc 7680
   aggggcatct aatctccgtt tcgatcgtga cggcggtacg gggagggcat caggaggtgc 7740
   gaccgccgtc gacgggaatc agcgctccgg tgatccagcc ggaaaactcc gggtcgagca 7800
   ggccgcacac ccacccggcg atctcggcgg gctggccgaa ccggcccaaa ggggtcgccg 7860
   cgagcagcga cgaccgcagc aacgcggact tcacggagtc caggcccatg tcgtcccaca 7920
   tcggtgtgtc cacgggtccg ggtaggacgg cgttgaccct gacgtccggg gccagttccc 7980
   gggccagcga tcgggtcaga ctgttcagcg cggccttgct ggcgccgtag aaggaccgcc 8040
   ccggcatggc cagcacggca ccgacggacg acacgttcac caccgcgccc cgatgctcac 8100
   gcaggtgggg tagggccgcg cggatcaggg cggccggtgc gcggacgttg gtggcgagca 8160
   gccggtcgaa gtcctcgtcc gacgccgtgt cgagcgtcgc gaagcgggcc agcccggcgt 8220
   tgttgaccag accgtgcacc gtaccgaagc gggtggtcgc ggcggcgacg acgtgttccg 8280
   ccgcgcccgg ctcggtgatg tcggcggcga cggtcgcgac ctgcccgcca ccgtctccgg 8340
   tcagctcccg caacgccgcc tcgcgacgcc cgaccgcgac cacgttcgcg tcgcgggcca 8400
   gcagtcgttc gccgatcgcc cgtcccagtc ccgtgccggc cccggtgacg atcaccgtac 8460
   gaccggcgag gccgtcgccc cgctcagcca cggcgggcca tgcctcggct tcggttgggg 8520
   acaccgacgg cgtggctggc tgggacggcc ggccgccgag gtgtgcacgg ccgatatccg 8580
   cgtccggtaa cgcgatgagc ggttgtgacc atggcctccg cctcgaccag actggtgttc 8640
   gagctccaat ctcccgcctg ggaggcggat cggcatccat tccgacagaa ctgagccaga 8700
   tgcgcccgcg gccctaccga ggcttgtgac gatgcgggca gttacgactt cgccgccgga 8760
   tctccggatc ccgtacggaa ggttctgtcg atgagttcgc ccacggccag caccagcgcg 8820
   ctcgacctca ccgacccgac gacgttcgtg cgccacgaca cgcacgtgtt ctgggccgag 8880
   gtgcgtgacc acaatcccgt ctactggtac cagggccgcg aggaccgccc cggcttctgg 8940
   gtggtatcgc gatatgtcga cgtggtcgcg tcctacaccg acgctgcgag gttgagctcg 9000
   gcgcgcggca ccgtgctgga cgtgctgctc cggggcgagg actccgccgg cgggcggatg 9060
   ctcgcggtca ccgatcggcc gcgccaccgt gaactgcgca acgtcatgct ccgcgcgttc 9120
   tcccccgggg tgctcggccg ggtcgtcgag caggtccatc gacgcgccga cgagctgatc 9180
   cggcgggtca ccggtaccgg cgccttcgac ttcgccaccg aggtggccga gagcattccg 9240
   atgggcacga tctgcgacct gctgtcgatc ccaccggcgg accggccgga cctgttgcgg 9300
   tggaacaaga acgccctctc ctccgacgag gccgacgcca acctgtatgc cgcgctggag 9360
   gcccgcaacc agatcctgct ctatttcatg gatctggccg agcagcggcg ggccagtccg 9420
   ggcgacgacg tgatcagcat gatcgccact gccaccgtcg gcggcgagcc gctctccatc 9480
   gacgacgtcg cgctcaactg ctacagcttg atcctcggcg gtgacgagtc ctcacggatg 9540
   tcggcgatct gcgcggtcaa ggcgttcgcg gacttccccg accagtggcg ggccgtacgc 9600
   gacggtgacg ttgcgatcga caccgcggtc gaggaggtgt tgcggtggtc cacgccggcc 9660
   atgcacttcg cccgtaccgc caccacggac ttcgagctgc ggggccagca ggtgcgggcc 9720
   ggagacatcg tcaccctgtg gaacctctcg gccaacttcg acgagcggga gttcgaccgg 9780
   ccctaccggt tcgaggtggg gcgtaccccg aacaagcacc tgtcgttcgg ccatggcccg 9840
   catttctgcc tcggcgccta ccttggccgc gccgagctgc aggcgttgct caccgcgctg 9900
   gtcggaaccg tttcgcgcat cgagtccgcc gggtcgcccc ggcgcgtcta ctcgaacttc 9960
   ctcaacggcc acagctcgct gcccgtcgcc ttcacgggtc gctgacccgg ccaggccctc 10020
   accccgatgt gcgcagggtg accttgtcga ccttgccgac ggcggtgcgg gggaactcct 10080
   cgatcggcac cacccgatcc ggaatcttgt aggtggccag accgcgctcg cggaggaagg 10140
   acttgacagc cgccggactg atcggctcgt cccggaggat gacgaaggcg acggtcttct 10200
   cgcccagcac cgggtcggcc acgccgacca ccgccgcgtc gcggatcgcg gcgtgggcga 10260
   gaaggtgctc ctcgatctcc tcggcgggta ccttctcccc gccgcggttg atgatgtcct 10320
   tgatccgccc gaccaccacg atgctgccgt ccggccgccg gcggaccatg tcgccggtgc 10380
   ggaagtagcc gtcgggggtg aacgagcgcc ggttctgttc ctcggcccgg tagtagccgc 10440
   ggatcgtgta gggaccccgc accagcagct cgccctcggc gccatcgggt acgggtcgac 10500
   cgtcggtgtc gacaacgagg atctcgtcgt gcgcggccag cggacgtccc tcggtggcga 10560
   gaacgacctc ctccggatcg tccaggcgcg tgtaggtcag cagcccttcc ccgacgccga 10620
   accactgtgt cagcacggca ccgaggctgg tccggacctc ggcggcgcgg gcggggtcga 10680
   acttggagct gcccacctgg agcatgaggt tggggaaccg gacgtccgac ttgcgggcgg 10740
   cgtcgagcca cagccgcacc acgggcggca cgagcgtcgt gagcgtcacc cgctcccgct 10800
   ccaacagctc gaacacctcg tccggccgga cgctggaggt gagcactgcg gtgccgccga 10860
   ccagcagtgc gccgaggacg ccgggacagc cgagcgcgga attgtggccg atcgggttga 10920
   ccgccaggta cacgtcctcc gcgccgaacc gcagcgcatc agccgtggcg cgcatgttgt 10980
   aggcgtagtc gtcgtgcgtg cgtgggatga gcttgggcag gccggtcgtg ccgccggaga 11040
   gcaggaacag cgcgggctcg ctggggtcga cggcaggcag gtcacgcccc cgccggtaga 11100
   ggtccgccag cgcggtgaac tcctcggcgt caccggcgac cacgacatgc ttcagtgcgg 11160
   gcacctcggc ccgtacctcg cgggccagcg cgcggtggtc gaagcccttg acctggtcga 11220
   cgacgaccag ggcgaccgcc tgcgaatgtg cgcacaggtg ggtcagctcg accttacggt 11280
   gtcccggcag ggccatgacg gggatggcgc cgatccgcag cagcgcgtag accaccacca 11340
   cgaactcggg cacgttgggc agctggacga cgactcggtc caacggttcg atgcccacgt 11400
   ccaacagccc gcccgcgacg tctgcggatg ccctcgacag ctcggcgtag gtcatccgcc 11460
   gctcgccgca gacgagtgcg gtcttgtcgg cgtgccgggc acatgcctcg gtcagcaggt 11520
   ctcccagcgg ccgtccctgc cagtacccct cgcggcggta gagcgcggca agttcctcgg 11580
   gccagggcac acaaccatcg agcatcgctt ccacccaaca gagtcgaggt agttgaacag 11640
   acacggcgtc tcgacgccat tgccacagcg tgacccaagc cgtgcggccc ggacggcacg 11700
   gcgacaggga cgcgaaagga gcgtccgtgg cggcacttgc cctgtcgcaa tcctttcgtg 11760
   gccgggcacc ggcgcgcgag cgagcagcta ggttcgggcc ccgtgcagcg ccctcgtacc 11820
   cagtaccgag aggacggagt gagcaggtgc ccaccgttgc ggaattgttt gagtccgttg 11880
   ttgcgcgctc gggcgaccac gtggcggtga tcttcggcga ccgcctcgtc agctatgccg 11940
   aactcgacgc ccgcgcaaac cggttggcgc gggtgctggt cgagcgcggc gtcggccccg 12000
   acaccaccgt ggcggtggcc atgccgaagt gcgacgagct gatggtggtg ctgctggcgg 12060
   tgctcaaggc cggcggcgcc tacctgccgc tggacccgca gtatcccgcc aagcgactgg 12120
   cgtacatggt gcaggacgcc cggcccacgc tgctggtccg gatggcgcag gtgtcgctgg 12180
   aactcggggt gccggtcgct gaacttgtcg tggacgaccc cgacacggcg cgggagaccg 12240
   agtcgcggcc agatcaccag ctgaccgacg ccagccgcaa cgctccgctg cgcccggaca 12300
   acctgatgta cgtcatctac acctcgggct cgacgggtac gcccaagggc gtcgccgtga 12360
   cccacaccgg cgtcgccgac atcgtggccg cccagaccgc ttccatcgcg ccccggccgg 12420
   gcgaccgcgt gctccagtgg gcgtcggtca gcttcgacgc ggcgttctgg gactggtcgg 12480
   cggcgttgct gtccggtgcc acgctgatca tggccccggc cgacgaactg ctcccgggcc 12540
   aaccgctgcg cgacaccctg cgccggtacg cggtcaccca cgccgtgctg cccccggtcg 12600
   cgctgagcgt caccgagccc gacgatgtgc tcgtcggcgg cacgctgatg tcgaccggcg 12660
   atgcgtgcac ccgcgcgctg gtggccaagt gggcgccggg ccggcggatg tacaacggct 12720
   acgggccgac cgagaccacg gtcgggtcga ccatcgccgg gcccatcgcc atctccgacg 12780
   agatcaccat cggtacgccg tggtcgggca acagggtgta cgtcctcgac gagcgcctgc 12840
   gcccggttcc gaacggccgc gacggcgagc tgtacctggc cggcaacggc ctagcccgcg 12900
   gctacctgaa ccggccgggc ctgacggcga gccggttcgt gctcgacccc ttcggcccac 12960
   ccggcggccg gatgtaccgc tccggcgacc ggggccaccg ccgcgcggac gggcagttgg 13020
   tcttcgccag ccgcggcgac gaccaggtga agatccgtgg cttccgcgtc gagctcggcg 13080
   aggtcgaggc gcggctggcg gcccaccctg cggtggatgt cgccgccgtg gtcgtggtcg 13140
   gcgacctcgc gggcgcccgc ctggccgcct tcgtcagcac cgtggccggc accggggttt 13200
   cggctgccga actgagcgca cactcggcgg agaccctgcc ggaacacatg gtgccgtcat 13260
   cggtgcaggt gttacagcgg ctcccggtca ccgccaacgg caagatcgac cgggcggcgt 13320
   tgcgggacct cgccgagtcg gcggcgcacc ccgacgcagc agcctcggcc ggtcctcccg 13380
   gcgaggatcc gacctcggtg gagagccggc tatgtctgct ggtcagggag cttctggcgg 13440
   tgccggacgt caggccgggc gacaacttct tcaaactggg cggccactcg gtgctggcga 13500
   cccggctcgc cggacggctc cgcgacgagt ggggaatcac cgtgccgatc cgggcggtgt 13560
   tcgaggcggc caccctgtcc gagctcgccc tggtcgtgca ggcgtcgaag gcatccatcc 13620
   ccaacgggcg cccctgacgc cgacgaggaa ccgaaccagc gttccggcca cacaggaaag 13680
   accccctcgg gcgatgccgg accggcaccg cccgaagggg gatccatcgg ctacctgggg 13740
   ctacagtgcc tggggcagtc gccacgccgg ggcgggcccc accaaccggc ggtgttgtgg 13800
   ttgtttccgt tgcgcgtcgc gaccgcgaag ccggcggacc ggcccgaggc gttgaccgcg 13860
   atgacgtacg agcccaggtc gttgtcgccg aggcgggtcg tgcgtccccg gcgcggctcc 13920
   cacatgaccg cgctgaacgg gtacgccgac gagccggtga agttgcggat gccgacgatc 13980
   tggccggcgt cgttgacccg gtacgccacc cgaccgggaa gctccaccgg ctgggcgttc 14040
   gcgctgggcc agtagatggc cgtggtggac tggccggttg cgttgatccg ccaaccgacg 14100
   atcaacccgt tctcgttgcg gtcggtggcg tacgtgtcgg cggagccgcc ggtgggaagt 14160
   tgctgcacgg cgagggtgcc gggctgccat accaccgccg tgtggtgcca ggactgctcg 14220
   ccgacctggg tgaccagccc accggccact ccgccgcgct cgtccagcgt gtgcgcgagg 14280
   gccaccggtc cggtgaccga cgtggtgggt ggcggcagcg tggtgtacgt gcggctggcc 14340
   gggttccaca ggtacgagcc cacgggaaga ccgacccccg tggcgttggt gccgaccacg 14400
   aggaccgtgc ccttgtcgtt gatgtcgacg gcctgggcac cgcccgcgcc gggcggcagc 14460
   ggcaggacgt cgagcccggt gcgcgcttcc cagacgaaag gctgaagcag cggctggctg 14520
   ccgtccggcg cgggcgtcgg cggcccgatg cccagcccga gcgtgccagc gacgacgccg 14580
   aagcggttga tcgcctgcgc tcgcgagcta cccgcctgcg acggcgtgat ttcgatcagg 14640
   cggccgccct tgtgccgcgg gttcgcgatg aaggcgtggg ccgtctgggt gcccgggagc 14700
   gacgtcacgc cgaccacgtc gccgcggtcg ttgaggtcgg tggcctcact cggcgtaccg 14760
   ggtccggcga gcgacccgag gtcgtggaac cggctgagcg cctcggcggg tgcgccaccc 14820
   ggcccggacg acgtgctgcc ggtgcccctg agatgggggt tcgcgatgag cgcgtgggtg 14880
   gtctgtgtgt cggcgagcgt ggcgacgccc agcacgtcgc cggacgcggc gaccggttcc 14940
   acctgatgcg aggtcgccgt cagtgccagt atcgcgacga ccgtcagggt gcggactccc 15000
   gtcaccaggt cacccgcacc ggtccgacgc caccgcgcgc caacaggttc aggtcgtagc 15060
   gggcgacctg gcggccgagc gcggcgccga cctcgtcgga gtagcggaag tggacacccg 15120
   ccaggacccg ggcgtcgatg ttctccttcg tcaacgtcga ccacggcgtg ccacgccggt 15180
   agttgcgggt caccacccgg ccgtcacccc gggcgaaggt gatcgagaac gccatcgggg 15240
   tgcagggtcc gaccagctgc gaaagcacct gttcggcggc cccagcggtg accgcgtggc 15300
   cgctggggta ctcgggatgc ggcggcgtcg gcaggaacga gttccaggtg gggtcggggg 15360
   cggtacgggg atcgccgtcg gtgtcggcgt cgcggatggc cgtgaccggc cgccagtgca 15420
   ggagggcgta cttggcctcg aaggtggcga tgctggaatc gacgatagcc gcgccgagcg 15480
   ccgccagtag ccgcaccttg ctcctgcggg accgggccga atcgccgacc agcaggcgaa 15540
   gcaccggcac gtactgcgac agggggtcca gccacgccag gtcgctctgc gcctcggtgc 15600
   gctcggaaac ctctccgccg agacggcgta cctcgtccag ggcccgccgg taaggggccg 15660
   tgcccagggc cggcggtggt ggcgggcgga accgatcggc ccggccgagc aggaacgggc 15720
   gggccagccc gtaaccggcc gccaccgtct gggtcgcccc gggcgtgagc cggtagaccc 15780
   ccggtgcctc gggcggcagc gtgaagggca cgttcaccga cgccaggtcg aggccgtcac 15840
   cggtacgcac cgcgaccatc gccgccgcgg cctcccgacc ggcggcgatg cccgccttct 15900
   tggcctcgct cgcggccagc ccggccagcg agtcggccag caccccgtcg agcttgtccg 15960
   cctggccggg gaccagtgcc ttcagcacac cgtgcaccgc cgttgcggtg gcggcatcct 16020
   cgaacaccgc ccgctgcccg ggtgcgcgca ccggtgccgc ccgccgtgcg ctgagcgccg 16080
   cccacgcggc aatccagctc atcgcccaca tccgctcggg cagcccggcc gtcggtgccg 16140
   ccgcgaacgt cgccagcatc tcgtcgaacc aggggcccac gacgtccggg cgggaaccgt 16200
   gccggggcga cgccgtggcg gcctcggcct tggtgggcag gatcaacagg cccgcggtgc 16260
   cggctgcggt gctggccgcg gcgatcagca ggccgcgtcg ggagagcccc ggaaccaggg 16320
   gggataggga cggttcggta gatcttgtcg tcatcgccat gcctttccat cggatacggg 16380
   acgggggttc ggtgaggggt ccggttcggg accggtcggg ttcagccaca ggctggttca 16440
   gagccggctg agcagcgccg cccggtcgac cttccccgtg ttggtcaggg gcagttcgtc 16500
   gagggcgacg atgcgcgtgg ggaccatgta cgacggcagg atcctctgga gggcaccgac 16560
   gacctcggcc gtgggaagcg gctcggccgt cggaacgtag aaggcggcca gcgtccgtcc 16620
   gcccggcccc gagggagcca ccaccgaggc ggcccggacg cccgcgcact gtaggagggc 16680
   gctctgcacc tcgcccagct cgattcggta gccgcgtacc ttcacctggt cgtcgaggcg 16740
   ccccaggaac tccaggtcac cgcttggcag ccaacgggcg cggtcaccgg tgtggtacat 16800
   gcgttcgccg gtgcggtggg cccacgggtc ggggaggaat ttggcggcgg tcaggccggg 16860
   ggaccgggca tagcccaggg cgacgcattc accggcgatg tacagatcgc cctccgcgcc 16920
   gttggggcag ggccccaggc cgtggtcgag tacgtagtag cgggcgttct gcatcggctt 16980
   cccgtatgga atgctcggcc acccggggtc gacgtcgcct acccggtagt ggttcgacca 17040
   gacggtgcac tccgtcgcgc cgcccagggc caccacctcg gcgttcggga aagcggcccg 17100
   gatctggtcg ggcagcggca ccggaatcca gtcgccgctg aggaggacca ggcggagcgt 17160
   gccacgcccg gccggctcgc ggacctggac gaagggcatg accatggcca gggcggcggg 17220
   tgccgagtcc cagatggtga tctcctcctg ttcgaggagg tccaccaggg tgtccggttc 17280
   ggccagttcc gccgctgacg ccagccgtac ggacccaccg gcggccagca cgccgaagat 17340
   gtcgtagacc gagagatcga agcagaacga ggtgacaaag aggatctggt cggtcggccc 17400
   gacccggtag ctgcggttca cccagtcgat gaggttcact gccgaccggt ggctgacgac 17460
   gactcccttg ggtgcgcccg tcgagccgga agtgaagatg gtgtagcaga cgtcgtcggc 17520
   cgtcgggccg gtggcgagcg gctcgctcgg gcgcagctcg atgtgatggc cggtccacag 17580
   cggctcgcgt cccgacgccg ggcggtcgcc ggatggcgcc acgcggatca cggcgtcgta 17640
   ccgctgggcc agctcgccgg tgaacgtctc gacctggcgg cggtggacgc tgccgccggt 17700
   caccccgggc aggaactcgg gaagacgttc gatgaagtgc ggtgcgaccc ggatgccgga 17760
   atagtcactg agatccgggt cgatcaggtc ggcgagcacg atgacgccgt cgtcggcgag 17820
   cagtccgagc agcgacccga ggacgctgag caaatagtcg aggtccggca ggaactgcac 17880
   ggtgctggcc agcaggatga cgtcgaacgg accctggacc cgctcggcta cctcgtgggc 17940
   gaagcacact acgccgtcga cctcgccgcc ggcgcggaac ccgccctcct ggctgcgcgt 18000
   cacggacacc ggtgaggggt cggcggcgac gtagcgcgac gcgcgcgggg ccagggcttc 18060
   gacgatcaga ccggacccgg atccgatctc gaggatcgag ggtcgtgccg cggccgtcgc 18120
   ggccagggcg aaggcagcca catggtcgcg gtactgctgc acatcgttct cgtcgaaggg 18180
   acgctccgaa cggcgcatgt tgaagccggc ggcctcgagc tggtcggggg cgctcgacac 18240
   gaagtcgaag aagttcacca cgaggtcggg ccggaaggtg gccgaccacg agaactcctc 18300
   ggcgatgtcg gggcagagca cgtggcgcag cgagcgcacc tgccaccgaa gctcctgcat 18360
   ggagcgcagg tgggtgcggt cggtcagcac cgcggccacg tcgagctggt cgagcagccc 18420
   gaccacccgg cgggcgggcc acgacggatc gatcggtacg taggcggcac cgcacttgag 18480
   cacggccagc agcccgacga gggccggtgc gtcacgctcg ccgaagagcg ccacggtgtc 18540
   gccccgtccc acgccgagat cgcgtagcag ccgcgccagg cggttggcgc gccggtgcag 18600
   ctcccggtgg gtcaggacct gcttgtcgga ggtcacgatc gccgggcggt ccgggtgccg 18660
   ttcggcggcc tcagcgagca gatcggggat cgtgacgtcg gcgcggaagt gtcgtgcggt 18720
   ctggttccat acgctccagg gcacgcccgg agacagggct ggcggggcgc tcatcgggcc 18780
   agcgacccga acgcctcgtg ggccgccagc gcgtcggtca gctcgttgac tgtcggatgg 18840
   tcgaagaaga gcttgagcga caagcgggtc ccggccagcg caccgagccc ggccatgatc 18900
   tcggcgatca gcagggagtg cccgccgatg gcgaagaagt cgtcgtcgtt gtcgaaccgt 18960
   tcgtgttgga gtgcggctgc ccacccggcc cgtacgtggg cacgcagcaa ccctcgcgcc 19020
   gagccgtcgt tgccgtcgcg atccgggttc gccacctcgg ccgatccgat gtcgttcacc 19080
   actcgcttcc ggtcctctcc cggtgccctg cctctgcaac tgcgggccgg tgccgcccgg 19140
   tccccgaccg tgcccggccg gtgccagtct caccgcggtg gtccgccgtt gtctgcggat 19200
   ctttcagagg tcagtcaggg ttggcgctga tgggcgtcgg cgcggcaatc ctcacgtgca 19260
   tgggaacgag aacgaagaat cggtggttcg tagaggtctc gcgcagtcgg ctcaaggagt 19320
   gtgccttgta cgccattccg cacgccggcg gcggggtggc cacagtgcgc agtctctgtg 19380
   tcgaactcgg cgacgcgttc gacaccttcg gcgtgcgcct gccgggacgg gagtcccggc 19440
   tcggtgaccc ggtggtgacg gtcatgtccc ggctcgccga cgaactcgcc ggcgaactgc 19500
   tccgccatgc ccaggggcgg ccggtggtgc tgtacggcca ctgctccggc tcggcgatcg 19560
   cctacgaggt agcgcgccgt atggaccccg cccagctgcg gggactcgcc gtctcctcgc 19620
   atccggcgcc cggcagcgtt cggcgtgcgc ccacctggga actgccgcga caggagttcc 19680
   tcaagcaggt cgtgaccgac gggtacctgc cgcccgagat catcgccaac gaggaactgc 19740
   tcgacatcta cgagcccgcc gtacgcgccg attacgagct cgtcgagacc tacgagttgg 19800
   acctgttcgg gggtgggccc gtcgaggctg tcgccgcgcc gacggtggcc atccacggtc 19860
   gcgacgacca caccatcgac ccggtccaca tcgatgcctg gtcggccctc accaccgggc 19920
   cgtttcgggt cgtgtcgctg gcggggggcc acaacctgct cctcggccga ccggcggagc 19980
   tggccgcggc catctgccag ggcctggcct cagacaaccg actccatgat caccggtagc 20040
   ggccggccca ccgcccgtca cgtggcacag gtgcctgccc tgctcggccg actggtcgag 20100
   cagggcagcc caccggtgat cgagggccat cagggaagtt gctcacgccc actcccggcc 20160
   catgatgtcc gagaccagcc cgtcgacatc gatggacaca tggaagtggc cgcccggtcg 20220
   caccgtcagc cggaactcgc ctgtggtgac gtccgcccac tccgtcagcc tcgactcgtc 20280
   caccagcggg tcgccgacgc cgtgataaca actcaccggg cacgacagcg gcgtggttcc 20340
   ggagccgggc cggtagttgg cgtgcgcgcg caggtccgag cgcagcgtcg gcatgaacaa 20400
   ctcgcgtagc tccgcgatct cggcgacccg ggggtcgatg ccgcccagag cgcagatggc 20460
   cgcccagaac tcgtcatcgg gcttcaggtg aacgtcccga ttcttcatct ggccgggtgg 20520
   gaggcaggcc gaggcgcaga gcctcctcgg ttccaccctc atgtcccgca gcaacatcgc 20580
   ggtctcgtac gccaccatgg cgcccaggct gtgtccgaac agcacctggt cgcggcaggg 20640
   cagctcaagc aactccacca cgatgtgccc ggccatctcg gccacgtcgt ccacgaggtg 20700
   ctcggcgaat cggtcaccgc gtccggggta ctggacggcc acgagttcga cccctggtgg 20760
   catcgcgttc gcccagtcgc tgaaggcggt cgccgagcct cctgagtgcg gaaaacacac 20820
   caagcgcccg gtgggcttct cgaccgtggt cagcgtgcgt atccagccct tcttccgcat 20880
   ccagctcatc gatccgtcct tccgattggt ttccgctttg cactggatgg tcgtcgttct 20940
   gacgtcactc aggtcattcg tgaaagaaat tggtcagaca tagctggtgc aacggttgct 21000
   ggccccaggg ttggttctgg ccgacatctt tcaggccgtt tgaggactca acaacccgtt 21060
   catagtgtgg ggctcacgaa cgacggcggc ttccgccggc cgtggctggg gagctgcgta 21120
   cgggaggacc caactgcgct ccgccgcgag tcagtgacga tcgagagtta cttattcggg 21180
   agtctggatg ggttacggcg gtgtttcgtc ggcgcaggag gcaatgtggc tggaacagaa 21240
   tcttgcacct gaggtgccga acagcaccac gaccatttgg gatgtgcggg gcgagctgca 21300
   cctgcctgat ctcgaggacg cgttgcggac cgccctgggc gagacgagcg cactcctggt 21360
   gaacttccgg cgggagagcg atgagctgcg gatcgtcccc cggaagctgg acgcgtggga 21420
   gcccttccgc attgacgcga gcgacgccgc cgaccctgcg gccgcggccc gggaatttct 21480
   caccgaacag attcgccgcc cgttcgacct cggtgcggat gcgctgttcc ggatcggaac 21540
   ggtccggctg ggcgacgggc atcatctgct gtgcctgatc atgcatcaca tcctgaccga 21600
   cgcgttcggc gtgttcaccc tgctctccca gcgcgtcgcg gagatctacc gcgccctgcg 21660
   cgccgattcc cctgtgccgg accggccgga cacgcctgcc gagatcgccg gcgaacggga 21720
   ggcgacgtac cggtcgtccg aaaggttcac ggacgcggcg cggttctggc gtgattacgt 21780
   cacggccggc ggcacggctg cccgactgcc gaccgggatg cgggcggcgg cgacccgtac 21840
   ggcgagcgtc gggttctggg acggtctgac cgcaccgctg ggcgtcgcca gctacaccgc 21900
   cagcgtggcg gcgagcgagg tcgcccggtg gcgggcggct gccgattcga ccgaaacgag 21960
   cgtcccggac ctgctcacct cggcggccac cgccttcctc caccgcctct gcaacacgcc 22020
   cagaccgatg ctctccttca ccgtcaacta ccgcaggggg gacatcaaac aggcctgcgg 22080
   cctgtactcg aacgccctgc cgctggtgat cgaggtgccg gcggcaggcg acttcgtcga 22140
   cctggcgaag tcggtgcgtc tcgagcgtct gcgggtgctg cggtacgccg agtacaacgt 22200
   ctccctgatc aagcgcgcgg ccggtcacgc gggcgacgta cgcagcccgt tcggccccgt 22260
   cgtcaacgtc attcccttcc tccgggaact cgacctcgcc ggcgccgtcg gacggttcgc 22320
   gggcggcacg ttcgggctac ccgacgaggt ccgtatctcc acctacgtgg acggcggccc 22380
   ggacagcgac ctgtacatcc gcttcgacgc gcccggacgg ctgtacaccc acgaggacat 22440
   cgccgggctg gccgagaggt tcgtgacctt cgtccgggcg gccctgaccg acccacgggc 22500
   gcgggtccgt gacatcagcg ccatcgggga gcaggaaaga gtcgcgctcc tcgaccgctg 22560
   tgccggcccg gtagtcgagc tggccggcgt gacggtgccc gtgctgctcg acatgcaggc 22620
   gctggccaca ccggatgcgg ccgccgtcgt gcacgagaac gccgtactga cctaccggga 22680
   gctggccgcg aggtccgacg ccctggcccg cagactcgcc gagtgcggcg cgggaccgga 22740
   acgtctcgtg gcggtcgccg tgccccggtc gatcggcctg gcggtcgcgt tgtgcgcggt 22800
   cctcaaggcc ggcgccgcct acctgcccat cgatccggac tctccggcgg agcgcgtgga 22860
   gttcatgctc ggcgacgctc ggccgacgct gctgctcacg gtcgccggga ccgacggact 22920
   ggccatcacg cccctgcacg gagcgcagcc acgctgggag gccagcgtta tcgagcggac 22980
   gaccgcagcg tcaccggcgg ccacgtcgca ggctggcggc accggcgagc acgccgcgta 23040
   cctgcggtac cggtcggggc cattcggtcc ggcggccggc gtcgtggtga cgcacgccgc 23100
   cctcgtcaac gagctgctct ggatgcagca gcgataccgg ttgaacgaca ccgaccgggt 23160
   gctgcacaag gccccggcat ccctcaatgc ctccgggtgg gagatcttct ggccgctgat 23220
   cgccggtgcc accgtcgtca tggcgaccca ggacgtccac ggggacgcga ccgcgctcgc 23280
   ggcgatgatc cggcggacgg gtgtcaccac cgtgcacgtc gtcccgtccg tgctcgccga 23340
   gctggtgcgc gacgaagccg ttgtcaccgc cggcaagggt ctgcggcacg tcatgtgcag 23400
   cggggaagtg ctcccggccg acctggcagc ccggttccac gagagctgcg acgtcccggt 23460
   gcacagtctt tacgggcccg tcgaggcgat gccggtcgtc acgtcgtgcg actaccgacc 23520
   cggctcgccc acggtaccgc tcggacgacc gatctcgaac gccggcgtct acgtgctgga 23580
   cagccaactc cagctgacgc cgccgggagt ggtcggcgag gtgtacgtca ccggcgtggg 23640
   tctggcccgc ggctacctgc accgcccggc cgcgaccgcg gaccacttcg tcgcgtgccc 23700
   gttcggcccc gccggcaccc gcatgtaccg caccggtgac ctggcgcgct ggaccgacga 23760
   cggggaactc gagcacgccg gcagcgccga ccagcgggtg cggatacgcg gcttctgggc 23820
   cgcgttcctc gagatcgaag cagttctgac cgcccacccg ggagtggacc aggccgccgt 23880
   gatcgcccgg cccgacggcc ccgaccgcgc cgcccagctg atcgcctacg tggtacccgc 23940
   gaccggcggg cggatgggtg cggccggcct ggacgtggac ttcagcgccg ggctggacgt 24000
   ggtcgagctg cggcggttcg tcgctgcccg gctgcccgaa cacctcgtac ccgccaccat 24060
   cgtcgtactg gaccggctgc cgctcacggc cgaagggaag cgggaccacg ccgcgctgcc 24120
   cgaaccccag gccacccgac gggcccaccg ggtggcccgg accgccgagg aacgcctcct 24180
   cgcggacgcc ttcgccgagg tgctgggcgc cggccgcatc ggtctggacg acgacttctt 24240
   cgccctgggc ggcgacagca tctcggcgat gcgggtggtg gccctcgccc gtgccggcgg 24300
   cctcgcgctc accgcgcgta ccgtcttcga atgccgcacc gtcgccgccc tggcggccgt 24360
   ggcggcgccg gccgacgaac cagcggtcgc cgctgcggcc gagggcgacg gcggacgcga 24420
   cgacgccggc ccgatcccac ttccgccgat ggcgcggctc ttcgccgaac gtggaccggg 24480
   cctggaccgg ctggcccagt ggctggtgct gacgctgccg gcggagatga gccccgacct 24540
   gctcaccgga gcggtccgag cggtcctgga ccggcacgat gtgctgcgct cccggctggc 24600
   cgacggtcag ctgctgatcc agccgccggg atcggtggac gtcgacaagc tcatccggca 24660
   ggtgccctgt tccggtgact ggagcgggcc ctcgtggccg gcgttactgg cggacgaggc 24720
   cgcgaaggcg gtcggtcagc tcagcgcctc ggccgggcag gtgatccgca tggtctggtt 24780
   cgcgccgccg ggcggtgcac ccggaagact gctcatcgcc gcccaccacc tggtcgtgga 24840
   cggaatgtcg tggcgggtgc tcgtcccgga cctcgccgag gccgcggcac agatccgatc 24900
   cgggcgtacg ccggcactgg ccccggtggg cacctcgctc cggtcctggc tggacgaact 24960
   cgcggccgaa gcggtacggc ccgagcgcgt ggccgagctg gaactctggc gcggcatcct 25020
   cgcaccctcc ggcgatccgc tgggtgcccg acccctcgat ccggcggtcg acctgaccag 25080
   gacggcccag acgctgcggg tcgaggttcc cgccgaactc agcagggccc tgctcacctc 25140
   ggtgccggcc accttccggt gcggtgtcga cgatgtgctg ctcaccgccc ttgcgctcgc 25200
   gttggccagc cgtcggcaca acagcgccgg agtcgtccgg ctggaagggc acggacgtca 25260
   ggaggaactc gttcccggca ccgacctgac ccgtacggtg ggctggttca ccacggtcca 25320
   cccggtccgg ttcgacctgt ccggcctcga cctcaccgac gcgatcaacg gtggcccggc 25380
   cgccgcggag gcactccggc gggtaaagga gagccggcgc gtcctgcccg atcggggcat 25440
   cggctactcg ctgctgcggt acctcaacca ggcgaccggc gccatgctcc gcgacctccc 25500
   cgccgacgag gtcggcttca actacctcgg tcggttctcc ttcgaccgtt cccggacggg 25560
   ccgcgagatg gcgtggacgt ccgcgccgga atccgccgca ctcgtggcgg caccggatcc 25620
   ggacatgccg ctgatctccg cactcgaact gaactccctg gtcgtcgaca ccgaccacgg 25680
   cgaacagcta caggcgctgt tcaccttcgc caccggggtg ctcgccaccg acgaggtccg 25740
   gcacctcgcc gacgactggc aggccgcgct gcgtggcatc cgagcacagg cggccacggg 25800
   cgcgagcggc ctgaccccat ccgacgtgcc gttggtgcgg gtgcggcagc aggacctgga 25860
   cgactggcag agccgtttcg ggcgggtcga cgacgtgtgg ccgctgactc cgctccagca 25920
   cgggctgctc taccacagcc tgctgggcga cagcgccgcc gcgtcgtacc agacccagtt 25980
   cgtcctccgg atcaacggac cggtggatcc ggcgcgtctt cgtggcgcgg cacagaccct 26040
   gctgaaccgg caccccaacc tgcgggtggc cttcctgacc aagtccaccg gcgagccggt 26100
   gcaggtggtc gtggacggcg tcgaggtgcc cttcgcgcac gtcgacctcg ccggtcaacc 26160
   gcaggccgag gagcgactgg cccggatcct ggccgaggaa cgggaggccc agttccgcca 26220
   cgacagcgca ccgctgctgc ggttcagcct catcaccctc accccgaccc aggccgaact 26280
   cgcgctgacc gcacatcacg tgctgttcga cggctggtcg ctgccgctca tcgaacaaca 26340
   gctgatgcac ctgtacgccg gcggggccga cgcgctggaa ctggtcgagc acggctatcg 26400
   cgagttcctg cgctggcact cgcggcagaa cgcggacgag gcgctcgcgg cctggaccga 26460
   ggaactggcc gacgcgacgc agcccacgct cctggcatcg gggttcgccc ccgccgagca 26520
   ggctgggacg agcaccgaga tcggccaggt cgacgtcctg ctcacccgcc aggaggcggc 26580
   atccgcagtg cgtcggccgg cggattgcgg catcacaccg aacgtcctcg tgcagggcgc 26640
   ctgggcggtc gtgctcgccc agctcaccgg aaacgccgac gtcctcttcg gcagctccgt 26700
   caccgtacgc ccgcccgaac tgccgggcgc gcacacggtc gtcgggatgt tcaccaacac 26760
   cgtgccggtg cgggtacggt gcgcgcccgc cgacagcctc gaaggcatgc tccaggccgt 26820
   acaggatgcc caggcgagga cgttggatca ctaccacgtc gggctcggcg acatccagcg 26880
   ggcgaccgga ctcactaccc tcttcgacac catcgtcatc ttcgagtcgt tcccggtgga 26940
   ccgggcggcg ttgtcgaagg ccagcgcgtc ggcggaactg accgtcaccg gcatccggcc 27000
   gttcgcgccg acgcactacc cgctgaccgt gctcgcggcg gcggatccgc tgttgagcct 27060
   gaccctccag taccaccccg acgcgcttgc accgagcacc gtgcaggcga tcgcggaccg 27120
   tttcgcccgg gtgttgcggc agttcgcggc cgacccgcgg acccgcatcg ccgccatcga 27180
   cgtgctctcc gaggccgagc ggcagctggt ggtgcacgac tggaacgcca ccgcgctgga 27240
   ggtgccgccc gagttcgtgc ccgagcagtt cgcccggcag gcggcagcga caccggacgc 27300
   ggtggccgtc gtggtggggg agcagcacct cacctacgcc gagttgagcg agcgggccaa 27360
   ccggatggcg cactggctga tcgcgcaggg aatcggcgcc gagagccggg tcgtggtcct 27420
   gctgccccgc tcggcggacc tggtggccgc gctgctggcg gtctggcagg ccggcggatg 27480
   ctacgtaccc gtggatcccg actatcccgc cgcgcgggtg cggtccgtga tcgacgactg 27540
   tgcggccacg ctggtgctgg acgagaacct gctggaccgg accgacctgt cgcgctaccc 27600
   ggcacacgcc cccgcgatcg ccgtcgcgcc cggccaggcg gcctacacca tctacacctc 27660
   cggctcgacc ggcaagccga agggcgtggt catcggccac gacgccctga ccaactttct 27720
   ggccagcatg cagcgaacgc tgcgcctgtc ggcgcaggac cgcttcgccg ccgtcaccac 27780
   gatcgccttc gatatcgcgg cgctcgagtt gtttctgccg ctcacgaccg gggcgcgcgt 27840
   ggtgctcgcc aaccgggagc acgtcgcgaa tccaccggcc atgctggacc tcgtcgagca 27900
   gaccggcgtc accgtcatgc aggcgacccc cgcgctgtgg cagatgctcg cgacgcacga 27960
   cgtgcgtcgc ctgtccggcg tacgcgtcct gaccggcggc gaggcactgc cccggccgct 28020
   cgccgagggc ctctgcgcgc acgccgcaca ggtgatcaac ttgtacgggc cgaccgagac 28080
   gaccgtctgg tcgacccttg cggacgtcgc gacctgcaag ccgccgtcga tcggtacgcc 28140
   catcgccaac acccaggtcc tgatcctgga ctcggcactg cggcccgtcc cacccggggt 28200
   cggtggagac ctgtggatcg caggggacgg cctggcccgc ggctatcacc ggcaaccggg 28260
   gctgaccgcc agccggttcg tggccaaccc gttcggccct caggtgcccg ggatgtaccg 28320
   caccggcgac ctggctcgct ggtcggagag cggaaggatc gagttcctcg gccgggccga 28380
   cttccagatc aaactccggg gattccggat cgagccgggc gacgtcgaac acgccctgac 28440
   ccggcatccc gcggtccgcg aggccgtcgt gacgatccgg gaggaccagc ccgacgacaa 28500
   gcggctcgtc gggtacgtgg tcgtcgccga ggatgccgag accccgccga cccgggagtt 28560
   gcagcagttc gtgcgggagc agctgcccga ctacatggtc ccgtcggcgg tggtcaccct 28620
   gcccgccatt ccgttgacgg cgaacggaaa gctggaccgt tcgcgactgc cccgacccga 28680
   ggcgagcagc acgaagtacc gggcgccgga atccgcccgg gaggtcgccc tctgcgggat 28740
   cttcgccgaa gtcctcggcg cggagcgggt cggcgtcgac gacgacttct tcgcgctcgg 28800
   cgggcactcg ctgctcgcca cccggcttgc cgctcggctg cggtccgacc tgggggtcga 28860
   ggtcccgatc cgcaccatct tcgccgcacc gaccgtcgcc gaactcgccc ggcggtggag 28920
   cggtctgtcg acctccgtcc ggaagcctct gcgcaggatg accgaaaggt aacagagaat 28980
   ggcgcccgtc tcgtatgcgc aacaacgaat gtggttcatc aatcggttcg agggccagac 29040
   cgcgacctac aacatcccga tcctgatccg gatgcgcgga cagatcgacc gagacgagct 29100
   gcgggctgcg ttcatggacg tcctgtcccg gcacgagatt ctgcgtaccg tctacgacga 29160
   gctcgacggg cagccgctgc aacggatcat cgagccggcc gagctgagcc tgccctggac 29220
   cgactgggga tgggtgtccc cacagcaggt ggacagcatc gtcgccgcgt cggtccgcga 29280
   gggcttcgac ctgtcggtgg acctgccgat ccgggcgtac ctgctgcgtc ccgagccgga 29340
   cgtggccctg ctcgcggtgg tgatccacca catcgccggc gacggcggct cgctcggccc 29400
   cttcacccgg gacctctcgg ccgcctaccg cgcccggctg gccggggtgg cgcccacctg 29460
   ggacgagctg ccggtgcagt actccgacta cgcgctctgg cagcgtgagc tgctgggcga 29520
   cgagtccgac ccggacagcg tcttctcgac ccagctgcgc tactggcggc aggaactgga 29580
   cggcgtctcc cggccggtcg cgctgcccgt cgaccggccg cgtcccgccc gggccagcta 29640
   tcgcggcgac aaccacggat tcgagattcc cgcggcgctg ttcgagaagg tcgagcgcct 29700
   cgcccagtcg tacaacgtga cggtgcccat ggtctttcag accgcgctgg tggtcctgct 29760
   gcaccgcctc ggcggcgacc gcgacatcac gctcggctcg ccgatcgcgg gccgcaccga 29820
   ccaggccctc gagaacctcg tgggcttctt cgtcaacacc tgggtgctgc gggtgggggt 29880
   gtgcaccggc gactcgttcg cgcaggtcct ggaacaggtc cggcagaagg ccatcgccgc 29940
   gtacgaccac caggacctgc cgttcgagcg gctggtggag ctgctgcgac ccgggcgctc 30000
   agtcgcgcat caccccctgt tccaggtcac cctcgcctgg cagaacagcg tccgccagga 30060
   actcgacctg cccggcgtca cggcctcgat ggagccggtc gccaccggca cggcaaagtt 30120
   cgacctgttc gtcaacctga tgccgaacca ttcgcgcagg tcggtgaccg tcgacctgga 30180
   atacgccacc gacctgttcg accccgccac cgtcgaggcg atcgcggcac ggtacctgcg 30240
   cgtgctggag caggtggtgg agagcccgga cctcggcgtc ggcatcctcg acgtacccgc 30300
   gatgccggcg acgcgcggcg agcggacggt cgacgccggc gaacgcgaga agatcctcta 30360
   ccgctggaac gacaccgacc gcagctttcc ctgcccggga ccgatccacc tgccgttcga 30420
   gcagcaggcc gccgcgcgtc ccgacgcgat cgccgtgcgc tggtcgggcg gcaccatgac 30480
   ctaccgcgag ctcaaccggc aggccaaccg gatcgcctgg accctcaagc gccgcggcgt 30540
   gcggccggag accgtggtgg gcgtggccgt gcggcgcggc ccgctcatgg tcgccgccgt 30600
   cctcggggtg ctcaaggccg gcggagcgta cctgcccatc ggctccacac tgccctccga 30660
   ccgggtcgcc ggcatgctgg ccgacgcgtc cgcgacgctg gtgctgacca ccgaggacac 30720
   caacaggtgg acgccaccgg atggcgtgga actcctcgac gtcggccggg ccggcctggc 30780
   cctgtcgctc gacggcgaga tcaaccccga gccggtggcg agtgccgaca acacggccta 30840
   catcatcttc acctcgggaa gcaccggaaa gcccaagggt gtcactgtgg cccaccggcc 30900
   ggtgcacaac ctgctgaact ggtgctaccg gaccttcgac ttcggccccg acgatgtgag 30960
   tctctgcgtc acctcgctgg acttcgacct ctccgtgttc gacatcttcg gcctgctcgg 31020
   ggcaggcggc gggttctaca tcgcggacgc cacccagcag cgcgatccgg agatgctgct 31080
   cgacgtcctg ctgtcggagc cgatcacctt ctggaactcg gtgccgggca cgctgaacca 31140
   actggccccg ctgctgccgc agaccgccgg cagcccaggg gtgcaggatc tgcgcctggt 31200
   ctttttctcc ggcgactaca ccccgctgtc cctgccggac caggtccgcg cggtcttccc 31260
   gcacgccgag atcatcagcc tcggcggcgc gaccgaggcg acggtctggt cgaacttctt 31320
   cccggtcggc gcgatcgacc cgcagtggcg cagcattccc tacgggcggc ccatcgacaa 31380
   cgcgcgctac tacgtcctcg acgagaagct ggagccgtgc gcggtcggtg tggagggcga 31440
   cctctacatc ggcgggccgg tgatcgctct cgggtacgtg aaccgtcccg acctgacggc 31500
   cgagcggttc atcgccgacc cgttcggcgg ggttcccggt cagcgcatct accgtacggg 31560
   cgaccgcgcc agcttcttcc cggacggcaa catctgtttc cttggtcgcg ccgacggcca 31620
   ggtgaaggtg cgggggttcc gcatcgaact gggcgagatc gagtacgccc tcggccgcca 31680
   cccggcggtg cgccagggta tcgcgatcac ccgccgggac tccgtcggtg acctgcggct 31740
   ggtcagctac gtcctgcccg atttggacac ggtggagagc gtggtcgccg ccgacgaaca 31800
   ggtccgcgaa tggcaggaga tctacgacca gggctacctg gaggtcaccg accaggattt 31860
   cggcgacgac ttcaacctct gggtgtcgag ctacaccggc gagcccatcc cggtcggcca 31920
   gatgcgggag tggcaggacg ccgccgtcga ccggatcctc agcttcacgc cgcggcgcgt 31980
   actggaggtg ggtgccggaa ccgggctgct gctcgcgcgg gttgcgggca gcgtggaggc 32040
   gtactgggcg accgacttct cggagccggt catcgagcgg ctgggccggc aggtgaccga 32100
   ggcgggctgg gccgagcggg tacggctgct gtgccgacgc gcggacgacc tggacgggat 32160
   cccacggatc ttcgacaccg tagtgctcaa ctcggtcgtg cagtacttcc cgaacgagcg 32220
   ctacctggag caggtgctcg acggcgtctg ggcgatgctg gagccaggtg gccggctcgt 32280
   gctcggcgac atccgccggg cccgctcgct gcgcgccttc caggtggccg tgcagcaggc 32340
   caagcacggc aacctcccgc cggcgcaact gcgcagcgcg gtcgaacagg gcctgctcct 32400
   ggagaaggaa ctggtcatcg acccggagtg gttccagcgc tgggccgagc gcgcgggcgc 32460
   ggccggcgtc gacgtgcgcc tgaaggaggg cgcgttccag aacgagttga cccgccaccg 32520
   ctacgagatc gtggtgcaca agccgggcac aacggaggcg ggccggccct acgcggtgga 32580
   taccgtcccg cgcctggagt ggaacggcga cctggacggc ctggccgagc gcatccgcac 32640
   gctgggcggg ccggtggtgc gcatcgccgg catccccaac gcgcgcgtcg cgcaggaggt 32700
   ggcggcggcg cgcgacctgg ggctggagga gtccgagccg ccgacgtcgg tgccggtgga 32760
   cccgcacgag atggcgacct gggccgcccg gcagggctgg tcgatcgcgc tgacctggtc 32820
   cggaagcgcc gtcgaccagt ttgaagcagt cctgttcacc gacgctacga ccgagcaccg 32880
   ggcactgtcg ggcacctacc tcccggtggc cgccgccggc acctggatca acgacccggc 32940
   cgccgcggcg ggcatcaccg cactcccggc ggtcctgcgg gagtacctgt cgaagacgct 33000
   gcccgagtac atggtgccat cggcgatcgt gccgatcggc cgggtgccgc tgacgccgaa 33060
   cggcaagctg gaccggcggg cgctgccggt gccggagtac gccgccgcca gtcgcggccg 33120
   gccgccgtgt acctccgagg aggagtcgct ctgcgagatc ttcgccggcg ttctcgggct 33180
   cgaccgcgtc ggcgtggacg acaacttctt cacgatcggc ggccactcgc ttctggcgac 33240
   ccgggtggtg agccggatcc gcgccgcgca cggtgtcgag atcccgatcc ggacgatctt 33300
   cgaggcgccg accgtcgccg aactggtggc ccatttgacc ggccagcgga cggtccggcc 33360
   gccgctgcgg ccgcagccgc ggccggaccg gctcccggtt tccttcgccc agcaacggct 33420
   gtggttcatc caccgcttcg aagggccatc gccgacctac atcattccgc tctccatgcg 33480
   tctgcgcgga aaactggacg tgcccgcgtt gcggcaggcg gtgcacgatg tcgtggtccg 33540
   gcacgagtcg ctgcgcaccg tcttcgacga ggtggacggg gcaccctacc agcgggtact 33600
   cgacccggac gggttggcga tcccgtggca ggagcacgag gtcaccgagt ccgcccttca 33660
   ggaggcgctg cgcctggagg cacgtcggcc cttcgacctg gccgcggaga ttccggtacg 33720
   cgccgcgctg ttccgcgtcg gcgagcacga cgcggtgctg ctgcttccgc tgcaccacat 33780
   cgcggccgac ggctggtcgc tggggccgct ggccgaggat ctgaccgccg cgtacaccgc 33840
   caggtgcgac gggcagcgac cgcaatgggc cgcactgccg gtccagtacg ccgactacac 33900
   gctgtggcag cgtgaccttc tcggcggcga tgacccacac agtctgtacc ggcggcagtt 33960
   ggactactgg acggggcaac tcgccggact gccggaatcg ctgcaactgc cgacggaccg 34020
   ggcccgaccc gccgtcgcgt cgttcgccgg cgaggtgctg cccgtagaac tcgacgaatc 34080
   gctgaccgag gacctccgca agttggccct gcgtaccggt accaccgtgt cgatggtgct 34140
   ccaggcgggt ctcgcggggc tgctcagcaa gctcggcgcc ggtaacgaca taccgatcgg 34200
   gtcgccgatc gccgggcgga cggacgaggc gctcaaccga ttggtgggct tcttcgtcaa 34260
   cacctgggtg gcgcgcgtcg acaccagcgg aagccccagc ctgatcgagc tggtggaccg 34320
   ggtacgggag gtgagcctcg ccgcctacga ccaccaggac atcccgttcg agcacctggt 34380
   cgaggcgctg aatccggtgc gctcgaccgc gcatcatccg ctgttccagg tgtgcctggc 34440
   ccttcagaac aacgtgcagc cggccttcga cctgcccgga ctgaaggtgt cccacgagcc 34500
   ggtcgacatg ggcgtctccc gtttcgacct gttcttgaat ctgatggagg agaccaccgg 34560
   cgaggggacg agccggatcg ccggagtcgt cgagtacgcc acggaactgt tcgaccggga 34620
   cacggtcacc gggctgctgg agcgctggcg gcgcctgctg cggcagtggg tcggcgctcc 34680
   ggacgccccg atcgacgcgg tggagatcct caaccgggac gacctggccg cccttgagcg 34740
   ctggaccggt cgcgctcgcg gcaccgatcg tgtcgtcggg acgattcccg agcgtttcgc 34800
   cgccgtggtg gccgagcagc cggaggctgt cgccctggtg gccgccgacg gcgaggagag 34860
   ctggacgtac ggcgaactcg accggtgggc caaccgcatc gcccaccatc tccacgcgcg 34920
   cggcgtcggt cggcagcacc gggtggcgtt ggtcatggag cggtccccgt tgctggtcgc 34980
   ggcggtgctg ggcacgctca aggcgggcgc ctgttatgtg ccggtggaac ccacctggcc 35040
   gcgggcccgg atcgacctcg tgctcgccga cctcgatccg gccctcgtga tcgacgagcg 35100
   gctggccgag gaggacctga ccggctaccc gacgcgcccg ctggacaccg ccgacgtcgg 35160
   cggggagcac ctcgcctacc tgatgtacac ctccggctcc acggggacgc cgaaaggtgt 35220
   cgaggtgagc catcgcaacg tgctcagtct cgcgctcgac ccgtgctggg ccgatgctga 35280
   ccaccagcga gtgctggtgc atgccccgcc gaccttcgac gcatcgacgt acgagatgtg 35340
   ggtgccgctg ctgcacggcg gagcggccgt ggtcgcgccg ccgggaaagc tggacgcggc 35400
   ccgactggcg acgctcatcg ccgagcgggg cgtaacggcg ctgtggctgc cggcgggcct 35460
   gttcgatcta atcacgcagc atcacccgaa gagcttcgtc caggtacgcg aggtgtgggc 35520
   cggcggcgac gtactgtccc cggcggccgt gcgacggctg gtacgcgacg acggcacgct 35580
   caccgtcgtg aacgggtacg gtcccaccga gaccaccacc ttcgcggccc ggtaccggat 35640
   gtccgcaccg gcgcggtgca aggacccctt gccgatcggc gagccgatgg cgggcagccg 35700
   cctctacgcc ctcgacgacc ggctccggca ggtgccgcag ggcgtcatcg gcgaactcta 35760
   cgtcggtggc gacggggtgg ctcgcgggta cgcgaaccat cccccgctga ccagcgaacg 35820
   attcgtcgcc gatcccttcg gacgcccggg cgagcggatg taccgcaccg gtgacctggt 35880
   ccggtggaac cacgacgggc aactcgagtt cctgggccgc gtcgacgagc aggtcaagat 35940
   ccgcggtttc cgggttgagc cgggagagat ccgagcggcc ctccggaaac gggacggcgt 36000
   ggcacaggcg gtggtggtcc cccgtacgga ccgactcggc gagcggcgcc tggtggccta 36060
   cgtggttccg gaggtgccgg cgggcgcgga cgaagactcg accgaacatg tcgagaagtg 36120
   gcgcgccatc tacgactcga tgtacgacga gaccgaggcc gacgctaccg agatcggcaa 36180
   cgacttcacc gggtggaaga gttcctacac gcgcgacaac atcccgctgt ccgagatgcg 36240
   ccggtggcgc gactccgtcg tcgaggaggt ccgcggtctg cgggcgcgcc ggatcctgga 36300
   gatcggggtc ggttcgggcc tcctgctcgg gccgttggcg ccggaagcgg aggcgtactg 36360
   gggcaccgac ttctcgttgc cggtcatcga gcgcctcgag gtgcaggtcg gcaccgatcc 36420
   gtgcctgaag gagaaggtgt cgctgcggtg ccagcacgcc gacgtcgccg acggactgcc 36480
   ggtgaagtac ttcgacaccg tcatcctgaa ctccgtcgtg cagtactttc cggacgccgc 36540
   ctatctctcc cgggtactgg acgtcgccct ggaccggctc gcgccgggtg gccggatcct 36600
   cgtcggtgac gtccggaact acggcaccct gcgggagttc ctgacggccg tccatcacgc 36660
   gcaacacccg caggacagcg cgtcggccgt ccgggcggcg gtcgagcgtg ccgtcctggc 36720
   cgagaaggaa cttgtcatcg atccggattt cttcaccgag tgggcacgga cccggcccga 36780
   cgtcgtggcc gtcgacatcc ggctgaagcc gggcgcggac cagaacgaac tcacccggca 36840
   ccgttacgag gtcatcctgc acaagcagcc gagccaacca ctgcgcctcg cggacgtgcg 36900
   gacggcgaac tggggctcgg aggtgcccga cctgtccggg ctggagaccg cactggcccg 36960
   gcacggcggt cgcctccggt tggcccggat cccgaacgcc aggctggtga gcgaggcggt 37020
   ccagtgcggc gtgcccacca acgtaggcgg cacgcccctg gacccgcacg agttggcgag 37080
   ctggggaggg cagcggggtt actcggtcca ctgcacgtgg tcggcggagg cgcccggctg 37140
   gttcgaggcg gtgatcatcc cggtcgacag cgggcactgc cgcgacggtg tgtaccggcc 37200
   ggtggggccc cggccacggc agttggtcaa cctgcccgcc gcggcgcgcc gggtgtctcg 37260
   gctgccctcg tggctgcgcg aggaactggc ggcggagctg ccggagcacc tggtgccggg 37320
   cgacatcgtg gtcatggaac gactgccgtt gaccaccaac gggaagatcg accacagcag 37380
   acttccggag gtggagcagg ccgaccggga gtacggctcg ccgcgcacac ccctggaggc 37440
   ggaactggcc ggtctcttcg ccgaggtcct cggcgtggac cgggtgggca tcgaggacga 37500
   cttcttcgac tgcggcggta gttctctcca ggtcatccgg ctcatctggc ggatccgcgc 37560
   ggagctgggc ttcgacattc cggtcagaac catcttccag catccgaccg tggccgaggt 37620
   cgcggaacat ctggcggccg gcagggagga cgtcgagttc gacgacccct tctcggtggt 37680
   gctaccgatc cggaccgacg gggaccgggc accgatctgg ttggtgccgc cgggtggcgg 37740
   actctcctgg gcctatctcg gcttcgccca gcacctggac cgctcgcgcc ccatctacgg 37800
   cctccaggcc cgtggatttg tcggagaccc gcgggcgacc tcgatcgaga gcatggtgga 37860
   cgactacgtc gaccagatcc tgcgggtcca gccggaaggt ccctacaacg tgctgggttg 37920
   gtcgctgggc ggcccgatcg cccaggcggt ggccgccgaa ctccagcgcc gggagtgcga 37980
   ggtggatctg ctggccgtcc tcgactcggg tccggcgagc tacttcgccg acttccagac 38040
   tcccgacgag aagttggtgc gcaggtacct ggcgcactac atgggccatc tcgcgggaat 38100
   ggcggagttc gagtcgctcg tgcagacatc caccaccctg ttcatcgagc acacggacct 38160
   gatgacgcaa ttcacgtcgc cgccgttccg tggcgatctg atcttctttg ccgccctcat 38220
   cgaccagggc acccgggaac gacggcaact cgaggtggaa ctggacgtcc aatggcggga 38280
   atacaccgag gggaccgtgc ggcgcttcga catcgaatgc gcccacaacg aaatgatgtg 38340
   gcccgagaac gcggccatga tcagccgtat cgtcaacgag atcatcgatt cggcccgata 38400
   accgcttccg ccctgacctg gagcggacgt gacaactacg gaggaaagat gagcgtcaac 38460
   ccgttcgatg acgaggacgg cgagttctac gtgctggtca acgacgagga gcagcactcg 38520
   ctgtggccca ccttcggtga cgtgcccgac ggctggcgaa tcgtgttcgg gccggccggc 38580
   cgagcggaga gcgtggcata cgtggaggag aactggaccg acatgcggcc caagagcctg 38640
   cgtgaggcga tgtcggcagc atgacgtacg tcgtgggctc gcaggcagac gcgtccgcct 38700
   gcgggcccac gattttgaac ttccgcgacg ccggtggcgg aaggtgcgta cagaagtctt 38760
   cacaggatgt ctaggtatag aactgggatg tggcaagccg gagatcgccg ggggagtgat 38820
   cagcccgggt caccccgccg gctccgcgtc gcgcgccaga cactttggag gccccatgac 38880
   tgttgcgtcg cccaggcaga gccgagaaga cctatcgctc gtcccggaca cccatgacgt 38940
   catccgcgtg gtcggggcac gggagaacaa cctcaaggac atccgactgg agatcccgaa 39000
   gcgccggctg accgtgttca ccggtgtctc cggctcgggg aagagttccc tggtgttcga 39060
   cacgatcgcc gcggagtcac agcggctgat caacgagacc tacagcgcat tcgtgcaagg 39120
   gttcatgccc acgctgtcgc gccccgacgt cgacgtgctc gatggcctca ccacggcgat 39180
   catcgtcagc caggagcgga tcggcaacaa tccgcgttcc accgtgggca ccgtgacgga 39240
   cgccaacgcg atgctgcgca tcctgttcag ccggctcggc gagccacgac tgggctcccc 39300
   gcggtcgtac tcgttcaaca ttccctcgat cagcgtcgcg aacgcggtca ccgtcggcca 39360
   agggagccgg gcggtcgagg agcaacgcgg cttcgacgtc gccggcggca tgtgcccacg 39420
   ctgcgaaggt acggggtcgg tcaacgacgt cgacctcacc cagctctacg acgccacgaa 39480
   gtcgctcgac gagggtgcca ttcgggtgcc cggttaccag gtcggcggct acaaccaccg 39540
   gctctacgcc ggttccgggc tgctcgaccc cggcaagccc gtcgagaagt acaccgagaa 39600
   ggagctcaac gacttcctcc accgggaacc gacccggctg aagatcggcg actaccacac 39660
   gaactacgaa gggctggtcg caagggtcca gaagtcgctg ctcaccaagg acatcgaggc 39720
   gcttcagccg cacgtacgcg ctttcgtgga gcgtgcggtc acgttcacgg cgtgcccaga 39780
   ctgcggcggt acccggctca acgagcaggc ccgggcatcc aggatcaagg gcatcaacat 39840
   cgccgacgcc tccgcgatgc agatcaacga cctcgccgaa tgggtccgcg acctggccga 39900
   gccatccgtc gcaccgctgc tggccacgct cctgcacacg ctcgactcgt tcgtgggtat 39960
   cgggctgggc tacctctccc tcgaccgggc ctccggcaca ctctccggcg gcgaggcaca 40020
   gcgggtcaag atgatccgcc acctcgggtc accgctcacc gacgtcacct acgtcttcga 40080
   cgagccgacg atcgggcttc acccccacga catccaacgg atgaacaaac tcctgctgcg 40140
   gctccgcgac aagggcaaca cggtgctcgt cgtggagcac aagccggaga ccatcgccat 40200
   cgccgaccgc gtcgtcgatc tcggtcccgg cgccggcacc gcaggcggca ccatctgctt 40260
   cgagggcacc gtcgcggaac tgcgttccgc cgacacgctg accggccgcc atcttgacga 40320
   caaggcgtcg ctgaaggaca gggtgcgtac cccgtcgggc tcgctcgagg tccggggcgc 40380
   gagcaaacac aacctgcgag acgtcgacgt cgacatcccg ctcggcgtgc tcgtcgtcgt 40440
   gaccggcgtg gcaggctccg gcaagagctc actgatcgaa gggtcggtat cgggacgcga 40500
   cggcgtggtc acgatcgacc aggggtcgat caagggctcc cgtcgaagca accccgccac 40560
   ctacaccggc ctcctcgatc ccatccgcaa ggcgttcgcc aaagccaacg gcgtgaagcc 40620
   ggcgctgttc agctcgaatt ccgagggggc ctgtgccacg tgcaagggtg caggcgtcat 40680
   ctacaccgac ctggcgatca tggcgggcgc ggcgaccacc tgtgacgact gcgagggcag 40740
   gcggttccag gacttcgtgc tccagtaccg tctcggcggt cgcgacatca gcgaggtgct 40800
   ggccatgtcc gtggccgagg ccgggcgttt cttcgctgcg ggtcaggcac gtacgcccgc 40860
   cgccgtcgtc atcctcgaac ggctcgccga cgtggggctt ggctaccttt ccctcggcca 40920
   gccactcccc acactctccg gaggcgagcg gcagcggctc aagctcgccg cgcacctggc 40980
   tgacgaaggt ggcatcgtcg tgctcgacga gccgaccacc ggcctgcacc tcgccgacgt 41040
   ggagcagtta ctcggtctgc tcgaccggct cgttgacgct ggccggtcgg tcatggtcat 41100
   cgagcatcac caggcggtca tggcccacgc cgactggatc atcgacctcg gtccggccgc 41160
   cggccacgac ggcggtcgtg tcgtcttcga aggctgcccg gccgacctcg tagccagccg 41220
   cgccaccctc accggcgaac acctcgcggc gtacgtaggg gtctgacggc ccgcctgaca 41280
   cggtttccgg ccgcctgccg tggcggccgg tcaccgctcg tacaagcacc cgctctgtcg 41340
   gcctcggccc acgcgtgcga tggcatccca tggcggtacc cgaccgccgc ctcaggctcc 41400
   atcctgccct gaccaggccg ttctccagca cgcggcgatc ggttcgggcc gatccgggta 41460
   gcccggcaca ggtgtttctg ctgtccccgt acctgaccga acccagctct tcgtggccct 41520
   ggcgaagttc tttcatcctt gtggcatgcg tgttgcgtga ccccatggtc gcgttgagta 41580
   ttgggaatcg taattggata tctcgcctgc ggggggatgc ggtgatggag gcgactggcg 41640
   ttgcggctgc gcgggtgcga gcaccggccg agccggcccg tcgcgttcgg ggtcgaggtc 41700
   gtcatggaca cacagccggg gcgatgccgc cacggatccg tgcccatgac gctctgacag 41760
   attcgttgac cgcgacccgt cgccgatggt gacgggagca gggcctttcg cgagatgttt 41820
   tgggggattt gcttgtgggc ggtcggaact ccgatgtcgt cttcatccgc tggcccgctc 41880
   aggcggacct gagaacccgg tgccggaagg agggagtgcc gcggctgctc gtcgtggagg 41940
   gcggtgcgca gccgccgatc agcaatgacc cggtcgagga ctgggtccgg ccgccggtgt 42000
   ccaggaacga cctggaggca cgggtcaagg cactacaaca acgcttcaac agccgacggc 42060
   tgccgaccat cgactctacc gggacgttga ccttcggccg tcactcgatc gtcatctcca 42120
   acacccaggc ggagctgatg cggctgctca tcgaacgctt cggcgaagtc gtgtaccgca 42180
   tcgagttcgt gcagtgcctc gccatccgga cgcccgcgct gaacagaaac tcgctcgacc 42240
   tgcacatcat gcggctgcgc cgccggctcc taccgatcga cctggcgatc cggaccgtgt 42300
   ggggtcgggg ctacatgttg gcgtatgcgg cctccgccga ggccatcatc agaaacgcca 42360
   gcggcatgtg atgatgcctg ccgaaaacag ctcaaatcgc cgtaccgtcg agctgaaggt 42420
   tgcggtgcgt ggctaccgcc ggtccggcgg aagcagcgag gagcgcgtcc agcgcgccga 42480
   gccggccctc gcggacgaac cattcccggc cgtaggctgc ggcgaagcat gaggggtcga 42540
   ggtctgccgc cccgacctgg ctggcgtgcg tggccatcgc ggccatcttc gcgagcaaat 42600
   cggcaccttc cgcgtcgaca gcgagcgaga tccgggacgc cggcaccccc atgtccgtca 42660
   ggtcgccggc agcacgctgc aacacgtgac gtggccccgc gcgcagagct gaggcatcca 42720
   cggtggcctg gtatcccgcc agctgaaggc tgcgggtgac cagagcgccc acccggtgca 42780
   cctgaacgtg gtccacgtgc cggtagatgc cgccgggatc atagtgaacc agcgtggtcg 42840
   caccctcacg gctggcgatc cgggccacct gtcgggcgac cctcgtggcg gacgccgcgc 42900
   cgagggtgcc ggcccggtac gggccgccgt gcgcaccgga atcggcgtag cccagcacct 42960
   ccaatctggc tacgccgagc aggtcacagg cacgttcgag ttcggcgaga cggcggctgc 43020
   gcaaactctc gccgcgtgac aacggcacgt gtggaacacc ggcggcacca tcggtggcca 43080
   tgacgaggat cacgcgggcg ccagcgtcgg ccaggcgacg catggtcagc ccggtgaaga 43140
   ttgcctcgtc gtccgggtga gcgtgcagga cgatgaccgt gtgcgaggag agcatgatgt 43200
   gatccctttt gatcgcggcg gttgagcacg gtcgcgacga cagcgcgtcc gccacgcgct 43260
   gcgacgcacc ccgctcgaac cggaagccga agaagccgac gtgtggtgcc cgcgcgatgt 43320
   gctgcgcgga gaacggctag gtcatagcta ccgcacccca ctcgcgtggt ggtacgtcac 43380
   ggaggtgtct cgtgaactcc cacaagtgac cttcgaggtc acggaccagg tactggcgca 43440
   gtccccacgg cttgtcctgc aggggctgca cgatgtcggc acctgccgct gtggagcgca 43500
   tgaagtgctc gtcgacgtcg gagacccaga cgatgacctg cttgcaggtg tgcccgccgg 43560
   cgcagctgac cgtgtagggc tcgccggtac gccggaccat gacgatccca ccgccggtgt 43620
   ccagatcggc gtgtcggatg gtcccgatgg ccggctgccg gagcagcagg cggaacccga 43680
   agacccgtac cagccagtcg acagcacgat cggggtccgt gtaggcgagt tcgacgacca 43740
   cgtgagggaa gccgcgggcc gggtcgtaca cagagtctcc ttcgttcggc gagcagcttg 43800
   ccgacgtcaa cggttcgacg tcaccgtttc gggacagggt cttcggatgt cggaagttct 43860
   cgacatccct gtcgaggcag gctacccacg ttttgagaca ccagggcccg cggcgaagca 43920
   tctgcgaaag aggtgccaac cgtcccatcg gacgccgccg ctcgcggtgg aggcggtccc 43980
   ggccgccagg gactgcaaga cggtccggac gtcacggtgc gtgccggcgt gcagttctga 44040
   tccggccgat ctgaggcgga cggttgattg gttggcagga atcaagccgg agagtgtcgg 44100
   atctggcgga tccagccggt ccgcgacccc tcgcggaacc cgctgttcca ggtgggcctg 44160
   caactgctgg gcagcgccac ccagagcacc gagccgaacc tgcccgggct caccgtcacc 44220
   tcgctggacg ccaacccggg cgggcacccg ttcgacctgt cgatcacctg caccgagagc 44280
   cccgaccagc tgcggctggt cgccgactac tcggtcgacc tgttcgacga gccgcggatg 44340
   gcgcggctgc tgcggcacct ggaacgggtg ttgcggaccg ccgtcgacgc ccccgacacc 44400
   ccgctgtcgg cgctgccgtt gctcgacgag gaggagcaaa cgctgctgct gcacacctgg 44460
   cagggaccgc agggggagcg cgcccgcgaa ccggtgcacg tgcagatcgc gcggctggcc 44520
   gccgaacgac ccgacctggt ggcggccacc ctgcacggtg ccgagctgac ctacggcgag 44580
   ctggatcgcc gcgccggggt gctggcgcac cggttccggg cgctcggcgt gggccgggac 44640
   gacatcgtgg cgttgctgct ggaacggggc ttcgacctga tcgtcggcat ggtcgccgcg 44700
   cagaaggccg gcggcgcgtt cgtggtgatg gacccgggcc acccggtacg ccgcatcgag 44760
   ttcatcctcg aagacaccgc ggccaaggcg gtggtcaccc gcagcgcgct ggccgaccgg 44820
   ctgcccgagg gtgccgccgc cacgccggtg ctcgtcgaca cggagtggga cgagttgtcg 44880
   gcggcggccg ccggcgcggc gccgctggac gaactggccg acgagaactc gctggcctac 44940
   gtgctctaca cctcgggctc gaccggcaaa cccaagggcg tgatgatcga gcatcacgca 45000
   ctgaacacgt tcctgctctg gctcggcaac atcttcgact tcggccccgg cgaccggttg 45060
   ctgcaacaca tggcgcccat cttcgacttc gccgagggcg agatcttcac cgcgctgacc 45120
   cgtggcgtga cgatggtctt cgtaccggag gaacaccgca ccgacccgga cgtgatcggg 45180
   cagctgctgg tctcggagag gatcacctac atcggcggac cgccggccat cctgggccgc 45240
   atcccgccgg gcagctatcc cgacctcaag tacatgatcg ccggaggtga ggcggtcacg 45300
   ggtgatctca tcaaccgctg gaacaccccg ggtcgccggt tcatcaacgg gtacggaccg 45360
   accgaggcgg ccgtcggctg catcttctac gagtgcgagc accgcacctg gatcggccaa 45420
   ccgccgatcg ggcgggccat gcccaaccgg gtcgcctacg tgctggaccg gtggaacaac 45480
   ctgcaaccgg tgggggtgcc cggtgaggtg atcaccggcg gggagggtct cgcccggggc 45540
   tacctcaacc ggccggagtt gaccgccgag aagttcatcg acaacccgta ccggccgggg 45600
   gagcggatgt accgcaccgg cgacctgggc gtgtggaccg aggacgggca gatccagttc 45660
   ctcggccgca tcgacaccca ggtcaagctc aacgggctgc gcatcgagct ggaggagatc 45720
   gagtcgacgc tcaccgcgca tcccggcgtc gccgaggcgg cggtggcgct gcgcgaggac 45780
   agccccggca gcaagcgcct ggtcggctac gtggtcccgg ccggcgcggc ggcaccaccg 45840
   accgaggaac tgcgcgagca cctgctcgcc gacctgccgc cgtacatggt gccgcacgtc 45900
   ttcgtggccc tggacgtgct accgctgacc agcgtcggca aggtggaccg caaccggctc 45960
   ccggttcccg aggagaccgg gtcggccgag gtggagctgg tcgcgccgcg caccccggag 46020
   gaggagcggg tcgccgagat cttcgccgcg gtgctcggtg tcgagcgggt cggcgccgac 46080
   gcgaacttct tcgagctggg cggcaactcg ctgatggcgg cccgggtgct gtcccggatc 46140
   gccgaactcg gcggggccag cgcgaccatg cgggacttct acaccaagcc gcgggtcagc 46200
   gagatcgcgc agctgctcgc cggaacgccg tcgagcaccg acccggcgac cccgaccgac 46260
   gcggcaccgg tggccgacca gacccgggcc cggttggagc aggagatcgc cgacctggaa 46320
   cagcggctac gggccgcgcg ggcggccctc gccgggcagg acggtccggt gtccggcggg 46380
   cacgagggac agcggctgcc gctgtcaccg acgcaggagc agctgtggtt cctcgaacag 46440
   ctcgcacccg gccagccgac ctaccacatc ccggtgccgg tacggctcac cggaccgctc 46500
   gacgtcgacc tgctcggtcg ggcgctgacc gccgtggtgg cccgccacga ggcgctgcgc 46560
   accacgttcg gcaccgacga cgacaccccg taccaactgg tgtcacccgc cggtgaggtg 46620
   ccgctgccgg tgcacgagct ggacgcgacg gcagccgacg accgggagca ggcgctgcgc 46680
   gtcgagctgg aacgcgacgc caccgaaccg ttcgacctgg gtcgcgacct gatgctgcgg 46740
   gcccggctga tccgcctcga cggccaggag cacgtgctca gcatggtgtt gcaccacatc 46800
   tgcgccgacg gctggtcggc cgggctgctg gtgcgtgatc tcgccgagct gtacgacgcg 46860
   ttgcggcacg accggcagcc gcagctgccc gccctgcggt tgcagtacgc cgaccacgcg 46920
   ctggcgcagc gccggcagct cgacgccggt gacctggagt cgcagttggc gtactggcgg 46980
   cggcagctgg ccgacgcgcc catcctggac ctgcccagcg accggccccg cccggccacg 47040
   gccaccaacc gtggcgccct gctggtgcgt cggatgcccg ccgacgtgct ggagggcctg 47100
   cgccgggtgg cccagcagga gcaggccagc acgtacgcgg tgctgctcgc cggcttcaag 47160
   acggtgctga tgcgctacac cggcgtcgac gacgtggtgg tcggcaccgc cggcgccggt 47220
   cgcggccgtc cggagctgga ggatctggtc ggtttcttcg tcaacatgct ggccctgcgt 47280
   accgacctct ccggcgaccc gagcttcggt gaggcggtcc gccgcaccat gggcaccctg 47340
   ctggccgcct gggaccacca ggacgcgccg ttcgagcggg tcgtcgggcg gctcggtcga 47400
   cgccgcgacg ccagccgcaa cccggtcttc caggtctcca tcgatctgct cagcggcgac 47460
   ttggtggatt tccggttccc cgggctggcc gccgagttcc tcgacgtcga tcccggggtc 47520
   tcccgcttcg acatggcgat caacgcgtac gaggagaccg acggcatcac cttccggatc 47580
   gagtacgcca cggacctgtt cgacaccgcc cggatcgagg cgatgttcgg ccacctggag 47640
   cgggttctgc gggccgccgt ggcggcaccg cagacgccgc tgtcggccct gccactgctc 47700
   gacgacaccg agcgaaccga actgctggcg ctggcccagg ggaagcgacg gccccaccgg 47760
   ccggcgaccc tgccggagct gttcgcggcg agcgtggccc gcaccccgca ggccaccgcg 47820
   gtcatcgccg ccgagggcac gctgagctac gccgaactcg acgagcgcag ccgtcggctc 47880
   gccgcgcggc tgcgcgccgc cggagtgggc accggggacg tcgtgccggt gctgctggaa 47940
   cgcagcctcg cggaggtggt gagcgtgctg gccgtgctgc gggccggtgc cgcgtacgcg 48000
   ccgatggacc cggccgcgcc gccgagccgg ctggccttcc aggtgcgcga caccggcgcc 48060
   cgggtggtgg tgggcgaccc gacgcggctc gacacggtgg tcggggtgac catcgtggac 48120
   ccggcggcgg agctgccgga ggcggccgac gagccgtggc tggccgagct gacccccgct 48180
   tcgctggcgt acgtgctgta cacctccggt tccaccggcg agcccaaggg cgtgctcatc 48240
   gagcacggca tggtcgccgg ctacctggag tggatggtgc cggacttcgg cgtcgagcca 48300
   cggtcccgga tgatgcactg ctgcgcgccg gtgttcgacc tggccgtcgg tgaactgttc 48360
   ggtgccctca ccgccggtgc gaccgtggtg gtggccggtc acgacgacgt gcaccgcccg 48420
   ggcgcgttga ccgcgctggc ccgcgagacc ggcgcgacgc acatcttcac cactcccacc 48480
   gtgctgggtc tgctcgatcc ggccgaactg ccgcaactgc ggtcggtgat gatggccggc 48540
   gagccggcgc cgccgtcgct ggtggccgcc tggcacgtcg acggtcggcg ggtgctcaac 48600
   ctgtacggcc cggccgagac cacggtgggc tgcacctggt acgagtgccc ggccggcacc 48660
   gcacccgcgg cgacggtacc gatcggtcgt ccgatgccga accggcgggt gtacgtcgtc 48720
   gacgacagcg gcgacctggc gccggtcggt gtgcccggtg agctggtcgt cgccggcaac 48780
   ggggtggcgc gtggctatct cgaccgaccc gagctgacgg cctcggcgtt cggcgccgac 48840
   gagcgggccg gggagggacg ggcgtaccgc accggtgacc tggcccggtg gaaccgggac 48900
   ggccagttgg agttcctcgg ccgccgcgac gggcaggtca agctgcgtgg gctgcgggtc 48960
   gagctgggcg aggtcgagca tgccctgcgc acggcggccg gcgtgtccgg ggccgccgcg 49020
   gcggtgcacg gcacgggcgc cgaggcggtc ctggtcgggt acgtggtcgg cgagttgtcg 49080
   gacgacgacg cggaggcgct gcggacgacg ctcgccggga agctgcccgg gtacatggtg 49140
   cccaacgtgg tgctgcgcct gccgcagctg ccgctgaccc gtaccggcaa ggtggaccgc 49200
   aagcggttgc ccgcgcccga gtccggtgcc gggcaggcgg cgtacgtgcc gcccggcacc 49260
   gacgacgagc ggctcatcgc cggggtcttc gccgacctgc tcggtctgga acgggccggc 49320
   cgcgaggacg agttcttcct cagcggcggc aactcgctgc aggcgatccg ggcggtgggc 49380
   cggctcaacg acgcactcgg tgtgccgctg accgtgcggg acttctacgc ggcaccgacc 49440
   gtcgcggccc tggccgagct ggccggacgc cgacgggccg ccgagcaggc ggcgcagtgg 49500
   caactgctcg acgagatcga gcagctcagc gacgacgagg tggaacggct gctggcggcg 49560
   gactgatcgc cggtcgcgtc cgcggccccc gccgccgtgc gcggcggggc cggcggtgtc 49620
   ctgaaaggag tgagatgtcc gacgagacct tcgacggcag ggtagcccga ctggacgagc 49680
   gccggcggct gctgctggaa cggatgcggc aacagcgccg gcagccaccg gcgacgatcg 49740
   gcgcccgccc ggccggcgac gagcgggtgc cgctgtcgcg ggcccaggaa cagttgtggt 49800
   tcctcgccca gctcgcgccg gccgagccga cgtacaacct ggtgcaggcc tcgtacctga 49860
   ccggcgaggt ggaccccgcc gcgctgcggc gggcgttgga cggcgtggtc gcccagcacg 49920
   aggcgttgcg cacggtcgtg gagagcgtcg acggcgtggc gtaccagcgg gtgctgccgc 49980
   cggggccggc cagcctggtc gtcgacgacg tgtcccggct gcccgaggag cagcgccggc 50040
   cggcggcgct caccctgctc cagcagcagg aggtgcaccg cccgttcgac ctggccaccg 50100
   gcccgttgtt ccgggcccgg ctggtccggc tcggtcccac cgagcacgct ctcgccgtcg 50160
   cggcgcacca catcgtggtc gacggctggt cgatgggcct gatcctgcgg gagctggccg 50220
   ccgcgtacga cgccgaccgg cacggcacgc gagcgcggct ggacccgccc gccctccagt 50280
   acccggactt cgcccgctgg cagcatcagc agctggccac cgccgaactg gacaaccacc 50340
   tgaggtactg ggcgcagcgg ctggccgacc tgcccaccct ggaactgccg gccgaccgac 50400
   cccggccgac caccgcgtcg ttccggggtg cgctgctgga gcaaccactg gatccgcagc 50460
   tgcacgccgg ggtgcaggcg ttggccaagg agaccggcgc gagcccgttc atggtgctgg 50520
   tggcggcctt cgcggccatg ctcgcccgct acaccggcac cgacgacctg gccgtcggca 50580
   ccaccttcag cgggcgcagt cgtccggagc tggagagtgt ggtcgggttc ttcgccaaca 50640
   tgggggtgct gcgtctggac acctcgggcg accccagttt cgccaccctg gtcgaccggg 50700
   cccgggacac ctgcctgggc gcgtgggagc accaggacgc cccgttcgag cgggtggtgc 50760
   agcgactcgc cccaccccgc gacccgggcc gcaacccgct gttccaggtg gccgtgcagc 50820
   tgctggacgg ctccacctgg ggcggaccga cgctgcccgg caccgacagc accccggtcg 50880
   acctgcggct ggaccggtcc cgcttcgaca tgatggtcag cctcgtcgac cacggcgacc 50940
   ggtactcggt gctcaccgag tactccaccg acctgttcga cgcccggcgg gtacgcgcca 51000
   tgctcgacca cgtcgcccgg ctgctcgacg cgggcctggc cgacccggct acgacgctgt 51060
   cgcggttgcc gatgctcgac cccgccgaac gcgagcgggt cctggccctc ggcgcgggtg 51120
   aacgacgtga cgtggcccct gccaccgtcc accagctggt ccgggcgcag gccgaacgca 51180
   accccgacgc ggtggccgtc cggcacggcg atcacgagct gacctaccgg gagctgctgg 51240
   accgggccgc gcggcgggcg gccggcctgc gcgacgcggg cctgcgaccc ggcgatcggg 51300
   tgccggcgct gctggaccgg ggcgtcgacg aggtgctgac gccgatggcg atctggcacg 51360
   ccggtggggt gtacgtgccg ctggacaccg ccgccccgcc gaaccggctg cgccggatcc 51420
   tgaccaactg tgccgcccgg ctcgtgctca cccgcaccga gcacgccgcc ggggtgccgg 51480
   agggcccgtg gcggaccctc ctgctcgaca ccggcgacct ggactccgcc gccgggctcg 51540
   ccccggaacc gtccaccggc ccggacgacc tggcgtacgt gctgcacacc tcgggcacga 51600
   cgggtgaccc gaagggtgtc cagctgccgc accggggttt cgtcacctac ctggactgga 51660
   tgcgcgacca gtggcgctgc gggcccggtg accgggtgtt gcacggctgc gcgccggtgt 51720
   tcgacctggc ggcgggtgag gtgctggcgg cgctcacctc cggcgccacg gtggtggtga 51780
   tcggcaagga ccggctgctc tcgccgggcg gtctcgccga ggtcctggcg gccgaacgga 51840
   tcacgcacct gttcctgacc ccgaccacgc tcggtctggt cgaggacgac accgaccggt 51900
   tcgacgcgtt gcgggaggtg ctggtcggtg gcgaggtctg ccccgccgac ctggtggccc 51960
   ggtgggcgcg cccggggcgg cgtcggttcg tcaacctgta cggcccgacc gaggccaccg 52020
   tgggctgcct cgcccacgac tgcacggact ggtcggcggc caccccaccg ccgatcggcg 52080
   cgacgatgcc gaaccgccgc acctacctgg tcgaccgctg ggacaacccg gtcccggtcg 52140
   gggtgccggg ggagatcctg gtcggtggcg cggcgctgag caccggctac ctcaacgacg 52200
   acgagctgac cggacgccgg ttcaccgcgg atccgttcga tccgcaggga cgggtctacc 52260
   gcaccggcga tcgtggtcac tgggacgcgc agggccggct gcacttcggc ggccggctgg 52320
   acggccaggt caagttgcgc ggcctacgcg tcgaactcga cgagatcgag gcactgctga 52380
   gccggcactc cggggtggcc cgcgccgccg tgaccgtggt ccgcgacggc aacggggtgc 52440
   agcggctcgt cgggtacgtg gtggacgccg ccgagccggc cagcgacgcg gacctgcgca 52500
   cgcacctgag cacggagctg cccgcccacc tggtgcccgg ccggttcgtc cgactgcccg 52560
   ccctgccgtt gaccaccagc ggcaaggtca accggcgggc gctgccggca ccggaggcgg 52620
   cggacaccga cgacaccggc gcggccccca ccaccggggc cgagcgccag gtcgcggaga 52680
   tcttcgcgga ggtgctgggg gtggcgcggg tgggcgcgga gacgtccttc ttcgacctcg 52740
   gcgggcactc gttgcaggcg gcgcacgtgc tggcccgcat cgccggacgg atcggcacgt 52800
   cggtgtcgct caagcagttc tacgccaccc cgaccgtccg ggcggtggcc cggttcgtcg 52860
   agcacgggcc ggtggtggac gcctcgccac tcgtcacgct caaggccacc ggttcgcggc 52920
   cccggctcta ctgcccgcac gcggtctccg gctcgccgta ctggtacctc ccgctgagcc 52980
   gggccctgca tccggagcag ccggtggacg gcttcgaggc gccggggctg gagggtggca 53040
   tcgagccggt cgacgacctg gtgacgctcg ccgggcggta cgtcggcgcg ctgcgcgaac 53100
   ggcagccgca cgggccgtac ctgctcgccg gctggtcgat ggggggcttc ctctgcttcg 53160
   agatggcccg acagctcgcc gccgtgggtg ccgcaccgga cctcgtggtg atgatcgact 53220
   ccaatgaacc cggcccgttg ccgctgccga gcgagcgaca ggtgatggag accttcgtcg 53280
   ccgacctggc cgggctctcc gcggcaccgg caccgccgct ggacgccgag gtggtcgacg 53340
   cggccgaccc gatcgcggcg ctcaccgggt tcctggtccg gcacggactg atcccgtcgg 53400
   acgtgcgggc cgacttcgtc gccaaccggt acgcggtgtt ccgcgccaac atgcgggcca 53460
   tctacggcta ccaaccggca ccgtacgccg gccggacggt gctggtgcag gcggagcggg 53520
   aggccagccg cgcggcgtgg cgccggtgga gtccggcgat ggaggtggtg acgctgccgg 53580
   gcgaccacta ctcgatgtgg tccccgcagc acctgcccgc gctggcagcc ctcatcgacg 53640
   accaggtggc acgggtgctc gacctcggct gaccggcacc ggggcccgcc cagaccatcg 53700
   gctgggcggg ccccggacga gcatcgtcac cggcggcgag cctggtcacc tcggcgcgga 53760
   acgcgctccg gtcggcgcgg acgactcctc gaccgggccc gttgccggct cgacgtcgac 53820
   ctcaccgggg ggtgcgttgc ccggcgccgc cacggcctcg acctgctcgg cggcgtcgcc 53880
   gctcggcgcc gtctcagccg acacccgggt ggacagccgc caggtccggt aggccagcag 53940
   gaatcccagc atcagcagac cggcgtcgac ccagaggctg cggctcaacc ccagcgcgga 54000
   acggctcggg tcgttctcgg cggtcgccag gtagacgccg ccgacgacga ccacgctcac 54060
   cacgctggcc agcagcgttc cggtcgagac cagggccgac agggcggcgg cgtagcgggg 54120
   gcccacggtc gcggccgtct gctccacgag cgtgctgaaa ccggccgcca tccccgcacc 54180
   ggcgagcagc agcagcgccg acaccaccgc gatcggccag ccgccggcca ccacgaccgc 54240
   gagtgcggcc agggccaggg cgaacaccag caggccggcg accggcagcg ggcggtggaa 54300
   acgtttcggc agccgtgtcc aggtgaggct gacggccgcg aaacccacgg ggtacgggat 54360
   gaacatcaac ccggcctgca acggtgtgta gcccagcccg ttctgcacgt gcagggtgag 54420
   cggaaacagc accccggcga aggcgaagtt gaggatgcag caggccagca gtcccgactt 54480
   gacgcctcgc ggtcgcagcg catcgatctc cagcaccggc tgtccgcccc gggcgcccag 54540
   ccgcagctcg taccggacga agaggacgag cccggccgtg ccggcggcga tgctggccag 54600
   tgcccacgcg ggccagccct gttcccgccc gaagaccagc ggtgccacca ccgcgcccat 54660
   cgagacggcg agcagcagga tgccgacgat gtcgggccgg aaccgcgagc tgggcgtgtg 54720
   ccccggcaga taacgcggcg ccgccgccag cagggccagc ccgatcggca cgttgaccag 54780
   gaacgcggcc ctccagccgc tgcccagcac gtcggcgctg acgatcacgc cgccggcgat 54840
   ctgaccggcg gccactccga gcgccagcac catcgagtag agcccgatgg cgcgggcccg 54900
   ggccgacccg gtgaagacga tctggatcac cgagagcacc tgcggcacca tcatggcccc 54960
   gaacgccccc tgcagcaggc gggcggcgac cagcgtgccc gcggtcgggg cgatcccgca 55020
   gagcagcgag ctggcggtga agccggtcag cccgatcatg aacagccgcc ggtgccccac 55080
   gtcgccgccg agtcgcgcgc cggtgaccac gagcaccgcg aacatgagcg tgtatccgga 55140
   caggatcagt tgcagaacgg cgccggaggt ctggaggtca tccttgatgg tcttggtggc 55200
   cacggcgacg atggagttgt ccatcgtcgc catgacctgt gccaccagga ggatgagcag 55260
   caccagccgg gcgccccgtg gtcgaccttc gtcctcagtg gccatcgaca ctcctcgctc 55320
   tttctggttg tcacgcccgt tgccgtggta gaggcttccg tgccgacagt ggttgcgcaa 55380
   ctacgtgcgg ctgggtgtcg cgggcgtgcg ggcatcccgc ggatcgtcgt cgtcggagac 55440
   cgccggtcgg gtcgccggca caacgttgcc ggtcgccgac cacgccaccg acgccgggct 55500
   ttcggtgtcg gtggggccgg tgcctggcgc gggaccggtc cgggacaccg gcacagtgcc 55560
   gacgccgcga cgcgcgagcg gcccgaccga ggcacccaac tccggcccga acaccagcag 55620
   caggacggtg gcggccaggc acagcacacc cagtgcgatg gccggcgcga cgagcccgcc 55680
   gccgagggag agaatcagca gcatggcgga cggtccgagg atgccaccga ccttcgtggc 55740
   cgccccggcg aagccggagc cggtggcccg catcgcggtc gggtagatct cggcgctgta 55800
   cggcagcagc atcgcgttga ccgcggacaa cgacagcacc agcaggccga tggaggccag 55860
   cagcagcacc gtcccgccgc tgtcggtggt ccagagggcc actccgccga gcccgaccgc 55920
   ggttatcgcg atgaacagcg ccagcgcgcg gcggccgctc caggccgcgt agagcagcac 55980
   gaccagcgcg agggccgggg tggagtagag cgccgagtcg gccagcagcc cgctcgcgac 56040
   accgccggcc aggcccgcct cgcgcagctg cgcgggcagc atcaccagga agccgaagtt 56100
   caccaggccc cagacgacac cgaccacgcc gatcaccgat gtcacgaccg gatacgcccg 56160
   cagcatcgcc cccacgccgg gacgcgtcgc ggcggcggca gtgtcggcgg ccgggggcgt 56220
   ggcacccagg tgggacccgt agcggcggag caccgcctcc gcctcggtac gccgcccggc 56280
   gcgcagcagg tacagcggcg actcggggat caacggcgcc atcagcagca gcagcagtcc 56340
   ggagggcagc ccgatcaacc agagtgcgcg ccaggtcagc gccggctcca ggtggcgtgc 56400
   cgcctcgctg gccgccaggt aaccgccgag gccaccgatc ccaccgacgg tcaccgccac 56460
   ccagccgcgg tgccggcgtg gggtcagctc ggcgatcagc gtgaacacca ggggcaacag 56520
   gccaccggcg gaggtgccca tcgtgaagca catgacgagg ttccactcga aggtgggcat 56580
   ggccccgcac gccgaggtcg cgatgaacag cagggtggcc aggagcaggg cggtccgtcg 56640
   accgtaccgg tcgccgagca ggccccagac gaccgacccg accgcggtgc cggtgagggc 56700
   caccaggggc aggatcgaga ccgattcgag ggacatgccg tactcgtcgc tcatgccggg 56760
   catgacgaag ccgagggtca gcggcttcat gatgtcgacc accagggcga tggtcagcac 56820
   cgcgcaggtg atccagtacg cgggtcgcag ccgcccgtgc tcgatcgact ccacggtgac 56880
   gctcgcggag tccggtcggc ggacccccgc gaacagcgcc cgcccggcca ccaccaggcc 56940
   gacgacgagc agcagcatgc cgaggatcat cgtcggcgtc cacatgtcca tcggctcgcc 57000
   ggggctcatg ccctccatgc ccggcatgct gtccatgtcg tccatgtcgt ccatgccgtc 57060
   catgccgggc tcggccgacc ccatccgggc atggtcgatc atcatcttca cgtcgggcag 57120
   ttggaacagc acgccggccg tcaccaacgc cacgccgagc aggaatccga gtgtcccgcg 57180
   gcgtttcgcg gatgctgtgt cggcagtgcg ggattcggac atggttgacc gacctccgag 57240
   ctgtcgcgag acgagaagaa cgtcggcgaa ttggcgacca ccgaaatcta caagggtgct 57300
   tcccgggtga caaccgtcaa ttatatgact catttcggta agtggttatg aattgctgcc 57360
   tccgggtggc tcaccctgtt tgctaccgat ttggtgggct ctgatcggtc agtcagtcga 57420
   cgagcgttgc ccgatcgccc cgtgggatgt taggctcacc ctaagtgatc aactggcggt 57480
   cgccggtgct gtccaatggt ccgcttacca cagcttttcg gcaggtcagt gccgttatgg 57540
   cggatcgttg gtcgcgggtg acgcgcaccg cgtcgtcgga tccgccgggc ggtggcgctg 57600
   tccgcgccag gccggcacgg cgcagcggct gacccgactt cagcaattaa ccgagcgtat 57660
   tcttcagtga attgctggca atctctccgt aacgctccgg gccgcacgga aatgctaggg 57720
   tcgggagcga tgcccgccgc ccatcgcctt tcgactgagg tcgacggcgg gctggatcgg 57780
   gcgtgagctg cgccgtttct cgaatcgcta tccgaccatc cgtacgcgac cgcgaccccg 57840
   gcacgcgccg accggcccgg atcgatcccc tcggtcactg tcggtgccgc agatgtgccc 57900
   catgtggacc ggtcggcccg atccggcccg tgttgtgcca gcatggacta catgaccatc 57960
   cagtccgacc cgatggcgtc gacggacgag gtacgcggtg agccggccga cgagttcgtc 58020
   ggcgccctcg tctcgctgag ccgggtcatg gtggccgtcg ccatgcggac gctggcgcgc 58080
   atcgacgccg acgtgacgct ccagcagttc cggacgctcg tggtgctggt gcccggcccg 58140
   cagcgggtga tcgacctggc gaagctgctc ggcgtgcagt cgtcgaccgc cacccggatg 58200
   tgcgaccgac tggtccgcaa ggggctggtg gcacggcacg agcgggtggc ggaccgtcgg 58260
   gctgcctggg tcgggctgac cgtcgccggc aaggaactcg tcggcctggg catgcgagcc 58320
   cgacaacagg aactggagcg gatgctggcc gacctgcgac ccgacgacac cgacgtcacc 58380
   accggcgcgc tggtggcggt actgcgcgcc accggtgagc tcaccgacgg tgcctggtgg 58440
   cggcagtggg cggattcggt ggtggagtga cgcaacgccg tcggtcccga gtggccggcg 58500
   cagccggggc cggccgtcgg cgcggctggg gtcagacggt gacgcggctg gccgcacggc 58560
   ggcgggccat gggcatcgac ggcacctcgg cggccggacg cggtaggtcg gacggtgtca 58620
   cgtcgaccgg cgacacgccg gccgagacgg cgtcctgcga cgccgcgttc cggagttgat 58680
   cgagggcggc gtcgaggctg tcgaagatcg ggaaaacccc gtcgagcttc atcgtgtgca 58740
   gcacggtgcg gatgaacctc gacggcgcgg cgaggcacag ccgcccgcca cgctcgcgta 58800
   cgtcgcgatg cgcccggacg agcatgccca gcccgagcga gtcgatgacc tgcacctggg 58860
   tcaggtccac caccaccgtc ccggccacgt cgaacgcgcc gcgcagcgcg gcccgcagcg 58920
   cgtcgccggt gtcctggtct ccgccgtcgg cgggcaccac caccgccgcc tcggcgaggc 58980
   ccgatccagc ccggtcgccg tcaccgaggg agctgcccgg cggcagcgcg ctgcaccggg 59040
   ggcagcggtg cgggccggtg gcgaacggcg acccgctcca cccgtggtcg gagaccagcg 59100
   tccagacgac ctcggcgtcg ggcagcacgc aggcggtgcc ggtggtggtg tcaccacaga 59160
   cgtcgcagat cagggtcatc aggttgtcgt ccggtacgac ggtcatcctg cttcctctcc 59220
   ggtgccgcgg ccggtcggcc gcggtgcggg actcggggcg ggggcgggtg aggcaccctc 59280
   gccgtacgga ctcctcacag ggagtcggag cgttccttgc cggtggtggc gaccacgatc 59340
   accaccgcga ccgcggcgag cccggcctgc acgaccaggc ccaggatgct gaacccctga 59400
   acgccggcga gccaggcggt gatggcaccg agcaacgagg cgacggcacc gatggtcatg 59460
   gtgagccaca gcggcgcagc cggacggccg ggcaggacca gtcggccgag cgcgccgacg 59520
   gcgaggccga cggcgagggc cgtgacgatc ccggcgctgg tcatgctgct cctcgtgctc 59580
   gcggtcagat gcgtcggctg cggtgtgtca ggtgcgcacg gtgacggcgg cgcggccgta 59640
   cgcggcagcg cgcgggaccg tacccggtgc cggtgtcccg tcggccggcg ggacacccgg 59700
   tgcggcggtg accgcctcgg tgaccggggc cgaggcggcg tcggtgtgca ccggcaggat 59760
   ctggtccagc cgggcggtct gcaggatgcg ggtgatgcgc gggttcgggt tgcggacggc 59820
   cagcacgccc ccggcacgga ccatccggcg gtgcacgtcg agcagtagac cgatggcggc 59880
   ggcgtcgatg tgccggcaac ccgccaggtc gatcacgacc tgaccgggac gcagggccag 59940
   gagctgatcg aggaccgcgc cggtctccgg caggcagctc agatcgaact cggtgatgga 60000
   cacctccacc agcggcaccg aacactcggg gcgtcgtggt gcggtcacgg ccaaggcccc 60060
   ctctcctgcg gtctccgagt ggctctcacc ctgcccagcg gggttggcgg ccaccgcgcg 60120
   gacggatgac agttgcgtga caaaaccccc ggttgtcgat cgtggttggc ccggcggtca 60180
   gggcttgggg atgattccgg tatgacagcg gtactggtga tcgaggacga cgaccggatc 60240
   cggttggcgt tgctgctcgc tctggaggac gagggctacg aggcccgggg agcggccacc 60300
   gccgaggagg ggctgcgtac gcaacgccgg gatcccgccg actacgtcct ggtcgacctg 60360
   atgctgcccg gcatcgacgg cttcgagggc atccgccagt tgcgccgcga cgacgacgtg 60420
   ccgatcgtcg tggtcagcgc ccgcgacgac actcacgaca tcgtcgccgc cctcgaggcc 60480
   ggcgccgacg actacgtggt caagccggtg gcgatcaagg aactgaccgc tcggctgcgc 60540
   gccctgcgcc gacgcgggcg caccacgacc gcgtcggacg gcccgcagcc ggtgccggtg 60600
   ctcgccttcg gtgaccttga ggtgagcccc gaggcggggg aggtgcgccg cgccggtgag 60660
   ccggtcgcgg tcacccgcac cgagttccgg ctgctgtgcg agctggccga acacgccggc 60720
   cgggtgctct cccggcagca gctgctcagc cgggtctggg ggtacgacag cggcgacgaa 60780
   cggcttgtcg acgtgcacgt cggtcggctg cgccagaaga tcgaagccga tccggccaac 60840
   ccccgacatt tggtgacgct gcgcgggctg ggttacaaac tgcaacgatg agacgcctcg 60900
   gactgcgtgc ccgggtcacc gccgcgttcg cggtcggtgc gttgctgctg tccgcctcga 60960
   tggccctggt gtcgtacgag ctgacccgcc gcaccctgct cgacgagcgg gaacgcaccg 61020
   tgctgcgggc ggcgtactac gacgcggcgg tggtccgcgc cgggctcgac accgacaacc 61080
   cggacgtcgc ggaggcgttg cgggcgttgg acaccggcac cggacggcga cccctggtgc 61140
   tgctcgacgg cgagtggtac gcccgcagcg ccgacaacgg gctcaccacg atcgttccgg 61200
   cccggcttca ggacctggtc gcggcggggg agccggcggt gcagcgggtg cgcgtcaacg 61260
   acaccccggt gatgctggtc ggtgtgccgc tgtccgagtc gaccgccttc tacgagatcg 61320
   cctcgctgcg ggaactggaa cagaccttcc aggtgctggc gctggcgctc accgcggtcg 61380
   cgatcatgat cgccgggtcc ggggcggccc tcggctggta cgccacccgg cacgggctgc 61440
   gtccgctgac cgccgtcgcc gacgccgccg agcggatcgc ggccggcgac ttcaccaccc 61500
   gcctcgcccc cgacaccgac ccggacctga cccgtctgtc cacctccttc aaccggatgg 61560
   tcgacgagtt ggccgggcgg atcgaccggg accggcgttt cgccgccgac gtcagtcacg 61620
   aactgcggtc gccgttgcag accctcgccg ccgccgcgag tgtgctgtcc cggcgccggg 61680
   agaaccagga cgagcggacc gccaccgcgg cccggctcgt cgccgacgag atcgaccgtt 61740
   tccagcaact ggtgaacgac ctgctggacc tggcccgtgg tgaccagccg gtgcaccggg 61800
   agtcggtgga cctgcccgag ctggcccggc aggcgtgccg ggaccgggac ctgccggagt 61860
   cgatggtgca gttggcaacg gggacaccgc ccgggtggcg ggtggaccgt cgccgggtcg 61920
   cccaggtgtt ggccaacctg ctggacaacg ccgagcggta cggtgcggga ccggtcgcgg 61980
   tgcggctgtc ccacgacgcg gacacgggcg tgatcgaggt cgacgacgag ggcccgggag 62040
   tgccgatgga ggaccgcgag gcgatcttcg accgtttcgt ccgggggcgg gcggccaact 62100
   accgtggtgg cggcgacggc accggcctgg gcctggccct ggtggcccag cacgccgccg 62160
   cgcacggcgg agacgcgtcg gtcagcgacc gcccggaggg cggtgcccgg ttccgggtca 62220
   ccctgccggg gagcctgtca tgacccgccc cggcaccggc cgggccgcgc ggacggtcgg 62280
   ggtcgcggcg ctcgtcaccg tgttcgccct gctcggcgcg tgcggggtgc ccgccgagga 62340
   tcgaccccgc gcggtgacgc cgccgccggt gccgttcccg gcgaccgcct cggccgcgcc 62400
   gacggcggcg gagaccggcg cggtgaccga ggtgctctac ttcgcccgcg acgaacgcct 62460
   ggtgccggtg atccgccggg ccgaccaggt gcccgcactc gacgcgcagc tgcgggcgct 62520
   gctcgccggg ccgaccccga ccgagcgtga cgacgggctg accagtgccc tgccgggagc 62580
   cttcaccagc gcggtggtcg agctggtcga cgggttggcc cgggtcaccg tcgggctgac 62640
   ggcggtggac accggccgca gcgacggccg gctggcgtac ggtcagatcg tctgcacgct 62700
   caccgcccgc accgatgtca cggcggtgct gttcctggaa ggcggcacgc cgctgagcgt 62760
   cccccgggcg gacggctcgc tgtcgtcggg accgctcacc gctgccgact acgccgtgtt 62820
   gatcaatcct cgttgacgag ggccggacca cccgacgcac gcgcagcgcg acgactcgta 62880
   tcctttccgc gaaccgtcgt gccgacgacc ggaaaggact tggtgtgaac gaccgggccg 62940
   agacgttgga gttccaggcc gaagcgcgtc agctgctccg gctgatggtc cactcgatct 63000
   actcgaacaa ggacgtcttc ctccgcgagt tgatctcgaa cgcctcagac gcgctggaca 63060
   agctgcgcct ggcgtcgatg gtcgacaagg acctcgccgt cgacaccggc gacctgcaca 63120
   tcgccatcga ggtcgaccgg gacgcccgca ccctcaccgt gcgggacaac ggcatcggca 63180
   tgagccgcga cgaggtggtc tccgtcatcg gcaccatcgc caagtccggc accgcggagc 63240
   tgctgcggca gctgcgggag gccaaggacg ccggcgcctc acaggagctg atcggtcagt 63300
   tcggcgtcgg cttctacgcc gcgttcatgg tcgccgagcg ggtcgagctg gtcacccgac 63360
   gggccggcga gaccggtggt acccgctggg agtccaccgg cgagggcacc tacaccatca 63420
   ccgagctgga cgacgccccc cagggcacgg cggtgaccct gcacctcaag cccgccgacg 63480
   ccgaggacaa cctgcacgac tacaccgccg aatggacgat ccgggggatc gtcaagcggt 63540
   actccgactt catcgcccac ccgatccgga tgaccgtcga gcggcccggc accgacgacg 63600
   cccccgccac caccgaggcg gtgacgctca actcgatgaa ggcgctgtgg gcccgcccgc 63660
   gcggtgaggt cgagcccgcc gagtaccacg agttctacaa gcacgtcagc cacgactggg 63720
   cggacccgct ggagaccatc cacatgcggg gggaggggac cttcgagtac gaggcgctgc 63780
   tgttcatccc cacccacgcg ccgctggacc tgttctcccc ccagggccgc cgaggggtgc 63840
   agctctacgt caagcgcgtg ttcatcatgg acgactgcga cgcgctgatg cccaactacc 63900
   tgcggttcgt caagggtgtg gtggacgccc acgacctgtc gctgaacatc tcccgggaga 63960
   tcctccagca ggaccgccag atccgcgccg tccgccgtcg cctggtcaag aagatcctcg 64020
   ccacggtcaa ggatctcaag accggccagc cggagcgcta ccgcaccttc tggaccgagt 64080
   tcgggacggt ggtcaaggaa ggtctgctgg aggacaccga caaccgggac accctgctgg 64140
   agatcctctc ggtggcctcc accaacgacc cggccgagca gaccgacctg gccggctacg 64200
   tggaacgcat gcgcgagggc cagcaggaca tctactacgc caccggcgag aaccggagca 64260
   ccatcgagaa ctccccgcac attgaggcgt tccgcgccaa gggctacgag gtgctgctgc 64320
   taaccgaccc ggtcgacgag gtttgggtgg agcgggtcgg cagctacgcg ggcaagacgc 64380
   tgcgctcggt cgccaagggg caggtcgacc tggacaccga gcaggagcgc accgaggccg 64440
   aagccgaacg ggagcggcag cgcaccgagt acgccgacct gctcggctgg atgggcgagg 64500
   tgttggccga caacgtcaag gaggtccggc tgtcgtcgcg gctgaccacc tcgccggcct 64560
   gcgtggtcgg cgacgcgcac gacctcacgc ccaccctgga gaagatgtac cgcgcgatgg 64620
   ggcaggaggt gccgaaggtc aagcggatcc 64650

<210>  2
<211>  178
<212>  PRT
<213>  Micromonospora sp., ML1 strain

<220>
<223>  ORF1 Protein

<400>  2
Ile Pro Ser Arg Glu Trp Gln Pro His Ser Cys Gly Pro Gly Ala Lys
1               5                   10                  15

Gly Pro Arg Glu Tyr Leu Trp Ala Trp Ile Thr Thr Ala Thr Ser Ala
            20                  25                  30

Gly Glu His Arg Trp Leu Leu Met Arg Arg Asn Arg Ser Thr Gly Glu
        35                  40                  45

Leu Ala Phe Tyr Leu Cys Trp Ser Pro Arg Pro Ala Pro Leu His Thr
    50                  55                  60

Leu Val Thr Val Ala Gly Ser Arg Trp Ser Ile Glu Glu Leu Phe Gln
65                  70                  75                  80

Ser Gly Lys Gly Gln Val Gly Leu Asp His Tyr Gln Val Arg Gly Trp
                85                  90                  95

Thr Gly Trp His Arg His Val Thr Leu Ala Met Leu Ala Leu Ala Val
            100                 105                 110

Leu Thr Ile Leu Ala Ala Arg Gln Pro Asp Pro Asp Pro Glu Ile Ile
        115                 120                 125

Ala Leu Thr Val Ala Glu Ile Arg Gly Leu Leu Asn Ala Phe Val Leu
    130                 135                 140

Ala Val Thr Leu Pro Pro Ala His Thr Leu His Trp Ser Thr Trp Arg
145                 150                 155                 160

Arg Thr Ser Gln Ala Arg Ala Arg Arg Ala His Tyr Gln Arg Arg Gln
                165                 170                 175

Ala Lys

<210>  3
<211>  46
<212>  PRT
<213>  Micromonospora sp., ML1 strain

<220>
<223>  ORF2 Protein

<400>  3
Met Arg Ser Cys Gly Arg Arg Leu Ala Arg Val Thr Arg Ile Gly Asn
                5                   10                  15

Pro Arg Gln Gly Leu Gln Ala Pro Arg Gly Gln Gln Ser Val Ser Arg
            20                  25                  30

Ala Gly Arg Gly Met Leu Thr Gly Tyr Val Asp Gln Arg Arg
        35                  40                  45


<210>  4
<211>  205
<212>  PRT
<213>  Micromonospora sp., ML1 strain

<220>
<223>  Tio3 Protein


<400>  4
Met Ser Val Ser Gly Ser Arg Arg Ser Glu Gly Ser Tyr Arg Arg Ser
                5                   10                  15

Gly Gly Cys Ala Gln Gly Arg Ser Pro Arg Pro Ala Arg Glu Asp Val
            20                  25                  30

Lys Phe Val Arg Trp Pro Ala Glu Arg His Leu Arg Asp Leu Cys Arg
        35                  40                  45

Gln Glu Arg Ile Pro Cys Leu Leu Val Val Glu Gly Gly Ala Glu Pro
    50                  55                  60

Pro Val Cys Ser Asp Pro Arg Glu Asp Trp Val Arg Thr Pro Ile Ser
65                  70                  75                  80

Pro Ser Asp Leu Asp Ala Arg Val Thr Ala Leu Arg Gln Arg Ser His
                85                  90                  95

Asp Arg Arg Thr Pro Val Leu Asp Pro Ala Gly Thr Leu Ser Phe Arg
            100                 105                 110

Thr Ala Ser Val Val Met Ser Ser Thr His Leu Glu Leu Met Asp Leu
        115                 120                 125

Phe Val Glu Arg Phe Gly Gln Val Val Tyr Arg Trp Glu Leu Met Thr
    130                 135                 140

Ile Leu Thr Lys Arg Asp Ser Thr Ser Thr Arg Asn Ala Leu Asp Leu
145                 150                 155                 160

His Ile Met Arg Met Arg Lys Arg Ile Ala Pro Leu Asp Leu Asp Ile
                165                 170                 175

Val Thr Ala Trp Gly Arg Gly Tyr Ala Leu Glu Glu Lys Ser Ser Trp

180                 185                 190

Ala Ala Val Ala Ser Lys Pro Ala Ala Val Asp His Cys
        195                 200                 205


<210>  5
<211>  223
<212>  PRT
<213>  Micromonospora sp., ML1 strain

<220>
<223>  Tio4 Protein

<400>  5
Met Ser Ala Gly Ser Arg Ser Ser Arg Asn Ala Val Ala Ala Leu Arg
                5                   10                  15

Val Val Pro Ala Ala Thr Ala Gly Val Pro Lys Gln Arg Pro Ala Glu
            20                  25                  30

Pro Ala Gln Val Met Leu Thr Arg Val Gly Leu Arg Leu Pro Glu Arg
        35                  40                  45

Met Ser Phe Glu His Trp Glu Arg Thr Gly Ala Gln Leu Ser Gly Ile
    50                  55                  60

Val Asp Ser Ser Ser Trp Cys Leu Gly Asp Trp Leu Val Tyr Gly Lys
65                  70                  75                  80

Glu Gln Tyr Ala Asp Arg Tyr Arg His Val Val Gln Ala Val Gly Leu
                85                  90                  95

Asp Tyr Gln Thr Leu Arg Asn Tyr Ala Trp Val Ala Gly Arg Phe Pro
            100                 105                 110

Leu Ala Arg Arg Arg Ser Gly Leu Ser Phe Gln His His Ala Glu Val
        115                 120                 125

Ala Ala Met Ser Pro Asp Asp Gln Asp Arg Trp Leu Asp Glu Ala Glu
    130                 135                 140

Thr Gln Met Trp Ser Val Lys Gln Leu Arg Ala Ala Ile Lys Ala Thr
145                 150                 155                 160

Arg Ser Asp Arg Thr Thr Glu Thr Ile Lys Thr Ala Leu Met Pro Arg
                165                 170                 175

Ile Pro Ile Pro Asp Arg Arg Leu Thr Arg Trp Arg Gln Ala Ala His
            180                 185                 190

Gln Ser Ser Val Glu Leu Asp Gln Trp Ile Val Ser Thr Leu Asp Arg
        195                 200                 205

Ala Ala Ala Glu Thr Leu Asp Lys Thr Thr His Asp Glu Thr Glu
    210                 215                 220

<210>  6
<211>  274
<212>  PRT
<213>  Micromonospora sp., ML1 strain

<220>
<223>  Tio5 Protein

<400>  6
Met Val Ser Thr Ser Leu Lys Pro Arg Leu Asp Ala Ala Ala Ala Gln
                5                   10                  15

Gln Gln Thr Gly Leu Ser Trp Trp Leu Ser Asp Ala Trp Gln Met Ala
            20                  25                  30

Leu Arg Asn Leu Arg His Leu Thr Arg Ser Pro Glu Leu Val Met Phe
        35                  40                  45

Ser Leu Val Gln Pro Val Met Phe Ile Leu Leu Phe Ala Tyr Val Phe
    50                  55                  60

Gly Gly Ala Ile Asn Val Glu Gly Gly Asn Tyr Pro Gln Phe Leu Leu
65                  70                  75                  80

Pro Gly Ile Leu Val Gln Met Val Leu Phe Gly Ser Val Ala Gly Thr
                85                  90                  95

Thr Ile Gly Ile Ser Thr Asp Met Asp Arg Gly Leu Met Asp Arg Phe
            100                 105                 110

Arg Ser Met Pro Met Ser Arg Ser Ala Val Leu Val Gly Arg Thr Leu
        115                 120                 125

Ser Glu Ile Leu Arg Asn Val Val Ser Met Leu Val Val Val Leu Met
    130                 135                 140

Gly Leu Leu Ile Gly Phe Arg Phe Gln Gly Gly Val Leu Ser Ala Leu
145                 150                 155                 160

Gly Gly Leu Ala Leu Leu Leu Leu Phe Gly Tyr Ala Leu Thr Trp Val
                165                 170                 175

Gly Ala Met Ile Gly Leu Ser Val Pro Asn Pro Glu Ala Ala Gln Ser
            180                 185                 190

Ala Gly Leu Ile Trp Ile Phe Pro Phe Ser Leu Ile Ser Ser Ala Phe
        195                 200                 205

Val Pro Thr Glu Ser Met Pro Thr Trp Leu Arg Val Tyr Ala Asp Ala
    210                 215                 220

Ser Pro Met Thr Ala Thr Val Asn Ala Leu Arg Asp Leu Phe Asn Gly
225                 230                 235                 240

Asn Pro Pro Gly Leu Asp Val Leu Leu Thr Val Ala Trp Ser Val Leu
                245                 250                 255

Leu Ile Thr Ile Phe Ala Pro Leu Ala Thr Arg Lys Tyr Ser Leu Arg
            260                 265                 270

Ser Arg


<210>  7
<211>  328
<212>  PRT
<213>  Micromonospora sp., ML1 strain

<220>
<223>  Tio6 Protein


<400>  7
Met Pro Ala Ala Ile Glu Val Ala Gly Leu Cys Lys Arg Phe Gly Ser
                5                   10                  15

Val Thr Ala Leu Asp His Leu Glu Leu Thr Val Pro Glu Gly Lys Val
            20                  25                  30

His Gly Leu Leu Gly Pro Asn Gly Ala Gly Lys Thr Thr Thr Val Arg
        35                  40                  45

Val Leu Thr Thr Leu Leu Arg Pro Asp Glu Gly Lys Ala Glu Ile Phe
    50                  55                  60

Gly Phe Asp Val Arg Arg Asn Ala Leu Gln Val Arg Ser Ile Ile Gly
65                  70                  75                  80

Leu Thr Gly Gln Tyr Thr Thr Val Asp Asp Met Leu Thr Gly Arg Glu
                85                  90                  95

Asn Leu His Met Val Gly Lys Leu Phe Arg Met Ser Thr Pro Asp Ile
            100                 105                 110

Lys Val Arg Ala Thr Glu Leu Leu Glu Arg Phe Asp Leu Thr Glu Ala
        115                 120                 125

Ala Gly Arg Gln Ala Lys Thr Tyr Ser Gly Gly Met Arg Arg Arg Leu
    130                 135                 140

Asp Ile Ala Ala Thr Leu Met Ser Arg Pro Gln Leu Ile Phe Leu Asp
145                 150                 155                 160

Glu Pro Thr Ala Ala Leu Asp Pro Arg Ser Arg Met Asp Val Trp Arg
                165                 170                 175

Ile Val Asn Glu Leu Val Glu Glu Gly Thr Thr Val Leu Leu Thr Thr
            180                 185                 190

Gln Tyr Leu Glu Glu Ala Asp His Leu Ala Asp Ser Leu Ser Val Ile
        195                 200                 205

Asp Ser Gly Lys Val Ile Ala Ser Gly Ala Pro Asp Glu Leu Lys Ala
    210                 215                 220

Glu Val Gly Gln Ala Trp Ala Asp Val Thr Leu Ile Asp Ala Ala Ala
225                 230                 235                 240

Val Glu Lys Thr Thr Gln Ile Leu Arg Glu Arg Leu Gly Thr Glu Pro
                245                 250                 255

Val Gly Asp Pro Glu Gly Met Gln Val His Ala Pro Leu Pro Glu Gly
            260                 265                 270

Met Thr Leu Asn Glu Leu Leu Asn Pro Leu Arg Asp Ser Gly Ile Ala
        275                 280                 285

Ile Ala Asp Val Ala Val Arg Arg Pro Thr Leu Asp Asp Val Phe Leu
    290                 295                 300

Ala Leu Thr Gly His Thr Ala Thr Ala Arg Ala Ser Glu Asp Val Asn
305                 310                 315                 320

Ser Lys Lys Lys Gly Gly Lys Arg

                325

<210>  8
<211>  236
<212>  PRT
<213>  Micromonospora sp., ML1 strain

<220>
<223>  Tio7 Protein

<400>  8
Met Tyr Phe Arg Pro Ala Asp Leu Ala His Glu His Gly Leu Ser Pro
                5                   10                  15

Gln Ser Val Arg Asn Tyr Glu Arg Asp Gly Leu Ile Pro Pro Ala Arg
            20                  25                  30

Arg Thr Glu Ser Gly Tyr Arg Arg Tyr Thr Glu Lys His Ala Ala Ala
        35                  40                  45

Leu Arg Ala Tyr Arg Ala Leu Ile Pro Ala His Gly Tyr Ala Glu Ser
    50                  55                  60

Gly Ala Ile Met Arg Asp Ile Thr Ala Gly Arg Leu Asn Glu Ala Leu
65                  70                  75                  80

Thr Thr Ile Asp Arg Ser His Ala Glu Leu Leu Arg Asp Arg Gly Thr
                85                  90                  95

Leu Asp Ala Val Ala Glu Val Leu Thr His Leu Thr Gly Arg Ser Thr
            100                 105                 110

Gly Thr Trp Arg Ala Pro Lys Ser Ile Gln Pro Tyr Ser Ile Gly Glu
        115                 120                 125

Leu Ala His Arg Leu Gly Val Asn Val Ala Thr Ile Arg Lys Trp Glu
    130                 135                 140

Thr Ser Gly Val Leu Ala Pro Val Arg Arg Pro Glu Thr Gly His Arg
145                 150                 155                 160

Met Tyr Asp Ala Ser Asp Val Arg Asp Ala Glu Leu Ala His Phe Leu
                165                 170                 175

Arg Arg Gly Arg Tyr Pro Leu Glu Leu Ile Ala Thr Val Val Gln Gln
            180                 185                 190

Val Arg Ala Ala Gly Asp Thr Gln Ala Leu Ala Ala Ala Leu Ala Asp
        195                 200                 205

Trp Arg Gln Arg Val Thr Thr Arg Gly Leu Ala Met Leu Asn Ala Ala
    210                 215                 220

Ala Gln Leu Ala Ser Tyr Leu Ala Met Glu Glu Asn
225                 230                 235

<210>  9
<211>  242
<212>  PRT
<213>  Micromonospora sp., ML1 strain

<220>
<223>  Tio8 Protein

<400>  9
Met Thr Thr Leu Thr Tyr Ala Asp Tyr Leu Gln Val Asp Thr Leu Leu
                5                   10                  15

Ser Leu Gln Glu Thr Arg Ala Pro Asp Thr Ala Ser Arg Ser Val Val
            20                  25                  30

Leu Ala Glu His Phe Phe Ile Val Thr His Gln Ala Cys Glu Leu Trp
        35                  40                  45

Leu Lys Gln Val Thr Tyr Asp Leu Asp Ala Ala Ala Asp Ala Met Thr
    50                  55                  60

Pro Pro Tyr Gly Pro Glu Asp Glu Glu Leu Ala Leu Glu Phe Leu Gln
65                  70                  75                  80

Arg Thr Ala Glu Leu Leu Arg Val Leu His Glu Gln Val Val Ala Leu
                85                  90                  95

Glu Arg Leu Pro Leu Arg His Phe Ala Glu Phe Arg Pro Phe Leu Asp
            100                 105                 110

Gly Ala Ser Gly Ala Gln Ser Ala Gln Phe Arg Leu Leu Ala Arg Leu
        115                 120                 125

Leu Gly Gly Glu Arg Gln Glu Gly Arg Leu Tyr Arg Ala Phe Glu Arg
    130                 135                 140

Leu Thr Glu His Asn Gly Ile Ser Val Ala Glu Val Cys Ala Arg Gly
145                 150                 155                 160

Pro Gly Ala Gly Val Leu Asn Arg Ile Ala Asp Ser Leu Leu Asp Val
                165                 170                 175

Ala Asn Gly Tyr Trp Arg Trp Lys Met Thr His Leu Ala Leu Val Ser
            180                 185                 190

Lys Met Val Gly Asn Arg Asn Gly Thr Gly Gly Ser Thr Gly Val Asp
        195                 200                 205

Tyr Leu Ala Arg Arg Ile Thr Leu Pro Phe Ala Gln Leu Arg Gln Leu

210                 215                 220

Arg Ala Gln Val His Asp Ala Leu Val Trp Glu Gln Pro Thr Ala His
225                 230                 235                 240

Thr Gln


<210>  10
<211>  421
<212>  PRT
<213>  Micromonospora sp., ML1 strain

<220>
<223>  Tio9 Protein

<400>  10
Met Pro Leu Pro Ser Pro Tyr Pro Asn Leu Leu Asp Gly Pro Ser Val
                5                   10                  15

Ala Ser Thr Ile Pro Ser Ser Glu Leu His Arg Val Phe Arg Asp Ala
            20                  25                  30

Gly Glu Arg Glu Arg Ile Ser Gly Val Pro Val Thr Lys Leu His Val
        35                  40                  45

Gly Glu Pro Tyr Phe Thr Pro Pro Pro Glu Val Ala Gln Ala Leu Ala
    50                  55                  60

Glu Ala Val Thr Ala Gly Arg Thr Asp Tyr Thr Ala Val Glu Gly Leu
65                  70                  75                  80

Leu Glu Leu Arg Glu Arg Leu Val Glu Lys Leu Ala Asp Gln Asn Gly
                85                  90                  95

Val Asp Thr Asp Val Ser Arg Leu Phe Val Thr Pro Gly Ser Cys Gln
            100                 105                 110

Gly Leu Ala Ala Leu Met Gln Ala Val Ala Glu Pro Asp Ala Glu Ile
        115                 120                 125

Leu Leu Pro Glu Leu His Trp Pro Ile His Leu Gln Gln Val Leu Leu
    130                 135                 140

Ala Gly Leu Arg Pro Val Phe Tyr Pro Leu Gly Arg Asp Tyr Arg Thr
145                 150                 155                 160

Asp Pro Glu Ser Val Leu Ala Ala Ser Thr Ala Asn Thr Arg Val Leu
                165                 170                 175

Leu Ile Asn Ser Pro Ala Asn Pro Thr Gly Ala Val Leu Asp Thr Ala
            180                 185                 190

Thr Leu Ser Thr Leu Ile Asp Met Ala Arg Ala Arg Gly Trp Gln Val

195                 200                 205

Ile Ser Asp Glu Ala Tyr Glu His Phe Val Phe Asp Asp Glu His Val
    210                 215                 220

Ser Thr Ala Ser Leu Glu Trp Asp Val Pro Val Ser Glu Arg Ile Val
225                 230                 235                 240

His Ser Ser Tyr Ser Phe Ser Lys Ser Leu Ala Met Thr Gly Tyr Arg
                245                 250                 255

Leu Gly Tyr Val Ser Thr Ala Asn Asp Arg Thr Ala His Ala Phe Arg
            260                 265                 270

Ile Val Gln Glu Ala Asn Leu Ile Ala Met Ser Thr Pro Val Gln Tyr
        275                 280                 285

Ala Gly Leu Glu Ala Leu Arg Leu Gly His Val Val Arg Glu Asn Arg
    290                 295                 300

Ala Met Val Leu Ser Asn Arg Asp Ala Val Leu Pro Ala Val Arg Asp
305                 310                 315                 320

Ala Gly Leu Leu Pro Glu Leu Pro Ala Gly Gly Trp Tyr Ala Val Leu
                325                 330                 335

Asp Val Ala Pro Thr Gly Leu Thr Ala Glu Glu Phe Ala Thr Gly Leu
            340                 345                 350

Leu Glu Thr Arg Asn Val Ala Val Val Pro Gly Thr Gly Phe Ala Leu
        355                 360                 365

Met Pro Arg Leu Asp Ala Arg Gly Arg Val Ile Thr Val Glu Pro Ala
    370                 375                 380

Pro Trp Ser Arg His Leu Ile Arg Ile Ala Met Cys Val Ala Pro Ser
385                 390                 395                 400

Val Leu Arg Ser Gly Val Arg Glu Leu Leu Glu Phe Ala Arg Glu Arg
                405                 410                 415

Pro Gly Thr Ala Arg
            420

<210>  11
<211>  264
<212>  PRT
<213>  Micromonospora sp., ML1 strain

<220>
<223>  Tio10 Protein

<400>  11
Val Ser Pro Thr Glu Ala Glu Ala Trp Pro Ala Val Ala Glu Arg Gly

5                   10                  15
Asp Gly Leu Ala Gly Arg Thr Val Ile Val Thr Gly Ala Gly Thr Gly
20                  25                  30

Leu Gly Arg Ala Ile Gly Glu Arg Leu Leu Ala Arg Asp Ala Asn Val
            35                  40                  45

Val Ala Val Gly Arg Arg Glu Ala Ala Leu Arg Glu Leu Thr Gly Asp
    50                  55                  60

Gly Gly Gly Gln Val Ala Thr Val Ala Ala Asp Ile Thr Glu Pro Gly
65                  70                  75                  80

Ala Ala Glu His Val Val Ala Ala Ala Thr Thr Arg Phe Gly Thr Val
                85                  90                  95

His Gly Leu Val Asn Asn Ala Gly Leu Ala Arg Phe Ala Thr Leu Asp
            100                 105                 110

Thr Ala Ser Asp Glu Asp Phe Asp Arg Leu Leu Ala Thr Asn Val Arg
        115                 120                 125

Ala Pro Ala Ala Leu Ile Arg Ala Ala Leu Pro His Leu Arg Glu His
    130                 135                 140

Arg Gly Ala Val Val Asn Val Ser Ser Val Gly Ala Val Leu Ala Met
145                 150                 155                 160

Pro Gly Arg Ser Phe Tyr Gly Ala Ser Lys Ala Ala Leu Asn Ser Leu
                165                 170                 175

Thr Arg Ser Leu Ala Arg Glu Leu Ala Pro Asp Val Arg Val Asn Ala
            180                 185                 190

Val Leu Pro Gly Pro Val Asp Thr Pro Met Trp Asp Asp Met Gly Leu
        195                 200                 205

Asp Ser Val Lys Ser Ala Leu Leu Arg Ser Ser Leu Leu Ala Ala Thr
    210                 215                 220

Pro Leu Gly Arg Phe Gly Gln Pro Ala Glu Ile Ala Gly Trp Val Cys
225                 230                 235                 240

Gly Leu Leu Asp Pro Glu Phe Ser Gly Trp Ile Thr Gly Ala Leu Ile
                245                 250                 255

Pro Val Asp Gly Gly Arg Thr Ser
            260

<210>  12
<211>  404
<212>  PRT
<213>  Micromonospora sp., ML1 strain

<220>
<223>  Tio11 Protein

<400>  12

Met Ser Ser Pro Thr Ala Ser Thr Ser Ala Leu Asp Leu Thr Asp Pro
                5                   10                  15

Thr Thr Phe Val Arg His Asp Thr His Val Phe Trp Ala Glu Val Arg
            20                  25                  30

Asp His Asn Pro Val Tyr Trp Tyr Gln Gly Arg Glu Asp Arg Pro Gly
        35                  40                  45

Phe Trp Val Val Ser Arg Tyr Val Asp Val Val Ala Ser Tyr Thr Asp
    50                  55                  60

Ala Ala Arg Leu Ser Ser Ala Arg Gly Thr Val Leu Asp Val Leu Leu
65                  70                  75                  80

Arg Gly Glu Asp Ser Ala Gly Gly Arg Met Leu Ala Val Thr Asp Arg
                85                  90                  95

Pro Arg His Arg Glu Leu Arg Asn Val Met Leu Arg Ala Phe Ser Pro
            100                 105                 110

Arg Val Leu Gly Arg Val Val Glu Gln Val His Arg Arg Ala Asp Glu
        115                 120                 125

Leu Ile Arg Arg Val Thr Gly Thr Gly Ala Phe Asp Phe Ala Thr Glu
    130                 135                 140

Val Ala Glu Ser Ile Pro Met Gly Thr Ile Cys Asp Leu Leu Ser Ile
145                 150                 155                 160

Pro Pro Ala Asp Arg Pro Asp Leu Leu Arg Trp Asn Lys Asn Ala Leu
                165                 170                 175

Ser Ser Asp Glu Ala Asp Ala Asn Leu Tyr Ala Ala Leu Glu Ala Arg
            180                 185                 190

Asn Gln Ile Leu Leu Tyr Phe Met Asp Leu Ala Glu Gln Arg Arg Ala
        195                 200                 205

Ser Pro Gly Asp Asp Val Ile Ser Met Ile Ala Thr Ala Thr Val Gly
    210                 215                 220

Gly Glu Pro Leu Ser Ile Asp Asp Val Ala Leu Asn Cys Tyr Ser Leu
225                 230                 235                 240

Ile Leu Gly Gly Asp Glu Ser Ser Arg Met Ser Ala Ile Cys Ala Val
                245                 250                 255

Lys Ala Phe Ala Asp Phe Pro Asp Gln Trp Arg Ala Val Arg Asp Gly
            260                 265                 270

Asp Val Ala Ile Asp Thr Ala Val Glu Glu Val Leu Arg Trp Ser Thr
        275                 280                 285

Pro Ala Met His Phe Ala Arg Thr Ala Thr Thr Asp Phe Glu Leu Arg

290                 295                 300

Gly Gln Gln Val Arg Ala Gly Asp Ile Val Thr Leu Trp Asn Leu Ser
305                 310                 315                 320

Ala Asn Phe Asp Glu Arg Glu Phe Asp Arg Pro Tyr Arg Phe Glu Val
                325                 330                 335

Gly Arg Thr Pro Asn Lys His Leu Ser Phe Gly His Gly Pro His Phe
            340                 345                 350

Cys Leu Gly Ala Tyr Leu Gly Arg Ala Glu Leu Gln Ala Leu Leu Thr
        355                 360                 365

Ala Leu Val Gly Thr Val Ser Arg Ile Glu Ser Ala Gly Ser Pro Arg
    370                 375                 380

Arg Val Tyr Ser Asn Phe Leu Asn Gly His Ser Ser Leu Pro Val Ala
385                 390                 395                 400

Phe Thr Gly Arg


<210>  13
<211>  523
<212>  PRT
<213>  Micromonospora sp., ML1 strain

<220>
<223>  Tio12 Protein

<400>  13
Val Pro Trp Pro Glu Glu Leu Ala Ala Leu Tyr Arg Arg Glu Gly Tyr
                5                   10                  15

Trp Gln Gly Arg Pro Leu Gly Asp Leu Leu Thr Glu Ala Cys Ala Arg
            20                  25                  30

His Ala Asp Lys Thr Ala Leu Val Cys Gly Glu Arg Arg Met Thr Tyr
        35                  40                  45

Ala Glu Leu Ser Arg Ala Ser Ala Asp Val Ala Gly Gly Leu Leu Asp
    50                  55                  60

Val Gly Ile Glu Pro Leu Asp Arg Val Val Val Gln Leu Pro Asn Val
65                  70                  75                  80

Pro Glu Phe Val Val Val Val Tyr Ala Leu Leu Arg Ile Gly Ala Ile
                85                  90                  95

Pro Val Met Ala Leu Pro Gly His Arg Lys Val Glu Leu Thr His Leu
            100                 105                 110

Cys Ala His Ser Gln Ala Val Ala Leu Val Val Val Asp Gln Val Lys

115                 120                 125

Gly Phe Asp His Arg Ala Leu Ala Arg Glu Val Arg Ala Glu Val Pro
    130                 135                 140

Ala Leu Lys His Val Val Val Ala Gly Asp Ala Glu Glu Phe Thr Ala
145                 150                 155                 160

Leu Ala Asp Leu Tyr Arg Arg Gly Arg Asp Leu Pro Ala Val Asp Pro
                165                 170                 175

Ser Glu Pro Ala Leu Phe Leu Leu Ser Gly Gly Thr Thr Gly Leu Pro
            180                 185                 190

Lys Leu Ile Pro Arg Thr His Asp Asp Tyr Ala Tyr Asn Met Arg Ala
        195                 200                 205

Thr Ala Asp Ala Leu Arg Phe Gly Ala Glu Asp Val Tyr Leu Ala Val
    210                 215                 220

Asn Pro Ile Gly His Asn Ser Ala Leu Gly Cys Pro Gly Val Leu Gly
225                 230                 235                 240

Ala Leu Leu Val Gly Gly Thr Ala Val Leu Thr Ser Ser Val Arg Pro
                245                 250                 255

Asp Glu Val Phe Glu Leu Leu Glu Arg Glu Arg Val Thr Leu Thr Thr
            260                 265                 270

Leu Val Pro Pro Val Val Arg Leu Trp Leu Asp Ala Ala Arg Lys Ser
        275                 280                 285

Asp Val Arg Phe Pro Asn Leu Met Leu Gln Val Gly Ser Ser Lys Phe
    290                 295                 300

Asp Pro Ala Arg Ala Ala Glu Val Arg Thr Ser Leu Gly Ala Val Leu
305                 310                 315                 320

Thr Gln Trp Phe Gly Val Gly Glu Gly Leu Leu Thr Tyr Thr Arg Leu
                325                 330                 335

Asp Asp Pro Glu Glu Val Val Leu Ala Thr Glu Gly Arg Pro Leu Ala
            340                 345                 350

Ala His Asp Glu Ile Leu Val Val Asp Thr Asp Gly Arg Pro Val Pro
        355                 360                 365

Asp Gly Ala Glu Gly Glu Leu Leu Val Arg Gly Pro Tyr Thr Ile Arg
    370                 375                 380

Gly Tyr Tyr Arg Ala Glu Glu Gln Asn Arg Arg Ser Phe Thr Pro Asp
385                 390                 395                 400

Gly Tyr Phe Arg Thr Gly Asp Met Val Arg Arg Arg Pro Asp Gly Ser
                405                 410                 415

Ile Val Val Val Gly Arg Ile Lys Asp Ile Ile Asn Arg Gly Gly Glu
            420                 425                 430

Lys Val Pro Ala Glu Glu Ile Glu Glu His Leu Leu Ala His Ala Ala
        435                 440                 445

Ile Arg Asp Ala Ala Val Val Gly Val Ala Asp Pro Val Leu Gly Glu
    450                 455                 460

Lys Thr Val Ala Phe Val Ile Leu Arg Asp Glu Pro Ile Ser Pro Ala
465                 470                 475                 480

Ala Val Lys Ser Phe Leu Arg Glu Arg Gly Leu Ala Thr Tyr Lys Ile
                485                 490                 495

Pro Asp Arg Val Val Pro Ile Glu Glu Phe Pro Arg Thr Ala Val Gly
            500                 505                 510

Lys Val Asp Lys Val Thr Leu Arg Thr Ser Gly
        515                 520


<210>  14
<211>  596
<212>  PRT
<213>  Micromonospora sp., ML1 strain

<220>
<223>  Tiol3 Protein


<400>  14
Val Pro Thr Val Ala Glu Leu Phe Glu Ser Val Val Ala Arg Ser Gly
                5                   10                  15

Asp His Val Ala Val Ile Phe Gly Asp Arg Leu Val Ser Tyr Ala Glu
            20                  25                  30

Leu Asp Ala Arg Ala Asn Arg Leu Ala Arg Val Leu Val Glu Arg Gly
        35                  40                  45

Val Gly Pro Asp Thr Thr Val Ala Val Ala Met Pro Lys Cys Asp Glu
    50                  55                  60

Leu Met Val Val Leu Leu Ala Val Leu Lys Ala Gly Gly Ala Tyr Leu
65                  70                  75                  80

Pro Leu Asp Pro Gln Tyr Pro Ala Lys Arg Leu Ala Tyr Met Val Gln
                85                  90                  95

Asp Ala Arg Pro Thr Leu Leu Val Arg Met Ala Gln Val Ser Leu Glu
            100                 105                 110

Leu Gly Val Pro Val Ala Glu Leu Val Val Asp Asp Pro Asp Thr Ala

115                 120                 125

Arg Glu Thr Glu Ser Arg Pro Asp His Gln Leu Thr Asp Ala Ser Arg
    130                 135                 140

Asn Ala Pro Leu Arg Pro Asp Asn Leu Met Tyr Val Ile Tyr Thr Ser
145                 150                 155                 160

Gly Ser Thr Gly Thr Pro Lys Gly Val Ala Val Thr His Thr Gly Val
                165                 170                 175

Ala Asp Ile Val Ala Ala Gln Thr Ala Ser Ile Ala Pro Arg Pro Gly
            180                 185                 190

Asp Arg Val Leu Gln Trp Ala Ser Val Ser Phe Asp Ala Ala Phe Trp
        195                 200                 205

Asp Trp Ser Ala Ala Leu Leu Ser Gly Ala Thr Leu Ile Met Ala Pro
    210                 215                 220

Ala Asp Glu Leu Leu Pro Gly Gln Pro Leu Arg Asp Thr Leu Arg Arg
225                 230                 235                 240

Tyr Ala Val Thr His Ala Val Leu Pro Pro Val Ala Leu Ser Val Thr
                245                 250                 255

Glu Pro Asp Asp Val Leu Val Gly Gly Thr Leu Met Ser Thr Gly Asp
            260                 265                 270

Ala Cys Thr Arg Ala Leu Val Ala Lys Trp Ala Pro Gly Arg Arg Met
        275                 280                 285

Tyr Asn Gly Tyr Gly Pro Thr Glu Thr Thr Val Gly Ser Thr Ile Ala
    290                 295                 300

Gly Pro Ile Ala Ile Ser Asp Glu Ile Thr Ile Gly Thr Pro Trp Ser
305                 310                 315                 320

Gly Asn Arg Val Tyr Val Leu Asp Glu Arg Leu Arg Pro Val Pro Asn
                325                 330                 335

Gly Arg Asp Gly Glu Leu Tyr Leu Ala Gly Asn Gly Leu Ala Arg Gly
            340                 345                 350

Tyr Leu Asn Arg Pro Gly Leu Thr Ala Ser Arg Phe Val Leu Asp Pro
        355                 360                 365

Phe Gly Pro Pro Gly Gly Arg Met Tyr Arg Ser Gly Asp Arg Gly His
    370                 375                 380

Arg Arg Ala Asp Gly Gln Leu Val Phe Ala Ser Arg Gly Asp Asp Gln
385                 390                 395                 400

Val Lys Ile Arg Gly Phe Arg Val Glu Leu Gly Glu Val Glu Ala Arg
                405                 410                 415

Leu Ala Ala His Pro Ala Val Asp Val Ala Ala Val Val Val Val Gly
            420                 425                 430

Asp Leu Ala Gly Ala Arg Leu Ala Ala Phe Val Ser Thr Val Ala Gly
        435                 440                 445

Thr Gly Val Ser Ala Ala Glu Leu Ser Ala His Ser Ala Glu Thr Leu
    450                 455                 460

Pro Glu His Met Val Pro Ser Ser Val Gln Val Leu Gln Arg Leu Pro
465                 470                 475                 480

Val Thr Ala Asn Gly Lys Ile Asp Arg Ala Ala Leu Arg Asp Leu Ala
                485                 490                 495

Glu Ser Ala Ala His Pro Asp Ala Ala Ala Ser Ala Gly Pro Pro Gly
            500                 505                 510

Glu Asp Pro Thr Ser Val Glu Ser Arg Leu Cys Leu Leu Val Arg Glu
        515                 520                 525

Leu Leu Ala Val Pro Asp Val Arg Pro Gly Asp Asn Phe Phe Lys Leu
    530                 535                 540

Gly Gly His Ser Val Leu Ala Thr Arg Leu Ala Gly Arg Leu Arg Asp
545                 550                 555                 560

Glu Trp Gly Ile Thr Val Pro Ile Arg Ala Val Phe Glu Ala Ala Thr
                565                 570                 575

Leu Ser Glu Leu Ala Leu Val Val Gln Ala Ser Lys Ala Ser Ile Pro
            580                 585                 590

Asn Gly Arg Pro
        595


<210> 15
<211> 424
<212> PRT
<213> Micromonospora sp., ML1 strain

<220>
<223> Tio14 Protein


<400> 15
Val Thr Gly Val Arg Thr Leu Thr Val Val Ala Ile Leu Ala Leu Thr
                5                   10                  15

Ala Thr Ser His Gln Val Glu Pro Val Ala Ala Ser Gly Asp Val Leu
            20                  25                  30

Gly Val Ala Thr Leu Ala Asp Thr Gln Thr Thr His Ala Leu Ile Ala
        35                  40                  45

Asn Pro His Leu Arg Gly Thr Gly Ser Thr Ser Ser Gly Pro Gly Gly
    50                  55                  60

Ala Pro Ala Glu Ala Leu Ser Arg Phe His Asp Leu Gly Ser Leu Ala
65                  70                  75                  80

Gly Pro Gly Thr Pro Ser Glu Ala Thr Asp Leu Asn Asp Arg Gly Asp
                85                  90                  95

Val Val Gly Val Thr Ser Leu Pro Gly Thr Gln Thr Ala His Ala Phe
            100                 105                 110

Ile Ala Asn Pro Arg His Lys Gly Gly Arg Leu Ile Glu Ile Thr Pro
        115                 120                 125

Ser Gln Ala Gly Ser Ser Arg Ala Gln Ala Ile Asn Arg Phe Gly Val
    130                 135                 140

Val Ala Gly Thr Leu Gly Leu Gly Ile Gly Pro Pro Thr Pro Ala Pro
145                 150                 155                 160

Asp Gly Ser Gln Pro Leu Leu Gln Pro Phe Val Trp Glu Ala Arg Thr
                165                 170                 175

Gly Leu Asp Val Leu Pro Leu Pro Pro Gly Ala Gly Gly Ala Gln Ala
            180                 185                 190

Val Asp Ile Asn Asp Lys Gly Thr Val Leu Val Val Gly Thr Asn Ala
        195                 200                 205

Thr Gly Val Gly Leu Pro Val Gly Ser Tyr Leu Trp Asn Pro Ala Ser
    210                 215                 220

Arg Thr Tyr Thr Thr Leu Pro Pro Pro Thr Thr Ser Val Thr Gly Pro
225                 230                 235                 240

Val Ala Leu Ala His Thr Leu Asp Glu Arg Gly Gly Val Ala Gly Gly
                245                 250                 255

Leu Val Thr Gln Val Gly Glu Gln Ser Trp His His Thr Ala Val Val
            260                 265                 270

Trp Gln Pro Gly Thr Leu Ala Val Gln Gln Leu Pro Thr Gly Gly Ser
        275                 280                 285

Ala Asp Thr Tyr Ala Thr Asp Arg Asn Glu Asn Gly Leu Ile Val Gly
    290                 295                 300

Trp Arg Ile Asn Ala Thr Gly Gln Ser Thr Thr Ala Ile Tyr Trp Pro
305                 310                 315                 320

Ser Ala Asn Ala Gln Pro Val Glu Leu Pro Gly Arg Val Ala Tyr Arg
                325                 330                 335

Val Asn Asp Ala Gly Gln Ile Val Gly Ile Arg Asn Phe Thr Gly Ser
            340                 345                 350

Ser Ala Tyr Pro Phe Ser Ala Val Met Trp Glu Pro Arg Arg Gly Arg

355                 360                 365

Thr Thr Arg Leu Gly Asp Asn Asp Leu Gly Ser Tyr Val Ile Ala Val
    370                 375                 380

Asn Ala Ser Gly Arg Ser Ala Gly Phe Ala Val Ala Thr Arg Asn Gly
385                 390                 395                 400

Asn Asn His Asn Thr Ala Gly Trp Trp Gly Pro Pro Arg Arg Gly Asp
                405                 410                 415

Cys Pro Arg His Cys Ser Pro Arg
            420


<210>  16
<211>  450
<212>  PRT
<213>  Micromonospora sp., ML1 strain

<220>
<223>  Tio15 Protein


<400>  16
Met Thr Thr Arg Ser Thr Glu Pro Ser Leu Ser Pro Leu Val Pro Gly
                5                   10                  15

Leu Ser Arg Arg Gly Leu Leu Ile Ala Ala Ala Ser Thr Ala Ala Gly
            20                  25                  30

Thr Ala Gly Leu Leu Ile Leu Pro Thr Lys Ala Glu Ala Ala Thr Ala
        35                  40                  45

Ser Pro Arg His Gly Ser Arg Pro Asp Val Val Gly Pro Trp Phe Asp
    50                  55                  60

Glu Met Leu Ala Thr Phe Ala Ala Ala Pro Thr Ala Gly Leu Pro Glu
65                  70                  75                  80

Arg Met Trp Ala Met Ser Trp Ile Ala Ala Trp Ala Ala Leu Ser Ala
                85                  90                  95

Arg Arg Ala Ala Pro Val Arg Ala Pro Gly Gln Arg Ala Val Phe Glu
            100                 105                 110

Asp Ala Ala Thr Ala Thr Ala Val His Gly Val Leu Lys Ala Leu Val
        115                 120                 125

Pro Gly Gln Ala Asp Lys Leu Asp Gly Val Leu Ala Asp Ser Leu Ala
    130                 135                 140

Gly Leu Ala Ala Ser Glu Ala Lys Lys Ala Gly Ile Ala Ala Gly Arg
145                 150                 155                 160

Glu Ala Ala Ala Ala Met Val Ala Val Arg Thr Gly Asp Gly Leu Asp
                165                 170                 175

Leu Ala Ser Val Asn Val Pro Phe Thr Leu Pro Pro Glu Ala Pro Gly
            180                 185                 190

Val Tyr Arg Leu Thr Pro Gly Ala Thr Gln Thr Val Ala Ala Gly Tyr
        195                 200                 205

Gly Leu Ala Arg Pro Phe Leu Leu Gly Arg Ala Asp Arg Phe Arg Pro
    210                 215                 220

Pro Pro Pro Pro Ala Leu Gly Thr Ala Pro Tyr Arg Arg Ala Leu Asp
225                 230                 235                 240

Glu Val Arg Arg Leu Gly Gly Glu Val Ser Glu Arg Thr Glu Ala Gln
                245                 250                 255

Ser Asp Leu Ala Trp Leu Asp Pro Leu Ser Gln Tyr Val Pro Val Leu
            260                 265                 270

Arg Leu Leu Val Gly Asp Ser Ala Arg Ser Arg Arg Ser Lys Val Arg
        275                 280                 285

Leu Leu Ala Ala Leu Gly Ala Ala Ile Val Asp Ser Ser Ile Ala Thr
    290                 295                 300

Phe Glu Ala Lys Tyr Ala Leu Leu His Trp Arg Pro Val Thr Ala Ile
305                 310                 315                 320

Arg Asp Ala Asp Thr Asp Gly Asp Pro Arg Thr Ala Pro Asp Pro Thr
                325                 330                 335

Trp Asn Ser Phe Leu Pro Thr Pro Pro His Pro Glu Tyr Pro Ser Gly
            340                 345                 350

His Ala Val Thr Ala Gly Ala Ala Glu Gln Val Leu Ser Gln Leu Val
        355                 360                 365

Gly Pro Cys Thr Pro Met Ala Phe Ser Ile Thr Phe Ala Arg Gly Asp
    370                 375                 380

Gly Arg Val Val Thr Arg Asn Tyr Arg Arg Gly Thr Pro Trp Ser Thr
385                 390                 395                 400

Leu Thr Lys Glu Asn Ile Asp Ala Arg Val Leu Ala Gly Val His Phe
                405                 410                 415

Arg Tyr Ser Asp Glu Val Gly Ala Ala Leu Gly Arg Gln Val Ala Arg
            420                 425                 430

Tyr Asp Leu Asn Leu Leu Ala Arg Gly Gly Val Gly Pro Val Arg Val
        435                 440                 445

Thr Trp

    450

<210>  17
<211>  768
<212>  PRT
<213>  Micromonospora sp., ML1 strain

<220>
<223>  Tio16 Protein

<400>  17
Val Pro Trp Ser Val Trp Asn Gln Thr Ala Arg His Phe Arg Ala Asp
                5                   10                  15

Val Thr Ile Pro Asp Leu Leu Ala Glu Ala Ala Glu Arg His Pro Asp
            20                  25                  30

Arg Pro Ala Ile Val Thr Ser Asp Lys Gln Val Leu Thr His Arg Glu
        35                  40                  45

Leu His Arg Arg Ala Asn Arg Leu Ala Arg Leu Leu Arg Asp Leu Gly
    50                  55                  60

Val Gly Arg Gly Asp Thr Val Ala Leu Phe Gly Glu Arg Asp Ala Pro
65                  70                  75                  80

Ala Leu Val Gly Leu Leu Ala Val Leu Lys Cys Gly Ala Ala Tyr Val
                85                  90                  95

Pro Ile Asp Pro Ser Trp Pro Ala Arg Arg Val Val Gly Leu Leu Asp
            100                 105                 110

Gln Leu Asp Val Ala Ala Val Leu Thr Asp Arg Thr His Leu Arg Ser
        115                 120                 125

Met Gln Glu Leu Arg Trp Gln Val Arg Ser Leu Arg His Val Leu Cys
    130                 135                 140

Pro Asp Ile Ala Glu Glu Phe Ser Trp Ser Ala Thr Phe Arg Pro Asp
145                 150                 155                 160

Leu Val Val Asn Phe Phe Asp Phe Val Ser Ser Ala Pro Asp Gln Leu
                165                 170                 175

Glu Ala Ala Gly Phe Asn Met Arg Arg Ser Glu Arg Pro Phe Asp Glu
            180                 185                 190

Asn Asp Val Gln Gln Tyr Arg Asp His Val Ala Ala Phe Ala Leu Ala
        195                 200                 205

Ala Thr Ala Ala Ala Arg Pro Ser Ile Leu Glu Ile Gly Ser Gly Ser
    210                 215                 220

Gly Leu Ile Val Glu Ala Leu Ala Pro Arg Ala Ser Arg Tyr Val Ala
225                 230                 235                 240

Ala Asp Pro Ser Pro Val Ser Val Thr Arg Ser Gln Glu Gly Gly Phe
                245                 250                 255

Arg Ala Gly Gly Glu Val Asp Gly Val Val Cys Phe Ala His Glu Val
            260                 265                 270

Ala Glu Arg Val Gln Gly Pro Phe Asp Val Ile Leu Leu Ala Ser Thr
        275                 280                 285

Val Gln Phe Leu Pro Asp Leu Asp Tyr Leu Leu Ser Val Leu Gly Ser
    290                 295                 300

Leu Leu Gly Leu Leu Ala Asp Asp Gly Val Ile Val Leu Ala Asp Leu
305                 310                 315                 320

Ile Asp Pro Asp Leu Ser Asp Tyr Ser Gly Ile Arg Val Ala Pro His
                325                 330                 335

Phe Ile Glu Arg Leu Pro Glu Phe Leu Pro Gly Val Thr Gly Gly Ser
            340                 345                 350

Val His Arg Arg Gln Val Glu Thr Phe Thr Gly Glu Leu Ala Gln Arg
        355                 360                 365

Tyr Asp Ala Val Ile Arg Val Ala Pro Ser Gly Asp Arg Pro Ala Ser
    370                 375                 380

Gly Arg Glu Pro Leu Trp Thr Gly His His Ile Glu Leu Arg Pro Ser
385                 390                 395                 400

Glu Pro Leu Ala Thr Gly Pro Thr Ala Asp Asp Val Cys Tyr Thr Ile
                405                 410                 415

Phe Thr Ser Gly Ser Thr Gly Ala Pro Lys Gly Val Val Val Ser His
            420                 425                 430

Arg Ser Ala Val Asn Leu Ile Asp Trp Val Asn Arg Ser Tyr Arg Val
        435                 440                 445

Gly Pro Thr Asp Gln Ile Leu Phe Val Thr Ser Phe Cys Phe Asp Leu
    450                 455                 460

Ser Val Tyr Asp Ile Phe Gly Val Leu Ala Ala Gly Gly Ser Val Arg
465                 470                 475                 480

Leu Ala Ser Ala Ala Glu Leu Ala Glu Pro Asp Thr Leu Val Asp Leu
                485                 490                 495

Leu Glu Gln Glu Glu Ile Thr Ile Trp Asp Ser Ala Pro Ala Ala Leu
            500                 505                 510

Ala Met Val Met Pro Phe Val Gln Val Arg Glu Pro Ala Gly Arg Gly
        515                 520                 525

Thr Leu Arg Leu Val Leu Leu Ser Gly Asp Trp Ile Pro Val Pro Leu
    530                 535                 540

Pro Asp Gln Ile Arg Ala Ala Phe Pro Asn Ala Glu Val Val Ala Leu
545                 550                 555                 560

Gly Gly Ala Thr Glu Cys Thr Val Trp Ser Asn His Tyr Arg Val Gly
                565                 570                 575

Asp Val Asp Pro Gly Trp Pro Ser Ile Pro Tyr Gly Lys Pro Met Gln
            580                 585                 590

Asn Ala Arg Tyr Tyr Val Leu Asp His Gly Leu Gly Pro Cys Pro Asn
        595                 600                 605

Gly Ala Glu Gly Asp Leu Tyr Ile Ala Gly Glu Cys Val Ala Leu Gly
    610                 615                 620

Tyr Ala Arg Ser Pro Gly Leu Thr Ala Ala Lys Phe Leu Pro Asp Pro
625                 630                 635                 640

Trp Ala His Arg Thr Gly Glu Arg Met Tyr His Thr Gly Asp Arg Ala
                645                 650                 655

Arg Trp Leu Pro Ser Gly Asp Leu Glu Phe Leu Gly Arg Leu Asp Asp
            660                 665                 670

Gln Val Lys Val Arg Gly Tyr Arg Ile Glu Leu Gly Glu Val Gln Ser
        675                 680                 685

Ala Leu Leu Gln Cys Ala Gly Val Arg Ala Ala Ser Val Val Ala Pro
    690                 695                 700

Ser Gly Pro Gly Gly Arg Thr Leu Ala Ala Phe Tyr Val Pro Thr Ala
705                 710                 715                 720

Glu Pro Leu Pro Thr Ala Glu Val Val Gly Ala Leu Gln Arg Ile Leu
                725                 730                 735

Pro Ser Tyr Met Val Pro Thr Arg Ile Val Ala Leu Asp Glu Leu Pro
            740                 745                 750

Leu Thr Asn Thr Gly Lys Val Asp Arg Ala Ala Leu Leu Ser Arg Leu
        755                 760                 765

<210>  18
<211>  94
<212>  PRT
<213>  Micromonospora sp., ML1 strain

<220>
<223>  Tio17 Protein

<400>  18
Val Ala Asn Pro Asp Arg Asp Gly Asn Asp Gly Ser Ala Arg Gly Leu
                5                   10                  15

Leu Arg Ala His Val Arg Ala Gly Trp Ala Ala Ala Leu Gln His Glu
            20                  25                  30

Arg Phe Asp Asn Asp Asp Asp Phe Phe Ala Ile Gly Gly His Ser Leu
        35                  40                  45

Leu Ile Ala Glu Ile Met Ala Gly Leu Gly Ala Leu Ala Gly Thr Arg
    50                  55                  60

Leu Ser Leu Lys Leu Phe Phe Asp His Pro Thr Val Asn Glu Leu Thr
65                  70                  75                  80

Asp Ala Leu Ala Ala His Glu Ala Phe Gly Ser Leu Ala Arg
                85                  90


<210>  19
<211>  259
<212>  PRT
<213>  Micromonospora sp., ML1 strain

<220>
<223>  Tio18 Protein


<400>  19
Met Gly Thr Arg Thr Lys Asn Arg Trp Phe Val Glu Val Ser Arg Ser
                5                   10                  15

Arg Leu Lys Glu Cys Ala Leu Tyr Ala Ile Pro His Ala Gly Gly Gly
            20                  25                  30

Val Ala Thr Val Arg Ser Leu Cys Val Glu Leu Gly Asp Ala Phe Asp
        35                  40                  45

Thr Phe Gly Val Arg Leu Pro Gly Arg Glu Ser Arg Leu Gly Asp Pro
    50                  55                  60

Val Val Thr Val Met Ser Arg Leu Ala Asp Glu Leu Ala Gly Glu Leu
65                  70                  75                  80

Leu Arg His Ala Gln Gly Arg Pro Val Val Leu Tyr Gly His Cys Ser
                85                  90                  95

Gly Ser Ala Ile Ala Tyr Glu Val Ala Arg Arg Met Asp Pro Ala Gln
            100                 105                 110

Leu Arg Gly Leu Ala Val Ser Ser His Pro Ala Pro Gly Ser Val Arg
        115                 120                 125

Arg Ala Pro Thr Trp Glu Leu Pro Arg Gln Glu Phe Leu Lys Gln Val
    130                 135                 140

Val Thr Asp Gly Tyr Leu Pro Pro Glu Ile Ile Ala Asn Glu Glu Leu
145                 150                 155                 160

Leu Asp Ile Tyr Glu Pro Ala Val Arg Ala Asp Tyr Glu Leu Val Glu
                165                 170                 175

Thr Tyr Glu Leu Asp Leu Phe Gly Gly Gly Pro Val Glu Ala Val Ala
            180                 185                 190

Ala Pro Thr Val Ala Ile His Gly Arg Asp Asp His Thr Ile Asp Pro
        195                 200                 205

Val His Ile Asp Ala Trp Ser Ala Leu Thr Thr Gly Pro Phe Arg Val
    210                 215                 220

Val Ser Leu Ala Gly Gly His Asn Leu Leu Leu Gly Arg Pro Ala Glu
225                 230                 235                 240

Leu Ala Ala Ala Ile Cys Gln Gly Leu Ala Ser Asp Asn Arg Leu His
                245                 250                 255

Asp His Arg

<210> 20
<211> 245
<212> PRT
<213> Micromonospora sp., ML1 strain

<220>
<223> Tio19 Protein

<400> 20
Met Arg Lys Lys Gly Trp Ile Arg Thr Leu Thr Thr Val Glu Lys Pro
                5                   10                  15

Thr Gly Arg Leu Val Cys Phe Pro His Ser Gly Gly Ser Ala Thr Ala
            20                  25                  30

Phe Ser Asp Trp Ala Asn Ala Met Pro Pro Gly Val Glu Leu Val Ala
        35                  40                  45

Val Gln Tyr Pro Gly Arg Gly Asp Arg Phe Ala Glu His Leu Val Asp
    50                  55                  60

Asp Val Ala Glu Met Ala Gly His Ile Val Val Glu Leu Leu Glu Leu
65                  70                  75                  80

Pro Cys Arg Asp Gln Val Leu Phe Gly His Ser Leu Gly Ala Met Val
                85                  90                  95

Ala Tyr Glu Thr Ala Met Leu Leu Arg Asp Met Arg Val Glu Pro Arg
            100                 105                 110

Arg Leu Cys Ala Ser Ala Cys Leu Pro Pro Gly Gln Met Lys Asn Arg
        115                 120                 125

Asp Val His Leu Lys Pro Asp Asp Glu Phe Trp Ala Ala Ile Cys Ala
    130                 135                 140

Leu Gly Gly Ile Asp Pro Arg Val Ala Glu Ile Ala Glu Leu Arg Glu
145                 150                 155                 160

Leu Phe Met Pro Thr Leu Arg Ser Asp Leu Arg Ala His Ala Asn Tyr
                165                 170                 175

Arg Pro Gly Ser Gly Thr Thr Pro Leu Ser Cys Pro Val Ser Cys Tyr
            180                 185                 190

His Gly Val Gly Asp Pro Leu Val Asp Glu Ser Arg Leu Thr Glu Trp
        195                 200                 205

Ala Asp Val Thr Thr Gly Glu Phe Arg Leu Thr Val Arg Pro Gly Gly
    210                 215                 220

His Phe His Val Ser Ile Asp Val Asp Gly Leu Val Ser Asp Ile Met
225                 230                 235                 240

Gly Arg Glu Trp Ala
                245

<210>  21
<211>  2594
<212>  PRT
<213>  Micromonospora sp., ML1 strain

<220>
<223>  Tio20 Protein

<400>  21
Met Gly Tyr Gly Gly Val Ser Ser Ala Gln Glu Ala Met Trp Leu Glu
  1               5                  10                  15

Gln Asn Leu Ala Pro Glu Val Pro Asn Ser Thr Thr Thr Ile Trp Asp
                20                  25                  30

Val Arg Gly Glu Leu His Leu Pro Asp Leu Glu Asp Ala Leu Arg Thr
            35                  40                  45

Ala Leu Gly Glu Thr Ser Ala Leu Leu Val Asn Phe Arg Arg Glu Ser
50                  55                  60

Asp Glu Leu Arg Ile Val Pro Arg Lys Leu Asp Ala Trp Glu Pro Phe
65                  70                  75                  80

Arg Ile Asp Ala Ser Asp Ala Ala Asp Pro Ala Ala Ala Ala Arg Glu
                85                  90                  95

Phe Leu Thr Glu Gln Ile Arg Arg Pro Phe Asp Leu Gly Ala Asp Ala
            100                 105                 110

Leu Phe Arg Ile Gly Thr Val Arg Leu Gly Asp Gly His His Leu Leu
        115                 120                 125

Cys Leu Ile Met His His Ile Leu Thr Asp Ala Phe Gly Val Phe Thr
    130                 135                 140

Leu Leu Ser Gln Arg Val Ala Glu Ile Tyr Arg Ala Leu Arg Ala Asp
145                 150                 155                 160

Ser Pro Val Pro Asp Arg Pro Asp Thr Pro Ala Glu Ile Ala Gly Glu
                165                 170                 175

Arg Glu Ala Thr Tyr Arg Ser Ser Glu Arg Phe Thr Asp Ala Ala Arg
            180                 185                 190

Phe Trp Arg Asp Tyr Val Thr Ala Gly Gly Thr Ala Ala Arg Leu Pro
        195                 200                 205

Thr Gly Met Arg Ala Gly Ala Thr Arg Thr Ala Ser Val Gly Phe Trp
    210                 215                 220

Asp Gly Leu Thr Ala Pro Leu Gly Val Ala Ser Tyr Thr Ala Ser Val
225                 230                 235                 240

Ala Ala Ser Glu Val Ala Arg Trp Arg Ala Ala Ala Asp Ser Thr Glu
                245                 250                 255

Thr Ser Val Pro Asp Leu Leu Thr Ser Ala Ala Thr Ala Phe Leu His
            260                 265                 270

Arg Leu Cys Asn Thr Pro Arg Pro Met Leu Ser Phe Thr Val Asn Tyr
        275                 280                 285

Arg Arg Gly Asp Ile Lys Gln Ala Cys Gly Leu Tyr Ser Asn Ala Leu
    290                 295                 300

Pro Leu Val Ile Glu Val Pro Ala Ala Gly Asp Phe Val Asp Leu Ala
305                 310                 315                 320

Lys Ser Val Arg Leu Glu Arg Leu Arg Val Leu Arg Tyr Ala Glu Tyr
                325                 330                 335

Asn Val Ser Leu Ile Lys Arg Ala Ala Gly His Ala Gly Asp Val Arg

340                 345                 350

Ser Pro Phe Gly Pro Val Val Asn Val Ile Pro Phe Leu Arg Glu Leu
        355                 360                 365

Asp Leu Ala Gly Ala Val Gly Arg Phe Ala Gly Gly Thr Phe Gly Leu
    370                 375                 380

Pro Asp Glu Val Arg Ile Ser Thr Tyr Val Asp Gly Gly Pro Asp Ser
385                 390                 395                 400

Asp Leu Tyr Ile Arg Phe Asp Ala Pro Gly Arg Leu Tyr Thr His Glu
                405                 410                 415

Asp Ile Ala Gly Leu Ala Glu Arg Phe Val Thr Phe Val Arg Ala Ala
            420                 425                 430

Leu Thr Asp Pro Arg Ala Arg Val Arg Asp Ile Ser Ala Ile Gly Glu
        435                 440                 445

Gln Glu Arg Val Ala Leu Leu Asp Arg Cys Ala Gly Pro Val Val Glu
    450                 455                 460

Leu Ala Gly Val Thr Val Pro Val Leu Leu Asp Met Gln Ala Leu Ala
465                 470                 475                 480

Thr Pro Asp Ala Ala Ala Val Val His Glu Asn Ala Val Leu Thr Tyr
                485                 490                 495

Arg Glu Leu Ala Ala Arg Ser Asp Ala Leu Ala Arg Arg Leu Ala Glu
            500                 505                 510

Cys Gly Ala Gly Pro Glu Arg Leu Val Ala Val Ala Val Pro Arg Ser
        515                 520                 525

Ile Gly Leu Ala Val Ala Leu Cys Ala Val Leu Lys Ala Gly Ala Ala
    530                 535                 540

Tyr Leu Pro Ile Asp Pro Asp Ser Pro Ala Glu Arg Val Glu Phe Met
545                 550                 555                 560

Leu Gly Asp Ala Arg Pro Thr Leu Leu Leu Thr Val Ala Gly Thr Asp
                565                 570                 575

Gly Leu Ala Ile Thr Pro Leu His Gly Ala Gln Pro Arg Trp Glu Ala
            580                 585                 590

Ser Val Ile Glu Arg Thr Thr Ala Ala Ser Pro Ala Ala Thr Ser Gln
        595                 600                 605

Ala Gly Gly Thr Gly Glu His Ala Ala Tyr Leu Arg Tyr Arg Ser Gly
    610                 615                 620

Pro Phe Gly Pro Ala Ala Gly Val Val Val Thr His Ala Ala Leu Val

625                 630                 635                 640

Asn Glu Leu Leu Trp Met Gln Gln Arg Tyr Arg Leu Asn Asp Thr Asp
                645                 650                 655

Arg Val Leu His Lys Ala Pro Ala Ser Leu Asn Ala Ser Gly Trp Glu
            660                 665                 670

Ile Phe Trp Pro Leu Ile Ala Gly Ala Thr Val Val Met Ala Thr Gln
        675                 680                 685

Asp Val His Gly Asp Ala Thr Ala Leu Ala Ala Met Ile Arg Arg Thr
    690                 695                 700

Gly Val Thr Thr Val His Val Val Pro Ser Val Leu Ala Glu Leu Val
705                 710                 715                 720

Arg Asp Glu Ala Val Val Thr Ala Gly Lys Gly Leu Arg His Val Met
                725                 730                 735

Cys Ser Gly Glu Val Leu Pro Ala Asp Leu Ala Ala Arg Phe His Glu
            740                 745                 750

Ser Cys Asp Val Pro Val His Ser Leu Tyr Gly Pro Val Glu Ala Met
        755                 760                 765

Pro Val Val Thr Ser Cys Asp Tyr Arg Pro Gly Ser Pro Thr Val Pro
    670                 675                 680

Leu Gly Arg Pro Ile Ser Asn Ala Gly Val Tyr Val Leu Asp Ser Gln
685                 690                 695                 700

Leu Gln Leu Thr Pro Pro Gly Val Val Gly Glu Val Tyr Val Thr Gly
                705                 710                 715

Val Gly Leu Ala Arg Gly Tyr Leu His Arg Pro Ala Ala Thr Ala Asp
            720                 725                 730

His Phe Val Ala Cys Pro Phe Gly Pro Ala Gly Thr Arg Met Tyr Arg
        735                 740                 745

Thr Gly Asp Leu Ala Arg Trp Thr Asp Asp Gly Glu Leu Glu His Ala
    750                 755                 760

Gly Ser Ala Asp Gln Arg Val Arg Ile Arg Gly Phe Trp Ala Ala Phe
765                 770                 775                 780

Leu Glu Ile Glu Ala Val Leu Thr Ala His Pro Gly Val Asp Gln Ala
                785                 790                 795

Ala Val Ile Ala Arg Pro Asp Gly Pro Asp Arg Ala Ala Gln Leu Ile
            800                 805                 810

Ala Tyr Val Val Pro Ala Thr Gly Gly Arg Met Gly Ala Ala Gly Leu
        815                 820                 825

Asp Val Asp Phe Ser Ala Gly Leu Asp Val Val Glu Leu Arg Arg Phe
    830                 835                 840

Val Ala Ala Arg Leu Pro Glu His Leu Val Pro Ala Thr Ile Val Val
845                 850                 855                 860

Leu Asp Arg Leu Pro Leu Thr Ala Glu Gly Lys Arg Asp His Ala Ala
                865                 870                 875

Leu Pro Glu Pro Gln Ala Thr Arg Arg Ala His Arg Val Ala Arg Thr
            880                 885                 890

Ala Glu Glu Arg Leu Leu Ala Asp Ala Phe Ala Glu Val Leu Gly Ala
        895                 900                 905

Gly Arg Ile Gly Leu Asp Asp Asp Phe Phe Ala Leu Gly Gly Asp Ser
    910                 915                 920

Ile Ser Ala Met Arg Val Val Ala Leu Ala Arg Ala Gly Gly Leu Ala
925                 930                 935                 940

Leu Thr Ala Arg Thr Val Phe Glu Cys Arg Thr Val Ala Ala Leu Ala
                945                 950                 955

Ala Val Ala Ala Pro Ala Asp Glu Pro Ala Val Ala Ala Ala Ala Glu
            960                 965                 970

Gly Asp Gly Gly Arg Asp Asp Ala Gly Pro Ile Pro Leu Pro Pro Met
        975                 980                 985

Ala Arg Leu Phe Ala Glu Arg Gly Pro Gly Leu Asp Arg Leu Ala Gln
    990                 995                 1000

Trp Leu Val Leu Thr Leu Pro Ala Glu Met Ser Pro Asp Leu Leu Thr
1005                1110                1115                1120

Gly Ala Val Arg Ala Val Leu Asp Arg His Asp Val Leu Arg Ser Arg
                1125                1130                1135

Leu Ala Asp Gly Gln Leu Leu Ile Gln Pro Pro Gly Ser Val Asp Val
            1140                1145                1150

Asp Lys Leu Ile Arg Gln Val Pro Cys Ser Gly Asp Trp Ser Gly Pro
        1155                1160                1665

Ser Trp Pro Ala Leu Leu Ala Asp Glu Ala Ala Lys Ala Val Gly Gln
    1170                1175                1180

Leu Ser Ala Ser Ala Gly Gln Val Ile Arg Met Val Trp Phe Ala Pro
1185                1190                1195                1200

Pro Gly Gly Ala Pro Gly Arg Leu Leu Ile Ala Ala His His Leu Val
                1205                1210                1215

Val Asp Gly Met Ser Trp Arg Val Leu Val Pro Asp Leu Ala Glu Ala
            1220                1225                1230

Ala Ala Gln Ile Arg Ser Gly Arg Thr Pro Ala Leu Ala Pro Val Gly
        1235                1240                1245

Thr Ser Leu Arg Ser Trp Leu Asp Glu Leu Ala Ala Glu Ala Val Arg
    1250                1255                1260

Pro Glu Arg Val Ala Glu Leu Glu Leu Trp Arg Gly Ile Leu Ala Pro
1265                1270                1275                1280

Ser Gly Asp Pro Leu Gly Ala Arg Pro Leu Asp Pro Ala Val Asp Leu
                1285                1290                1295

Thr Arg Thr Ala Gln Thr Leu Arg Val Glu Val Pro Ala Glu Leu Ser
            1300                1305                1310

Arg Ala Leu Leu Thr Ser Val Pro Ala Thr Phe Arg Cys Gly Val Asp
        1315                1320                1325

Asp Val Leu Leu Thr Ala Leu Ala Leu Ala Leu Ala Ser Arg Arg His
    1330                1335                1340

Asn Ser Ala Gly Val Val Arg Leu Glu Gly His Gly Arg Gln Glu Glu
1345                1350                1355                1360

Leu Val Pro Gly Thr Asp Leu Thr Arg Thr Val Gly Trp Phe Thr Thr
                1365                1370                1375

Val His Pro Val Arg Phe Asp Leu Ser Gly Leu Asp Leu Thr Asp Ala
            1380                1385                1390

Ile Asn Gly Gly Pro Ala Ala Ala Glu Ala Leu Arg Arg Val Lys Glu
        1395                1400                1405

Ser Arg Arg Val Leu Pro Asp Arg Gly Ile Gly Tyr Ser Leu Leu Arg
    1410                1415                1420

Tyr Leu Asn Gln Ala Thr Gly Ala Met Leu Arg Asp Leu Pro Ala Asp
1425                1430                1435                1440

Glu Val Gly Phe Asn Tyr Leu Gly Arg Phe Ser Phe Asp Arg Ser Arg
                1445                1450                1455

Thr Gly Arg Glu Met Ala Trp Thr Ser Ala Pro Glu Ser Ala Ala Leu
            1460                1465                1470

Val Ala Ala Pro Asp Pro Asp Met Pro Leu Ile Ser Ala Leu Glu Leu
        1475                1480                1485

Asn Ser Leu Val Val Asp Thr Asp His Gly Glu Gln Leu Gln Ala Leu
    1490                1495                1500

Phe Thr Phe Ala Thr Gly Val Leu Ala Thr Asp Glu Val Arg His Leu
1505                1510                1515                1520

Ala Asp Asp Trp Gln Ala Ala Leu Arg Gly Ile Arg Ala Gln Ala Ala
                1525                1530                1535

Thr Gly Ala Ser Gly Leu Thr Pro Ser Asp Val Pro Leu Val Arg Val
            1540                1545                1550

Arg Gln Gln Asp Leu Asp Asp Trp Gln Ser Arg Phe Gly Arg Val Asp
        1555                1560                1565

Asp Val Trp Pro Leu Thr Pro Leu Gln His Gly Leu Leu Tyr His Ser
    1570                1575                1580

Leu Leu Gly Asp Ser Ala Ala Ala Ser Tyr Gln Thr Gln Phe Val Leu
1585                1590                1595                1600

Arg Ile Asn Gly Pro Val Asp Pro Ala Arg Leu Arg Gly Ala Ala Gln
                1605                1610                1615

Thr Leu Leu Asn Arg His Pro Asn Leu Arg Val Ala Phe Leu Thr Lys
            1620                1625                1630

Ser Thr Gly Glu Pro Val Gln Val Val Val Asp Gly Val Glu Val Pro
        1635                1640                1645

Phe Ala His Val Asp Leu Ala Gly Gln Pro Gln Ala Glu Glu Arg Leu
    1650                1655                1660

Ala Arg Ile Leu Ala Glu Glu Arg Glu Ala Gln Phe Arg His Asp Ser
1665                1670                1675                1680

Ala Pro Leu Leu Arg Phe Ser Leu Ile Thr Leu Thr Pro Thr Gln Ala
                1685                1690                1695

Glu Leu Ala Leu Thr Ala His His Val Leu Phe Asp Gly Trp Ser Leu
            1700                1705                1710

Pro Leu Ile Glu Gln Gln Leu Met His Leu Tyr Ala Gly Gly Ala Asp
        1715                1720                1725

Ala Leu Glu Leu Val Glu His Gly Tyr Arg Glu Phe Leu Arg Trp His
    1730                1735                1740

Ser Arg Gln Asn Ala Asp Glu Ala Leu Ala Ala Trp Thr Glu Glu Leu
1745                1750                1755                1760

Ala Asp Ala Thr Gln Pro Thr Leu Leu Ala Ser Gly Phe Ala Pro Ala
                1765                1770                1775

Glu Gln Ala Gly Thr Ser Thr Glu Ile Gly Gln Val Asp Val Leu Leu
            1780                1785                1790

Thr Arg Gln Glu Ala Ala Ser Ala Val Arg Arg Ala Ala Asp Cys Gly
        1795                1800                1805

Ile Thr Pro Asn Val Leu Val Gln Gly Ala Trp Ala Val Val Leu Ala
    1810                1815                1820

Gln Leu Thr Gly Asn Ala Asp Val Leu Phe Gly Ser Ser Val Thr Val
1825                1830                1835                1840

Arg Pro Pro Glu Leu Pro Gly Ala His Thr Val Val Gly Met Phe Thr
                1845                1850                1855

Asn Thr Val Pro Val Arg Val Arg Cys Ala Pro Ala Asp Ser Leu Glu
            1860                1865                1870

Gly Met Leu Gln Ala Val Gln Asp Ala Gln Ala Arg Thr Leu Asp His
        1875                1880                1885

Tyr His Val Gly Leu Gly Asp Ile Gln Arg Ala Thr Gly Leu Thr Thr
    1890                1895                1900

Leu Phe Asp Thr Ile Val Ile Phe Glu Ser Phe Pro Val Asp Arg Ala
1905                1910                1915                1920

Ala Leu Ser Lys Ala Ser Ala Ser Ala Glu Leu Thr Val Thr Gly Ile
                1925                1930                1935

Arg Pro Phe Ala Pro Thr His Tyr Pro Leu Thr Val Leu Ala Ala Ala
            1940                1945                1950

Asp Pro Leu Leu Ser Leu Thr Leu Gln Tyr His Pro Asp Ala Leu Ala
        1955                1960                1965

Pro Ser Thr Val Gln Ala Ile Ala Asp Arg Phe Ala Arg Val Leu Arg
    1970                1975                1980

Gln Phe Ala Ala Asp Pro Arg Thr Arg Ile Ala Ala Ile Asp Val Leu
1985                1990                1995                2000

Ser Glu Ala Glu Arg Gln Leu Val Val His Asp Trp Asn Ala Thr Ala
                2005                2010                2015

Leu Glu Val Pro Pro Glu Phe Val Pro Glu Gln Phe Ala Arg Gln Ala
            2020                2025                2030

Ala Ala Thr Pro Asp Ala Val Ala Val Val Val Gly Glu Gln His Leu
        2035                2040                2045

Thr Tyr Ala Glu Leu Ser Glu Arg Ala Asn Arg Met Ala His Trp Leu
    2050                2055                2060

Ile Ala Gln Gly Ile Gly Ala Glu Ser Arg Val Val Val Leu Leu Pro
2065                2070                2075                2080

Arg Ser Ala Asp Leu Val Ala Ala Leu Leu Ala Val Trp Gln Ala Gly

2085                2090                2095

Gly Cys Tyr Val Pro Val Asp Pro Asp Tyr Pro Ala Ala Arg Val Arg
            2100                2105                2110

Ser Val Ile Asp Asp Cys Ala Ala Thr Leu Val Leu Asp Glu Asn Leu
        2115                2120                2125

Leu Asp Arg Thr Asp Leu Ser Arg Tyr Pro Ala His Ala Pro Ala Ile
    2130                2135                2140

Ala Val Ala Pro Gly Gln Ala Ala Tyr Thr Ile Tyr Thr Ser Gly Ser
2145                2150                2155                2160

Thr Gly Lys Pro Lys Gly Val Val Ile Gly His Asp Ala Leu Thr Asn
                2165                2170                2175

Phe Leu Ala Ser Met Gln Arg Thr Leu Arg Leu Ser Ala Gln Asp Arg
            2180                2185                2190

Phe Ala Ala Val Thr Thr Ile Ala Phe Asp Ile Ala Ala Leu Glu Leu
        2195                2200                2205

Phe Leu Pro Leu Thr Thr Gly Ala Arg Val Val Leu Ala Asn Arg Glu
    2210                2215                2220

His Val Ala Asn Pro Pro Ala Met Leu Asp Leu Val Glu Gln Thr Gly
2225                2230                2235                2240

Val Thr Val Met Gln Ala Thr Pro Ala Leu Trp Gln Met Leu Ala Thr
                2245                2250                2255

His Asp Val Arg Arg Leu Ser Gly Val Arg Val Leu Thr Gly Gly Glu
            2260                2265                2270

Ala Leu Pro Arg Pro Leu Ala Glu Gly Leu Cys Ala His Ala Ala Gln
        2275                2280                2285

Val Ile Asn Leu Tyr Gly Pro Thr Glu Thr Thr Val Trp Ser Thr Leu
    2290                2295                2300

Ala Asp Val Ala Thr Cys Lys Pro Pro Ser Ile Gly Thr Pro Ile Ala
2305                2310                2315                2320

Asn Thr Gln Val Leu Ile Leu Asp Ser Ala Leu Arg Pro Val Pro Pro
                2325                2330                2335

Gly Val Gly Gly Asp Leu Trp Ile Ala Gly Asp Gly Leu Ala Arg Gly
            2340                2345                2350

Tyr His Arg Gln Pro Gly Leu Thr Ala Ser Arg Phe Val Ala Asn Pro
        2355                2360                2365

Phe Gly Pro Gln Gly Ala Arg Met Tyr Arg Thr Gly Asp Leu Ala Arg

2370                2375                2380

Trp Ser Glu Ser Gly Arg Ile Glu Phe Leu Gly Arg Ala Asp Phe Gln
2385                2390                2395                2400

Ile Lys Leu Arg Gly Phe Arg Ile Glu Pro Gly Asp Val Glu His Ala
                2405                2410                2415

Leu Thr Arg His Pro Ala Val Arg Glu Ala Val Val Thr Ile Arg Glu
            2420                2425                2430

Asp Gln Pro Asp Asp Lys Arg Leu Val Gly Tyr Val Val Ala Glu
        2435                2440                2445

Asp Ala Glu Thr Pro Pro Thr Arg Glu Leu Gln Gln Phe Val Arg Glu
    2450                2455                2460

Gln Leu Pro Asp Tyr Met Val Pro Ser Ala Val Val Thr Leu Pro Ala
2465                2470                2475                2480

Ile Pro Leu Thr Ala Asn Gly Lys Leu Asp Arg Ser Arg Leu Pro Arg
                2485                2490                2495

Pro Glu Ala Ser Ser Thr Lys Tyr Arg Ala Pro Glu Ser Ala Arg Glu
            2500                2505                2510

Val Ala Leu Cys Gly Ile Phe Ala Glu Val Leu Gly Ala Glu Arg Val
        2515                2520                2525

Gly Val Asp Asp Asp Phe Phe Ala Leu Gly Gly His Ser Leu Leu Ala
    2530                2535                2540

Thr Arg Leu Ala Ala Arg Leu Arg Ser Asp Leu Gly Val Glu Val Pro
2545                2550                2555                2560

Ile Arg Thr Ile Phe Ala Ala Pro Thr Val Ala Glu Leu Ala Arg Arg
                2565                2570                2575

Trp Ser Gly Leu Ser Thr Ser Val Arg Lys Pro Leu Arg Arg Met Thr
            2580                2585                2590

Glu Arg

<210>  22
<211>  3140
<212>  PRT
<213>  Micromonospora sp., ML1 strain

<220>
<223>  Tio21 Protein

<400>  22
Met Ala Pro Val Ser Tyr Ala Gln Gln Arg Met Trp Phe Ile Asn Arg

1               5                   10                  15

Phe Glu Gly Gln Thr Ala Thr Tyr Asn Ile Pro Ile Leu Ile Arg Met
            20                  25                  30

Arg Gly Gln Ile Asp Arg Asp Glu Leu Arg Ala Ala Phe Met Asp Val
        35                  50                  55

Leu Ser Arg His Glu Ile Leu Arg Thr Val Tyr Asp Glu Leu Asp Gly
    50                  55                  60

Gln Pro Leu Gln Arg Ile Ile Glu Pro Ala Glu Leu Ser Leu Pro Trp
65                  70                  75                  80

Thr Asp Trp Gly Trp Val Ser Pro Gln Gln Val Asp Ser Ile Val Ala
                85                  90                  95

Ala Ser Val Arg Glu Gly Phe Asp Leu Ser Val Asp Leu Pro Ile Arg
            100                 105                 110

Ala Tyr Leu Leu Arg Pro Glu Pro Asp Val Ala Leu Leu Ala Val Val
        115                 120                 125

Ile His His Ile Ala Gly Asp Gly Gly Ser Leu Gly Pro Phe Thr Arg
    130                 135                 140

Asp Leu Ser Ala Ala Tyr Arg Ala Arg Leu Ala Gly Val Ala Pro Thr
145                 150                 155                 160

Trp Asp Glu Leu Pro Val Gln Tyr Ser Asp Tyr Ala Leu Trp Gln Arg
                165                 170                 175

Glu Leu Leu Gly Asp Glu Ser Asp Pro Asp Ser Val Phe Ser Thr Gln
            180                 185                 190

Leu Arg Tyr Trp Arg Gln Glu Leu Asp Gly Val Ser Arg Pro Val Ala
        195                 200                 205

Leu Pro Val Asp Arg Pro Arg Pro Ala Arg Ala Ser Tyr Arg Gly Asp
    210                 215                 220

Asn His Gly Phe Glu Ile Pro Ala Ala Leu Phe Glu Lys Val Glu Arg
225                 230                 235                 240

Leu Ala Gln Ser Tyr Asn Val Thr Val Pro Met Val Phe Gln Thr Ala
                245                 250                 255

Leu Val Val Leu Leu His Arg Leu Gly Gly Asp Arg Asp Ile Thr Leu
            260                 265                 270

Gly Ser Pro Ile Ala Gly Arg Thr Asp Gln Ala Leu Glu Asn Leu Val
        275                 280                 285

Gly Phe Phe Val Asn Thr Trp Val Leu Arg Val Gly Val Cys Thr Gly
    290                 295                 300

Asp Ser Phe Ala Gln Val Leu Glu Gln Val Arg Gln Lys Ala Ile Ala
305                 310                 315                 320

Ala Tyr Asp His Gln Asp Leu Pro Phe Glu Arg Leu Val Glu Leu Leu
                325                 330                 335

Arg Pro Gly Arg Ser Val Ala His His Pro Leu Phe Gln Val Thr Leu
            340                 345                 350

Ala Trp Gln Asn Ser Val Arg Gln Glu Leu Asp Leu Pro Gly Val Thr
        355                 360                 365

Ala Ser Met Glu Pro Val Ala Thr Gly Thr Ala Lys Phe Asp Leu Phe
    370                 375                 380

Val Asn Leu Met Pro Asn His Ser Arg Arg Ser Val Thr Val Asp Leu
385                 390                 395                 400

Glu Tyr Ala Thr Asp Leu Phe Asp Pro Ala Thr Val Glu Ala Ile Ala
                405                 410                 415

Ala Arg Tyr Leu Arg Val Leu Glu Gln Val Val Glu Ser Pro Asp Leu
            420                 425                 430

Gly Val Gly Ile Leu Asp Val Pro Ala Met Pro Ala Thr Arg Gly Glu
        435                 440                 445

Arg Thr Val Asp Ala Gly Glu Arg Glu Lys Ile Leu Tyr Arg Trp Asn
    450                 455                 460

Asp Thr Asp Arg Ser Phe Pro Cys Pro Gly Pro Ile His Leu Pro Phe
465                 470                 475                 480

Glu Gln Gln Ala Ala Ala Arg Pro Asp Ala Ile Ala Val Arg Trp Ser
                485                 490                 495

Gly Gly Thr Met Thr Tyr Arg Glu Leu Asn Arg Gln Ala Asn Arg Ile
            500                 505                 510

Ala Trp Thr Leu Lys Arg Arg Gly Val Arg Pro Glu Thr Val Val Gly
        515                 520                 525

Val Ala Val Arg Arg Gly Pro Leu Met Val Ala Ala Val Leu Gly Val
    530                 535                 540

Leu Lys Ala Gly Gly Ala Tyr Leu Pro Ile Gly Ser Thr Leu Pro Ser
545                 550                 555                 560

Asp Arg Val Ala Gly Met Leu Ala Asp Ala Ser Ala Thr Leu Val Leu
                565                 570                 575

Thr Thr Glu Asp Thr Asn Arg Trp Thr Pro Pro Asp Gly Val Glu Leu
            580                 585                 590

Leu Asp Val Gly Arg Ala Gly Leu Ala Leu Ser Leu Asp Gly Glu Ile
        595                 600                 605

Asn Pro Glu Pro Val Ala Ser Ala Asp Asn Thr Ala Tyr Ile Ile Phe
    610                 615                 620

Thr Ser Gly Ser Thr Gly Lys Pro Lys Gly Val Thr Val Ala His Arg
625                 630                 635                 640

Pro Val His Asn Leu Leu Asn Trp Cys Tyr Arg Thr Phe Asp Phe Gly
                645                 650                 655

Pro Asp Asp Val Ser Leu Cys Val Thr Ser Leu Asp Phe Asp Leu Ser
            660                 665                 670

Val Phe Asp Ile Phe Gly Leu Leu Gly Ala Gly Gly Gly Phe Tyr Ile
        675                 680                 685

Ala Asp Ala Thr Gln Gln Arg Asp Pro Glu Met Leu Leu Asp Val Leu
    690                 695                 700

Leu Ser Glu Pro Ile Thr Phe Trp Asn Ser Val Pro Gly Thr Leu Asn
705                 710                 715                 720

Gln Leu Ala Pro Leu Leu Pro Gln Thr Ala Gly Ser Pro Gly Val Gln
                725                 730                 735

Asp Leu Arg Leu Val Phe Phe Ser Gly Asp Tyr Thr Pro Leu Ser Leu
            740                 745                 750

Pro Asp Gln Val Arg Ala Val Phe Pro His Ala Glu Ile Ile Ser Leu
        755                 760                 765

Gly Gly Ala Thr Glu Ala Thr Val Trp Ser Asn Phe Phe Pro Val Gly
    770                 775                 780

Ala Ile Asp Pro Gln Trp Arg Ser Ile Pro Tyr Gly Arg Pro Ile Asp
785                 790                 795                 800

Asn Ala Arg Tyr Tyr Val Leu Asp Glu Lys Leu Glu Pro Cys Ala Val
                805                 810                 815

Gly Val Glu Gly Asp Leu Tyr Ile Gly Gly Pro Val Ile Ala Leu Gly
            820                 825                 830

Tyr Val Asn Arg Pro Asp Leu Thr Ala Glu Arg Phe Ile Ala Asp Pro
        835                 840                 845

Phe Gly Gly Val Pro Gly Gln Arg Ile Tyr Arg Thr Gly Asp Arg Ala
    850                 855                 860

Ser Phe Phe Pro Asp Gly Asn Ile Cys Phe Leu Gly Arg Ala Asp Gly
865                 870                 875                 880

Gln Val Lys Val Arg Gly Phe Arg Ile Glu Leu Gly Glu Ile Glu Tyr
                885                 890                 895

Ala Leu Gly Arg His Pro Ala Val Arg Gln Gly Ile Ala Ile Thr Arg
            900                 905                 910

Arg Asp Ser Val Gly Asp Leu Arg Leu Val Ser Tyr Val Leu Pro Asp
        915                 920                 925

Leu Asp Thr Val Glu Ser Val Val Ala Ala Asp Glu Gln Val Arg Glu
    930                 935                 940

Trp Gln Glu Ile Tyr Asp Gln Gly Tyr Leu Glu Val Thr Asp Gln Asp
945                 950                 955                 960

Phe Gly Asp Asp Phe Asn Leu Trp Val Ser Ser Tyr Thr Gly Glu Pro
                965                 970                 975

Ile Pro Val Gly Gln Met Arg Glu Trp Gln Asp Ala Ala Val Asp Arg
            980                 985                 990

Ile Leu Ser Phe Thr Pro Arg Arg Val Leu Glu Val Gly Ala Gly Thr
        995                 1000                1005

Gly Leu Leu Leu Ala Arg Val Ala Gly Ser Val Glu Ala Tyr Trp Ala
    1010                1015                1020

Thr Asp Phe Ser Glu Pro Val Ile Glu Arg Leu Gly Arg Gln Val Thr
1025                1030                1035                1040

Glu Ala Gly Trp Ala Glu Arg Val Arg Leu Leu Cys Arg Arg Ala Asp
                1045                1050                1055

Asp Leu Asp Gly Ile Pro Arg Ile Phe Asp Thr Val Val Leu Asn Ser
            1060                1065                1070

Val Val Gln Tyr Phe Pro Asn Glu Arg Tyr Leu Glu Gln Val Leu Asp
        1075                1080                1085

Gly Val Trp Ala Met Leu Glu Pro Gly Gly Arg Leu Val Leu Gly Asp
    1090                1095                1100

Ile Arg Arg Ala Arg Ser Leu Arg Ala Phe Gln Val Ala Val Gln Gln
1105                1110                1115                1120

Ala Lys His Gly Asn Leu Pro Pro Ala Gln Leu Arg Ser Ala Val Glu
                1125                1130                1135

Gln Gly Leu Leu Leu Glu Lys Glu Leu Val Ile Asp Pro Glu Trp Phe
            1140                1145                1150

Gln Arg Trp Ala Glu Arg Ala Gly Ala Ala Gly Val Asp Val Arg Leu
        1155                1160                1165

Lys Glu Gly Ala Phe Gln Asn Glu Leu Thr Arg His Arg Tyr Glu Ile

1170                1175                1180

Val Val His Lys Pro Gly Thr Thr Glu Ala Gly Arg Pro Tyr Ala Val
1185                1190                1195                1200

Asp Thr Val Pro Arg Leu Glu Trp Asn Gly Asp Leu Asp Gly Leu Ala
                1205                1210                1215

Glu Arg Ile Arg Thr Leu Gly Gly Pro Val Val Arg Ile Ala Gly Ile
            1220                1225                1230

Pro Asn Ala Arg Val Ala Gln Glu Val Ala Ala Ala Arg Asp Leu Gly
        1235                1240                1245

Leu Glu Glu Ser Glu Pro Pro Thr Ser Val Pro Val Asp Pro His Glu
    1250                1255                1260

Met Ala Thr Trp Ala Ala Arg Gln Gly Trp Ser Ile Ala Leu Thr Trp
1265                1270                1275                1280

Ser Gly Ser Ala Val Asp Gln Phe Glu Ala Val Leu Phe Thr Asp Ala
                1285                1290                1295

Thr Thr Glu His Arg Ala Leu Ser Gly Thr Tyr Leu Pro Val Ala Ala
            1300                1305                1310

Ala Gly Thr Trp Ile Asn Asp Pro Ala Ala Ala Ala Gly Ile Thr Ala
        1315                1320                1325

Leu Pro Ala Val Leu Arg Glu Tyr Leu Ser Lys Thr Leu Pro Glu Tyr
    1330                1335                1340

Met Val Pro Ser Ala Ile Val Pro Ile Gly Arg Val Pro Leu Thr Pro
1345                1350                1355                1360

Asn Gly Lys Leu Asp Arg Ala Ala Leu Pro Val Pro Glu Tyr Ala Ala
                1365                1370                1375

Ala Ser Arg Gly Arg Pro Pro Cys Thr Ser Glu Glu Glu Ser Leu Cys
            1380                1385                1390

Glu Ile Phe Ala Gly Val Leu Gly Leu Asp Arg Val Gly Val Asp Asp
        1395                1400                1405

Asn Phe Phe Thr Ile Gly Gly His Ser Leu Leu Ala Thr Arg Val Val
    1410                1415                1420

Ser Arg Ile Arg Ala Ala His Gly Val Glu Ile Pro Ile Arg Thr Ile
1425                1430                1435                1440

Phe Glu Ala Pro Thr Val Ala Glu Leu Val Ala His Leu Thr Gly Gln
                1445                1450                1455

Arg Thr Val Arg Pro Pro Leu Arg Pro Gln Pro Arg Pro Asp Arg Leu

1460                 1465                 1470

Pro Val Ser Phe Ala Gln Gln Arg Leu Trp Phe Ile His Arg Phe Glu
        1475                1480                1485

Gly Pro Ser Pro Thr Tyr Ile Ile Pro Leu Ser Met Arg Leu Arg Gly
    1490                1495                1500

Lys Leu Asp Val Pro Ala Leu Arg Gln Ala Val His Asp Val Val Val
1505                1510                1515                1520

Arg His Glu Ser Leu Arg Thr Val Phe Asp Glu Val Asp Gly Ala Pro
                1525                1530                1535

Tyr Gln Arg Val Leu Asp Pro Asp Gly Leu Ala Ile Pro Trp Gln Glu
            1540                1545                1550

His Glu Val Thr Glu Ser Ala Leu Gln Glu Ala Leu Arg Leu Glu Ala
        1555                1560                1565

Arg Arg Pro Phe Asp Leu Ala Ala Glu Ile Pro Val Arg Ala Ala Leu
    1570                1575                1580

Phe Arg Val Gly Glu His Asp Ala Val Leu Leu Leu Pro Leu His His
1585                1590                1595                1600

Ile Ala Ala Asp Gly Trp Ser Leu Gly Pro Leu Ala Glu Asp Leu Thr
                1605                1610                1615

Ala Ala Tyr Thr Ala Arg Cys Asp Gly Gln Arg Pro Gln Trp Ala Ala
            1620                1625                1630

Leu Pro Val Gln Tyr Ala Asp Tyr Thr Leu Trp Gln Arg Asp Leu Leu
        1635                1640                1645

Gly Gly Asp Asp Pro His Ser Leu Tyr Arg Arg Gln Leu Asp Tyr Trp
    1650                1655                1660

Thr Gly Gln Leu Ala Gly Leu Pro Glu Ser Leu Gln Leu Pro Thr Asp
1665                1670                1675                1680

Arg Ala Arg Pro Ala Val Ala Ser Phe Ala Gly Glu Val Leu Pro Val
                1685                1690                1695

Glu Leu Asp Glu Ser Leu Thr Glu Asp Leu Arg Lys Leu Ala Leu Arg
            1700                1705                1710

Thr Gly Thr Thr Val Ser Met Val Leu Gln Ala Gly Leu Ala Gly Leu
        1715                1720                1725

Leu Ser Lys Leu Gly Ala Gly Asn Asp Ile Pro Ile Gly Ser Pro Ile
    1730                1735                1740

Ala Gly Arg Thr Asp Glu Ala Leu Asn Arg Leu Val Gly Phe Phe Val
1745                1750                1755                1760

Asn Thr Trp Val Ala Arg Val Asp Thr Ser Gly Ser Pro Ser Leu Ile
                1765                1770                1775

Glu Leu Val Asp Arg Val Arg Glu Val Ser Leu Ala Ala Tyr Asp His
            1780                1785                1790

Gln Asp Ile Pro Phe Glu His Leu Val Glu Ala Leu Asn Pro Val Arg
        1795                1800                1805

Ser Thr Ala His His Pro Leu Phe Gln Val Cys Leu Ala Leu Gln Asn
    1810                1815                1820

Asn Val Gln Pro Ala Phe Asp Leu Pro Gly Leu Lys Val Ser His Glu
1825                1830                1835                1840

Pro Val Asp Met Gly Val Ser Arg Phe Asp Leu Phe Leu Asn Leu Met
                1845                1850                1855

Glu Glu Thr Thr Gly Glu Gly Thr Ser Arg Ile Ala Gly Val Val Glu
            1860                1865                1870

Tyr Ala Thr Glu Leu Phe Asp Arg Asp Thr Val Thr Gly Leu Leu Glu
        1875                1880                1885

Arg Trp Arg Arg Leu Leu Arg Gln Trp Val Gly Ala Pro Asp Ala Pro
    1890                1895                1900

Ile Asp Ala Val Glu Ile Leu Asn Arg Asp Asp Leu Ala Ala Leu Glu
1905                1910                1915                1920

Arg Trp Thr Gly Arg Ala Arg Gly Thr Asp Arg Val Val Gly Thr Ile
                1925                1930                1935

Pro Glu Arg Phe Ala Ala Val Val Ala Glu Gln Pro Glu Ala Val Ala
            1940                1945                1950

Leu Val Ala Ala Asp Gly Glu Glu Ser Trp Thr Tyr Gly Glu Leu Asp
        1955                1960                1965

Arg Trp Ala Asn Arg Ile Ala His His Leu His Ala Arg Gly Val Gly
    1970                1975                1980

Arg Gln His Arg Val Ala Leu Val Met Glu Arg Ser Pro Leu Leu Val
1985                1990                1995                2000

Ala Ala Val Leu Gly Thr Leu Lys Ala Gly Ala Cys Tyr Val Pro Val
                2005                2010                2015

Glu Pro Thr Trp Pro Arg Ala Arg Ile Asp Leu Val Leu Ala Asp Leu
            2020                2025                2030

Asp Pro Ala Leu Val Ile Asp Glu Arg Leu Ala Glu Glu Asp Leu Thr
        2035                2040                2045

Gly Tyr Pro Thr Arg Pro Leu Asp Thr Ala Asp Val Gly Gly Glu His
    2050                2055                2060

Leu Ala Tyr Leu Met Tyr Thr Ser Gly Ser Thr Gly Thr Pro Lys Gly
2065                2070                2075                2080

Val Glu Val Ser His Arg Asn Val Leu Ser Leu Ala Leu Asp Pro Cys
                2085                2090                2095

Trp Ala Asp Ala Asp His Gln Arg Val Leu Val His Ala Pro Pro Thr
            2100                2105                2110

Phe Asp Ala Ser Thr Tyr Glu Met Trp Val Pro Leu Leu His Gly Gly
        2115                2120                2125

Ala Ala Val Val Ala Pro Pro Gly Lys Leu Asp Ala Ala Arg Leu Ala
    2130                2135                2140

Thr Leu Ile Ala Glu Arg Gly Val Thr Ala Leu Trp Leu Pro Ala Gly
2145                2150                2155                2160

Leu Phe Asp Leu Ile Thr Gln His His Pro Lys Ser Phe Val Gln Val
                2165                2170                2175

Arg Glu Val Trp Ala Gly Gly Asp Val Leu Ser Pro Ala Ala Val Arg
            2180                2185                2190

Arg Leu Val Arg Asp Asp Gly Thr Leu Thr Val Val Asn Gly Tyr Gly
        2195                2200                2205

Pro Thr Glu Thr Thr Thr Phe Ala Ala Arg Tyr Arg Met Ser Ala Pro
    2210                2215                2220

Ala Arg Cys Lys Asp Pro Leu Pro Ile Gly Glu Pro Met Ala Gly Ser
2225                2230                2235                2240

Arg Leu Tyr Ala Leu Asp Asp Arg Leu Arg Gln Val Pro Gln Gly Val
                2245                2250                2255

Ile Gly Glu Leu Tyr Val Gly Gly Asp Gly Val Ala Arg Gly Tyr Ala
            2260                2265                2270

Asn His Pro Pro Leu Thr Ser Glu Arg Phe Val Ala Asp Pro Phe Gly
        2275                2280                2285

Arg Pro Gly Glu Arg Met Tyr Arg Thr Gly Asp Leu Val Arg Trp Asn
    2290                2295                2300

His Asp Gly Gln Leu Glu Phe Leu Gly Arg Val Asp Glu Gln Val Lys
2305                2310                2315                2320

Ile Arg Gly Phe Arg Val Glu Pro Gly Glu Ile Arg Ala Ala Leu Arg
                2325                2330                2335

Lys Arg Asp Gly Val Ala Gln Ala Val Val Val Pro Arg Thr Asp Arg
            2340                2345                2350

Leu Gly Glu Arg Arg Leu Val Ala Tyr Val Val Pro Glu Val Pro Ala
        2355                2360                2365

Gly Ala Asp Glu Asp Ser Thr Glu His Val Glu Lys Trp Arg Ala Ile
    2370                2375                2380

Tyr Asp Ser Met Tyr Asp Glu Thr Glu Ala Asp Ala Thr Glu Ile Gly
2385                2390                2395                2400

Asn Asp Phe Thr Gly Trp Lys Ser Ser Tyr Thr Arg Asp Asn Ile Pro
                2405                2410                2415

Leu Ser Glu Met Arg Arg Trp Arg Asp Ser Val Val Glu Glu Val Arg
2420 2425 2403

Gly Leu Arg Ala Arg Arg Ile Leu Glu Ile Gly Val Gly Ser Gly Leu
        2435                2440                2445

Leu Leu Gly Pro Leu Ala Pro Glu Ala Glu Ala Tyr Trp Gly Thr Asp
    2450                2455                2460

Phe Ser Leu Pro Val Ile Glu Arg Leu Glu Val Gln Val Gly Thr Asp
2465                2470                2475                2480

Pro Cys Leu Lys Glu Lys Val Ser Leu Arg Cys Gln His Ala Asp Val
                2485                2490                2495

Ala Asp Gly Leu Pro Val Lys Tyr Phe Asp Thr Val Ile Leu Asn Ser
            2500                2505                2510

Val Val Gln Tyr Phe Pro Asp Ala Ala Tyr Leu Ser Arg Val Leu Asp
        2515                2520                2525

Val Ala Leu Asp Arg Leu Ala Pro Gly Gly Arg Ile Leu Val Gly Asp
    2530                2535                2540

Val Arg Asn Tyr Gly Thr Leu Arg Glu Phe Leu Thr Ala Val His His
2545                2550                2555                2560

Ala Gln His Pro Gln Asp Ser Ala Ser Ala Val Arg Ala Ala Val Glu
                2565                2570                2575

Arg Ala Val Leu Ala Glu Lys Glu Leu Val Ile Asp Pro Asp Phe Phe
            2580                2585                2590

Thr Glu Trp Ala Arg Thr Arg Pro Asp Val Val Ala Val Asp Ile Arg
        2595                2600                2605

Leu Lys Pro Gly Ala Asp Gln Asn Glu Leu Thr Arg His Arg Tyr Glu
    2610                2615                2620

Val Ile Leu His Lys Gln Pro Ser Gln Pro Leu Arg Leu Ala Asp Val
2625                2630                2635                2640

Arg Thr Ala Asn Trp Gly Ser Glu Val Pro Asp Leu Ser Gly Leu Glu
                2645                2650                2655

Thr Ala Leu Ala Arg His Gly Gly Arg Leu Arg Leu Ala Arg Ile Pro
            2660                2665                2670

Asn Ala Arg Leu Val Ser Glu Ala Val Gln Cys Gly Val Pro Thr Asn
        2675                2680                2685

Val Gly Gly Thr Pro Leu Asp Pro His Glu Leu Ala Ser Trp Gly Gly
    2690                2695                2700

Gln Arg Gly Tyr Ser Val His Cys Thr Trp Ser Ala Glu Ala Pro Gly
2705                2710                2715                2720

Trp Phe Glu Ala Val Ile Ile Pro Val Asp Ser Gly His Cys Arg Asp
                2725                2730                2735

Gly Val Tyr Arg Pro Val Gly Pro Arg Pro Arg Gln Leu Val Asn Leu
            2740                2745                2750

Pro Ala Ala Ala Arg Arg Val Ser Arg Leu Pro Ser Trp Leu Arg Glu
        2755                2760                2765

Glu Leu Ala Ala Glu Leu Pro Glu His Leu Val Pro Gly Asp Ile Val
    2770                2775                2780

Val Met Glu Arg Leu Pro Leu Thr Thr Asn Gly Lys Ile Asp His Ser
2785                2790                2795                2800

Arg Leu Pro Glu Val Glu Gln Ala Asp Arg Glu Tyr Gly Ser Pro Arg
                2805                2810                2815

Thr Pro Leu Glu Ala Glu Leu Ala Gly Leu Phe Ala Glu Val Leu Gly
            2820                2825                2830

Val Asp Arg Val Gly Ile Glu Asp Asp Phe Phe Asp Cys Gly Gly Ser
        2835                2840                2845

Ser Leu Gln Val Ile Arg Leu Ile Trp Arg Ile Arg Ala Glu Leu Gly
    2850                2855                2860

Phe Asp Ile Pro Val Arg Thr Ile Phe Gln His Pro Thr Val Ala Glu
2865                2870                2875                2880

Val Ala Glu His Leu Ala Ala Gly Arg Glu Asp Val Glu Phe Asp Asp
                2885                2890                2895

Pro Phe Ser Val Val Leu Pro Ile Arg Thr Asp Gly Asp Arg Ala Pro
            2900                2905                2910

Ile Trp Leu Val Pro Pro Gly Gly Gly Leu Ser Trp Ala Tyr Leu Gly

2915                2920                2925

Phe Ala Gln His Leu Asp Arg Ser Arg Pro Ile Tyr Gly Leu Gln Ala
    2930                2935                2940

Arg Gly Phe Val Gly Asp Pro Arg Ala Thr Ser Ile Glu Ser Met Val
2945                2950                2955                2960

Asp Asp Tyr Val Asp Gln Ile Leu Arg Val Gln Pro Glu Gly Pro Tyr
                2965                2970                2975

Asn Val Leu Gly Trp Ser Leu Gly Gly Pro Ile Ala Gln Ala Val Ala
            2980                2985                2990

Ala Glu Leu Gln Arg Arg Glu Cys Glu Val Asp Leu Leu Ala Val Leu
        2995                3000                3005

Asp Ser Gly Pro Ala Ser Tyr Phe Ala Asp Phe Gln Thr Pro Asp Glu
    3010                3015                3020

Lys Leu Val Arg Arg Tyr Leu Ala His Tyr Met Gly His Leu Ala Gly
3025                3030                3035                3040

Met Ala Glu Phe Glu Ser Leu Val Gln Thr Ser Thr Thr Leu Phe Ile
                3045                3050                3055

Glu His Thr Asp Leu Met Thr Gln Phe Thr Ser Pro Pro Phe Arg Gly
            3060                3065                3070

Asp Leu Ile Phe Phe Ala Ala Leu Ile Asp Gln Gly Thr Arg Glu Arg
        3075                3080                3085

Arg Gln Leu Glu Val Glu Leu Asp Val Gln Trp Arg Glu Tyr Thr Glu
    3090                3095                3100

Gly Thr Val Arg Arg Phe Asp Ile Glu Cys Ala His Asn Glu Met Met
3105                3110                3115                3120

Trp Pro Glu Asn Ala Ala Met Ile Ser Arg Ile Val Asn Glu Ile Ile
                3125                3130                3135

Asp Ser Ala Arg
            3140

<210>  23
<211>  71
<212>  PRT
<213>  Micromonospora sp., ML1 strain

<220>
<223>  Tio22 Protein

<400>  23
Met Ser Val Asn Pro Phe Asp Asp Glu Asp Gly Glu Phe Tyr Val Leu
1               5                   10                  15

Val Asn Asp Glu Glu Gln His Ser Leu Trp Pro Thr Phe Gly Asp Val
            20                  25                  30

Pro Asp Gly Trp Arg Ile Val Phe Gly Pro Ala Gly Arg Ala Glu Ser
        35                  40                  45

Val Ala Tyr Val Glu Glu Asn Trp Thr Asp Met Arg Pro Lys Ser Leu
    50                  55                  60

Arg Glu Ala Met Ser Ala Ala
65                  70


<210>  24
<211>  874
<212>  PRT
<213>  Micromonospora sp., ML1 strain

<220>
<223>  Tio23 Protein

<400>  24
Val Arg Arg Cys Arg Gln His Asp Val Arg Arg Gly Leu Ala Gly Arg
1               5                   10                  15

Arg Val Arg Leu Arg Ala His Asp Phe Glu Leu Pro Arg Arg Arg Trp
            20                  25                  30

Arg Lys Val Arg Thr Glu Val Phe Thr Gly Cys Leu Gly Ile Glu Leu
        35                  40                  45

Gly Cys Gly Lys Pro Glu Ile Ala Gly Gly Val Ile Ser Pro Gly His
    50                  55                  60

Pro Ala Gly Ser Ala Ser Arg Ala Arg His Phe Gly Gly Pro Met Thr
65                  70                  75                  80

Val Ala Ser Pro Arg Gln Ser Arg Glu Asp Leu Ser Leu Val Pro Asp
                85                  90                  95

Thr His Asp Val Ile Arg Val Val Gly Ala Arg Glu Asn Asn Leu Lys
            100                 105                 110

Asp Ile Arg Leu Glu Ile Pro Lys Arg Arg Leu Thr Val Phe Thr Gly
        115                 120                 125

Val Ser Gly Ser Gly Lys Ser Ser Leu Val Phe Asp Thr Ile Ala Ala
    130                 135                 140

Glu Ser Gln Arg Leu Ile Asn Glu Thr Tyr Ser Ala Phe Val Gln Gly

145                 150                 155                 160

Phe Met Pro Thr Leu Ser Arg Pro Asp Val Asp Val Leu Asp Gly Leu
                165                 170                 175

Thr Thr Ala Ile Ile Val Ser Gln Glu Arg Ile Gly Asn Asn Pro Arg
            180                 185                 190

Ser Thr Val Gly Thr Val Thr Asp Ala Asn Ala Met Leu Arg Ile Leu
        195                 200                 205

Phe Ser Arg Leu Gly Glu Pro Arg Leu Gly Ser Pro Arg Ser Tyr Ser
    210                 215                 220

Phe Asn Ile Pro Ser Ile Ser Val Ala Asn Ala Val Thr Val Gly Gln
225                 230                 235                 240

Gly Ser Arg Ala Val Glu Glu Gln Arg Gly Phe Asp Val Ala Gly Gly
                245                 250                 255

Met Cys Pro Arg Cys Glu Gly Thr Gly Ser Val Asn Asp Val Asp Leu
            260                 265                 270

Thr Gln Leu Tyr Asp Ala Thr Lys Ser Leu Asp Glu Gly Ala Ile Arg
        275                 280                 285

Val Pro Gly Tyr Gln Val Gly Gly Tyr Asn His Arg Leu Tyr Ala Gly
    290                 295                 300

Ser Gly Leu Leu Asp Pro Gly Lys Pro Val Glu Lys Tyr Thr Glu Lys
305                 310                 315                 320

Glu Leu Asn Asp Phe Leu His Arg Glu Pro Thr Arg Leu Lys Ile Gly
                325                 330                 335

Asp Tyr His Thr Asn Tyr Glu Gly Leu Val Ala Arg Val Gln Lys Ser
            340                 345                 350

Leu Leu Thr Lys Asp Ile Glu Ala Leu Gln Pro His Val Arg Ala Phe
        355                 360                 365

Val Glu Arg Ala Val Thr Phe Thr Ala Cys Pro Asp Cys Gly Gly Thr
    370                 375                 380

Arg Leu Asn Glu Gln Ala Arg Ala Ser Arg Ile Lys Gly Ile Asn Ile
385                 390                 395                 400

Ala Asp Ala Ser Ala Met Gln Ile Asn Asp Leu Ala Glu Trp Val Arg
                405                 410                 415

Asp Leu Ala Glu Pro Ser Val Ala Pro Leu Leu Ala Thr Leu Leu His
            420                 425                 430

Thr Leu Asp Ser Phe Val Gly Ile Gly Leu Gly Tyr Leu Ser Leu Asp

435                 440                 445

Arg Ala Ser Gly Thr Leu Ser Gly Gly Glu Ala Gln Arg Val Lys Met
    450                 455                 460

Ile Arg His Leu Gly Ser Pro Leu Thr Asp Val Thr Tyr Val Phe Asp
465                 470                 475                 480

Glu Pro Thr Ile Gly Leu His Pro His Asp Ile Gln Arg Met Asn Lys
                485                 490                 495

Leu Leu Leu Arg Leu Arg Asp Lys Gly Asn Thr Val Leu Val Val Glu
            500                 505                 510

His Lys Pro Glu Thr Ile Ala Ile Ala Asp Arg Val Val Asp Leu Gly
        515                 520                 525

Pro Gly Ala Gly Thr Ala Gly Gly Thr Ile Cys Phe Glu Gly Thr Val
    530                 535                 540

Ala Glu Leu Arg Ser Ala Asp Thr Leu Thr Gly Arg His Leu Asp Asp
545                 550                 555                 560

Lys Ala Ser Leu Lys Asp Arg Val Arg Thr Pro Ser Gly Ser Leu Glu
                565                 570                 575

Val Arg Gly Ala Ser Lys His Asn Leu Arg Asp Val Asp Val Asp Ile
            580                 585                 590

Pro Leu Gly Val Leu Val Val Val Thr Gly Val Ala Gly Ser Gly Lys
        595                 600                 605

Ser Ser Leu Ile Glu Gly Ser Val Ser Gly Arg Asp Gly Val Val Thr
    610                 615                 620

Ile Asp Gln Gly Ser Ile Lys Gly Ser Arg Arg Ser Asn Pro Ala Thr
625                 630                 635                 640

Tyr Thr Gly Leu Leu Asp Pro Ile Arg Lys Ala Phe Ala Lys Ala Asn
                645                 650                 655

Gly Val Lys Pro Ala Leu Phe Ser Ser Asn Ser Glu Gly Ala Cys Ala
            660                 665                 670

Thr Cys Lys Gly Ala Gly Val Ile Tyr Thr Asp Leu Ala Ile Met Ala
        675                 680                 685

Gly Ala Ala Thr Thr Cys Asp Asp Cys Glu Gly Arg Arg Phe Gln Asp
    690                 695                 700

Phe Val Leu Gln Tyr Arg Leu Gly Gly Arg Asp Ile Ser Glu Val Leu
705                 710                 715                 720

Ala Met Ser Val Ala Glu Ala Gly Arg Phe Phe Ala Ala Gly Gln Ala
                725                 730                 735

Arg Thr Pro Ala Ala Val Val Ile Leu Glu Arg Leu Ala Asp Val Gly
            740                 745                 750

Leu Gly Tyr Leu Ser Leu Gly Gln Pro Leu Pro Thr Leu Ser Gly Gly
        755                 760                 765

Glu Arg Gln Arg Leu Lys Leu Ala Ala His Leu Ala Asp Glu Gly Gly
    770                 775                 780

Ile Val Val Leu Asp Glu Pro Thr Thr Gly Leu His Leu Ala Asp Val
785                 790                 795                 800

Glu Gln Leu Leu Gly Leu Leu Asp Arg Leu Val Asp Ala Gly Arg Ser
                805                 810                 815

Val Met Val Ile Glu His His Gln Ala Val Met Ala His Ala Asp Trp
            820                 825                 830

Ile Ile Asp Leu Gly Pro Ala Ala Gly His Asp Gly Gly Arg Val Val
        835                 840                 845

Phe Glu Gly Cys Pro Ala Asp Leu Val Ala Ser Arg Ala Thr Leu Thr
    850                 855                 860

Gly Glu His Leu Ala Ala Tyr Val Gly Val
865                 870

<210>  25
<211>  178
<212>  PRT
<213>  Micromonospora sp., ML1 strain

<220>
<223>  Tio24 Protein

<400>  25
Val Gly Gly Arg Asn Ser Asp Val Val Phe Ile Arg Trp Pro Ala Gln
1               5                   10                  15

Ala Asp Leu Arg Thr Arg Cys Arg Lys Glu Gly Val Pro Arg Leu Leu
            20                  25                  30

Val Val Glu Gly Gly Ala Gln Pro Pro Ile Ser Asn Asp Pro Val Glu
        35                  40                  45

Asp Trp Val Arg Pro Pro Val Ser Arg Asn Asp Leu Glu Ala Arg Val
    50                  55                  60

Lys Ala Leu Gln Gln Arg Phe Asn Ser Arg Arg Leu Pro Thr Ile Asp
65                  70                  75                  80

Ser Thr Gly Thr Leu Thr Phe Gly Arg His Ser Ile Val Ile Ser Asn

85                  90                  95

Thr Gln Ala Glu Leu Met Arg Leu Leu Ile Glu Arg Phe Gly Glu Val
            100                 105                 110

Val Tyr Arg Ile Glu Phe Val Gln Cys Leu Ala Ile Arg Thr Pro Ala
        115                 120                 125

Leu Asn Arg Asn Ser Leu Asp Leu His Ile Met Arg Leu Arg Arg Arg
    130                 135                 140

Leu Leu Pro Ile Asp Leu Ala Ile Arg Thr Val Trp Gly Arg Gly Tyr
145                 150                 155                 160

Met Leu Ala Tyr Ala Ala Ser Ala Glu Ala Ile Ile Arg Asn Ala Ser
                165                 170                 175

Gly Met

<210>  26
<211>  287
<212>  PRT
<213>  Micromonospora sp., ML1 strain

<220>
<223>  Tio25 Protein

<400>  26
Val Ala Asp Ala Leu Ser Ser Arg Pro Cys Ser Thr Ala Ala Ile Lys
1               5                   10                  15

Arg Asp His Ile Met Leu Ser Ser His Thr Val Ile Val Leu His Ala
            20                  25                  30

His Pro Asp Asp Glu Ala Ile Phe Thr Gly Leu Thr Met Arg Arg Leu
        35                  40                  45

Ala Asp Ala Gly Ala Arg Val Ile Leu Val Met Ala Thr Asp Gly Ala
    50                  55                  60

Ala Gly Val Pro His Val Pro Leu Ser Arg Gly Glu Ser Leu Arg Ser
65                  70                  75                  80

Arg Arg Leu Ala Glu Leu Glu Arg Ala Cys Asp Leu Leu Gly Val Ala
                85                  90                  95

Arg Leu Glu Val Leu Gly Tyr Ala Asp Ser Gly Ala His Gly Gly Pro
            100                 105                 110

Tyr Arg Ala Gly Thr Leu Gly Ala Ala Ser Ala Thr Arg Val Ala Arg
        115                 120                 125

Gln Val Ala Arg Ile Ala Ser Arg Glu Gly Ala Thr Thr Leu Val His

130                 135                 140

Tyr Asp Pro Gly Gly Ile Tyr Arg His Val Asp His Val Gln Val His
145                 150                 155                 160

Arg Val Gly Ala Leu Val Thr Arg Ser Leu Gln Leu Ala Gly Tyr Gln
                165                 170                 175

Ala Thr Val Asp Ala Ser Ala Leu Arg Ala Gly Pro Arg His Val Leu
            180                 185                 190

Gln Arg Ala Ala Gly Asp Leu Thr Asp Met Gly Val Pro Ala Ser Arg
        195                 200                 205

Ile Ser Leu Ala Val Asp Ala Glu Gly Ala Asp Leu Leu Ala Lys Met
    210                 215                 220

Ala Ala Met Ala Thr His Ala Ser Gln Val Gly Ala Ala Asp Leu Asp
225                 230                 235                 240

Pro Ser Cys Phe Ala Ala Ala Tyr Gly Arg Glu Trp Phe Val Arg Glu
                245                 250                 255

Gly Arg Leu Gly Ala Leu Asp Ala Leu Leu Ala Ala Ser Ala Gly Pro
            260                 265                 270

Ala Val Ala Thr His Arg Asn Leu Gln Leu Asp Gly Thr Ala Ile
        275                 280                 285


<210>  27
<211>  134
<212>  PRT
<213>  Micromonospora sp., ML1 strain

<220>
<223>  Tio26 Protein


<400>  27
Val Val Val Glu Leu Ala Tyr Thr Asp Pro Asp Arg Ala Val Asp Trp
  1               5                  10                  15

Leu Val Arg Val Phe Gly Phe Arg Leu Leu Leu Arg Gln Pro Ala Ile
            20                  25                  30

Gly Thr Ile Arg His Ala Asp Leu Asp Thr Gly Gly Gly Ile Val Met
        35                  40                  45

Val Arg Arg Thr Gly Glu Pro Tyr Thr Val Ser Cys Ala Gly Gly His
    50                  55                  60

Thr Cys Lys Gln Val Ile Val Trp Val Ser Asp Val Asp Glu His Phe
65                  70                  75                  80

Met Arg Ser Thr Ala Ala Gly Ala Asp Ile Val Gln Pro Leu Gln Asp
                85                  90                  95

Lys Pro Trp Gly Leu Arg Gln Tyr Leu Val Arg Asp Leu Glu Gly His
            100                 105                 110

Leu Trp Glu Phe Thr Arg His Leu Arg Asp Val Pro Pro Arg Glu Trp
        115                 120                 125

Gly Ala Val Ala Met Thr
    130


<210>  28
<211>  1804
<212>  PRT
<213>  Micromonospora sp., ML1 strain

<220>
<223>  Tio27 Protein

<400>  28
Val Gly Leu Gln Leu Leu Gly Ser Ala Thr Gln Ser Thr Glu Pro Asn
1               5                   10                  15

Leu Pro Gly Leu Thr Val Thr Ser Leu Asp Ala Asn Pro Gly Gly His
            20                  25                  30

Pro Phe Asp Leu Ser Ile Thr Cys Thr Glu Ser Pro Asp Gln Leu Arg
        35                  40                  45

Leu Val Ala Asp Tyr Ser Val Asp Leu Phe Asp Glu Pro Arg Met Ala
    50                  55                  60

Arg Leu Leu Arg His Leu Glu Arg Val Leu Arg Thr Ala Val Asp Ala
65                  70                  75                  80

Pro Asp Thr Pro Leu Ser Ala Leu Pro Leu Leu Asp Glu Glu Glu Gln
                85                  90                  95

Thr Leu Leu Leu His Thr Trp Gln Gly Pro Gln Gly Glu Arg Ala Arg
            100                 105                 110

Glu Pro Val His Val Gln Ile Ala Arg Leu Ala Ala Glu Arg Pro Asp
        115                 120                 125

Leu Val Ala Ala Thr Leu His Gly Ala Glu Leu Thr Tyr Gly Glu Leu
    130                 135                 140

Asp Arg Arg Ala Gly Val Leu Ala His Arg Phe Arg Ala Leu Gly Val
145                 150                 155                 160

Gly Arg Asp Asp Ile Val Ala Leu Leu Leu Glu Arg Gly Phe Asp Leu
                165                 170                 175

Ile Val Gly Met Val Ala Ala Gln Lys Ala Gly Gly Ala Phe Val Val
            180                 185                 190

Met Asp Pro Gly His Pro Val Arg Arg Ile Glu Phe Ile Leu Glu Asp
        195                 200                 205

Thr Ala Ala Lys Ala Val Val Thr Arg Ser Ala Leu Ala Asp Arg Leu
    210                 215                 220

Pro Glu Gly Ala Ala Ala Thr Pro Val Leu Val Asp Thr Glu Trp Asp
225                 230                 235                 240

Glu Leu Ser Ala Ala Ala Ala Gly Ala Ala Pro Leu Asp Glu Leu Ala
                245                 250                 255

Asp Glu Asn Ser Leu Ala Tyr Val Leu Tyr Thr Ser Gly Ser Thr Gly
            260                 265                 270

Lys Pro Lys Gly Val Met Ile Glu His His Ala Leu Asn Thr Phe Leu
        275                 280                 285

Leu Trp Leu Gly Asn Ile Phe Asp Phe Gly Pro Gly Asp Arg Leu Leu
    290                 295                 300

Gln His Met Ala Pro Ile Phe Asp Phe Ala Glu Gly Glu Ile Phe Thr
305                 310                 315                 320

Ala Leu Thr Arg Gly Val Thr Met Val Phe Val Pro Glu Glu His Arg
                325                 330                 335

Thr Asp Pro Asp Val Ile Gly Gln Leu Leu Val Ser Glu Arg Ile Thr
            340                 345                 350

Tyr Ile Gly Gly Pro Pro Ala Ile Leu Gly Arg Ile Pro Pro Gly Ser
        355                 360                 365

Tyr Pro Asp Leu Lys Tyr Met Ile Ala Gly Gly Glu Ala Val Thr Gly
    370                 375                 380

Asp Leu Ile Asn Arg Trp Asn Thr Pro Gly Arg Arg Phe Ile Asn Gly
385                 390                 395                 400

Tyr Gly Pro Thr Glu Ala Ala Val Gly Cys Ile Phe Tyr Glu Cys Glu
                405                 410                 415

His Arg Thr Trp Ile Gly Gln Pro Pro Ile Gly Arg Ala Met Pro Asn
            420                 425                 430

Arg Val Ala Tyr Val Leu Asp Arg Trp Asn Asn Leu Gln Pro Val Gly
        435                 440                 445

Val Pro Gly Glu Val Ile Thr Gly Gly Glu Gly Leu Ala Arg Gly Tyr
    450                 455                 460

Leu Asn Arg Pro Glu Leu Thr Ala Glu Lys Phe Ile Asp Asn Pro Tyr
465                 470                 475                 480

Arg Pro Gly Glu Arg Met Tyr Arg Thr Gly Asp Leu Gly Val Trp Thr
                485                 490                 495

Glu Asp Gly Gln Ile Gln Phe Leu Gly Arg Ile Asp Thr Gln Val Lys
            500                 505                 510

Leu Asn Gly Leu Arg Ile Glu Leu Glu Glu Ile Glu Ser Thr Leu Thr
        515                 520                 525

Ala His Pro Gly Val Ala Glu Ala Ala Val Ala Leu Arg Glu Asp Ser
    530                 535                 540

Pro Gly Ser Lys Arg Leu Val Gly Tyr Val Val Pro Ala Gly Ala Ala
545                 550                 555                 560

Ala Pro Pro Thr Glu Glu Leu Arg Glu His Leu Leu Ala Asp Leu Pro
                565                 570                 575

Pro Tyr Met Val Pro His Val Phe Val Ala Leu Asp Val Leu Pro Leu
            580                 585                 590

Thr Ser Val Gly Lys Val Asp Arg Asn Arg Leu Pro Val Pro Glu Glu
        595                 600                 605

Thr Gly Ser Ala Glu Val Glu Leu Val Ala Pro Arg Thr Pro Glu Glu
    610                 615                 620

Glu Arg Val Ala Glu Ile Phe Ala Ala Val Leu Gly Val Glu Arg Val
625                 630                 635                 640

Gly Ala Asp Ala Asn Phe Phe Glu Leu Gly Gly Asn Ser Leu Met Ala
                645                 650                 655

Ala Arg Val Leu Ser Arg Ile Ala Glu Leu Gly Gly Ala Ser Ala Thr
            660                 665                 670

Met Arg Asp Phe Tyr Thr Lys Pro Arg Val Ser Glu Ile Ala Gln Leu
        675                 680                 685

Leu Ala Gly Thr Pro Ser Ser Thr Asp Pro Ala Thr Pro Thr Asp Ala
    690                 695                 700

Ala Pro Val Ala Asp Gln Thr Arg Ala Arg Leu Glu Gln Glu Ile Ala
705                 710                 715                 720

Asp Leu Glu Gln Arg Leu Arg Ala Ala Arg Ala Ala Leu Ala Gly Gln
                725                 730                 735

Asp Gly Pro Val Ser Gly Gly His Glu Gly Gln Arg Leu Pro Leu Ser
            740                 745                 750

Pro Thr Gln Glu Gln Leu Trp Phe Leu Glu Gln Leu Ala Pro Gly Gln
        755                 760                 765

Pro Thr Tyr His Ile Pro Val Pro Val Arg Leu Thr Gly Pro Leu Asp
    770                 775                 780

Val Asp Leu Leu Gly Arg Ala Leu Thr Ala Val Val Ala Arg His Glu
785                 790                 795                 800

Ala Leu Arg Thr Thr Phe Gly Thr Asp Asp Asp Thr Pro Tyr Gln Leu
                805                 810                 815

Val Ser Pro Ala Gly Glu Val Pro Leu Pro Val His Glu Leu Asp Ala
            820                 825                 830

Thr Ala Ala Asp Asp Arg Glu Gln Ala Leu Arg Val Glu Leu Glu Arg
        835                 840                 845

Asp Ala Thr Glu Pro Phe Asp Leu Gly Arg Asp Leu Met Leu Arg Ala
    850                 855                 860

Arg Leu Ile Arg Leu Asp Gly Gln Glu His Val Leu Ser Met Val Leu
865                 870                 875                 880

His His Ile Cys Ala Asp Gly Trp Ser Ala Gly Leu Leu Val Arg Asp
                885                 890                 895

Leu Ala Glu Leu Tyr Asp Ala Leu Arg His Asp Arg Gln Pro Gln Leu
            900                 905                 910

Pro Ala Leu Arg Leu Gln Tyr Ala Asp His Ala Leu Ala Gln Arg Arg
        915                 920                 925

Gln Leu Asp Ala Gly Asp Leu Glu Ser Gln Leu Ala Tyr Trp Ala Arg
    930                 935                 940

Gln Leu Ala Asp Ala Pro Ile Leu Asp Leu Pro Ser Asp Arg Pro Arg
945                 950                 955                 960

Pro Ala Thr Ala Thr Asn Arg Gly Ala Leu Leu Val Arg Arg Met Pro
                965                 970                 975

Ala Asp Val Leu Glu Gly Leu Arg Arg Val Ala Gln Gln Glu Gln Ala
            980                 985                 990

Ser Thr Tyr Ala Val Leu Leu Ala Gly Phe Lys Thr Val Leu Met Arg
        995                 1000                1005

Tyr Thr Gly Val Asp Asp Val Val Val Gly Thr Ala Gly Ala Gly Arg
    1010                1015                1020

Gly Arg Pro Glu Leu Glu Asp Leu Val Gly Phe Phe Val Asn Met Leu
1025                1030                1035                1040

Ala Leu Arg Thr Asp Leu Ser Gly Asp Pro Ser Phe Gly Glu Ala Val
                1045                1050                1055

Arg Arg Thr Met Gly Thr Leu Leu Ala Ala Trp Asp His Gln Asp Ala
            1060                1065                1070

Pro Phe Glu Arg Val Val Gly Arg Leu Gly Arg Arg Arg Asp Ala Ser
        1075                1080                1085

Arg Asn Pro Val Phe Gln Val Ser Ile Asp Leu Leu Ser Gly Asp Leu
    1090                1095                1100

Val Asp Phe Arg Phe Pro Gly Leu Ala Ala Glu Phe Leu Asp Val Asp
1105                1110                1115                1120

Pro Gly Val Ser Arg Phe Asp Met Ala Ile Asn Ala Tyr Glu Glu Thr
                1125                1130                1135

Asp Gly Ile Thr Phe Arg Ile Glu Tyr Ala Thr Asp Leu Phe Asp Thr
            1140                1145                1150

Ala Arg Ile Glu Ala Met Phe Gly His Leu Glu Arg Val Leu Arg Ala
        1155                1160                1165

Ala Val Ala Ala Pro Gln Thr Pro Leu Ser Ala Leu Pro Leu Leu Asp
    1170                1175                1180

Asp Thr Glu Arg Thr Glu Leu Leu Ala Leu Ala Gln Gly Lys Arg Arg
1185                1190                1195                1200

Pro His Arg Pro Ala Thr Leu Pro Glu Leu Phe Ala Ala Ser Val Ala
                1205                1210                1215

Arg Thr Pro Gln Ala Thr Ala Val Ile Ala Ala Glu Gly Thr Leu Ser
            1220                1225                1230

Tyr Ala Glu Leu Asp Glu Arg Ser Arg Arg Leu Ala Ala Arg Leu Arg
        1235                1240                1245

Ala Ala Gly Val Gly Thr Gly Asp Val Val Pro Val Leu Leu Glu Arg
    1250                1255                1260

Ser Leu Ala Glu Val Val Ser Val Leu Ala Val Leu Arg Ala Gly Ala
1265                1270                1275                1280

Ala Tyr Ala Pro Met Asp Pro Ala Ala Pro Pro Ser Arg Leu Ala Phe
                1285                1290                1295

Gln Val Arg Asp Thr Gly Ala Arg Val Val Val Gly Asp Pro Thr Arg
            1300                1305                1310

Leu Asp Thr Val Val Gly Val Thr Ile Val Asp Pro Ala Ala Glu Leu
        1315                1320                1325

Pro Glu Ala Ala Asp Glu Pro Trp Leu Ala Glu Leu Thr Pro Ala Ser

1330                1335                1340

Leu Ala Tyr Val Leu Tyr Thr Ser Gly Ser Thr Gly Glu Pro Lys Gly
1345                1350                1355                1360

Val Leu Ile Glu His Gly Met Val Ala Gly Tyr Leu Glu Trp Met Val
                1365                1370                1375

Pro Asp Phe Gly Val Glu Pro Arg Ser Arg Met Met His Cys Cys Ala
            1380                1385                1390

Pro Val Phe Asp Leu Ala Val Gly Glu Leu Phe Gly Ala Leu Thr Ala
        1395                1400                1405

Gly Ala Thr Val Val Val Ala Gly His Asp Asp Val His Arg Pro Gly
    1410                1415                1420

Ala Leu Thr Ala Leu Ala Arg Glu Thr Gly Ala Thr His Ile Phe Thr
1425                1430                1435                1440

Thr Pro Thr Val Leu Gly Leu Leu Asp Pro Ala Glu Leu Pro Gln Leu
                1445                1450                1455

Arg Ser Val Met Met Ala Gly Glu Pro Ala Pro Pro Ser Leu Val Ala
            1460                1465                1470

Ala Trp His Val Asp Gly Arg Arg Val Leu Asn Leu Tyr Gly Pro Ala
        1475                1480                1485

Glu Thr Thr Val Gly Cys Thr Trp Tyr Glu Cys Pro Ala Gly Thr Ala
    1490                1495                1500

Pro Ala Ala Thr Val Pro Ile Gly Arg Pro Met Pro Asn Arg Arg Val
1505                1510                1515                1520

Tyr Val Val Asp Asp Ser Gly Asp Leu Ala Pro Val Gly Val Pro Gly
                1525                1530                1535

Glu Leu Val Val Ala Gly Asn Gly Val Ala Arg Gly Tyr Leu Asp Arg
            1540                1545                1550

Pro Glu Leu Thr Ala Ser Ala Phe Gly Ala Asp Glu Arg Ala Gly Glu
        1555                1560                1565

Gly Arg Ala Tyr Arg Thr Gly Asp Leu Ala Arg Trp Asn Arg Asp Gly
    1570                1575                1580

Gln Leu Glu Phe Leu Gly Arg Arg Asp Gly Gln Val Lys Leu Arg Gly
1585                1590                1595                1600

Leu Arg Val Glu Leu Gly Glu Val Glu His Ala Leu Arg Thr Ala Ala
                1605                1610                1615

Gly Val Ser Gly Ala Ala Ala Ala Val His Gly Thr Gly Ala Glu Ala

1620                1625                1630

Val Leu Val Gly Tyr Val Val Gly Glu Leu Ser Asp Asp Asp Ala Glu
        1635                1640                1645

Ala Leu Arg Thr Thr Leu Ala Gly Lys Leu Pro Gly Tyr Met Val Pro
    1650                1655                1660

Asn Val Val Leu Arg Leu Pro Gln Leu Pro Leu Thr Arg Thr Gly Lys
1665                1670                1675                1680

Val Asp Arg Lys Arg Leu Pro Ala Pro Glu Ser Gly Ala Gly Gln Ala
                1685                1690                1695

Ala Tyr Val Pro Pro Gly Thr Asp Asp Glu Arg Leu Ile Ala Gly Val
            1700                1705                1710

Phe Ala Asp Leu Leu Gly Leu Glu Arg Ala Gly Arg Glu Asp Glu Phe
        1715                1720                1725

Phe Leu Ser Gly Gly Asn Ser Leu Gln Ala Ile Arg Ala Val Gly Arg
    1730                1735                1740

Leu Asn Asp Ala Leu Gly Val Pro Leu Thr Val Arg Asp Phe Tyr Ala
1745                1750                1755                1760

Ala Pro Thr Val Ala Ala Leu Ala Glu Leu Ala Gly Arg Arg Arg Ala
                1765                1770                1775

Ala Glu Gln Ala Ala Gln Trp Gln Leu Leu Asp Glu Ile Glu Gln Leu
            1780                1785                1790

Ser Asp Asp Glu Val Glu Arg Leu Leu Ala Ala Asp
        1795                1800


<210>  29
<211>  1345
<212>  PRT
<213>  Micromonospora sp., ML1 strain

<220>
<223>  Tio28 Protein

<400>  29
Met Ser Asp Glu Thr Phe Asp Gly Arg Val Ala Arg Leu Asp Glu Arg
1               5                   10                  15

Arg Arg Leu Leu Leu Glu Arg Met Arg Gln Gln Arg Arg Gln Pro Pro
            20                  25                  30

Ala Thr Ile Gly Ala Arg Pro Ala Gly Asp Glu Arg Val Pro Leu Ser
        35                  40                  45

Arg Ala Gln Glu Gln Leu Trp Phe Leu Ala Gln Leu Ala Pro Ala Glu
        50                  55                  60

Pro Thr Tyr Asn Leu Val Gln Ala Ser Tyr Leu Thr Gly Glu Val Asp
65                  70                  75                  80

Pro Ala Ala Leu Arg Arg Ala Leu Asp Gly Val Val Ala Gln His Glu
                85                  90                  95

Ala Leu Arg Thr Val Val Glu Ser Val Asp Gly Val Ala Tyr Gln Arg
            100                 105                 110

Val Leu Pro Pro Gly Pro Ala Ser Leu Val Val Asp Asp Val Ser Arg
        115                 120                 125

Leu Pro Glu Glu Gln Arg Arg Pro Ala Ala Leu Thr Leu Leu Gln Gln
    130                 135                 140

Gln Glu Val His Arg Pro Phe Asp Leu Ala Thr Gly Pro Leu Phe Arg
145                 150                 155                 160

Ala Arg Leu Val Arg Leu Gly Pro Thr Glu His Ala Leu Ala Val Ala
                165                 170                 175

Ala His His Ile Val Val Asp Gly Trp Ser Met Gly Leu Ile Leu Arg
            180                 185                 190

Glu Leu Ala Ala Ala Tyr Asp Ala Asp Arg His Gly Thr Arg Ala Arg
        195                 200                 205

Leu Asp Pro Pro Ala Leu Gln Tyr Pro Asp Phe Ala Arg Trp Gln His
    210                 215                 220

Gln Gln Leu Ala Thr Ala Glu Leu Asp Asn His Leu Arg Tyr Trp Ala
225                 230                 235                 240

Gln Arg Leu Ala Asp Leu Pro Thr Leu Glu Leu Pro Ala Asp Arg Pro
                245                 250                 255

Arg Pro Thr Thr Ala Ser Phe Arg Gly Ala Leu Leu Glu Gln Pro Leu
            260                 265                 270

Asp Pro Gln Leu His Ala Gly Val Gln Ala Leu Ala Lys Glu Thr Gly
        275                 280                 285

Ala Ser Pro Phe Met Val Leu Val Ala Ala Phe Ala Ala Met Leu Ala
    290                 295                 300

Arg Tyr Thr Gly Thr Asp Asp Leu Ala Val Gly Thr Thr Phe Ser Gly
305                 310                 315                 320

Arg Ser Arg Pro Glu Leu Glu Ser Val Val Gly Phe Phe Ala Asn Met
                325                 330                 335

Gly Val Leu Arg Leu Asp Thr Ser Gly Asp Pro Ser Phe Ala Thr Leu
            340                 345                 350

Val Asp Arg Ala Arg Asp Thr Cys Leu Gly Ala Trp Glu His Gln Asp
        355                 360                 365

Ala Pro Phe Glu Arg Val Val Gln Arg Leu Ala Pro Pro Arg Asp Pro
    370                 375                 380

Gly Arg Asn Pro Leu Phe Gln Val Ala Val Gln Leu Leu Asp Gly Ser
385                 390                 395                 400

Thr Trp Gly Gly Pro Thr Leu Pro Gly Thr Asp Ser Thr Pro Val Asp
                405                 410                 415

Leu Arg Leu Asp Arg Ser Arg Phe Asp Met Met Val Ser Leu Val Asp
            420                 425                 430

His Gly Asp Arg Tyr Ser Val Leu Thr Glu Tyr Ser Thr Asp Leu Phe
        435                 440                 445

Asp Ala Arg Arg Val Arg Ala Met Leu Asp His Val Ala Arg Leu Leu
    450                 455                 560

Asp Ala Gly Leu Ala Asp Pro Ala Thr Thr Leu Ser Arg Leu Pro Met
465                 470                 475                 480

Leu Asp Pro Ala Glu Arg Glu Arg Val Leu Ala Leu Gly Ala Gly Glu
                485                 490                 495

Arg Arg Asp Val Ala Pro Ala Thr Val His Gln Leu Val Arg Ala Gln
            500                 505                 510

Ala Glu Arg Asn Pro Asp Ala Val Ala Val Arg His Gly Asp His Glu
        515                 520                 525

Leu Thr Tyr Arg Glu Leu Leu Asp Arg Ala Ala Arg Arg Ala Ala Gly
    530                 535                 540

Leu Arg Asp Ala Gly Leu Arg Pro Gly Asp Arg Val Pro Ala Leu Leu
545                 550                 555                 560

Asp Arg Gly Val Asp Glu Val Leu Thr Pro Met Ala Ile Trp His Ala
                565                 570                 575

Gly Gly Val Tyr Val Pro Leu Asp Thr Ala Ala Pro Pro Asn Arg Leu
            580                 585                 590

Arg Arg Ile Leu Thr Asn Cys Ala Ala Arg Leu Val Leu Thr Arg Thr
        595                 600                 605

Glu His Ala Ala Gly Val Pro Glu Gly Pro Trp Arg Thr Leu Leu Leu
    610                 615                 620

Asp Thr Gly Asp Leu Asp Ser Ala Ala Gly Leu Ala Pro Glu Pro Ser

625                 630                 635                 640

Thr Gly Pro Asp Asp Leu Ala Tyr Val Leu His Thr Ser Gly Thr Thr
                645                 650                 655

Gly Asp Pro Lys Gly Val Gln Leu Pro His Arg Gly Phe Val Thr Tyr
            660                 665                 670

Leu Asp Trp Met Arg Asp Gln Trp Arg Cys Gly Pro Gly Asp Arg Val
        675                 680                 685

Leu His Gly Cys Ala Pro Val Phe Asp Leu Ala Ala Gly Glu Val Leu
    690                 695                 700

Ala Ala Leu Thr Ser Gly Ala Thr Val Val Val Ile Gly Lys Asp Arg
705                 710                 715                 720

Leu Leu Ser Pro Gly Gly Leu Ala Glu Val Leu Ala Ala Glu Arg Ile
                725                 730                 735

Thr His Leu Phe Leu Thr Pro Thr Thr Leu Gly Leu Val Glu Asp Asp
            740                 745                 750

Thr Asp Arg Phe Asp Ala Leu Arg Glu Val Leu Val Gly Gly Glu Val
        755                 760                 765

Cys Pro Ala Asp Leu Val Ala Arg Trp Ala Arg Pro Gly Arg Arg Arg
    770                 775                 780

Phe Val Asn Leu Tyr Gly Pro Thr Glu Ala Thr Val Gly Cys Leu Ala
785                 790                 795                 800

His Asp Cys Thr Asp Trp Ser Ala Ala Thr Pro Pro Pro Ile Gly Ala
                805                 810                 815

Thr Met Pro Asn Arg Arg Thr Tyr Leu Val Asp Arg Trp Asp Asn Pro
            820                 825                 830

Val Pro Val Gly Val Pro Gly Glu Ile Leu Val Gly Gly Ala Ala Leu
        835                 840                 845

Ser Thr Gly Tyr Leu Asn Asp Asp Glu Leu Thr Gly Arg Arg Phe Thr
    850                 855                 860

Ala Asp Pro Phe Asp Pro Gln Gly Arg Val Tyr Arg Thr Gly Asp Arg
865                 870                 875                 880

Gly His Trp Asp Ala Gln Gly Arg Leu His Phe Gly Gly Arg Leu Asp
                885                 890                 895

Gly Gln Val Lys Leu Arg Gly Leu Arg Val Glu Leu Asp Glu Ile Glu
            900                 905                 910

Ala Leu Leu Ser Arg His Ser Gly Val Ala Arg Ala Ala Val Thr Val

915                 920                 925

Val Arg Asp Gly Asn Gly Val Gln Arg Leu Val Gly Tyr Val Val Asp
    930                 935                 940

Ala Ala Glu Pro Ala Ser Asp Ala Asp Leu Arg Thr His Leu Ser Thr
945                 950                 955                 960

Glu Leu Pro Ala His Leu Val Pro Gly Arg Phe Val Arg Leu Pro Ala
                965                 970                 975

Leu Pro Leu Thr Thr Ser Gly Lys Val Asn Arg Arg Ala Leu Pro Ala
            980                 985                 990

Pro Glu Ala Ala Asp Thr Asp Asp Thr Gly Ala Ala Pro Thr Thr Gly
        995                 1000                1005

Ala Glu Arg Gln Val Ala Glu Ile Phe Ala Glu Val Leu Gly Val Ala
    1010                1015                1020

Arg Val Gly Ala Glu Thr Ser Phe Phe Asp Leu Gly Gly His Ser Leu
1025                1030                1035                1040

Gln Ala Ala His Val Leu Ala Arg Ile Ala Gly Arg Ile Gly Thr Ser
                1045                1050                1055

Val Ser Leu Lys Gln Phe Tyr Ala Thr Pro Thr Val Arg Ala Val Ala
            1060                1065                1070

Arg Phe Val Glu His Gly Pro Val Val Asp Ala Ser Pro Leu Val Thr
        1075                1080                1085

Leu Lys Ala Thr Gly Ser Arg Pro Arg Leu Tyr Cys Pro His Ala Val
    1090                1095                1100

Ser Gly Ser Pro Tyr Trp Tyr Leu Pro Leu Ser Arg Ala Leu His Pro
1105                1110                1115                1120

Glu Gln Pro Val Asp Gly Phe Glu Ala Pro Gly Leu Glu Gly Gly Ile
                1125                1130                1135

Glu Pro Val Asp Asp Leu Val Thr Leu Ala Gly Arg Tyr Val Gly Ala
            1140                1145                1150

Leu Arg Glu Arg Gln Pro His Gly Pro Tyr Leu Leu Ala Gly Trp Ser
        1155                1160                1165

Met Gly Gly Phe Leu Cys Phe Glu Met Ala Arg Gln Leu Ala Ala Val
    1170                1175                1180

Gly Ala Ala Pro Asp Leu Val Val Met Ile Asp Ser Asn Glu Pro Gly
1185                1190                1195                1200

Pro Leu Pro Leu Pro Ser Glu Arg Gln Val Met Glu Thr Phe Val Ala
                1205                1210                1215

Asp Leu Ala Gly Leu Ser Ala Ala Pro Ala Pro Pro Leu Asp Ala Glu
            1220                1225                1230

Val Val Asp Ala Ala Asp Pro Ile Ala Ala Leu Thr Gly Phe Leu Val
        1235                1240                1245

Arg His Gly Leu Ile Pro Ser Asp Val Arg Ala Asp Phe Val Ala Asn
    1250                1255                1260

Arg Tyr Ala Val Phe Arg Ala Asn Met Arg Ala Ile Tyr Gly Tyr Gln
1265                1270                1275                1280

Pro Ala Pro Tyr Ala Gly Arg Thr Val Leu Val Gln Ala Glu Arg Glu
                1285                1290                1295

Ala Ser Arg Ala Ala Trp Arg Arg Trp Ser Pro Ala Met Glu Val Val
            1300                1305                1310

Thr Leu Pro Gly Asp His Tyr Ser Met Trp Ser Pro Gln His Leu Pro
        1315                1320                1325

Ala Leu Ala Ala Leu Ile Asp Asp Gln Val Ala Arg Val Leu Asp Leu
    1330                1335                1340

Gly
1345


<210>  30
<211>  519
<212>  PRT
<213>  Micromonospora sp., ML1 strain

<220>
<223>  ORF29 Protein


<400>  30
Met Ala Thr Glu Asp Glu Gly Arg Pro Arg Gly Ala Arg Leu Val Leu
  1               5                  10                  15

Leu Ile Leu Leu Val Ala Gln Val Met Ala Thr Met Asp Asn Ser Ile
             20                  25                  30

Val Ala Val Ala Thr Lys Thr Ile Lys Asp Asp Leu Gln Thr Ser Gly
         35                  40                  45

Ala Val Leu Gln Leu Ile Leu Ser Gly Tyr Thr Leu Met Phe Ala Val
     50                  55                  60

Leu Val Val Thr Gly Ala Arg Leu Gly Gly Asp Val Gly His Arg Arg
 65                  70                  75                  80

Leu Phe Met Ile Gly Leu Thr Gly Phe Thr Ala Ser Ser Leu Leu Cys

                85                  90                  95

Gly Ile Ala Pro Thr Ala Gly Thr Leu Val Ala Ala Arg Leu Leu Gln
            100                 105                 110

Gly Ala Phe Gly Ala Met Met Val Pro Gln Val Leu Ser Val Ile Gln
        115                 120                 125

Ile Val Phe Thr Gly Ser Ala Arg Ala Arg Ala Ile Gly Leu Tyr Ser
    130                 135                 140

Met Val Leu Ala Leu Gly Val Ala Ala Gly Gln Ile Ala Gly Gly Val
145                 150                 155                 160

Ile Val Ser Ala Asp Val Leu Gly Ser Gly Trp Arg Ala Ala Phe Leu
                165                 170                 175

Val Asn Val Pro Ile Gly Leu Ala Leu Leu Ala Ala Ala Pro Arg Tyr
            180                 185                 190

Leu Pro Gly His Thr Pro Ser Ser Arg Phe Arg Pro Asp Ile Val Gly
        195                 200                 205

Ile Leu Leu Leu Ala Val Ser Met Gly Ala Val Val Ala Pro Leu Val
    210                 215                 220

Phe Gly Arg Glu Gln Gly Trp Pro Ala Trp Ala Leu Ala Ser Ile Ala
225                 230                 235                 240

Ala Gly Thr Ala Gly Leu Val Leu Phe Val Arg Tyr Glu Leu Arg Leu
                245                 250                 255

Gly Ala Arg Gly Gly Gln Pro Val Leu Glu Ile Asp Ala Leu Arg Pro
            260                 265                 270

Arg Gly Val Lys Ser Gly Leu Leu Ala Cys Cys Ile Leu Asn Phe Ala
        275                 280                 285

Phe Ala Gly Val Leu Phe Pro Leu Thr Leu His Val Gln Asn Gly Leu
    290                 295                 300

Gly Tyr Thr Pro Leu Gln Ala Gly Leu Met Phe Ile Pro Tyr Pro Val
305                 310                 315                 320

Gly Phe Ala Ala Val Ser Leu Thr Trp Thr Arg Leu Pro Lys Arg Phe
                325                 330                 335

His Arg Pro Leu Pro Val Ala Gly Leu Leu Val Phe Ala Leu Ala Leu
            340                 345                 350

Ala Ala Leu Ala Val Val Val Ala Gly Gly Trp Pro Ile Ala Val Val
        355                 360                 365

Ser Ala Leu Leu Leu Leu Ala Gly Ala Gly Met Ala Ala Gly Phe Ser

370                 375                 380

Thr Leu Val Glu Gln Thr Ala Ala Thr Val Gly Pro Arg Tyr Ala Ala
385                 390                 395                 400

Ala Leu Ser Ala Leu Val Ser Thr Gly Thr Leu Leu Ala Ser Val Val
                405                 410                 415

Ser Val Val Val Val Gly Gly Val Tyr Leu Ala Thr Ala Glu Asn Asp
            420                 425                 430

Pro Ser Arg Ser Ala Leu Gly Leu Ser Arg Ser Leu Trp Val Asp Ala
        435                 440                 445

Gly Leu Leu Met Leu Gly Phe Leu Leu Ala Tyr Arg Thr Trp Arg Leu
    450                 455                 460

Ser Thr Arg Val Ser Ala Glu Thr Ala Pro Ser Gly Asp Ala Ala Glu
465                 470                 475                 480

Gln Val Glu Ala Val Ala Ala Pro Gly Asn Ala Pro Pro Gly Glu Val
                485                 490                 495

Asp Val Glu Pro Ala Thr Gly Pro Val Glu Glu Ser Ser Ala Pro Thr
            500                 505                 510

Gly Ala Arg Ser Ala Pro Arg
        515


<210>  31
<211>  613
<212>  PRT
<213>  Micromonospora sp., ML1 strain

<220>
<223>  ORF30 Protein


<400>  31
Met Ser Glu Ser Arg Thr Ala Asp Thr Ala Ser Ala Lys Arg Arg Gly
1               5                   10                  15

Thr Leu Gly Phe Leu Leu Gly Val Ala Leu Val Thr Ala Gly Val Leu
            20                  25                  30

Phe Gln Leu Pro Asp Val Lys Met Met Ile Asp His Ala Arg Met Gly
        35                  40                  45

Ser Ala Glu Pro Gly Met Asp Gly Met Asp Asp Met Asp Asp Met Asp
    50                  55                  60

Ser Met Pro Gly Met Glu Gly Met Ser Pro Gly Glu Pro Met Asp Met
65                  70                  75                  80

Trp Thr Pro Thr Met Ile Leu Gly Met Leu Leu Leu Val Val Gly Leu
                85                  90                  95

Val Val Ala Gly Arg Ala Leu Phe Ala Gly Val Arg Arg Pro Asp Ser
            100                 105                 110

Ala Ser Val Thr Val Glu Ser Ile Glu His Gly Arg Leu Arg Pro Ala
        115                 120                 125

Tyr Trp Ile Thr Cys Ala Val Leu Thr Ile Ala Leu Val Val Asp Ile
    130                 135                 140

Met Lys Pro Leu Thr Leu Gly Phe Val Met Pro Gly Met Ser Asp Glu
145                 150                 155                 160

Tyr Gly Met Ser Leu Glu Ser Val Ser Ile Leu Pro Leu Val Ala Leu
                165                 170                 175

Thr Gly Thr Ala Val Gly Ser Val Val Trp Gly Leu Leu Gly Asp Arg
            180                 185                 190

Tyr Gly Arg Arg Thr Ala Leu Leu Leu Ala Thr Leu Leu Phe Ile Ala
        195                 200                 205

Thr Ser Ala Cys Gly Ala Met Pro Thr Phe Glu Trp Asn Leu Val Met
    210                 215                 220

Cys Phe Thr Met Gly Thr Ser Ala Gly Gly Leu Leu Pro Leu Val Phe
225                 230                 235                 240

Thr Leu Ile Ala Glu Leu Thr Pro Arg Arg His Arg Gly Trp Val Ala
                245                 250                 255

Val Thr Val Gly Gly Ile Gly Gly Leu Gly Gly Tyr Leu Ala Ala Ser
            260                 265                 270

Glu Ala Ala Arg His Leu Glu Pro Ala Leu Thr Trp Arg Ala Leu Trp
        275                 280                 285

Leu Ile Gly Leu Pro Ser Gly Leu Leu Leu Leu Leu Met Ala Pro Leu
    290                 295                 300

Ile Pro Glu Ser Pro Leu Tyr Leu Leu Arg Ala Gly Arg Arg Thr Glu
305                 310                 315                 320

Ala Glu Ala Val Leu Arg Arg Tyr Gly Ser His Leu Gly Ala Thr Pro
                325                 330                 335

Pro Ala Ala Asp Thr Ala Ala Ala Ala Thr Arg Pro Gly Val Gly Ala
            340                 345                 350

Met Leu Arg Ala Tyr Pro Val Val Thr Ser Val Ile Gly Val Val Gly
        355                 360                 365

Val Val Trp Gly Leu Val Asn Phe Gly Phe Leu Val Met Leu Pro Ala

370                 375                 380

Gln Leu Arg Glu Ala Gly Leu Ala Gly Gly Val Ala Ser Gly Leu Leu
385                 390                 395                 400

Ala Asp Ser Ala Leu Tyr Ser Thr Pro Ala Leu Ala Leu Val Val Leu
                405                 410                 415

Leu Tyr Ala Ala Trp Ser Gly Arg Arg Ala Leu Ala Leu Phe Ile Ala
            420                 425                 430

Ile Thr Ala Val Gly Leu Gly Gly Val Ala Leu Trp Thr Thr Asp Ser
        435                 440                 445

Gly Gly Thr Val Leu Leu Leu Ala Ser Ile Gly Leu Leu Val Leu Ser
    450                 455                 460

Leu Ser Ala Val Asn Ala Met Leu Leu Pro Tyr Ser Ala Glu Ile Tyr
465                 470                 475                 480

Pro Thr Ala Met Arg Ala Thr Gly Ser Gly Phe Ala Gly Ala Ala Thr
                485                 490                 495

Lys Val Gly Gly Ile Leu Gly Pro Ser Ala Met Leu Leu Ile Leu Ser
            500                 505                 510

Leu Gly Gly Gly Leu Val Ala Pro Ala Ile Ala Leu Gly Val Leu Cys
        515                 520                 525

Leu Ala Ala Thr Val Leu Leu Leu Val Phe Gly Pro Glu Leu Gly Ala
    530                 535                 540

Ser Val Gly Pro Leu Ala Arg Arg Gly Val Gly Thr Val Pro Val Ser
545                 550                 555                 560

Arg Thr Gly Pro Ala Pro Gly Thr Gly Pro Thr Asp Thr Glu Ser Pro
                565                 570                 575

Ala Ser Val Ala Trp Ser Ala Thr Gly Asn Val Val Pro Ala Thr Arg
            580                 585                 590

Pro Ala Val Ser Asp Asp Asp Asp Pro Arg Asp Ala Arg Thr Pro Ala
        595                 600                 605

Thr Pro Ser Arg Thr
    610


<210>  32
<211>  191
<212>  PRT
<213>  Micromonospora sp., ML1 strain

<220>
<223>  ORF31 Protein

<400>  32
Val Pro His Val Asp Arg Ser Ala Arg Ser Gly Pro Cys Cys Ala Ser
1               5                   10                  15

Met Asp Tyr Met Thr Ile Gln Ser Asp Pro Met Ala Ser Thr Asp Glu
            20                  25                  30

Val Arg Gly Glu Pro Ala Asp Glu Phe Val Gly Ala Leu Val Ser Leu
        35                  40                  45

Ser Arg Val Met Val Ala Val Ala Met Arg Thr Leu Ala Arg Ile Asp
    50                  55                  60

Ala Asp Val Thr Leu Gln Gln Phe Arg Thr Leu Val Val Leu Val Pro
65                  70                  75                  80

Gly Pro Gln Arg Val Ile Asp Leu Ala Lys Leu Leu Gly Val Gln Ser
                85                  90                  95

Ser Thr Ala Thr Arg Met Cys Asp Arg Leu Val Arg Lys Gly Leu Val
            100                 105                 110

Ala Arg His Glu Arg Val Ala Asp Arg Arg Ala Ala Trp Val Gly Leu
        115                 120                 125

Thr Val Ala Gly Lys Glu Leu Val Gly Leu Gly Met Arg Ala Arg Gln
    130                 135                 140

Gln Glu Leu Glu Arg Met Leu Ala Asp Leu Arg Pro Asp Asp Thr Asp
145                 150                 155                 160

Val Thr Thr Gly Ala Leu Val Ala Val Leu Arg Ala Thr Gly Glu Leu
                165                 170                 175

Thr Asp Gly Ala Trp Trp Arg Gln Trp Ala Asp Ser Val Val Glu
            180                 185                 190

<210>  33
<211>  224
<212>  PRT
<213>  Micromonospora sp., ML1 strain

<220>
<223>  ORF32 Protein

<400>  33
Met Thr Val Val Pro Asp Asp Asn Leu Met Thr Leu Ile Cys Asp Val
1               5                   10                  15

Cys Gly Asp Thr Thr Thr Gly Thr Ala Cys Val Leu Pro Asp Ala Glu
            20                  25                  30

Val Val Trp Thr Leu Val Ser Asp His Gly Trp Ser Gly Ser Pro Phe
        35                  40                  45

Ala Thr Gly Pro His Arg Cys Pro Arg Cys Ser Ala Leu Pro Pro Gly
    50                  55                  60

Ser Ser Leu Gly Asp Gly Asp Arg Ala Gly Ser Gly Leu Ala Glu Ala
65                  70                  75                  80

Ala Val Val Val Pro Ala Asp Gly Gly Asp Gln Asp Thr Gly Asp Ala
                85                  90                  95

Leu Arg Ala Ala Leu Arg Gly Ala Phe Asp Val Ala Gly Thr Val Val
            100                 105                 110

Val Asp Leu Thr Gln Val Gln Val Ile Asp Ser Leu Gly Leu Gly Met
        115                 120                 125

Leu Val Arg Ala His Arg Asp Val Arg Glu Arg Gly Gly Arg Leu Cys
    130                 135                 140

Leu Ala Ala Pro Ser Arg Phe Ile Arg Thr Val Leu His Thr Met Lys
145                 150                 155                 160

Leu Asp Gly Val Phe Pro Ile Phe Asp Ser Leu Asp Ala Ala Leu Asp
                165                 170                 175

Gln Leu Arg Asn Ala Ala Ser Gln Asp Ala Val Ser Ala Gly Val Ser
            180                 185                 190

Pro Val Asp Val Thr Pro Ser Asp Leu Pro Arg Pro Ala Ala Glu Val
        195                 200                 205

Pro Ser Met Pro Met Ala Arg Arg Arg Ala Ala Ser Arg Val Thr Val
    210                 215                 220


<210>  34
<211>  89
<212>  PRT
<213>  Micromonospora sp., ML1 strain

<220>
<223>  ORF33 Protein


<400>  34
Met Thr Ser Ala Gly Ile Val Thr Ala Leu Ala Val Gly Leu Ala Val
1               5                   10                  15

Gly Ala Leu Gly Arg Leu Val Leu Pro Gly Arg Pro Ala Ala Pro Leu
            20                  25                  30

Trp Leu Thr Met Thr Ile Gly Ala Val Ala Ser Leu Leu Gly Ala Ile
        35                  40                  45

Thr Ala Trp Leu Ala Gly Val Gln Gly Phe Ser Ile Leu Gly Leu Val
    50                  55                  60

Val Gln Ala Gly Leu Ala Ala Val Ala Val Val Ile Val Val Ala Thr
65                  70                  75                  80

Thr Gly Lys Glu Arg Ser Asp Ser Leu
                85


<210>  35
<211>  168
<212>  PRT
<213>  Micromonospora sp., ML1 strain

<220>
<223>  ORF34 Protein

<400>  35
Val Ala Ala Asn Pro Ala Gly Gln Gly Glu Ser His Ser Glu Thr Ala
1               5                   10                  15

Gly Glu Gly Ala Leu Ala Val Thr Ala Pro Arg Arg Pro Glu Cys Ser
            20                  25                  30

Val Pro Leu Val Glu Val Ser Ile Thr Glu Phe Asp Leu Ser Cys Leu
        35                  40                  45

Pro Glu Thr Gly Ala Val Leu Asp Gln Leu Leu Ala Leu Arg Pro Gly
    50                  55                  60

Gln Val Val Ile Asp Leu Ala Gly Cys Arg His Ile Asp Ala Ala Ala
65                  70                  75                  80

Ile Gly Leu Leu Leu Asp Val His Arg Arg Met Val Arg Ala Gly Gly
                85                  90                  95

Val Leu Ala Val Arg Asn Pro Asn Pro Arg Ile Thr Arg Ile Leu Gln
            100                 105                 110

Thr Ala Arg Leu Asp Gln Ile Leu Pro Val His Thr Asp Ala Ala Ser
        115                 120                 125

Ala Pro Val Thr Glu Ala Val Thr Ala Ala Pro Gly Val Pro Pro Ala
    130                 135                 140

Asp Gly Thr Pro Ala Pro Gly Thr Val Pro Arg Ala Ala Ala Tyr Gly
145                 150                 155                 160

Arg Ala Ala Val Thr Val Arg Thr
                165

<210>  36
<211>  229
<212>  PRT
<213>  Micromonospora sp., ML1 strain

<220>
<223>  ORF35 Protein

<400>  36
Met Thr Ala Val Leu Val Ile Glu Asp Asp Asp Arg Ile Arg Leu Ala
1               5                   10                  15

Leu Leu Leu Ala Leu Glu Asp Glu Gly Tyr Glu Ala Arg Gly Ala Ala
            20                  25                  30

Thr Ala Glu Glu Gly Leu Arg Thr Gln Arg Arg Asp Pro Ala Asp Tyr
        35                  40                  45

Val Leu Val Asp Leu Met Leu Pro Gly Ile Asp Gly Phe Glu Gly Ile
    50                  55                  60

Arg Gln Leu Arg Arg Asp Asp Asp Val Pro Ile Val Val Val Ser Ala
65                  70                  75                  80

Arg Asp Asp Thr His Asp Ile Val Ala Ala Leu Glu Ala Gly Ala Asp
                85                  90                  95

Asp Tyr Val Val Lys Pro Val Ala Ile Lys Glu Leu Thr Ala Arg Leu
            100                 105                 110

Arg Ala Leu Arg Arg Arg Gly Arg Thr Thr Thr Ala Ser Asp Gly Pro
        115                 120                 125

Gln Pro Val Pro Val Leu Ala Phe Gly Asp Leu Glu Val Ser Pro Glu
    130                 135                 140

Ala Gly Glu Val Arg Arg Ala Gly Glu Pro Val Ala Val Thr Arg Thr
145                 150                 155                 160

Glu Phe Arg Leu Leu Cys Glu Leu Ala Glu His Ala Gly Arg Val Leu
                165                 170                 175

Ser Arg Gln Gln Leu Leu Ser Arg Val Trp Gly Tyr Asp Ser Gly Asp
            180                 185                 190

Glu Arg Leu Val Asp Val His Val Gly Arg Leu Arg Gln Lys Ile Glu
        195                 200                 205

Ala Asp Pro Ala Asn Pro Arg His Leu Val Thr Leu Arg Gly Leu Gly
    210                 215                 220

Tyr Lys Leu Gln Arg
225

<210>  37
<211>  427
<212>  PRT
<213>  Micromonospora sp., ML1 strain

<220>
<223>  ORF36 Protein

<400>  37
Met Ala Leu Val Ser Tyr Glu Leu Thr Arg Arg Thr Leu Leu Asp Glu
1               5                   10                  15

Arg Glu Arg Thr Val Leu Arg Ala Ala Tyr Tyr Asp Ala Ala Val Val
            20                  25                  30

Arg Ala Gly Leu Asp Thr Asp Asn Pro Asp Val Ala Glu Ala Leu Arg
        35                  40                  45

Ala Leu Asp Thr Gly Thr Gly Arg Arg Pro Leu Val Leu Leu Asp Gly
    50                  55                  60

Glu Trp Tyr Ala Arg Ser Ala Asp Asn Gly Leu Thr Thr Ile Val Pro
65                  70                  75                  80

Ala Arg Leu Gln Asp Leu Val Ala Ala Gly Glu Pro Ala Val Gln Arg
                85                  90                  95

Val Arg Val Asn Asp Thr Pro Val Met Leu Val Gly Val Pro Leu Ser
            100                 105                 110

Glu Ser Thr Ala Phe Tyr Glu Ile Ala Ser Leu Arg Glu Leu Glu Gln
        115                 120                 125

Thr Phe Gln Val Leu Ala Leu Ala Leu Thr Ala Val Ala Ile Met Ile
    130                 135                 140

Ala Gly Ser Gly Ala Ala Leu Gly Trp Tyr Ala Thr Arg His Gly Leu
145                 150                 155                 160

Arg Pro Leu Thr Ala Val Ala Asp Ala Ala Glu Arg Ile Ala Ala Gly
                165                 170                 175

Asp Phe Thr Thr Arg Leu Ala Pro Asp Thr Asp Pro Asp Leu Thr Arg
            180                 185                 190

Leu Ser Thr Ser Phe Asn Arg Met Val Asp Glu Leu Ala Gly Arg Ile
        195                 200                 205

Asp Arg Asp Arg Arg Phe Ala Ala Asp Val Ser His Glu Leu Arg Ser
    210                 215                 220

Pro Leu Gln Thr Leu Ala Ala Ala Ala Ser Val Leu Ser Arg Arg Arg
225                 230                 235                 240

Glu Asn Gln Asp Glu Arg Thr Ala Thr Ala Ala Arg Leu Val Ala Asp
                245                 250                 255

Glu Ile Asp Arg Phe Gln Gln Leu Val Asn Asp Leu Leu Asp Leu Ala
            260                 265                 270

Arg Gly Asp Gln Pro Val His Arg Glu Ser Val Asp Leu Pro Glu Leu
        275                 280                 285

Ala Arg Gln Ala Cys Arg Asp Arg Asp Leu Pro Glu Ser Met Val Gln
    290                 295                 300

Leu Ala Thr Gly Thr Pro Pro Gly Trp Arg Val Asp Arg Arg Arg Val
305                 310                 315                 320

Ala Gln Val Leu Ala Asn Leu Leu Asp Asn Ala Glu Arg Tyr Gly Ala
                325                 330                 335

Gly Pro Val Ala Val Arg Leu Ser His Asp Ala Asp Thr Gly Val Ile
            340                 345                 350

Glu Val Asp Asp Glu Gly Pro Gly Val Pro Met Glu Asp Arg Glu Ala
        355                 360                 365

Ile Phe Asp Arg Phe Val Arg Gly Arg Ala Ala Asn Tyr Arg Gly Gly
    370                 375                 380

Gly Asp Gly Thr Gly Leu Gly Leu Ala Leu Val Ala Gln His Ala Ala
385                 390                 395                 400

Ala His Gly Gly Asp Ala Ser Val Ser Asp Arg Pro Glu Gly Gly Ala
                405                 410                 415

Arg Phe Arg Val Thr Leu Pro Gly Ser Leu Ser
            420                 425


<210>  38
<211>  178
<212>  PRT
<213>  Micromonospora sp., ML1 strain

<220>
<223>  ORF37 Protein


<400>  38
Val Phe Ala Leu Leu Gly Ala Cys Gly Val Pro Ala Glu Asp Arg Pro
 1               5                  10                  15

Arg Ala Val Thr Pro Pro Pro Val Pro Phe Pro Ala Thr Ala Ser Ala
            20                  25                  30

Ala Pro Thr Ala Ala Glu Thr Gly Ala Val Thr Glu Val Leu Tyr Phe

35                  40                  45

Ala Arg Asp Glu Arg Leu Val Pro Val Ile Arg Arg Ala Asp Gln Val
    50                  55                  60

Pro Ala Leu Asp Ala Gln Leu Arg Ala Leu Leu Ala Gly Pro Thr Pro
65                  70                  75                  80

Thr Glu Arg Asp Asp Gly Leu Thr Ser Ala Leu Pro Gly Ala Phe Thr
                85                  90                  95

Ser Ala Val Val Glu Leu Val Asp Gly Leu Ala Arg Val Thr Val Gly
            100                 105                 110

Leu Thr Ala Val Asp Thr Gly Arg Ser Asp Gly Arg Leu Ala Tyr Gly
        115                 120                 125

Gln Ile Val Cys Thr Leu Thr Ala Arg Thr Asp Val Thr Ala Val Leu
    130                 135                 140

Phe Leu Glu Gly Gly Thr Pro Leu Ser Val Pro Arg Ala Asp Gly Ser
145                 150                 155                 160

Leu Ser Ser Gly Pro Leu Thr Ala Ala Asp Tyr Ala Val Leu Ile Asn
                165                 170                 175

Pro Arg


<210>  39
<211>  574
<212>  PRT
<213>  Micromonospora sp., ML1 strain

<220>
<223>  ORF38 Protein

<400>  39
Val Asn Asp Arg Ala Glu Thr Leu Glu Phe Gln Ala Glu Ala Arg Gln
1               5                   10                  15

Leu Leu Arg Leu Met Val His Ser Ile Tyr Ser Asn Lys Asp Val Phe
            20                  25                  30

Leu Arg Glu Leu Ile Ser Asn Ala Ser Asp Ala Leu Asp Lys Leu Arg
        35                  40                  45

Leu Ala Ser Met Val Asp Lys Asp Leu Ala Val Asp Thr Gly Asp Leu
    50                  55                  60

His Ile Ala Ile Glu Val Asp Arg Asp Ala Arg Thr Leu Thr Val Arg
65                  70                  75                  80

Asp Asn Gly Ile Gly Met Ser Arg Asp Glu Val Val Ser Val Ile Gly

                85                  90                  95

Thr Ile Ala Lys Ser Gly Thr Ala Glu Leu Leu Arg Gln Leu Arg Glu
            100                 105                 110

Ala Lys Asp Ala Gly Ala Ser Gln Glu Leu Ile Gly Gln Phe Gly Val
        115                 120                 125

Gly Phe Tyr Ala Ala Phe Met Val Ala Glu Arg Val Glu Leu Val Thr
    130                 135                 140

Arg Arg Ala Gly Glu Thr Gly Gly Thr Arg Trp Glu Ser Thr Gly Glu
145                 150                 155                 160

Gly Thr Tyr Thr Ile Thr Glu Leu Asp Asp Ala Pro Gln Gly Thr Ala
                165                 170                 175

Val Thr Leu His Leu Lys Pro Ala Asp Ala Glu Asp Asn Leu His Asp
            180                 185                 190

Tyr Thr Ala Glu Trp Thr Ile Arg Gly Ile Val Lys Arg Tyr Ser Asp
        195                 200                 205

Phe Ile Ala His Pro Ile Arg Met Thr Val Glu Arg Pro Gly Thr Asp
    210                 215                 220

Asp Ala Pro Ala Thr Thr Glu Ala Val Thr Leu Asn Ser Met Lys Ala
225                 230                 235                 240

Leu Trp Ala Arg Pro Arg Gly Glu Val Glu Pro Ala Glu Tyr His Glu
                245                 250                 255

Phe Tyr Lys His Val Ser His Asp Trp Ala Asp Pro Leu Glu Thr Ile
            260                 265                 270

His Met Arg Gly Glu Gly Thr Phe Glu Tyr Glu Ala Leu Leu Phe Ile
        275                 280                 285

Pro Thr His Ala Pro Leu Asp Leu Phe Ser Pro Gln Gly Arg Arg Gly
    290                 295                 300

Val Gln Leu Tyr Val Lys Arg Val Phe Ile Met Asp Asp Cys Asp Ala
305                 310                 315                 320

Leu Met Pro Asn Tyr Leu Arg Phe Val Lys Gly Val Val Asp Ala His
                325                 330                 335

Asp Leu Ser Leu Asn Ile Ser Arg Glu Ile Leu Gln Gln Asp Arg Gln
            340                 345                 350

Ile Arg Ala Val Arg Arg Arg Leu Val Lys Lys Ile Leu Ala Thr Val
        355                 360                 365

Lys Asp Leu Lys Thr Gly Gln Pro Glu Arg Tyr Arg Thr Phe Trp Thr
    370                 375                 380

Glu Phe Gly Thr Val Val Lys Glu Gly Leu Leu Glu Asp Thr Asp Asn
385                 390                 395                 400

Arg Asp Thr Leu Leu Glu Ile Leu Ser Val Ala Ser Thr Asn Asp Pro
                405                 410                 415

Ala Glu Gln Thr Asp Leu Ala Gly Tyr Val Glu Arg Met Arg Glu Gly
            420                 425                 430

Gln Gln Asp Ile Tyr Tyr Ala Thr Gly Glu Asn Arg Ser Thr Ile Glu
        435                 440                 445

Asn Ser Pro His Ile Glu Ala Phe Arg Ala Lys Gly Tyr Glu Val Leu
    450                 455                 460

Leu Leu Thr Asp Pro Val Asp Glu Val Trp Val Glu Arg Val Gly Ser
465                 470                 475                 480

Tyr Ala Gly Lys Thr Leu Arg Ser Val Ala Lys Gly Gln Val Asp Leu
                485                 490                 495

Asp Thr Glu Gln Glu Arg Thr Glu Ala Glu Ala Glu Arg Glu Arg Gln
            500                 505                 510

Arg Thr Glu Tyr Ala Asp Leu Leu Gly Trp Met Gly Glu Val Leu Ala
        515                 520                 525

Asp Asn Val Lys Glu Val Arg Leu Ser Ser Arg Leu Thr Thr Ser Pro
    530                 535                 540

Ala Cys Val Val Gly Asp Ala His Asp Leu Thr Pro Thr Leu Glu Lys
545                 550                 555                 560

Met Tyr Arg Ala Met Gly Gln Glu Val Pro Lys Val Lys Arg
                565                 570